(19) Europäisches Patentamt / European Patent Office / Office européen des brevets

(11) **EP 4 745 151 A1**

(12) **EUROPEAN PATENT APPLICATION**
published in accordance with Art. 153(4) EPC

(43) Date of publication:
**20.05.2026 Bulletin 2026/21**

(21) Application number: **24838863.9**

(22) Date of filing: **11.07.2024**

(51) International Patent Classification (IPC):
***C07K 7/08*** *(2006.01)* ***C07K 14/81*** *(2006.01)*
***C07K 7/06*** *(2006.01)* ***A61K 38/08*** *(2019.01)*

(52) Cooperative Patent Classification (CPC):
**A61K 38/08; C07K 7/06; C07K 7/08; C07K 14/81**

(86) International application number:
**PCT/CN2024/104918**

(87) International publication number:
**WO 2025/011611 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.07.2023 CN 202310843734**
**16.10.2023 CN 202311332360**

(71) Applicants:
- **Jiangsu Hengrui Pharmaceuticals Co., Ltd.**
  **Lianyungang, Jiangsu 222047 (CN)**
- **Tianjin Hengrui Medicine Co., Ltd.**
  **Tianjin 300303 (CN)**

(72) Inventors:
- **GE, Xinyue**
  **Tianjin 300303 (CN)**
- **ZHOU, Shunguang**
  **Tianjin 300303 (CN)**
- **GUO, Feihu**
  **Tianjin 300303 (CN)**
- **SUN, Jiyun**
  **Tianjin 300303 (CN)**
- **LI, Zhongqing**
  **Tianjin 300303 (CN)**
- **SONG, Zihui**
  **Tianjin 300303 (CN)**
- **ZHANG, Mengyi**
  **Tianjin 300303 (CN)**

(74) Representative: **Dragotti & Associati S.P.A.**
**Via Nino Bixio, 7**
**20129 Milano (MI) (IT)**

# (54) FIBROBLAST ACTIVATION PROTEIN LIGAND AND USE THEREOF

(57) The present application relates to a fibroblast activation protein ligand and a use thereof, and in particular to a compound as shown in formula (I) or a pharmaceutically acceptable salt thereof, a pharmaceutical composition containing the compound, and a use thereof in diagnosis or treatment of diseases related to a fibroblast activation protein, such as tumours or cancers.

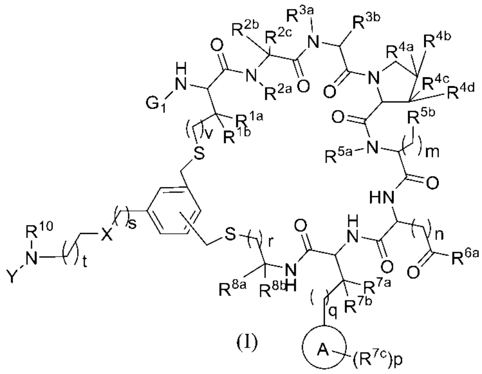

(I)

EP 4 745 151 A1

## Description

## Technical Field

The present disclosure relates to a fibroblast activation protein ligand and the use thereof, and belongs to the field of pharmaceuticals.

## Background Art

In recent years, the tumour microenvironment has attracted interest as a therapeutic target for cancer treatment. The tumour microenvironment can account for a large portion of a tumour. For example, in pancreatic ductal carcinoma, the tumour microenvironment has been reported to constitute up to 80% of the tumour mass. Carcer-associated fibroblasts are a component of the tumour microenvironment and are abundantly present in the stroma of tumour entities such as breast cancer and colon cancer. Fibroblast activation protein (FAP) is a type II transmembrane serine protease, which can serve as a tumour-associated fibroblast marker. FAP is not expressed or is expressed at low levels in normal tissues, but is highly expressed in over 90% of epithelial-derived tumour tissues, such as breast cancer, colorectal cancer, lung cancer, ovarian cancer and pancreatic cancer. Due to the limited expression in normal tissues, FAP has been identified as a potential pan-tumour target for cancer treatment. In cancer indications of mesenchymal origin, especially sarcomas and mesotheliomas, FAP is expressed on tumour cells in addition to cancer-associated fibroblasts. FAP expression is also found in chronic inflammatory diseases such as rheumatoid arthritis and osteoarthritis, and cardiac remodelling after myocardial infarction.

The first method of targeting FAP for cancer treatment involves the use of the monoclonal antibody sibrotuzumab, which is tested in the treatment of colorectal cancer in both unconjugated and 131I-conjugated forms. Other methods targeting FAP, including bispecific antibodies, antibody fragment constructs, chimeric antigen receptor T cells and antibody-drug conjugates, are currently under investigation, most of which are in preclinical development or phase I clinical trials. In addition, small molecule FAP inhibitors (FAPIs) have been found, and conjugated with radionuclides to produce excellent imaging agents suitable for various cancer indications (J Nucl Med 2022; 63: 415-423).

FAP-2286 contains a peptide that effectively and selectively binds to FAP, and is attached to DOTA via a linker. This compound is currently in the clinical stage. More FAP inhibitors have been developed, showing broad application prospects.

## Summary of the Invention

The present disclosure provides a compound as shown in formula (I) or a pharmaceutically acceptable salt thereof,

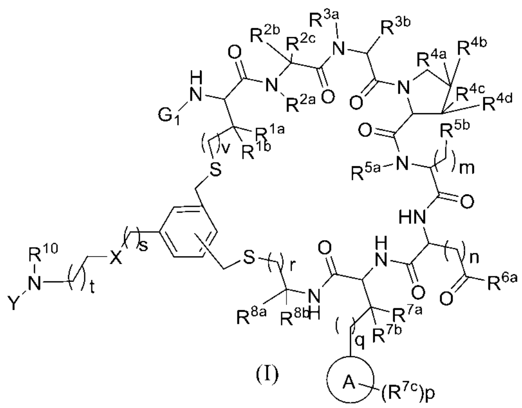

(I)

wherein the $R^{1a}$ and $R^{1b}$ are each independently selected from hydrogen or methyl;

the v is selected from 0 or 1;

the $G_1$ is selected from $-(CO)-L-R^{1c}$, the L is selected from $C_{3-20}$ alkylene, the $R^{1c}$ is selected from methyl or $-NH-(C=NH)-NR^{1d}R^{1f}$, and the $R^{1d}$ and $R^{1f}$ are each independently selected from hydrogen or methyl; or the $G_1$ is selected from

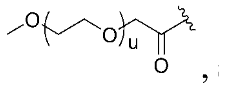

and the u is selected from an integer from 1 to 5;
the $R^{2a}$, $R^{2b}$ and $R^{2e}$ are each independently selected from hydrogen or $C_{1-3}$ alkyl;
the $R^{3a}$ and $R^{3b}$ are each independently selected from hydrogen and $C_{1-3}$ alkyl, or the $R^{3a}$ and $R^{3b}$ together with the atom to which each is attached form 5- to 6-membered nitrogen-containing heterocycloalkyl, the $C_{1-3}$ alkyl or 5- to 6-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl;
the $R^{4a}$, $R^{4b}$, $R^{4c}$ and $R^{4d}$ are each independently selected from hydrogen, hydroxyl, amino, halogen, $C_{1-3}$ alkyl or $C_{1-3}$ haloalkyl;
the $R^{5a}$ is selected from hydrogen or methyl;
the $R^{5b}$ is selected from $C_{1-6}$ alkyl, 6- to 10-membered aryl or 5- to 6-membered heteroaryl, the $C_{1-6}$ alkyl, 6- to 10-membered aryl or 5- to 6-membered heteroaryl is optionally substituted with 1 or 2 $R^B$, and the $R^B$ groups are the same or different, and are selected from hydroxyl, amino, halogen and $-CONH_2$;
the m is selected from 1, 2 or 3; the hydrogen on $CH_2$ is optionally substituted with one or more groups selected from hydroxyl, halogen, $C_{1-3}$ alkyl, 6- to 10-membered aryl or 5- to 6-membered heteroaryl (i.e., in

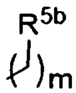

,

m is selected from 1, 2 or 3, the hydrogen on $CH_2$ is optionally substituted with one or more groups selected from hydroxyl, halogen, $C_{1-3}$ alkyl, 6- to 10-membered aryl or 5- to 6-membered heteroaryl);
the $R^{6a}$ is selected from hydroxyl or amino;
the n is selected from 1, 2 or 3;
the $R^{7a}$ and $R^{7b}$ are each independently selected from hydrogen and $C_{1-3}$ alkyl;
the q is selected from 0 or 1;
the ring A is selected from phenyl, pyridyl, naphthyl or thienyl;
each $R^{7c}$ is independently selected from halogen, nitro, amino, cyano, hydroxyl, $C_{1-6}$ alkyl, $C_{1-6}$ alkoxy, hydroxy $C_{1-6}$ alkyl, trifluoromethyl, difluoromethyl and trifluoroethyl;
the p is selected from 0, 1, 2 or 3;
the $R^{8a}$ and $R^{8b}$ are each independently selected from hydrogen, -COOH, - $CONH_2$, $CH_2$-OH, $-CONHR^{8c}$ and $-(CO)-(NR^{8c})-R^{8d}$, and the $R^{8c}$ and $R^{8d}$ are each independently selected from $C_{1-6}$ alkyl;
the r is selected from 0 or 1;
the s is selected from 0 or 1;
the X is selected from O, S or $NR^9$, and the $R^9$ is selected from hydrogen or $C_{1-6}$ alkyl;
the t is selected from 1, 2, 3, 4, 5 or 6;
the $R^{10}$ is selected from hydrogen or $C_{1-6}$ alkyl;
the Y is a chelating agent or hydrogen, and the chelating agent optionally complexes a nuclide.

In an alternative embodiment, the Y is a chelating agent, and the chelating agent optionally complexes a nuclide.
In an alternative embodiment, the Y is hydrogen.
In an alternative embodiment, the compound as shown in formula (I) or the pharmaceutically acceptable salt thereof is a compound as shown in formula (II) or a pharmaceutically acceptable salt thereof,

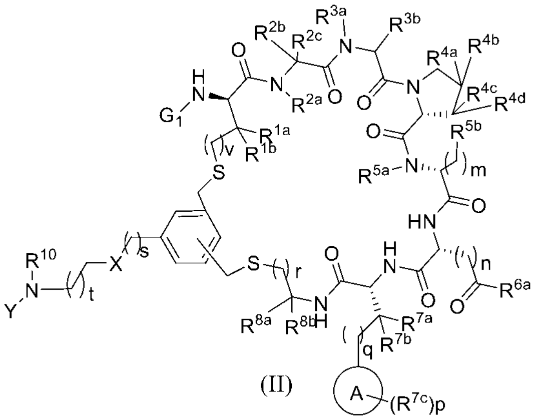

(II)

wherein the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t, $R^{10}$ and Y are as defined in formula (I).

In an alternative embodiment, in the compound as shown in formula (I) or the pharmaceutically acceptable salt thereof, and the compound as shown in formula (II) or the pharmaceutically acceptable salt thereof provided by the present disclosure, the $R^{2a}$ is hydrogen or methyl, and the $R^{2b}$ and $R^{2c}$ are each independently selected from hydrogen or methyl; optionally, the $R^{2b}$ is hydrogen, the $R^{2c}$ is methyl, or the $R^{2b}$ and $R^{2c}$ are both methyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{4a}$ and $R^{4b}$ are each independently selected from hydrogen and hydroxyl; optionally, the $R^{4a}$ is hydrogen, and the $R^{4b}$ is hydroxyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{2a}$ is hydrogen or methyl; the $R^{2b}$ and $R^{2c}$ are each independently selected from hydrogen or methyl, with at least one being methyl; the $R^{4a}$ is hydrogen, and the $R^{4b}$ is hydroxyl; the $R^{4c}$ and $R^{4d}$ are each independently selected from hydrogen.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{3a}$ and $R^{3b}$ are each independently selected from hydrogen and $C_{1-3}$ alkyl, the $C_{1-3}$ alkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{3a}$ is methyl, and the $R^{3b}$ is hydrogen.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{3a}$ and $R^{3b}$ together with the atom to which each is attached form 5-membered nitrogen-containing heterocycloalkyl, the 5-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{3a}$ and $R^{3b}$ together with the atom to which each is attached form 5-membered nitrogen-containing heterocycloalkyl, and the 5-membered nitrogen-containing heterocycloalkyl is unsubstituted.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{7a}$ and $R^{7b}$ are each independently selected from hydrogen and $C_{1-3}$ alkyl;

the q is selected from 0 or 1;
the ring A is selected from phenyl or naphthyl;
each $R^{7c}$ is independently selected from halogen, nitro, amino, cyano, hydroxyl, $C_{1-6}$ alkyl, $C_{1-6}$ alkoxy, hydroxy $C_{1-6}$ alkyl, trifluoromethyl, difluoromethyl and trifluoroethyl;
the p is selected from 0, 1, 2 or 3.

In an alternative embodiment, in the compound as shown in formula (I) or the pharmaceutically acceptable salt thereof

thereof, and the compound as shown in formula (II) or the pharmaceutically acceptable salt thereof provided by the present disclosure, the $R^{7a}$ and $R^{7b}$ are each independently selected from hydrogen;

the q is 0;
the ring A is phenyl;
each $R^{7c}$ is independently selected from fluorine, chlorine, bromine and iodine, or trifluoromethyl, difluoromethyl and trifluoroethyl;
the p is selected from 0, 1 or 2.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{1a}$ and $R^{1b}$ are each independently selected from hydrogen or methyl;

the v is selected from 0 or 1;
the $G_1$ is selected from -(CO)-L-$R^{1c}$, the L is selected from $C_{3-20}$ alkylene, and the $R^{1c}$ is selected from methyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{1a}$ and $R^{1b}$ are each independently selected from hydrogen;

the v is selected from 0;
the $G_1$ is selected from -(CO)-L-$R^{1c}$, the L is selected from $C_{4-14}$ alkylene, and the $R^{1c}$ is selected from methyl;

In an alternative embodiment, in the compound as shown in formula (I) or the pharmaceutically acceptable salt thereof, and the compound as shown in formula (II) or the pharmaceutically acceptable salt thereof provided by the present disclosure, the $R^{1a}$ and $R^{1b}$ are each independently selected from hydrogen or methyl;

the v is selected from 0 or 1;
the $G_1$ is selected from -(CO)-L-$R^{1c}$, the L is selected from $C_{3-20}$ alkylene, the $R^{1c}$ is selected from -NH-(C=NH)-$NR^{1d}R^{1f}$, i.e.,

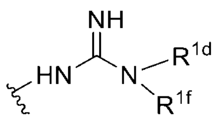
,

and the $R^{1d}$ and $R^{1f}$ are each independently selected from hydrogen or methyl.

In an alternative embodiment, in the compound as shown in formula (I) or the pharmaceutically acceptable salt thereof, and the compound as shown in formula (II) or the pharmaceutically acceptable salt thereof provided by the present disclosure,

the $R^{1a}$ and $R^{1b}$ are each independently selected from hydrogen;
the v is selected from 0;
the $G_1$ is selected from -(CO)-L-$R^{1c}$, the L is selected from $C_{4-14}$ alkylene, the $R^{1c}$ is selected from -NH(C=NH)-$NR^{1d}R^{1f}$, and the $R^{1d}$ and $R^{1f}$ are each independently selected from hydrogen or methyl.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{1a}$ and $R^{1b}$ are each independently selected from hydrogen or methyl;

the v is selected from 0 or 1;

the $G_1$ is selected from

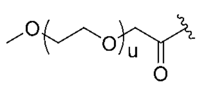
,

and the u is selected from an integer from 1 to 5.

In an alternative embodiment, in the compound as shown in formula (I) or the pharmaceutically acceptable salt thereof, and the compound as shown in formula (II) or the pharmaceutically acceptable salt thereof provided by the present disclosure, the $R^{1a}$ and $R^{1b}$ are each independently selected from hydrogen;

the v is selected from 0;
the $G_1$ is selected from

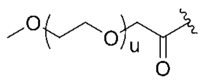;

the u is selected from 1 or 2.

In an alternative embodiment, in the compound as shown in formula (I) or the pharmaceutically acceptable salt thereof, and the compound as shown in formula (II) or the pharmaceutically acceptable salt thereof provided by the present disclosure,

the $R^{5a}$ is selected from hydrogen;
the $R^{5b}$ is selected from $C_{1-6}$ alkyl, the $C_{1-6}$ alkyl is optionally substituted with 1 or 2 $R^B$, and the $R^B$ groups are the same or different, and are selected from hydroxyl or halogen;
the m is selected from 0 or 1; the hydrogen on $CH_2$ is optionally substituted with one or more groups selected from hydroxyl, halogen, hydroxyl and $C_{1-3}$ alkyl;
the n is 2;
the $R^{6a}$ is amino;
the $R^{8a}$ and $R^{8b}$ are each independently selected from hydrogen, -COOH and - $CONH_2$, with at least one being -COOH or -$CONH_2$, and the r is 1.

In an alternative embodiment, the compound as shown in formula (I) or the pharmaceutically acceptable salt thereof, or the compound as shown in formula (II) or the pharmaceutically acceptable salt thereof provided by the present disclosure is a compound as shown in formula (III) or a pharmaceutically acceptable salt thereof,

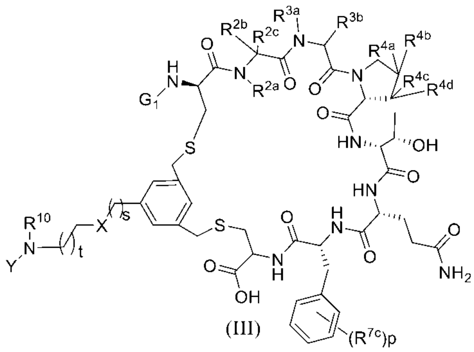

(III)

wherein the $R^{2a}$ is hydrogen or methyl;
the $R^{2b}$ and $R^{2c}$ are each independently selected from hydrogen or methyl, with at least one being methyl;
the $R^{3a}$ and $R^{3b}$ are each independently selected from hydrogen and $C_{1-3}$ alkyl, the $C_{1-3}$ alkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl; or the $R^{3a}$ and $R^{3b}$ together with the atom to which each is attached form 5-membered nitrogen-containing heterocycloalkyl, the 5-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl;
the $R^{4a}$ is hydrogen, fluorine or trifluoromethyl, and the $R^{4b}$ is fluorine or hydroxyl;
the $R^{4c}$ and $R^{4d}$ are each independently selected from hydrogen;
each $R^{7c}$ is independently selected from fluorine, chlorine, bromine and iodine, or trifluoromethyl, difluoromethyl and trifluoroethyl;

the p is selected from 0, 1 or 2;
the $G_1$, s, X, t, $R^{10}$ and Y are as defined in formula (I).

In an alternative embodiment, in the compound as shown in formula (III) or the pharmaceutically acceptable salt thereof provided by the present disclosure, the $R^{4a}$ is hydrogen, and the $R^{4b}$ is hydroxyl.
In an alternative embodiment, in the compound as shown in formula (III) or the pharmaceutically acceptable salt thereof provided by the present disclosure, the $R^{3a}$ is hydrogen, and the $R^{3b}$ is methyl.
In an alternative embodiment, in the compound as shown in formula (III) or the pharmaceutically acceptable salt thereof provided by the present disclosure, the $R^{3a}$ and $R^{3b}$ together with the atom to which each is attached form 5-membered nitrogen-containing heterocycloalkyl, and the 5-membered nitrogen-containing heterocycloalkyl is unsubstituted.
In an alternative embodiment, in the compound as shown in formula (I) or the pharmaceutically acceptable salt thereof, the compound as shown in formula (II) or the pharmaceutically acceptable salt thereof, and the compound as shown in formula (III) or the pharmaceutically acceptable salt thereof provided by the present disclosure, the s is 1; X is S; $R^{10}$ is hydrogen.
In an alternative embodiment, in the compound as shown in formula (III) or the pharmaceutically acceptable salt thereof provided by the present disclosure, the $G_1$ is selected from -(CO)-L-$R^{1c}$, the L is selected from $C_{4-14}$ alkylene, the $R^{1c}$ is selected from methyl, and the L may be selected from, for example, $C_4$ alkylene, $C_6$ alkylene, $C_8$ alkylene, $C_{10}$ alkylene, $C_{12}$ alkylene or $C_{14}$ alkylene.
In an alternative embodiment, in the compound as shown in formula (III) or the pharmaceutically acceptable salt thereof provided by the present disclosure, the $G_1$ is selected from -(CO)-L-$R^{1c}$, the L is selected from -(CO)-L-$R^{1c}$, the L is selected from $C_{4-14}$ alkylene, the $R^{1c}$ is selected from -NH-(C=NH)-$NR^{1d}R^{1f}$, the $R^{1d}$ and $R^{1f}$ are each independently selected from hydrogen or methyl, and the L may be selected from, for example, $C_4$ alkylene, $C_3$ alkylene or $C_6$ alkylene.
In an alternative embodiment, in the compound as shown in formula (III) or the pharmaceutically acceptable salt thereof provided by the present disclosure, the $G_1$ is selected from

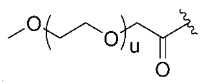,

and the u is selected from 1 or 2.
In the compound provided by the present disclosure, the fragment attached to Y specifically refers to the fragment corresponding to

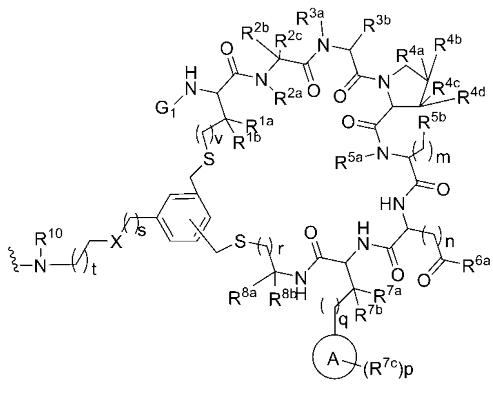,

and is selected from:

group 1:

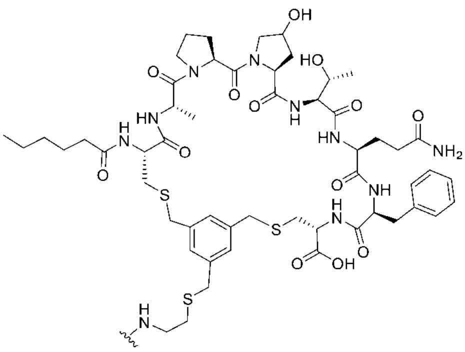,
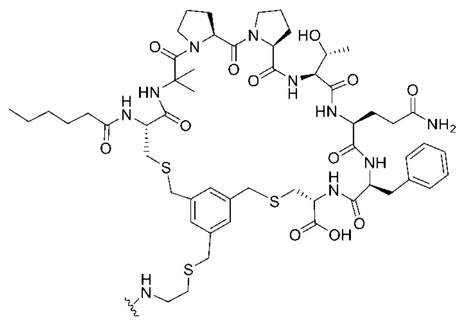,
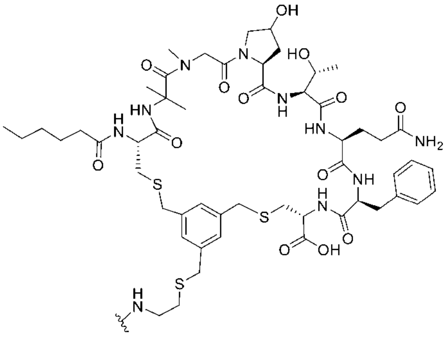,
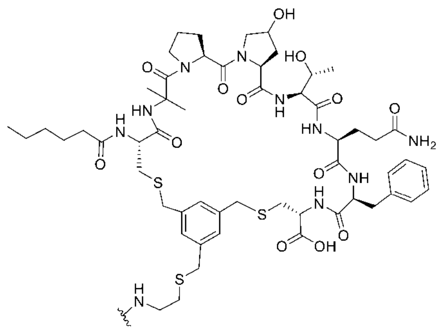,
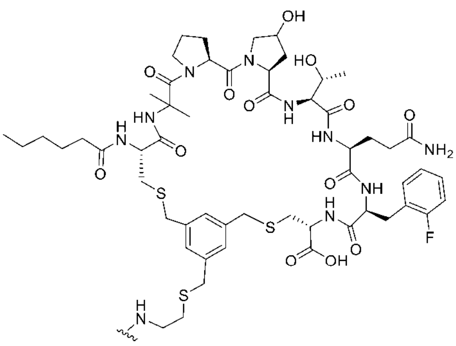,
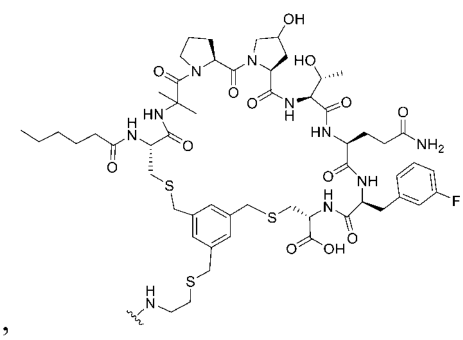,

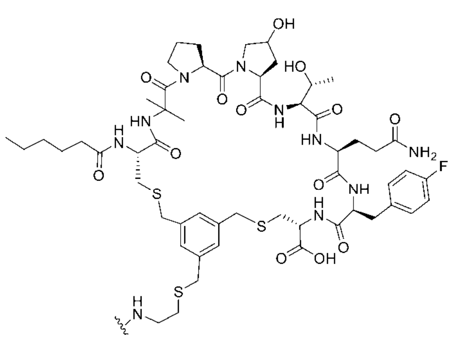,

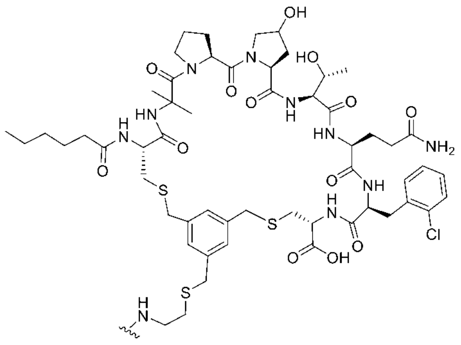,

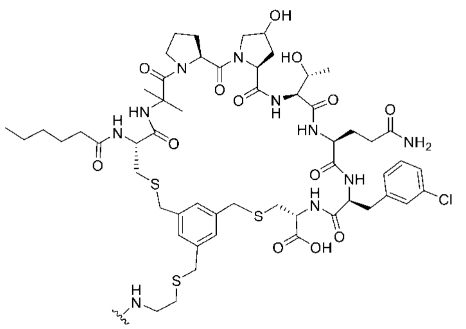,

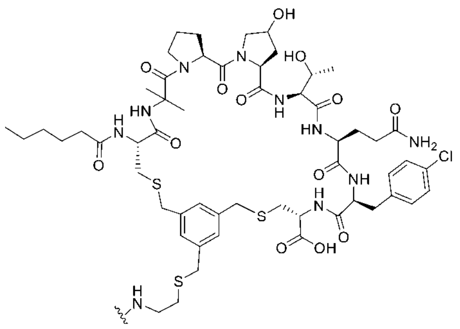,

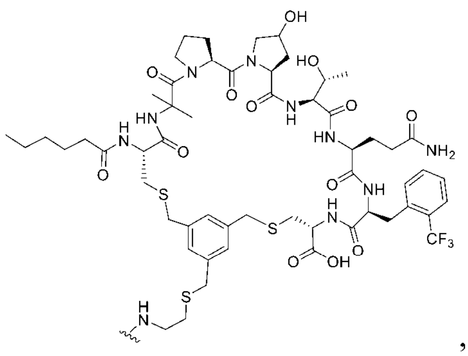,

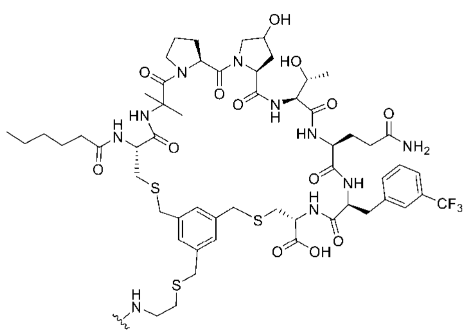,

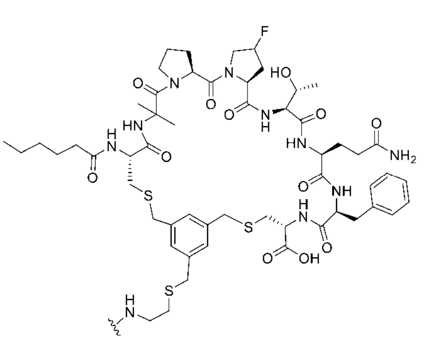
,
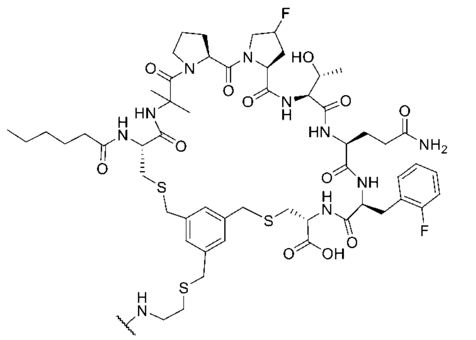
,
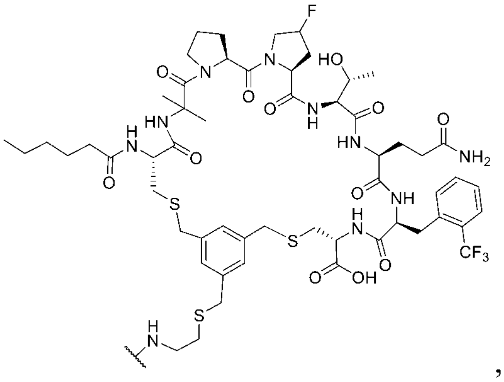
,
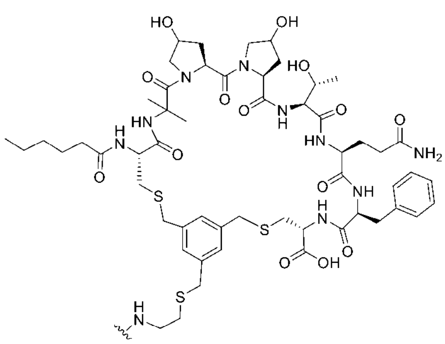
,
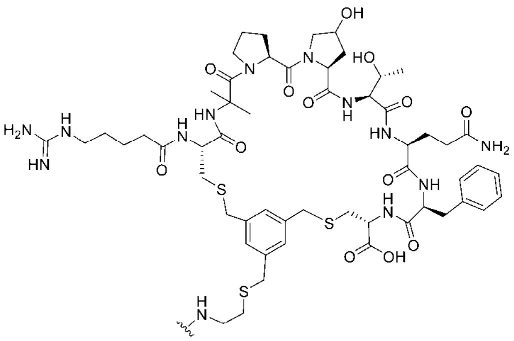
,
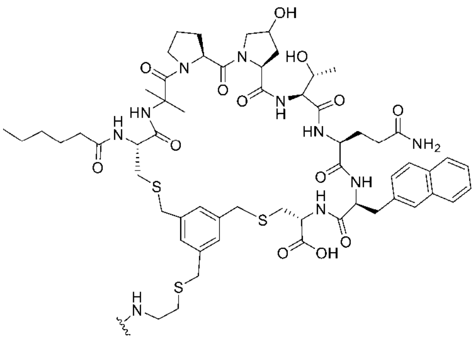
,
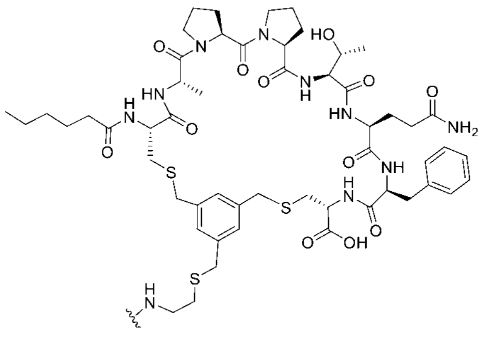
,
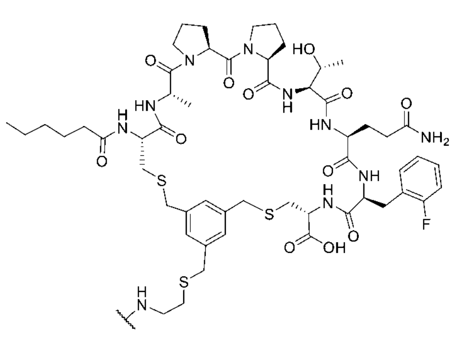
or
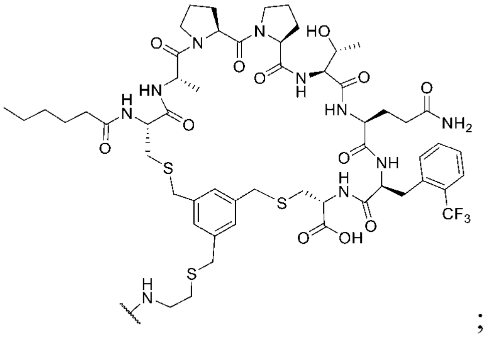
;

group 2:

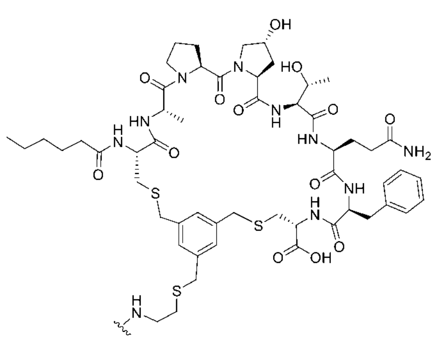,

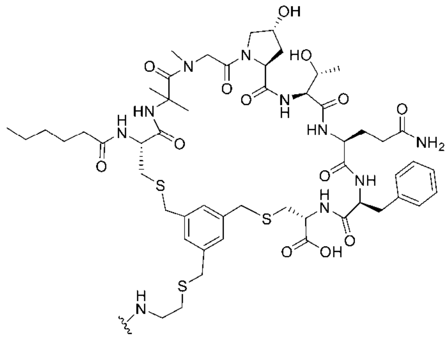,

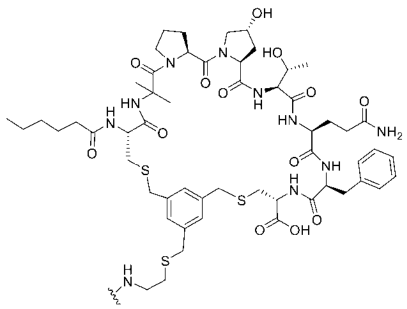,

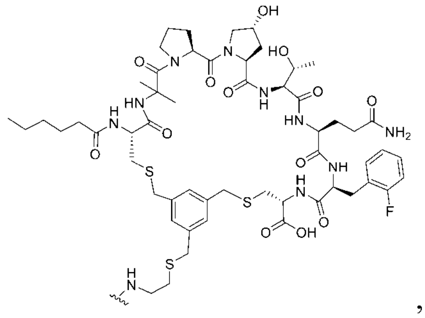,

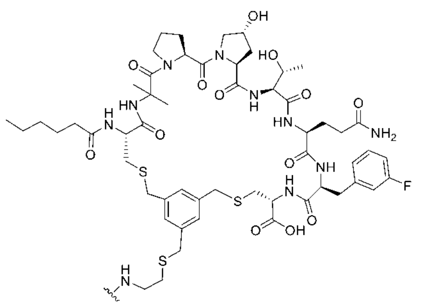,

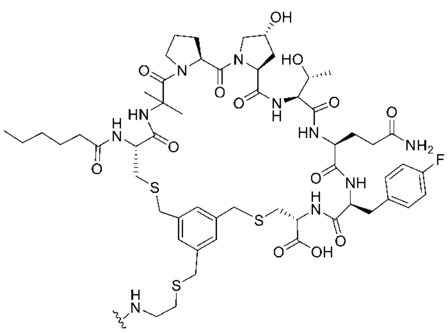,

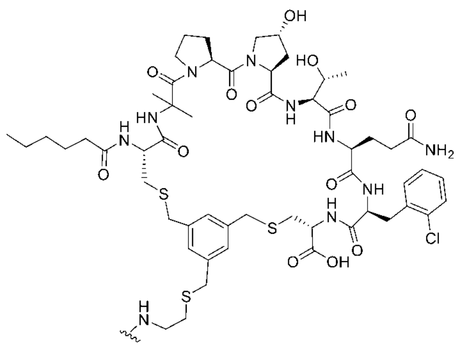,

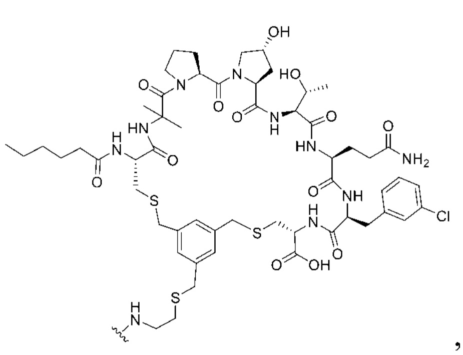,

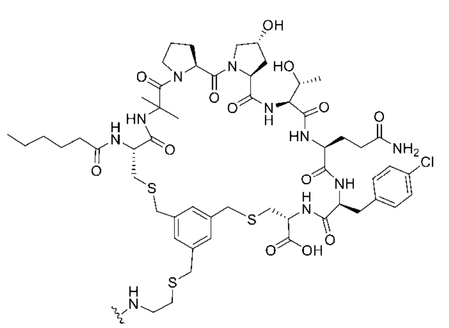,

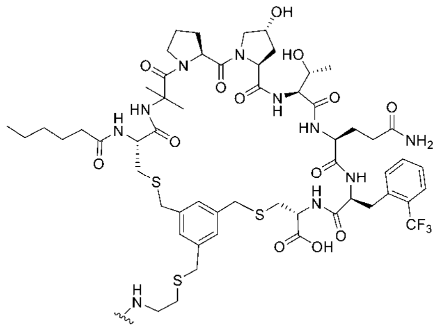,

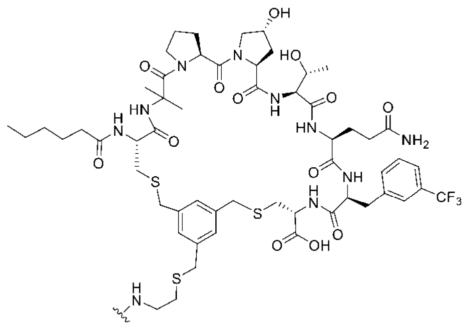,

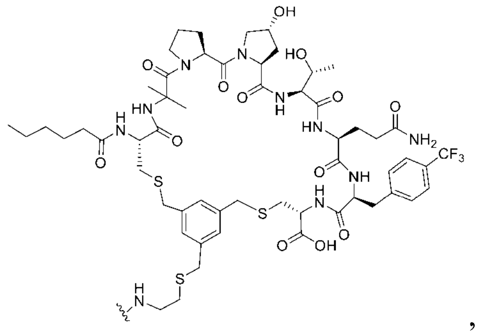,

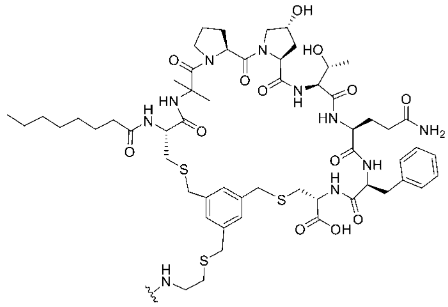,

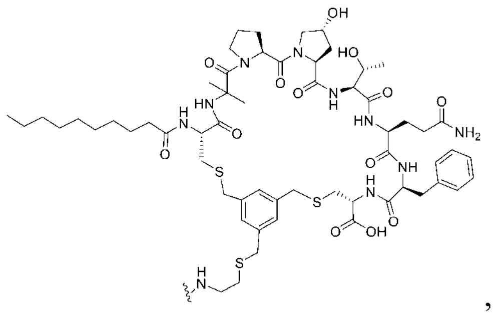,

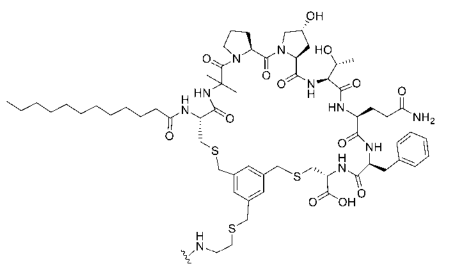,

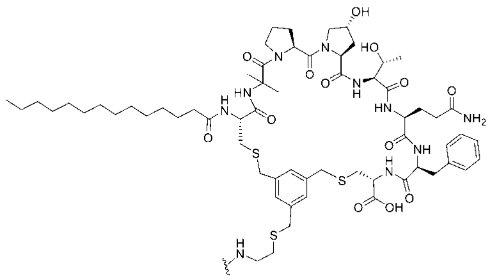,

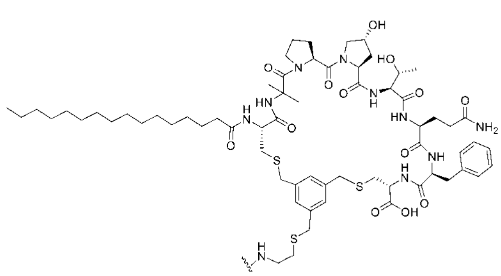,

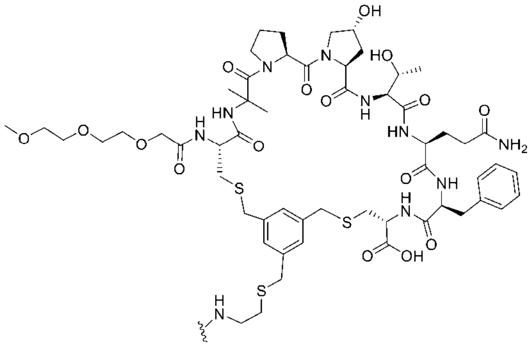,

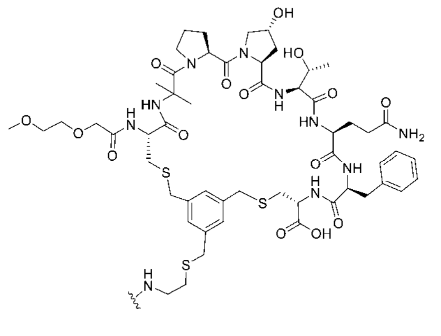,

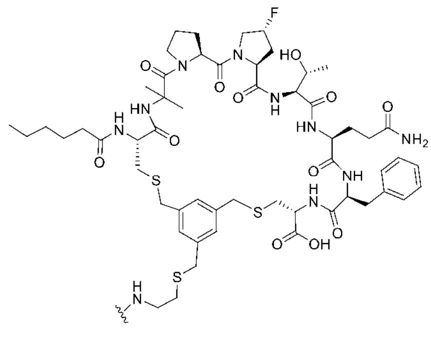,

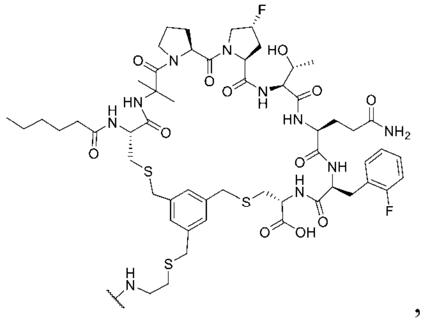,

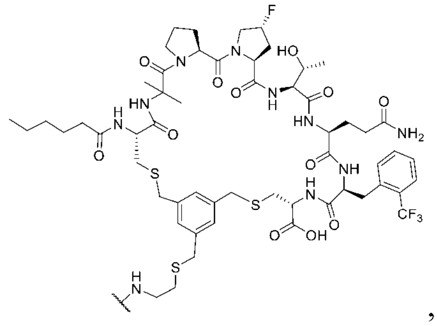,

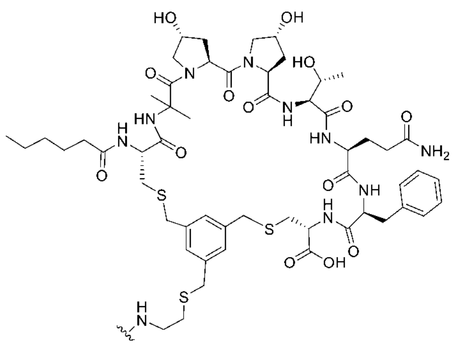,

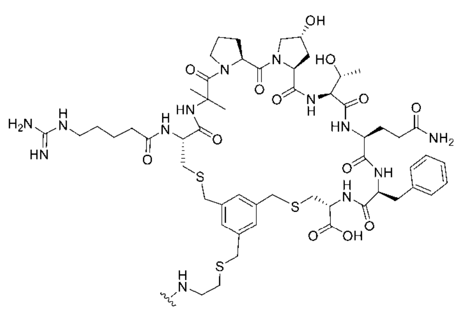

or

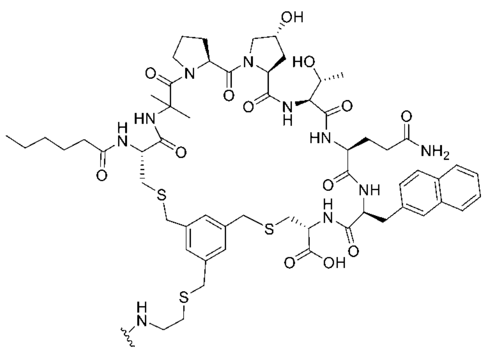;

group 3:

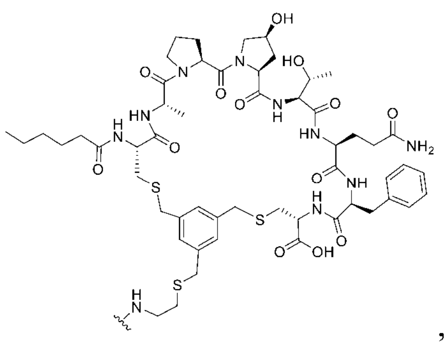
,
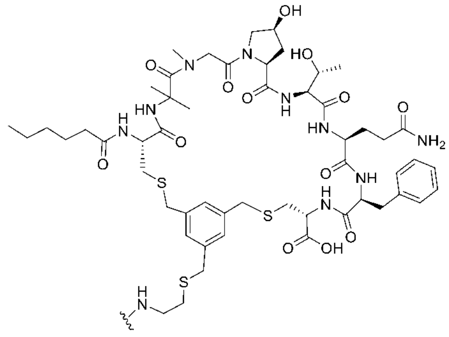
,

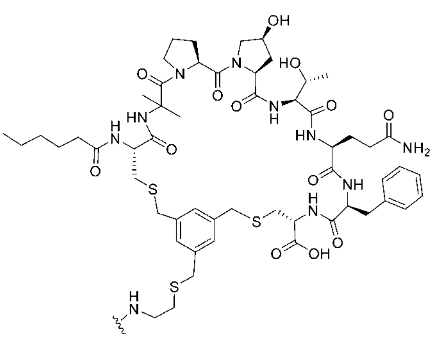
,

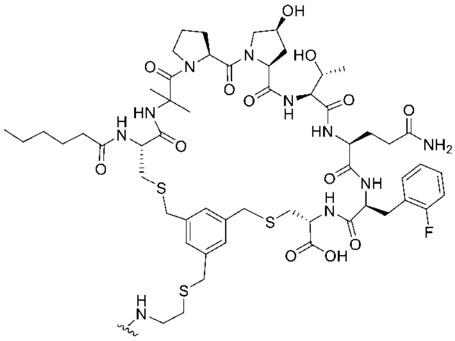
,

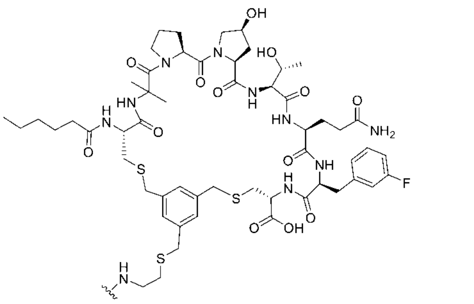
,

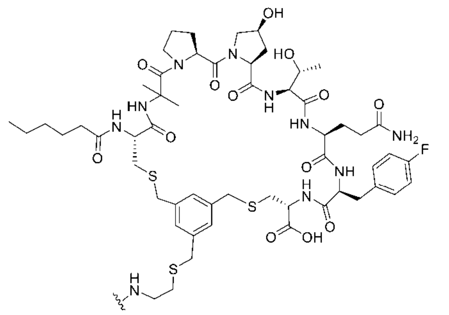
,

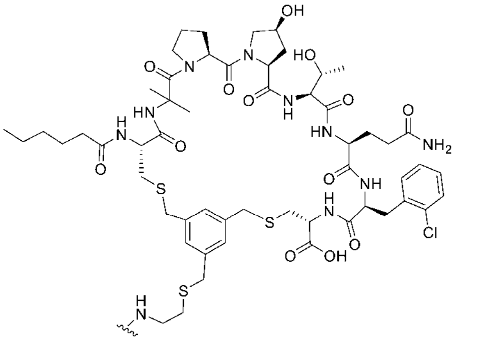
,

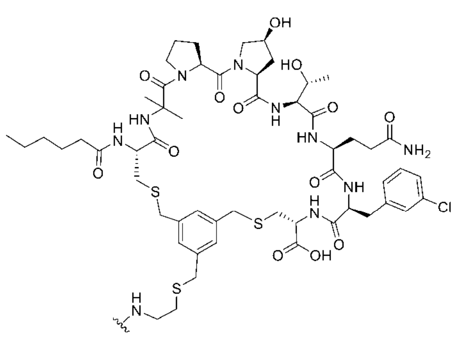
,

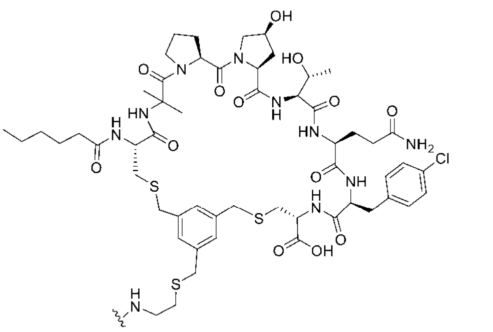
,

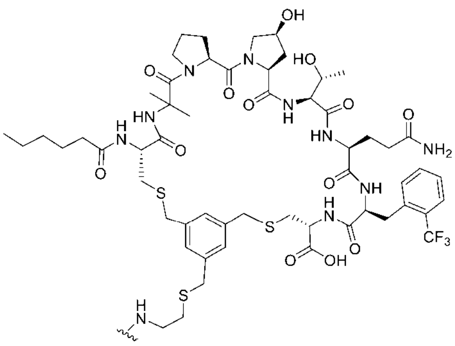
,

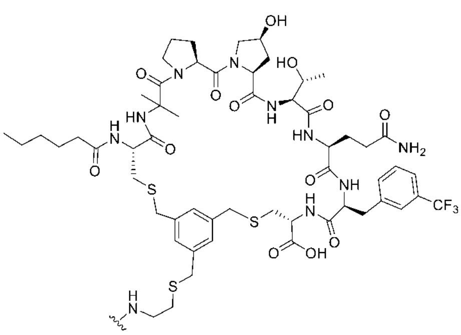
,

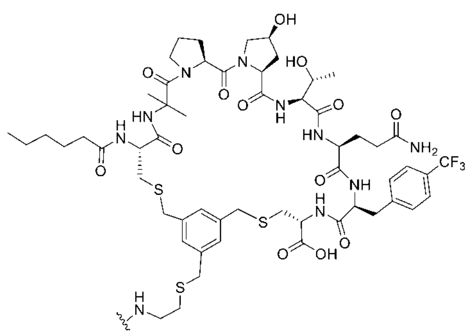
,

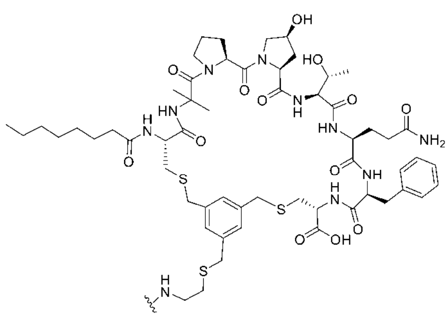

,

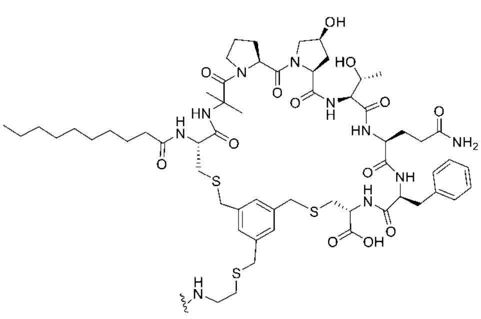

,

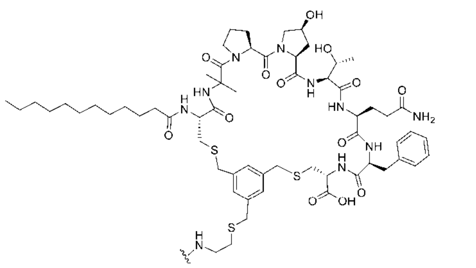

,

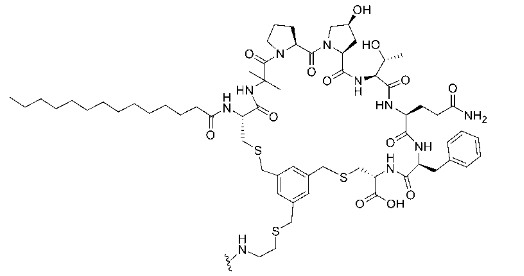

,

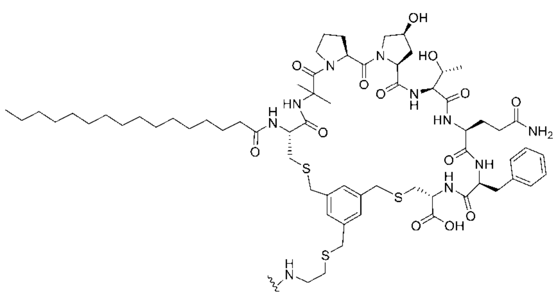

,

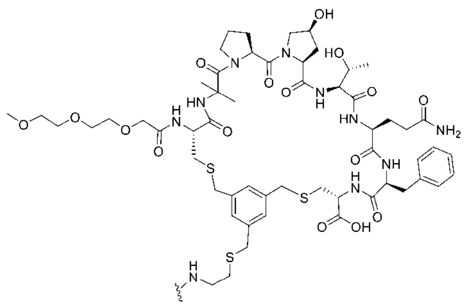

,

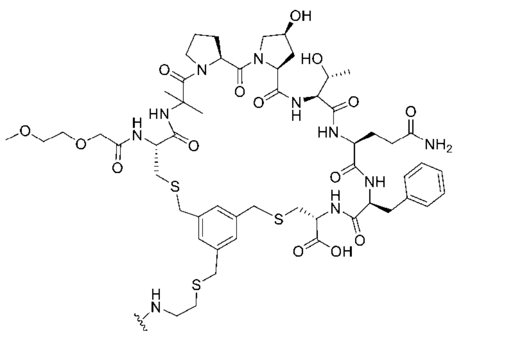

,

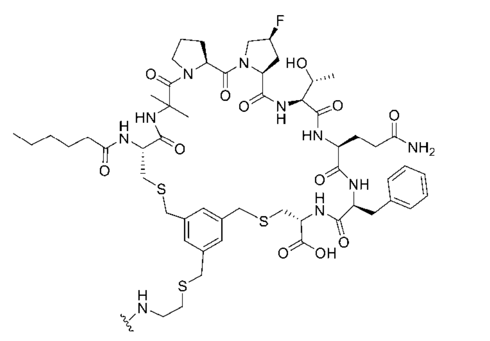

,

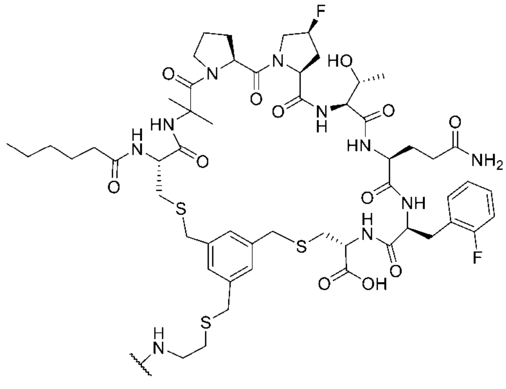

,

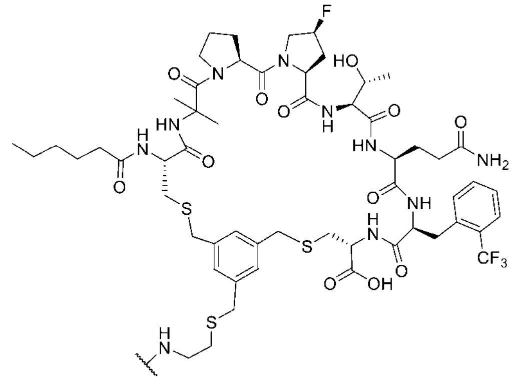

,

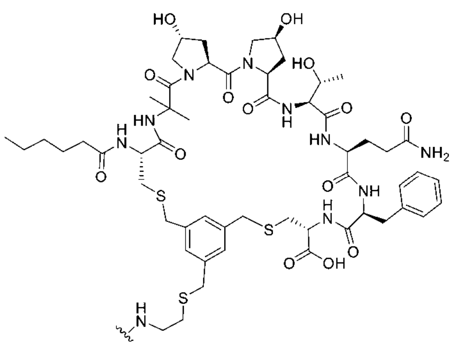,
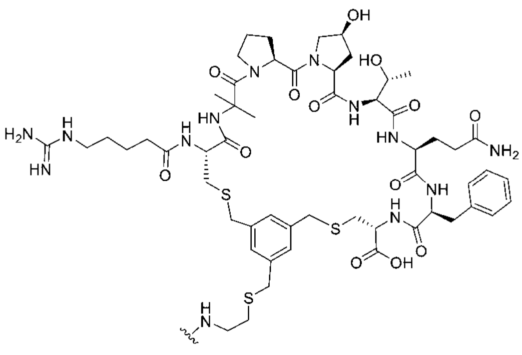
or
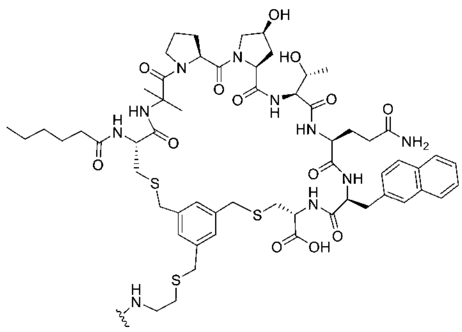

In the compound provided by the present disclosure, the fragment attached to Y specifically refers to: the fragment corresponding to

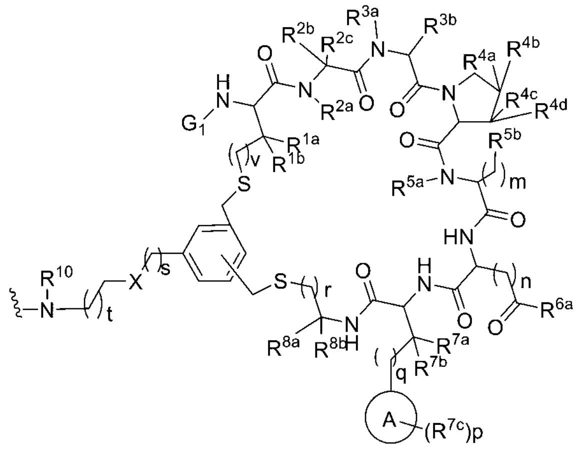,

and is selected from:

group 1'

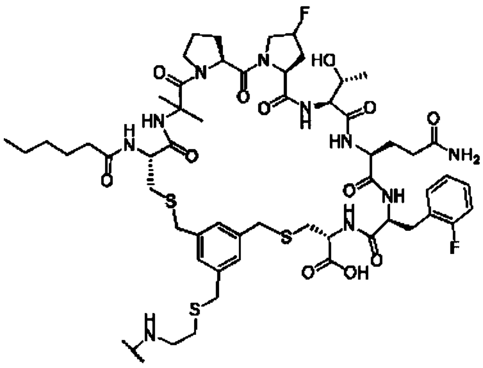 , 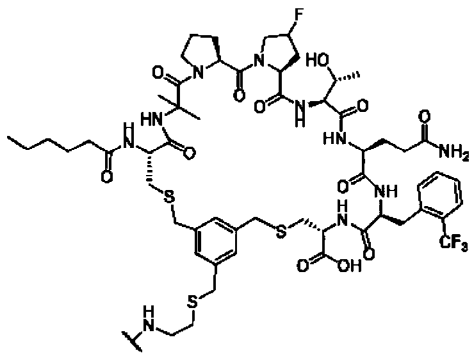 ,

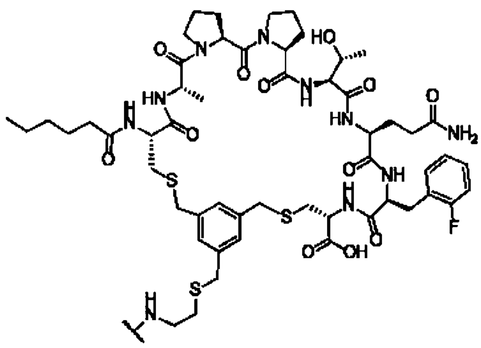 , 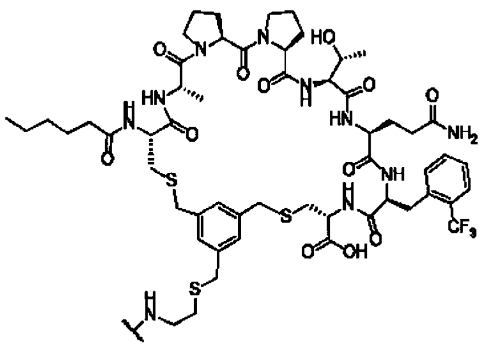 ;

group 2':

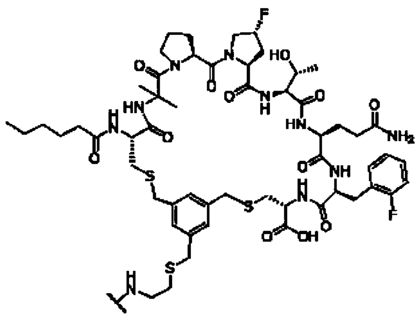 , 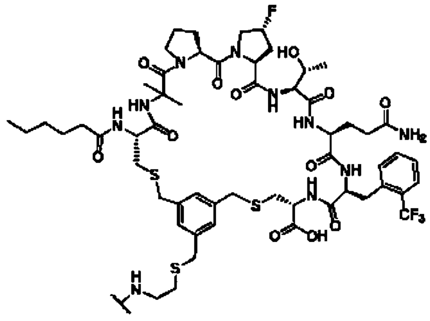 ;

group 3':

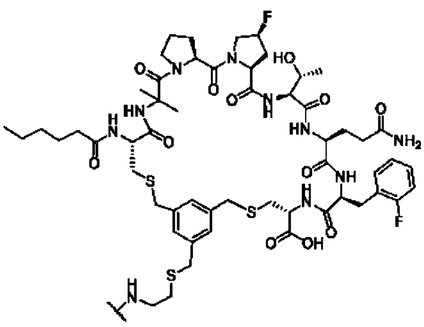 , 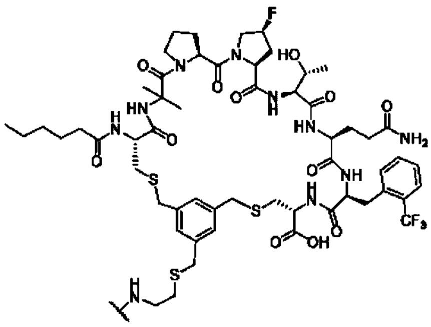 .

The chelating agent described in the present disclosure is selected from DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, $NxS_4$-x($N_4$, $N_2S_2$, $N_3S$), Hynic, and 99mTc$(CO)_3$-chelating agent.

In an alternative embodiment, the chelating agent is selected from

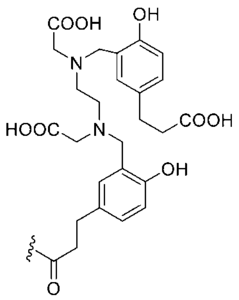 , 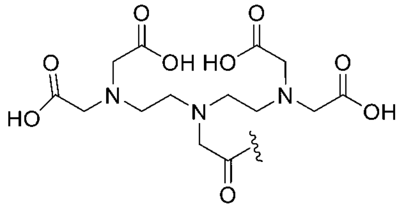 , 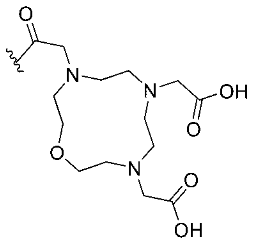 ,

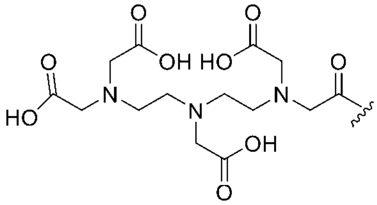 , 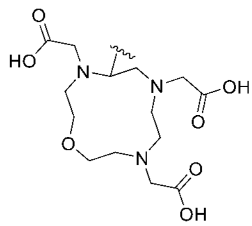 , 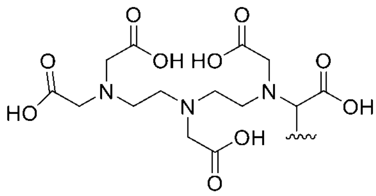 ,

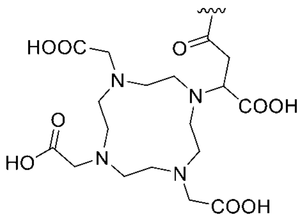 , 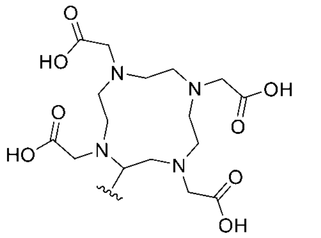 , 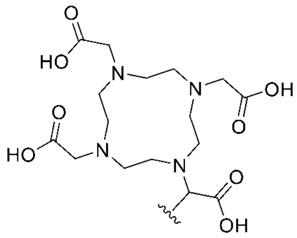 ,

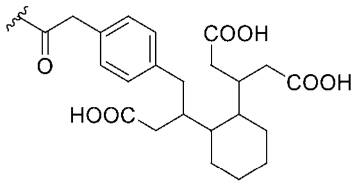 , 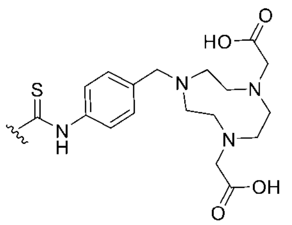 , 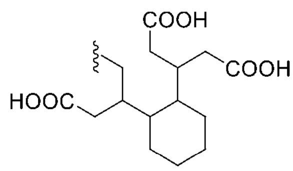 ,

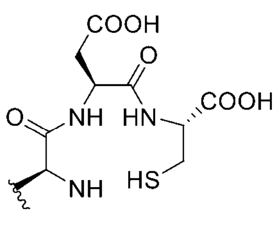 , 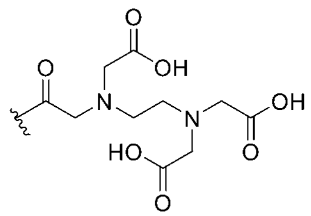 , 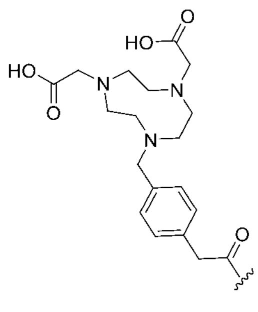 ,

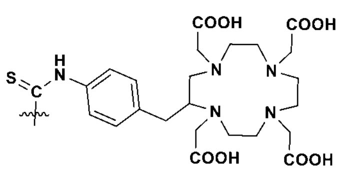 , 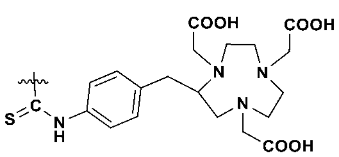 , 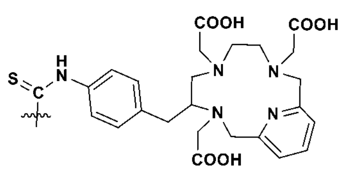 ,

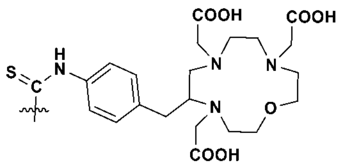,

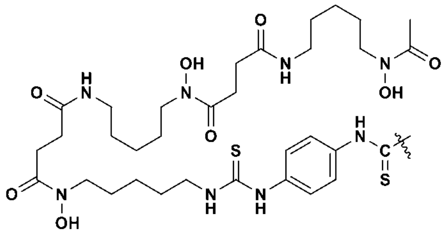,

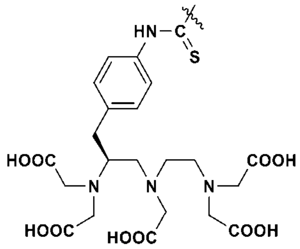,

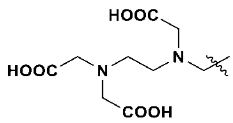,

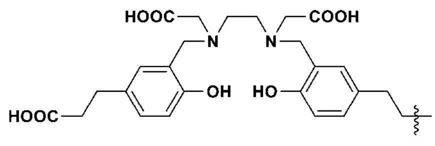,

and

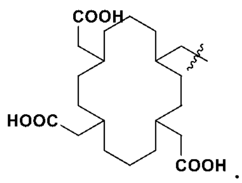.

In some embodiments, the chelating agent is selected from DOTA, DOTAGA, NOTA, NODAGA, NODA-MPAA, HBED, CB-TE2A, DFO, THP and $N_4$.

In some embodiments, the chelating agent is selected from DOTA, DOTAGA, NOTA and NODAGA.

The present disclosure provides a compound as shown below or a pharmaceutically acceptable salt thereof, which is selected from:

group 4:

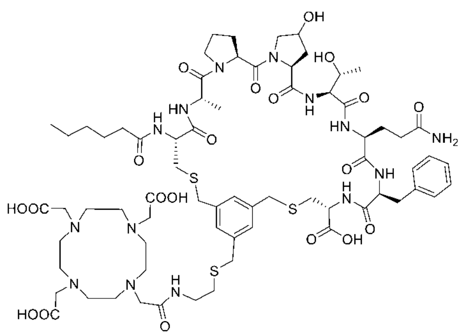

Hex-[Cys-(tMeBn(DOTA-AET))-Ala-Pro-(4-OH-Pro)-Thr-Gln-Phe-Cys]-OH,

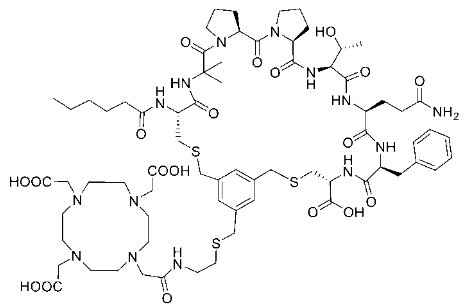

Hex-[Cys-(tMeBn(DOTA-AET))-Aib-Pro-Pro-Thr-Gln-Phe-Cys]-OH,

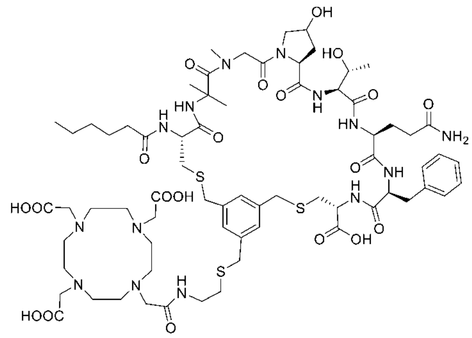

Hex-[Cys-(tMeBn(DOTA-AET))-Aib-Sar-(4-OH-Pro)-Thr-Gln-Phe-Cys]-OH,

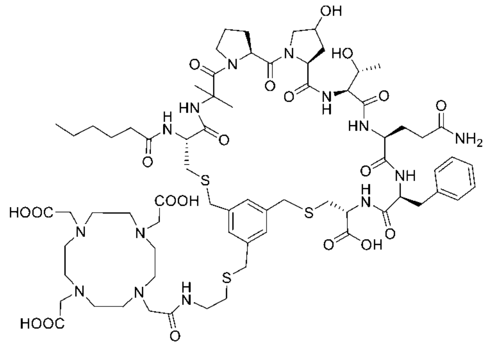

Hex-[Cys-(tMeBn(DOTA-AET))-Aib-Pro-(4-OH-Pro)-Thr-Gln-Phe-Cys]-OH,

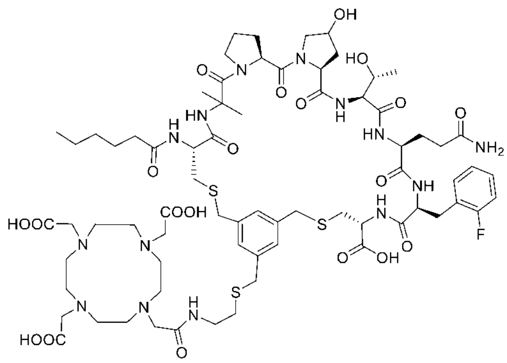

Hex-[Cys-(tMeBn(DOTA-AET))-Aib-Pro-(4-OH-Pro)-Thr-Gln-(2-F-Phe)-Cys]-OH,

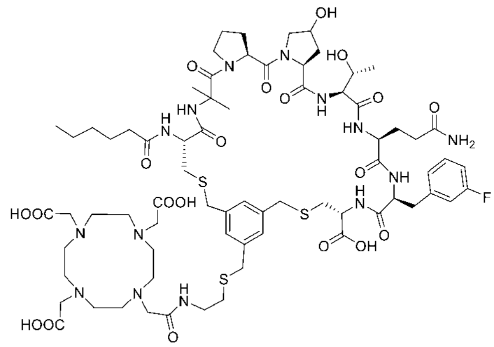

Hex-[Cys-(tMeBn(DOTA-AET))-Aib-Pro-(4-OH-Pro)-Thr-Gln-(3-F-Phe)-Cys]-OH,

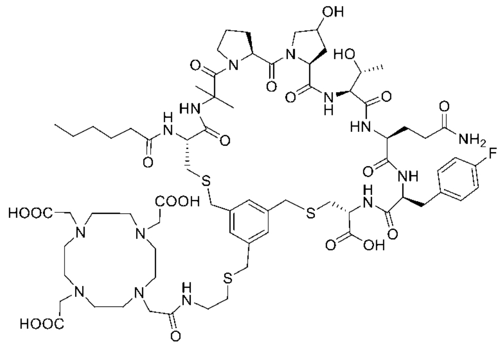

Hex-[Cys-(tMeBn(DOTA-AET))-Aib-Pro-(4-OH-Pro)-Thr-Gln-(4-F-Phe)-Cys]-OH,

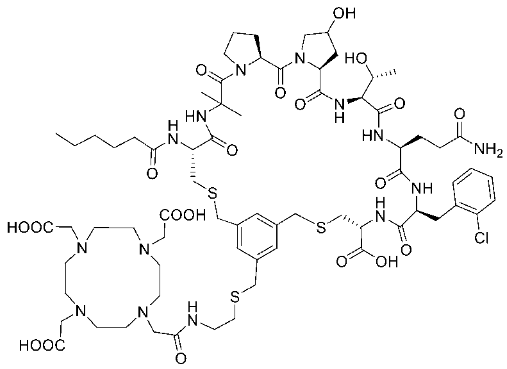

Hex-[Cys-(tMeBn(DOTA-AET))-Aib-Pro-(4-OH-Pro)-Thr-Gln-(2-Cl-Phe)-Cys]-OH,

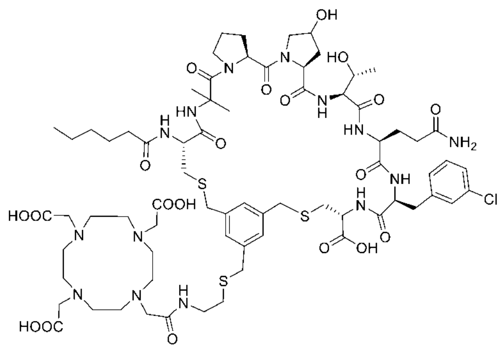

Hex-[Cys-(tMeBn(DOTA-AET))-Aib-Pro-(4-OH-Pro)-Thr-Gln-(3-Cl-Phe)-Cys]-OH,

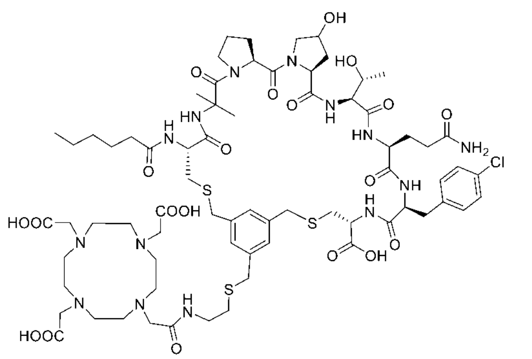

Hex-[Cys-(tMeBn(DOTA-AET))-Aib-Pro-(4-OH-Pro)-Thr-Gln-(4-Cl-Phe)-Cys]-OH,

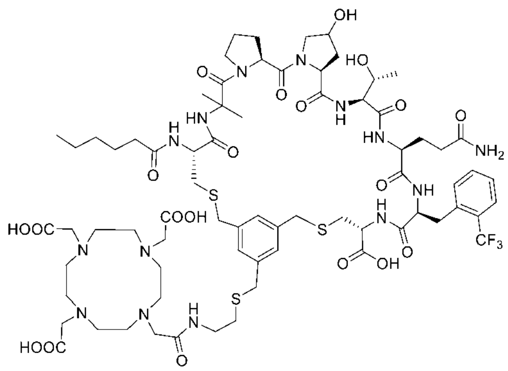

Hex-[Cys-(tMeBn(DOTA-AET))-Aib-Pro-(4-OH-Pro)-Thr-Gln-(2-$CF_3$-Phe)-Cys]-OH,

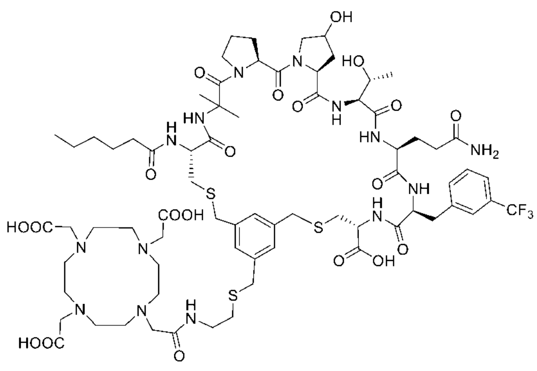

Hex-[Cys-(tMeBn(DOTA-AET))-Aib-Pro-(4-OH-Pro)-Thr-Gln-(3-$CF_3$-Phe)-Cys]-OH,

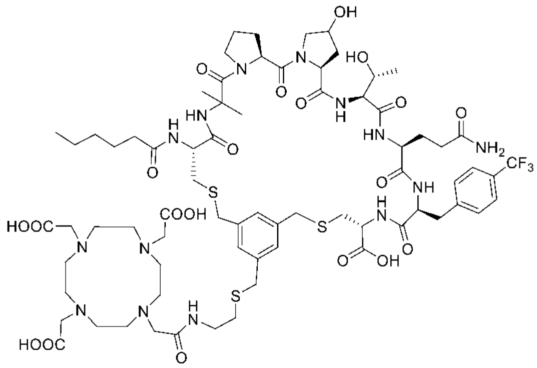

Hex-[Cys-(tMeBn(DOTA-AET))-Aib-Pro-(4-OH-Pro)-Thr-Gln-(4-$CF_3$-Phe)-Cys]-OH,

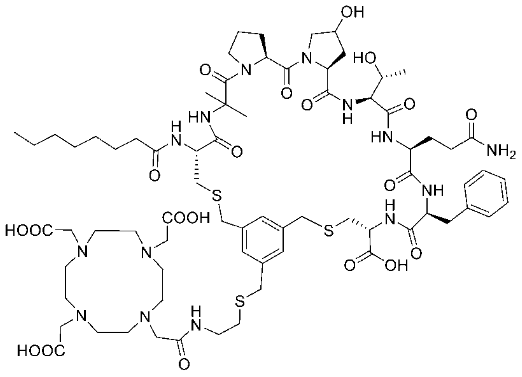

Oct-[Cys-(tMeBn(DOTA-AET))-Aib-Pro-(4-OH-Pro)-Thr-Gln-Phe-Cys]-OH,

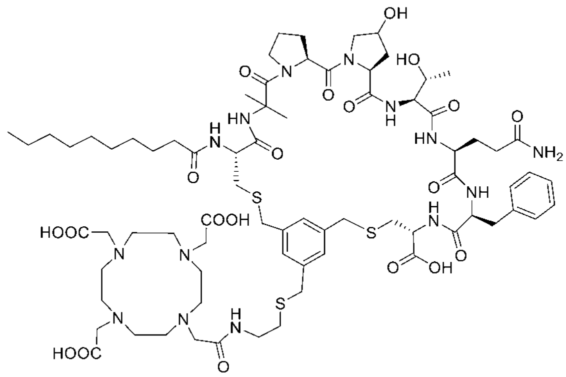

Dec-[Cys-(tMeBn(DOTA-AET))-Aib-Pro-(4-OH-Pro)-Thr-Gln-Phe-Cys]-OH,

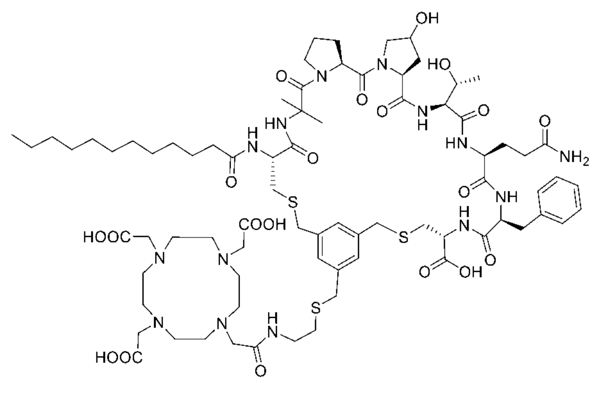

Dod-[Cys-(tMeBn(DOTA-AET))-Aib-Pro-(4-OH-Pro)-Thr-Gln-Phe-Cys]-OH,

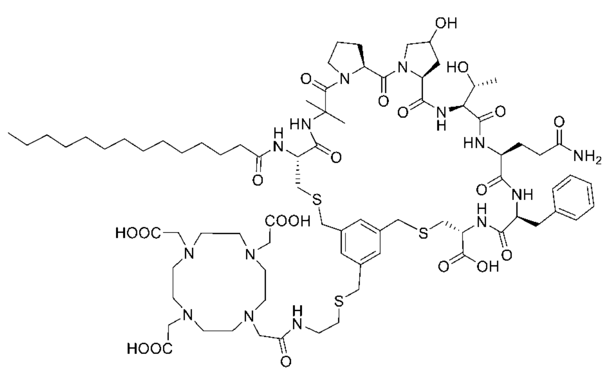

Tet-[Cys-(tMeBn(DOTA-AET))-Aib-Pro-(4-OH-Pro)-Thr-Gln-Phe-Cys]-OH,

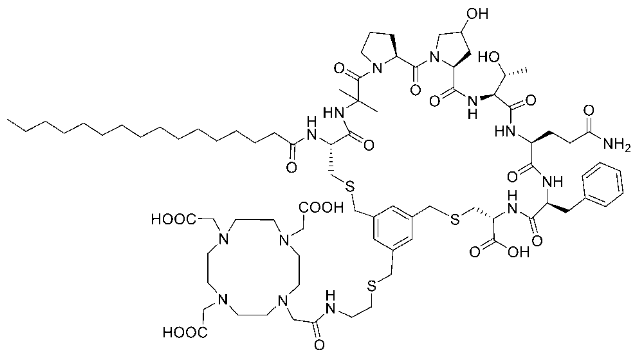

Pal-[Cys-(tMeBn(DOTA-AET))-Aib-Pro-(4-OH-Pro)-Thr-Gln-Phe-Cys]-OH,

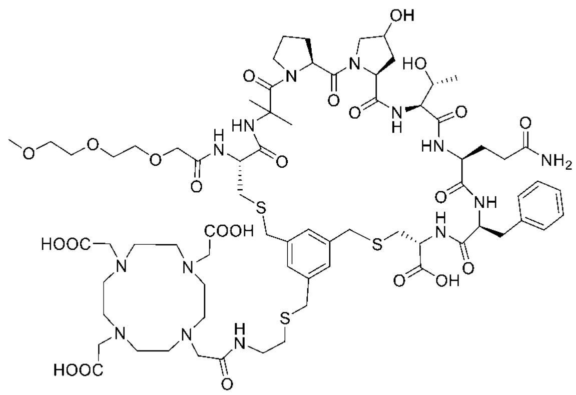

{2-[2-(2-Methoxyethoxy)ethoxy]-Ace}-[Cys-(tMeBn(DOTA-AET))-Aib-Pro-(4-OH-Pro)-Thr-Gln-Phe-Cys]-OH,

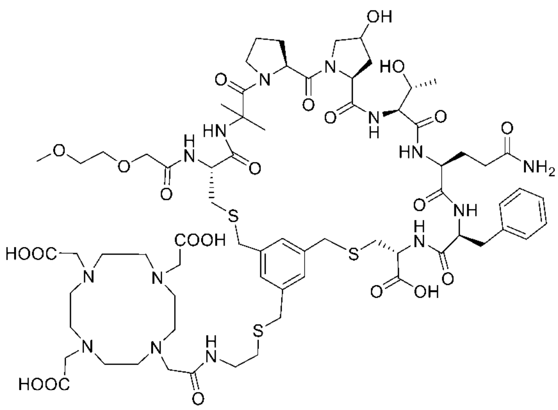

[2-(2-Methoxyethoxy)-Ace]-[Cys-(tMeBn(DOTA-AET))-Aib-Pro-(4-OH-Pro)-Thr-Gln-Phe-Cys]-OH,

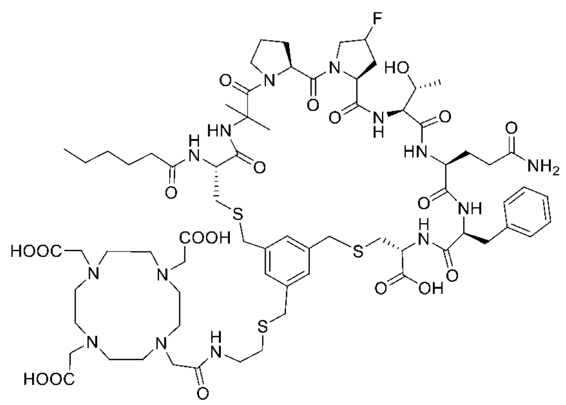

Hex-[Cys-(tMeBn(DOTA-AET))-Aib-Pro-(4-F-Pro)-Thr-Gln-Phe-Cys]-OH,

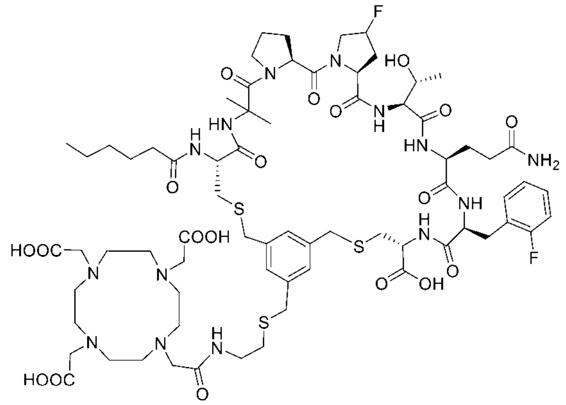

Hex-[Cys-(tMeBn(DOTA-AET))-Aib-Pro-(4-F-Pro)-Thr-Gln-(2-F-Phe)-Cys]-OH,

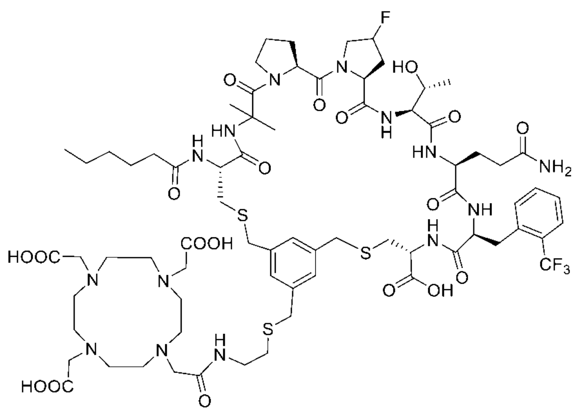

Hex-[Cys-(tMeBn(DOTA-AET))-Aib-Pro-(4-F-Pro)-Thr-Gln-(2-$CF_3$-Phe)-Cys]-OH,

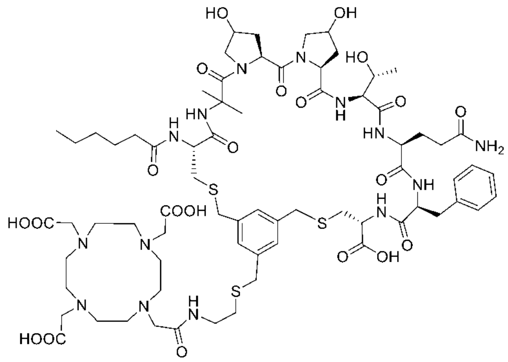

Hex-[Cys-(tMeBn(DOTA-AET))-Aib-(4-OH-Pro)-(4-OH-Pro)-Thr-Gln-Phe-Cys]-OH,

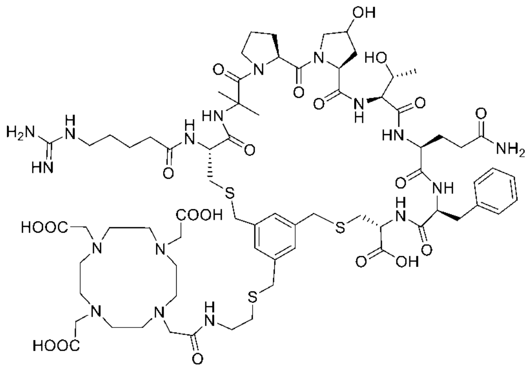

(5-guanidineValy)-[Cys-(tMeBn(DOTA-AET))-Aib-Pro-(4-OH-Pro)-Thr-Gln-Phe-Cys]-OH,

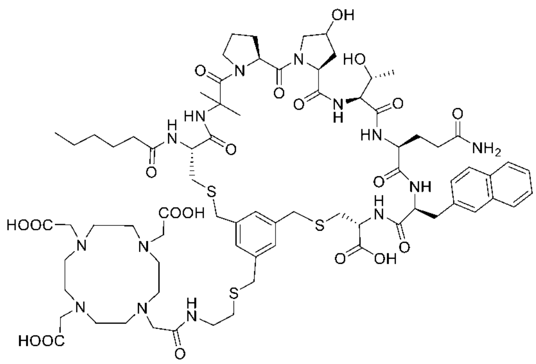

Hex-[Cys-(tMeBn(DOTA-AET))-Aib-Pro-(4-OH-Pro)-Thr-Gln-(2-Nal)-Cys]-OH,

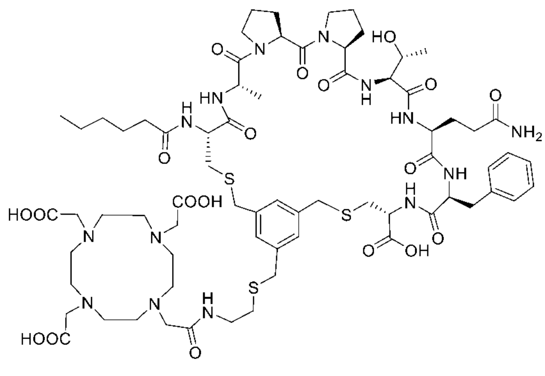

Hex-[Cys-(tMeBn(DOTA-AET))-Ala-Pro-Pro-Thr-Gln-Phe-Cys]-OH,

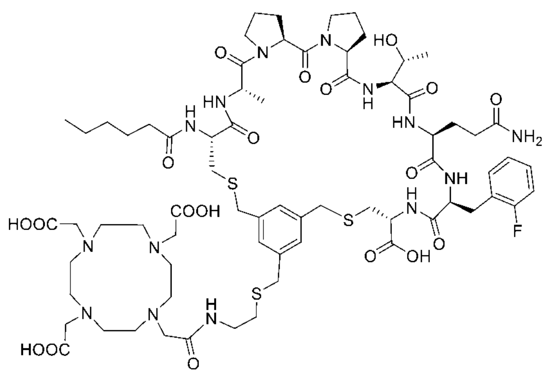

Hex-[Cys-(tMeBn(DOTA-AET))-Ala-Pro-Pro-Thr-Gln-(2-F-Phe)-Cys]-OH,

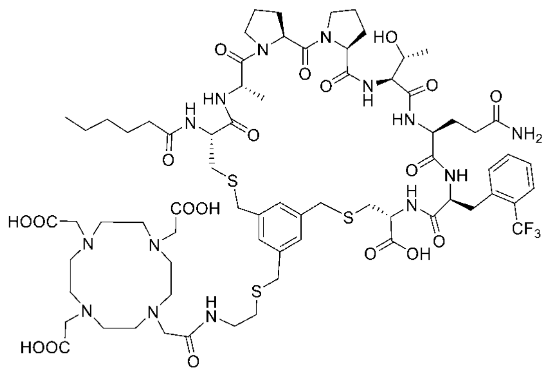

Hex-[Cys-(tMeBn(DOTA-AET))-Ala-Pro-Pro-Thr-Gln-(2-$CF_3$-Phe)-Cys]-OH,

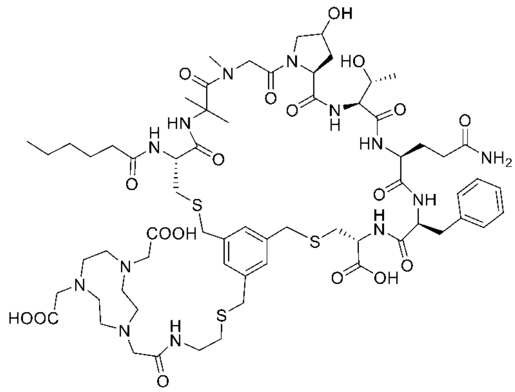

Hex-[Cys-(tMeBn(NOTA-AET))-Aib-Sar-(4-OH-Pro)-Thr-Gln-Phe-Cys]-OH,

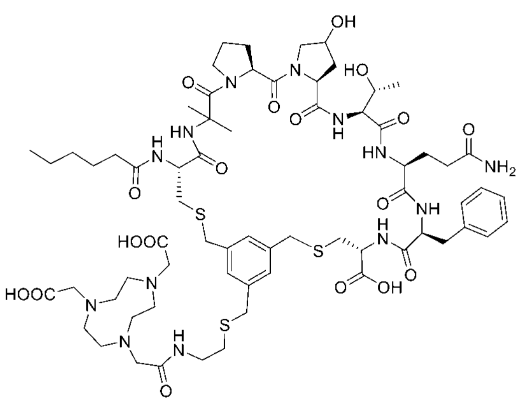

Hex-[Cys-(tMeBn(NOTA-AET))-Aib-Pro-(4-OH-Pro)-Thr-Gln-Phe-Cys]-OH,

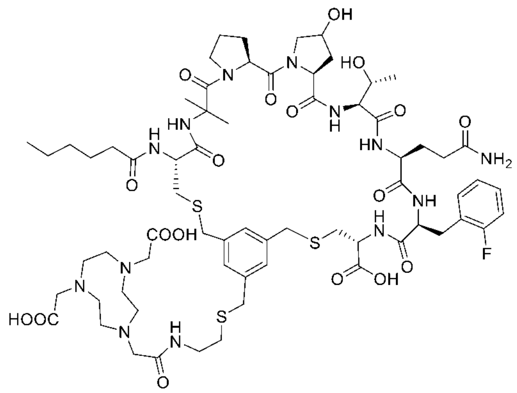

Hex-[Cys-(tMeBn(NOTA-AET))-Aib-Pro-(4-OH-Pro)-Thr-Gln-(2-F-Phe)-Cys]-OH,

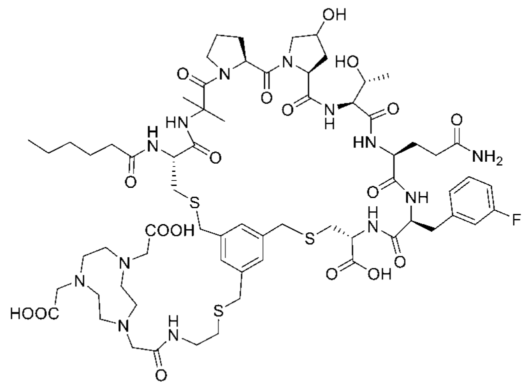

Hex-[Cys-(tMeBn(NOTA-AET))-Aib-Pro-(4-OH-Pro)-Thr-Gln-(3-F-Phe)-Cys]-OH,

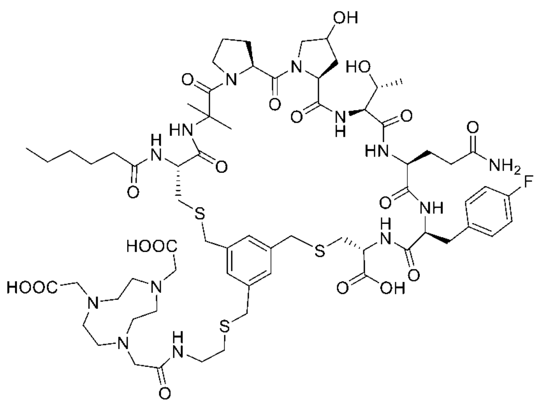

Hex-[Cys-(tMeBn(NOTA-AET))-Aib-Pro-(4-OH-Pro)-Thr-Gln-(4-F-Phe)-Cys]-OH,

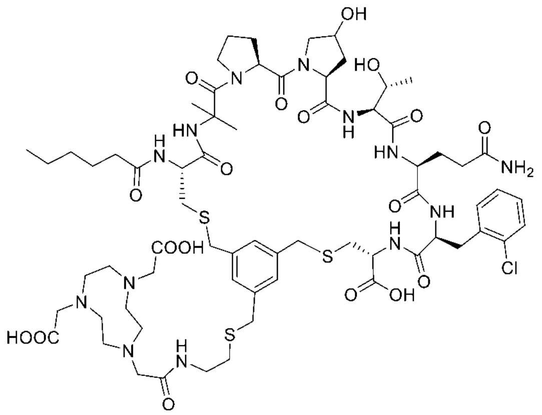

Hex-[Cys-(tMeBn(NOTA-AET))-Aib-Pro-(4-OH-Pro)-Thr-Gln-(2-Cl-Phe)-Cys]-OH,

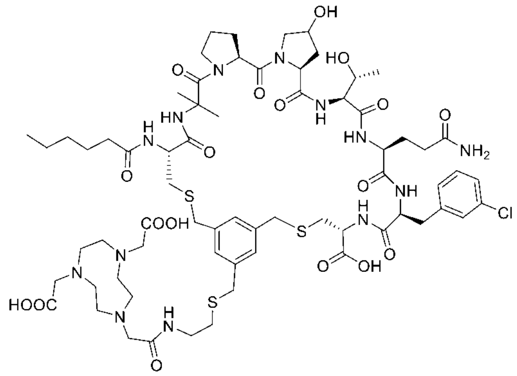

Hex-[Cys-(tMeBn(NOTA-AET))-Aib-Pro-(4-OH-Pro)-Thr-Gln-(3-Cl-Phe)-Cys]-OH,

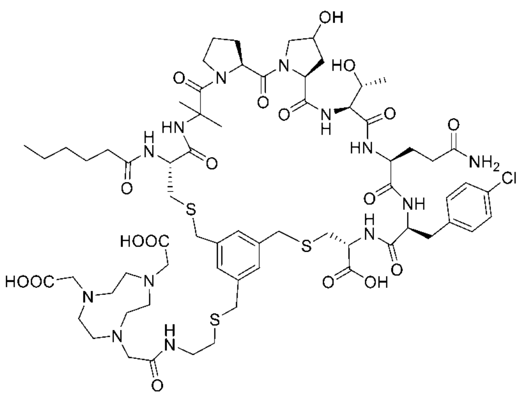

Hex-[Cys-(tMeBn(NOTA-AET))-Aib-Pro-(4-OH-Pro)-Thr-Gln-(4-Cl-Phe)-Cys]-OH,

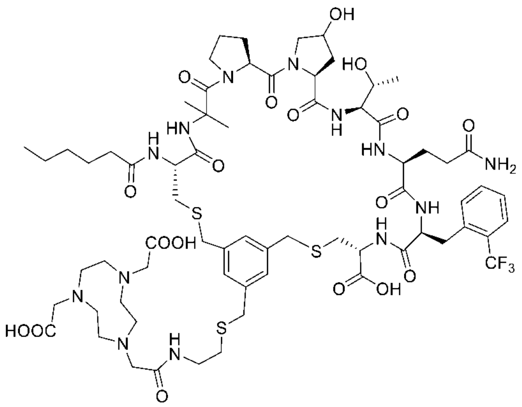

Hex-[Cys-(tMeBn(NOTA-AET))-Aib-Pro-(4-OH-Pro)-Thr-Gln-(2-$CF_3$-Phe)-Cys]-OH,

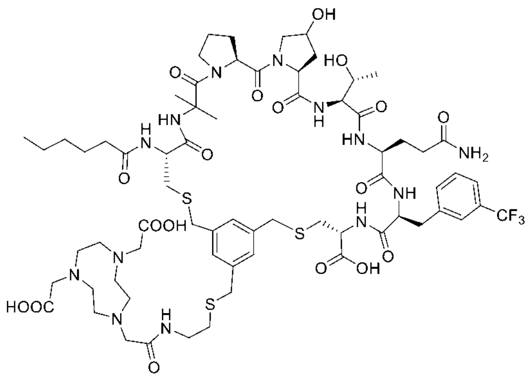

Hex-[Cys-(tMeBn(NOTA-AET))-Aib-Pro-(4-OH-Pro)-Thr-Gln-(3-$CF_3$-Phe)-Cys]-OH,

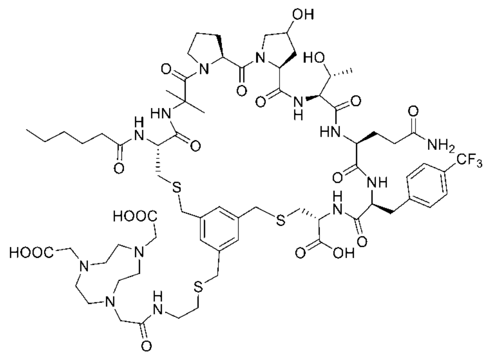

Hex-[Cys-(tMeBn(NOTA-AET))-Aib-Pro-(4-OH-Pro)-Thr-Gln-(4-$CF_3$-Phe)-Cys]-OH,

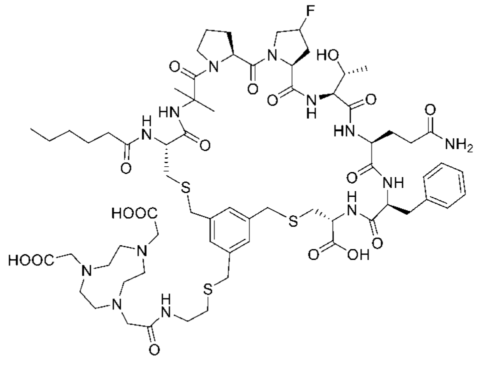

Hex-[Cys-(tMeBn(NOTA-AET))-Aib-Pro-(4-F-Pro)-Thr-Gln-Phe-Cys]-OH,

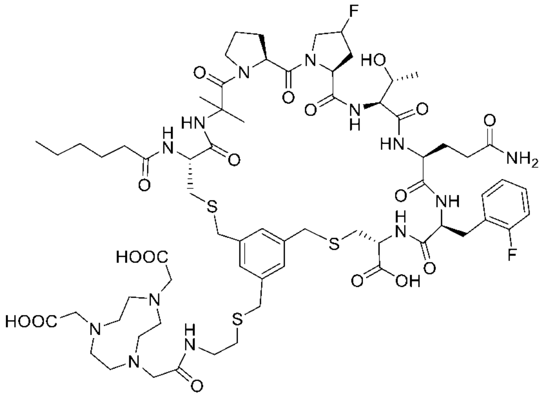

Hex-[Cys-(tMeBn(NOTA-AET))-Aib-Pro-(4-F-Pro)-Thr-Gln-(2-F-Phe)-Cys]-OH,

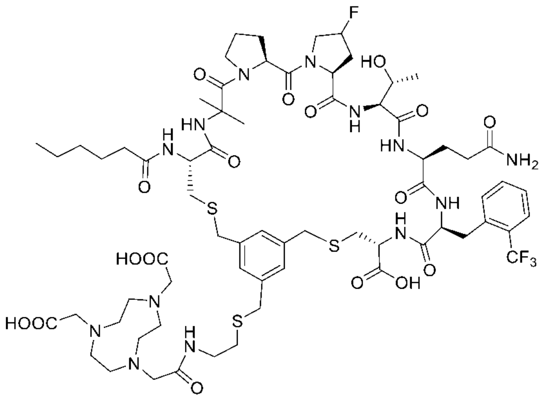

Hex-[Cys-(tMeBn(NOTA-AET))-Aib-Pro-(4-F-Pro)-Thr-Gln-(2-$CF_3$-Phe)-Cys]-OH,

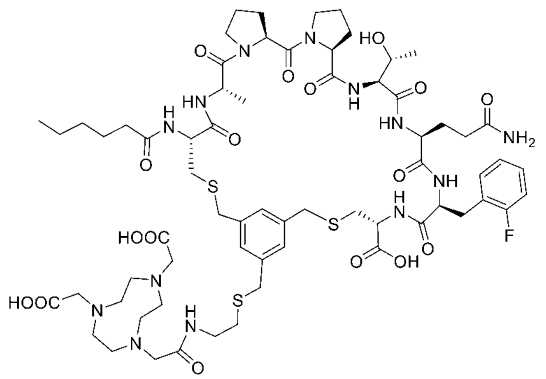

Hex-[Cys-(tMeBn(NOTA-AET))-Ala-Pro-Pro-Thr-Gln-(2-F-Phe)-Cys]-OH, and

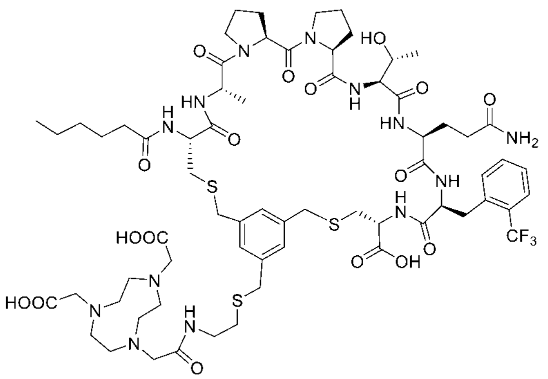

Hex-[Cys-(tMeBn(NOTA-AET))-Ala-Pro-Pro-Thr-Gln-(2-$CF_3$-Phe)-Cys]-OH; group 5:

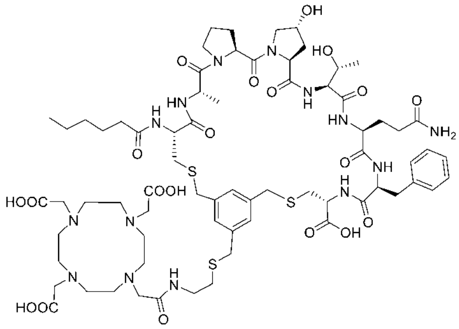

Hex-[Cys-(tMeBn(DOTA-AET))-Ala-Pro-((4R)-OH-Pro)-Thr-Gln-Phe-Cys]-OH,

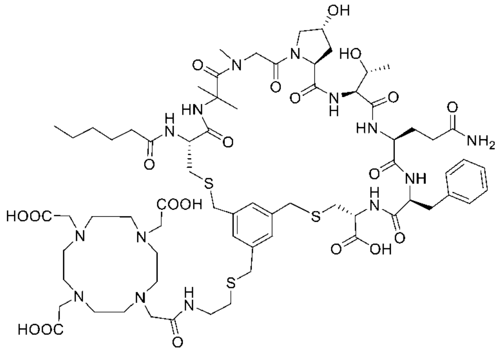

Hex-[Cys-(tMeBn(DOTA-AET))-Aib-Sar-((4R)-OH-Pro)-Thr-Gln-Phe-Cys]-OH,

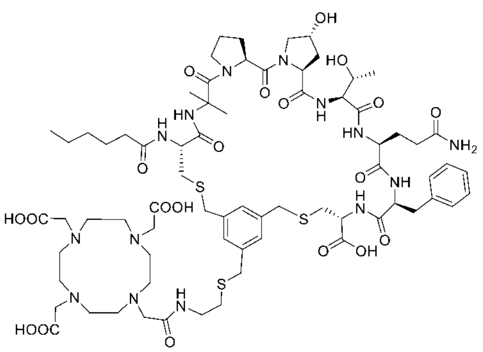

Hex-[Cys-(tMeBn(DOTA-AET))-Aib-Pro-((4R)-OH-Pro)-Thr-Gln-Phe-Cys]-OH,

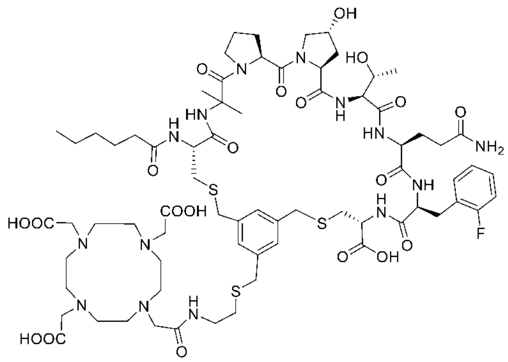

Hex-[Cys-(tMeBn(DOTA-AET))-Aib-Pro-((4R)-OH-Pro)-Thr-Gln-(2-F-Phe)-Cys]-OH,

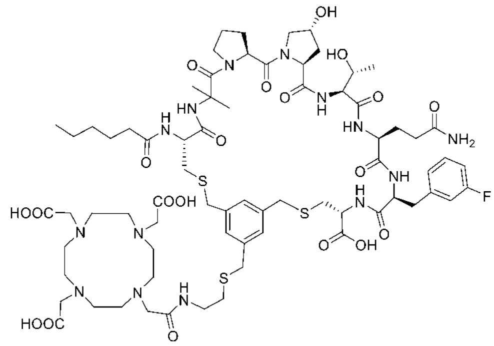

Hex-[Cys-(tMeBn(DOTA-AET))-Aib-Pro-((4R)-OH-Pro)-Thr-Gln-(3-F-Phe)-Cys]-OH,

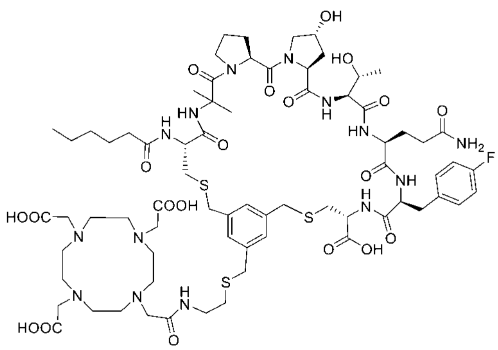

Hex-[Cys-(tMeBn(DOTA-AET))-Aib-Pro-((4R)-OH-Pro)-Thr-Gln-(4-F-Phe)-Cys]-OH,

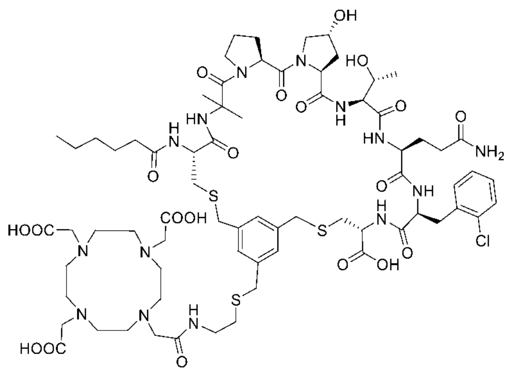

Hex-[Cys-(tMeBn(DOTA-AET))-Aib-Pro-((4R)-OH-Pro)-Thr-Gln-(2-Cl-Phe)-Cys]-OH,

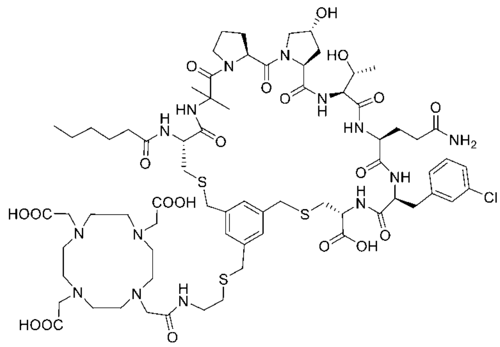

Hex-[Cys-(tMeBn(DOTA-AET))-Aib-Pro-((4R)-OH-Pro)-Thr-Gln-(3-Cl-Phe)-Cys]-OH,

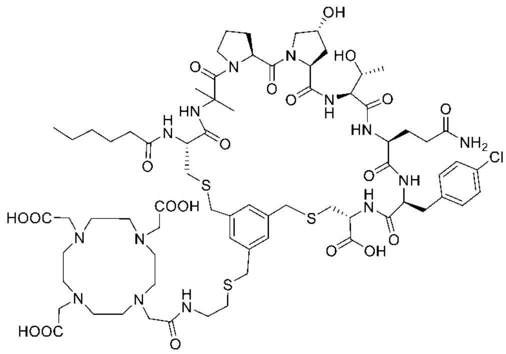

Hex-[Cys-(tMeBn(DOTA-AET))-Aib-Pro-((4R)-OH-Pro)-Thr-Gln-(4-Cl-Phe)-Cys]-OH,

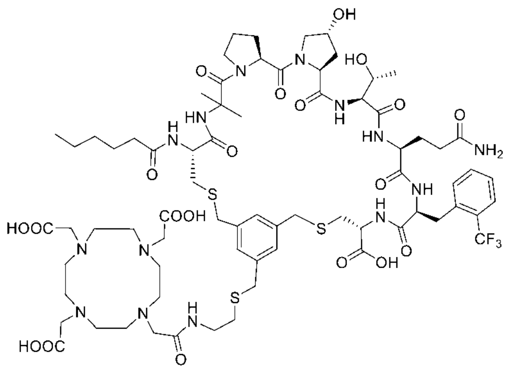

Hex-[Cys-(tMeBn(DOTA-AET))-Aib-Pro-((4R)-OH-Pro)-Thr-Gln-(2-$CF_3$-Phe)-Cys]-OH,

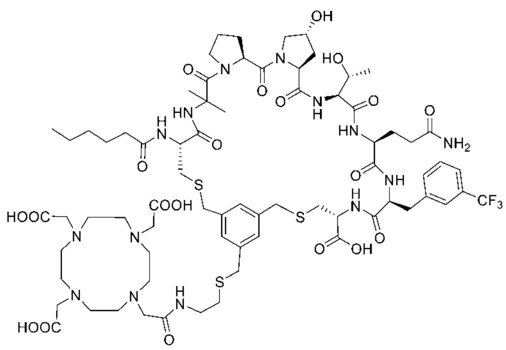

Hex-[Cys-(tMeBn(DOTA-AET))-Aib-Pro-((4R)-OH-Pro)-Thr-Gln-(3-$CF_3$-Phe)-Cys]-OH,

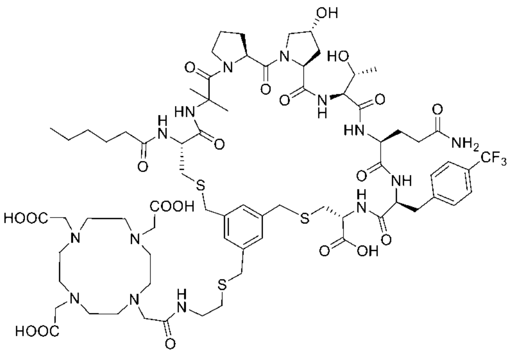

Hex-[Cys-(tMeBn(DOTA-AET))-Aib-Pro-((4R)-OH-Pro)-Thr-Gln-(4-$CF_3$-Phe)-Cys]-OH,

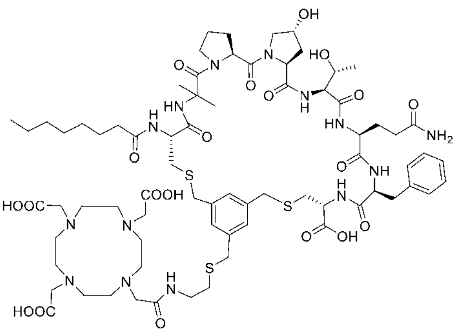

Oct-[Cys-(tMeBn(DOTA-AET))-Aib-Pro-((4R)-OH-Pro)-Thr-Gln-Phe-Cys]- OH,

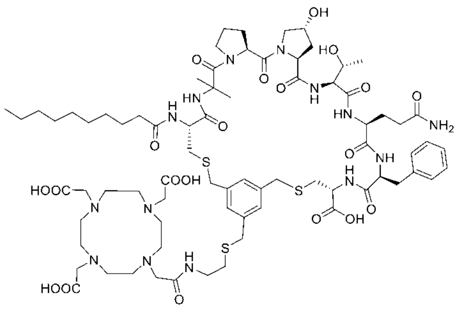

Dec-[Cys-(tMeBn(DOTA-AET))-Aib-Pro-((4R)-OH-Pro)-Thr-Gln-Phe-Cys]-OH,

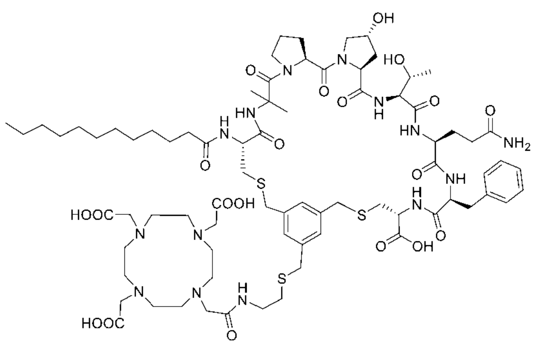

Dod-[Cys-(tMeBn(DOTA-AET))-Aib-Pro-((4R)-OH-Pro)-Thr-Gln-Phe-Cys]-OH,

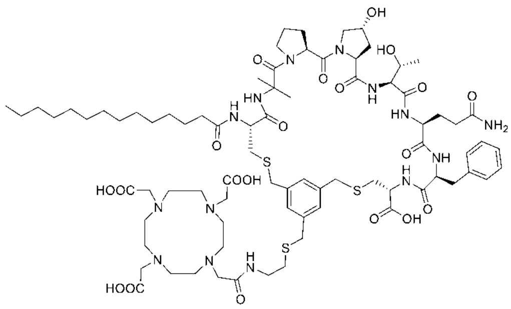

Tet-[Cys-(tMeBn(DOTA-AET))-Aib-Pro-((4R)-OH-Pro)-Thr-Gln-Phe-Cys]-OH,

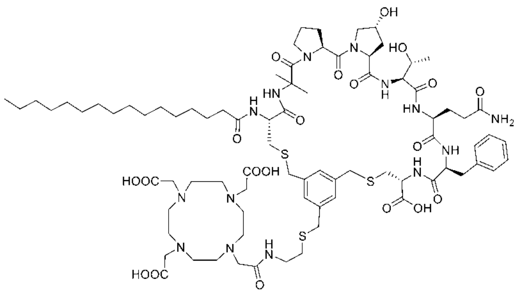

Pal-[Cys-(tMeBn(DOTA-AET))-Aib-Pro-((4R)-OH-Pro)-Thr-Gln-Phe-Cys]-OH,

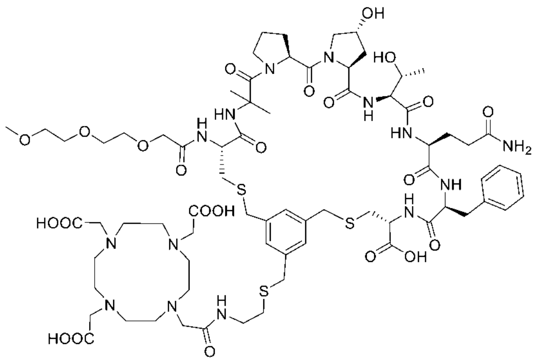

{2-[2-(2-Methoxyethoxy)ethoxy]-Ace}-[Cys-(tMeBn(DOTA-AET))-Aib-Pro-((4R)-OH-Pro)-Thr-Gln-Phe-Cys]-OH,

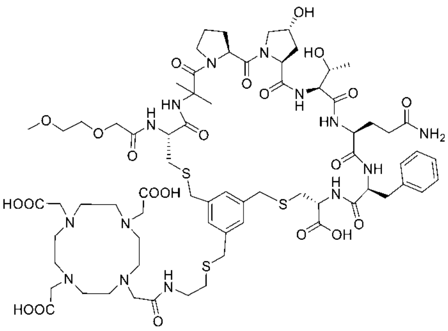

[2-(2-Methoxyethoxy)-Ace]-[Cys-(tMeBn(DOTA-AET))-Aib-Pro-((4R)-OH-Pro)-Thr-Gln-Phe-Cys]-OH,

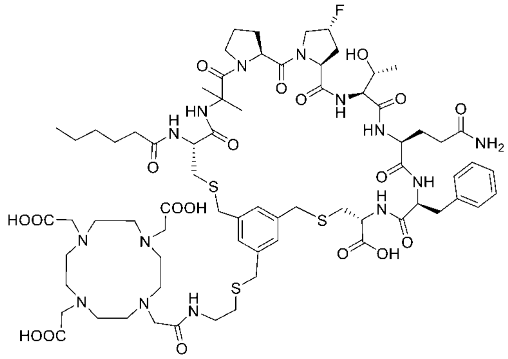

Hex-[Cys-(tMeBn(DOTA-AET))-Aib-Pro-((4R)-F-Pro)-Thr-Gln-Phe-Cys]-OH,

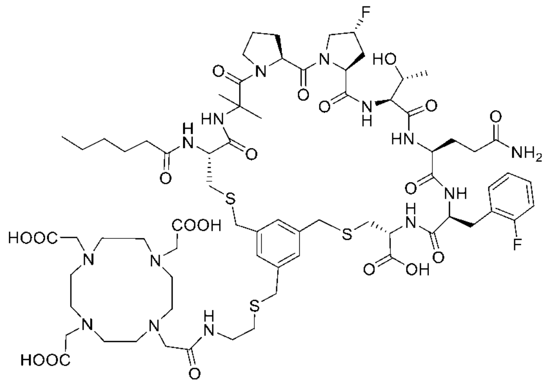

Hex-[Cys-(tMeBn(DOTA-AET))-Aib-Pro-((4R)-F-Pro)-Thr-Gln-(2-F-Phe)-Cys]-OH,

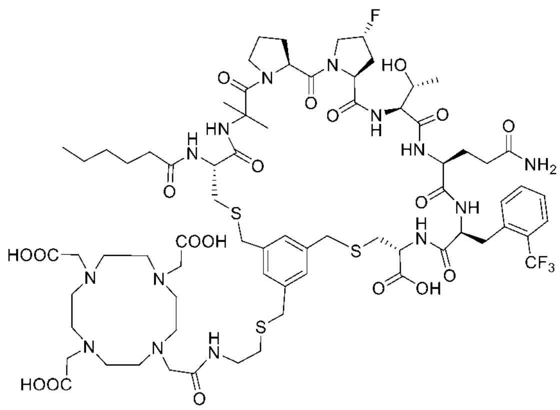

Hex-[Cys-(tMeBn(DOTA-AET))-Aib-Pro-((4R)-F-Pro)-Thr-Gln-(2-$CF_3$-Phe)-Cys]-OH,

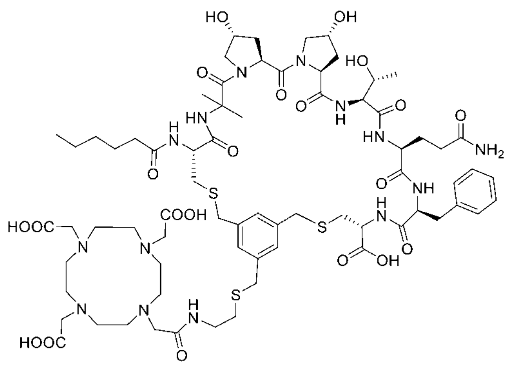

Hex-[Cys-(tMeBn(DOTA-AET))-Aib-((4R)-OH-Pro)-((4R)-OH-Pro)-Thr-Gln-Phe-Cys]-OH,

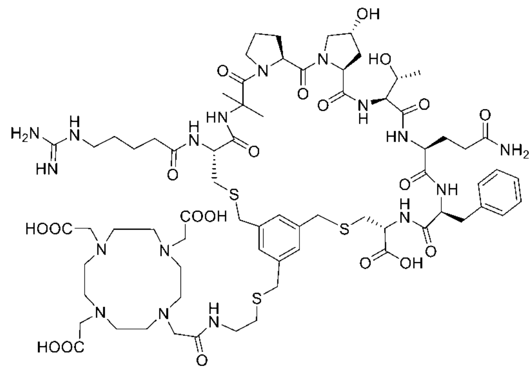

(5-guanidineValy)-[Cys-(tMeBn(DOTA-AET))-Aib-Pro-((4R)-OH-Pro)-Thr-Gln-Phe-Cys]-OH,

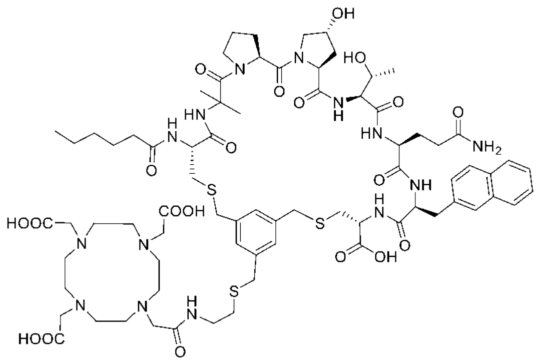

Hex-[Cys-(tMeBn(DOTA-AET))-Aib-Pro-((4R)-OH-Pro)-Thr-Gln-(2-Nal)-Cys]-OH,

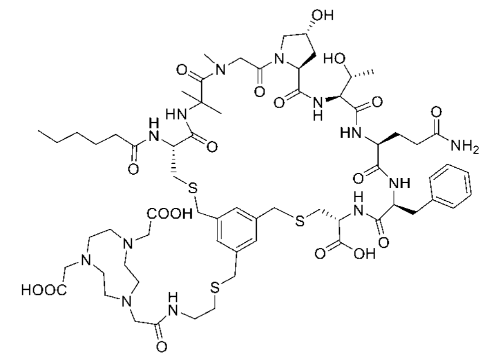

Hex-[Cys-(tMeBn(NOTA-AET))-Aib-Sar-((4R)-OH-Pro)-Thr-Gln-Phe-Cys]-OH,

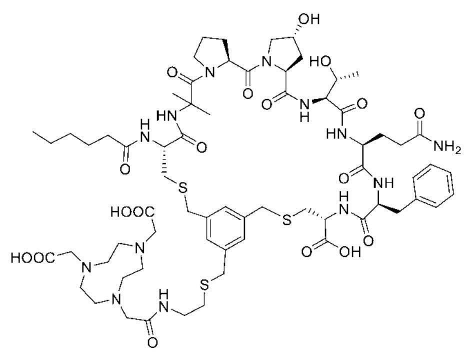

Hex-[Cys-(tMeBn(NOTA-AET))-Aib-Pro-((4R)-OH-Pro)-Thr-Gln-Phe-Cys]-OH,

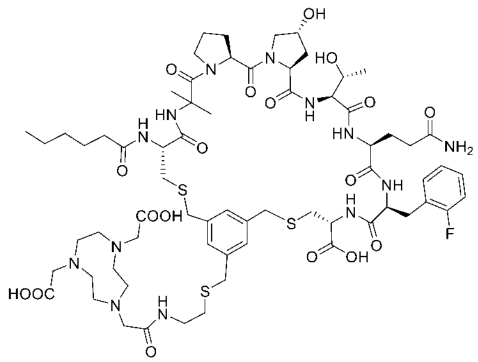

Hex-[Cys-(tMeBn(NOTA-AET))-Aib-Pro-((4R)-OH-Pro)-Thr-Gln-(2-F-Phe)-Cys]-OH,

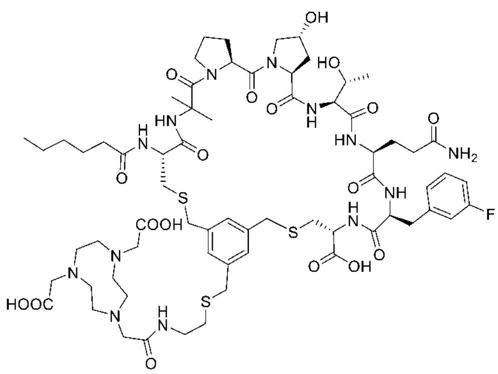

Hex-[Cys-(tMeBn(NOTA-AET))-Aib-Pro-((4R)-OH-Pro)-Thr-Gln-(3-F-Phe)-Cys]-OH,

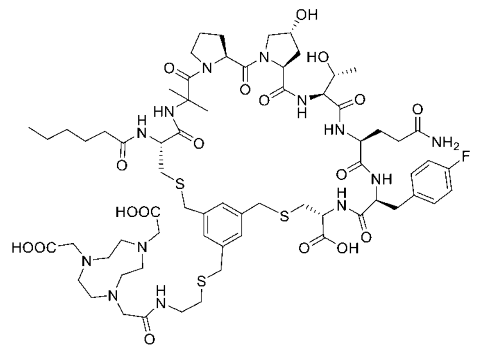

Hex-[Cys-(tMeBn(NOTA-AET))-Aib-Pro-((4R)-OH-Pro)-Thr-Gln-(4-F-Phe)-Cys]-OH,

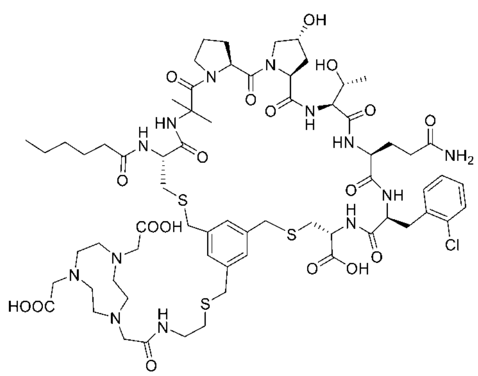

Hex-[Cys-(tMeBn(NOTA-AET))-Aib-Pro-((4R)-OH-Pro)-Thr-Gln-(2-Cl-Phe)-Cys]-OH,

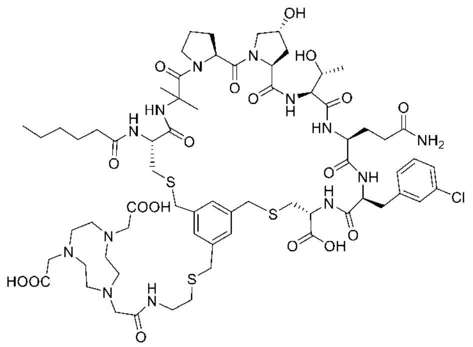

Hex-[Cys-(tMeBn(NOTA-AET))-Aib-Pro-((4R)-OH-Pro)-Thr-Gln-(3-Cl-Phe)-Cys]-OH,

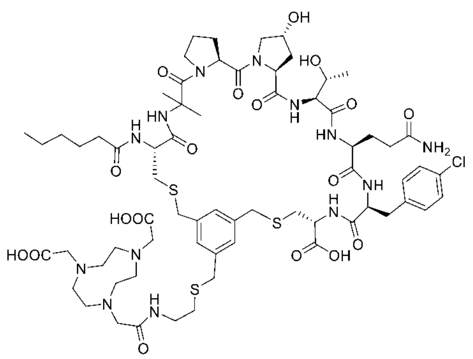

Hex-[Cys-(tMeBn(NOTA-AET))-Aib-Pro-((4R)-OH-Pro)-Thr-Gln-(4-Cl-Phe)-Cys]-OH,

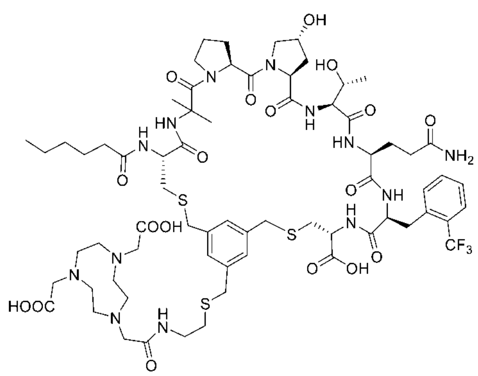

Hex-[Cys-(tMeBn(NOTA-AET))-Aib-Pro-((4R)-OH-Pro)-Thr-Gln-(2-$CF_3$-Phe)-Cys]-OH,

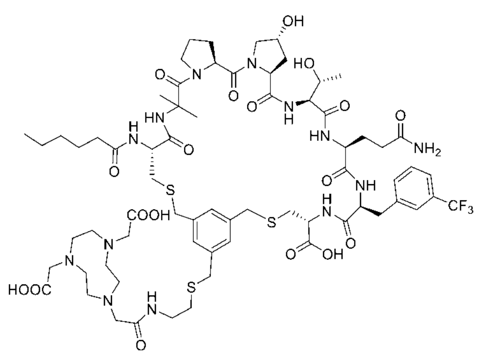

Hex-[Cys-(tMeBn(NOTA-AET))-Aib-Pro-((4R)-OH-Pro)-Thr-Gln-(3-$CF_3$-Phe)-Cys]-OH,

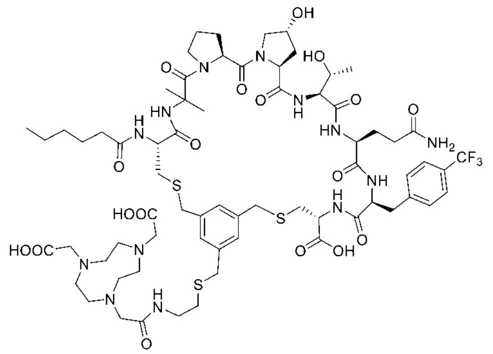

Hex-[Cys-(tMeBn(NOTA-AET))-Aib-Pro-((4R)-OH-Pro)-Thr-Gln-(4-$CF_3$-Phe)-Cys]-OH,

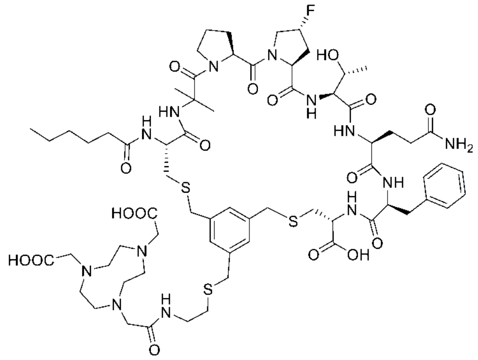

Hex-[Cys-(tMeBn(NOTA-AET))-Aib-Pro-((4R)-F-Pro)-Thr-Gln-Phe-Cys]-OH,

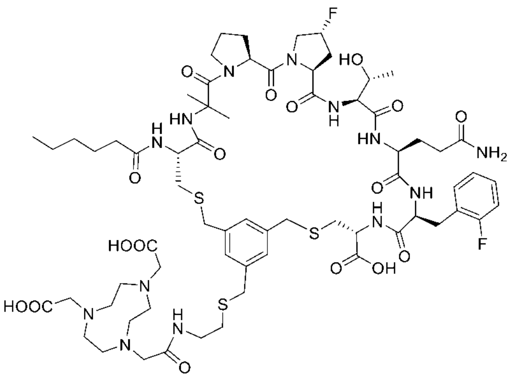

Hex-[Cys-(tMeBn(NOTA-AET))-Aib-Pro-((4R)-F-Pro)-Thr-Gln-(2-F-Phe)-Cys]-OH,

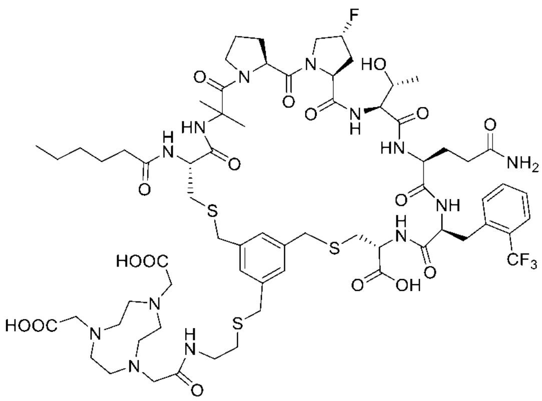

Hex-[Cys-(tMeBn(NOTA-AET))-Aib-Pro-((4R)-F-Pro)-Thr-Gln-(2-$CF_3$-Phe)-Cys]-OH; and group 6:

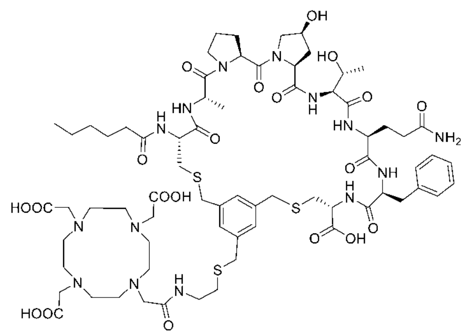

Hex-[Cys-(tMeBn(DOTA-AET))-Ala-Pro-((4S)-OH-Pro)-Thr-Gln-Phe-Cys]-OH,

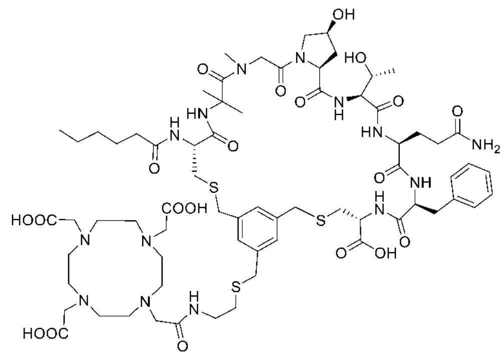

Hex-[Cys-(tMeBn(DOTA-AET))-Aib-Sar-((4S)-OH-Pro)-Thr-Gln-Phe-Cys]-OH,

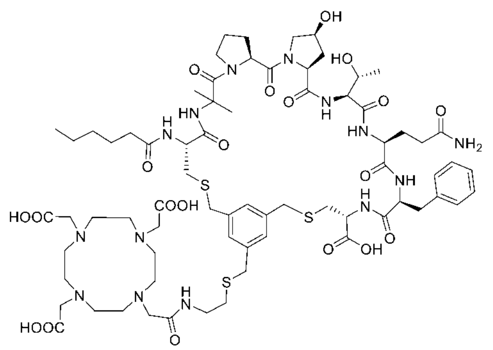

Hex-[Cys-(tMeBn(DOTA-AET))-Aib-Pro-((4S)-OH-Pro)-Thr-Gln-Phe-Cys]-OH,

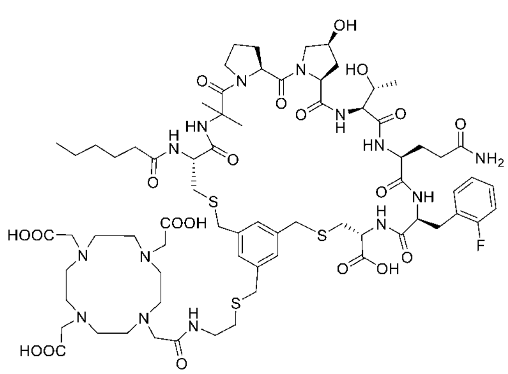

Hex-[Cys-(tMeBn(DOTA-AET))-Aib-Pro-((4S)-OH-Pro)-Thr-Gln-(2-F-Phe)-Cys]-OH,

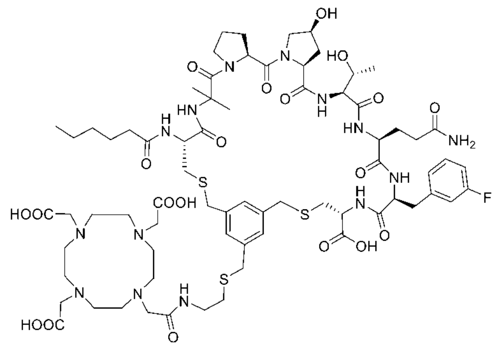

Hex-[Cys-(tMeBn(DOTA-AET))-Aib-Pro-((4S)-OH-Pro)-Thr-Gln-(3-F-Phe)-Cys]-OH,

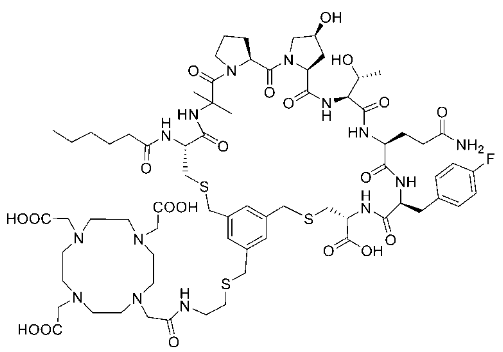

Hex-[Cys-(tMeBn(DOTA-AET))-Aib-Pro-((4S)-OH-Pro)-Thr-Gln-(4-F-Phe)-Cys]-OH,

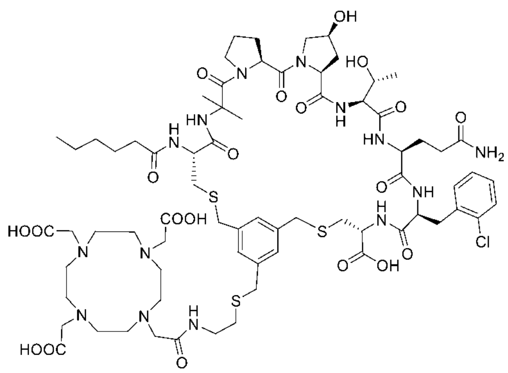

Hex-[Cys-(tMeBn(DOTA-AET))-Aib-Pro-((4S)-OH-Pro)-Thr-Gln-(2-Cl-Phe)-Cys]-OH,

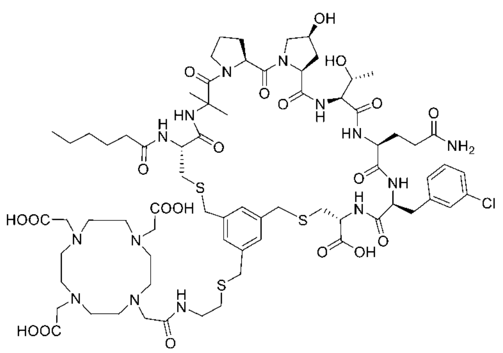

Hex-[Cys-(tMeBn(DOTA-AET))-Aib-Pro-((4S)-OH-Pro)-Thr-Gln-(3-Cl-Phe)-Cys]-OH,

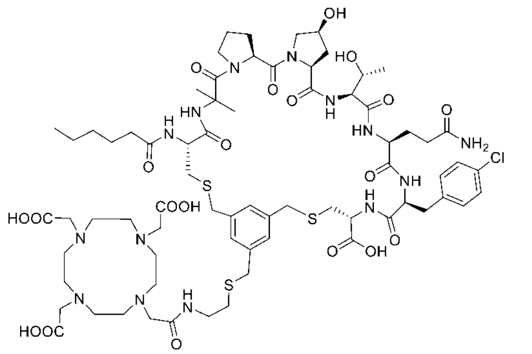

Hex-[Cys-(tMeBn(DOTA-AET))-Aib-Pro-((4S)-OH-Pro)-Thr-Gln-(4-Cl-Phe)-Cys]-OH,

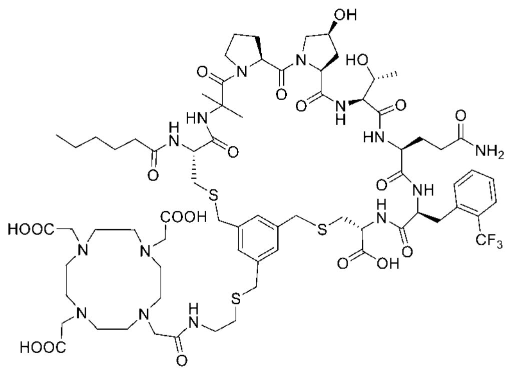

Hex-[Cys-(tMeBn(DOTA-AET))-Aib-Pro-((4S)-OH-Pro)-Thr-Gln-(2-$CF_3$-Phe)-Cys]-OH,

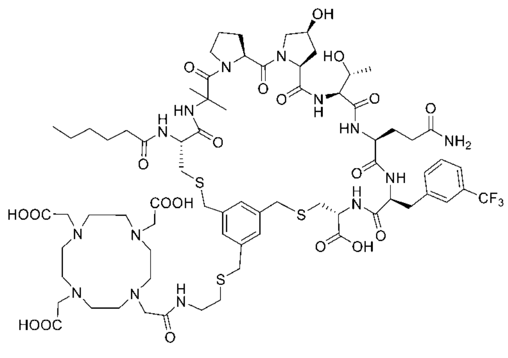

Hex-[Cys-(tMeBn(DOTA-AET))-Aib-Pro-((4S)-OH-Pro)-Thr-Gln-(3-$CF_3$-Phe)-Cys]-OH,

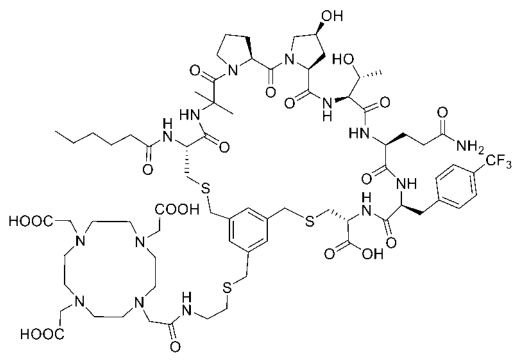

Hex-[Cys-(tMeBn(DOTA-AET))-Aib-Pro-((4S)-OH-Pro)-Thr-Gln-(4-$CF_3$-Phe)-Cys]-OH,

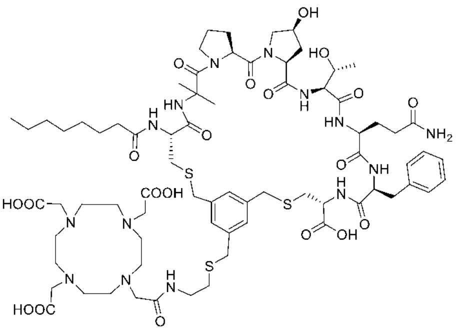

Oct- [Cys-(tMeBn(DOTA-AET))-Aib-Pro-((4S)-OH-Pro)-Thr-Gln-Phe-Cys] - OH,

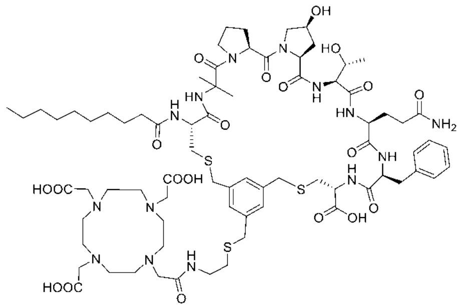

Dec-[Cys-(tMeBn(DOTA-AET))-Aib-Pro-((4S)-OH-Pro)-Thr-Gln-Phe-Cys]-OH,

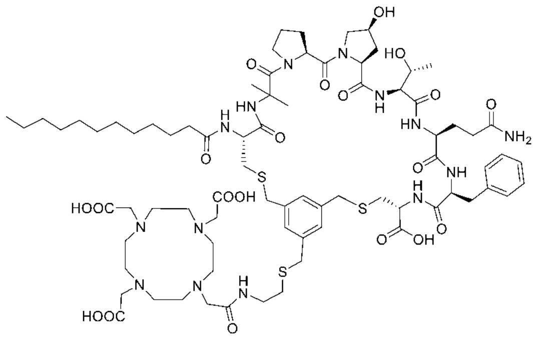

Dod-[Cys-(tMeBn(DOTA-AET))-Aib-Pro-((4S)-OH-Pro)-Thr-Gln-Phe-Cys]-OH,

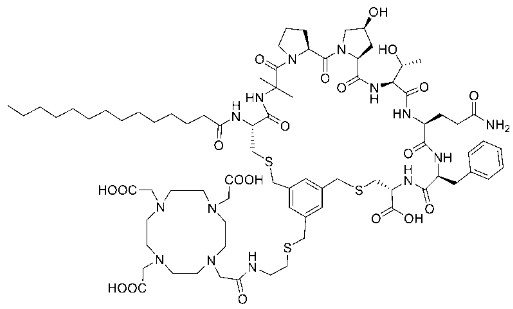

Tet-[Cys-(tMeBn(DOTA-AET))-Aib-Pro-((4S)-OH-Pro)-Thr-Gln-Phe-Cys]-OH,

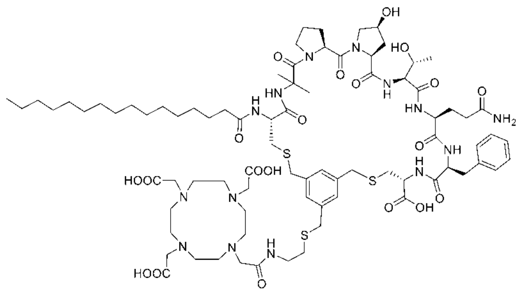

Pal-[Cys-(tMeBn(DOTA-AET))-Aib-Pro-((4S)-OH-Pro)-Thr-Gln-Phe-Cys]-OH,

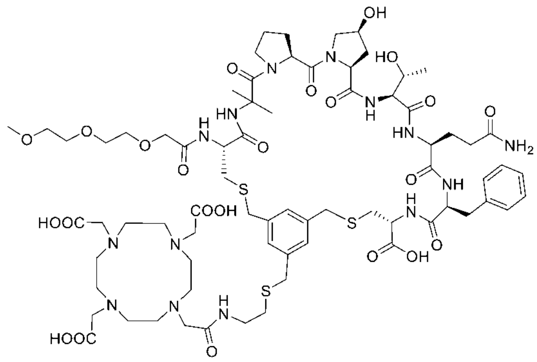

{2-[2-(2-Methoxyethoxy)ethoxy]-Ace}-[Cys-(tMeBn(DOTA-AET))-Aib-Pro-((4S)-OH-Pro)-Thr-Gln-Phe-Cys]-OH,

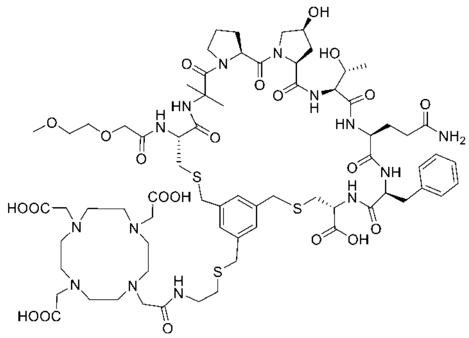

[2-(2-Methoxyethoxy)-Ace]-[Cys-(tMeBn(DOTA-AET))-Aib-Pro-((4S)-OH-Pro)-Thr-Gln-Phe-Cys]-OH,

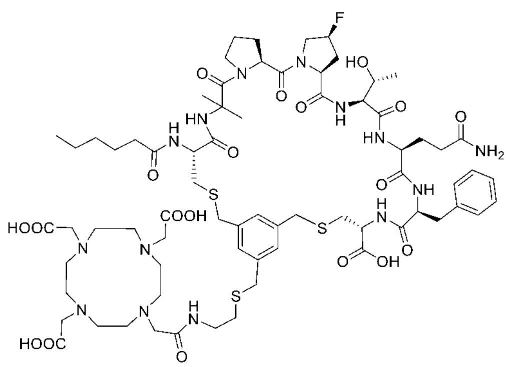

Hex-[Cys-(tMeBn(DOTA-AET))-Aib-Pro-((4S)-F-Pro)-Thr-Gln-Phe-Cys]-OH,

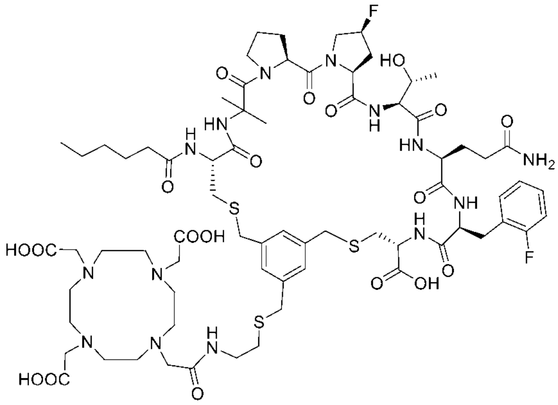

Hex-[Cys-(tMeBn(DOTA-AET))-Aib-Pro-((4S)-F-Pro)-Thr-Gln-(2-F-Phe)-Cys]-OH,

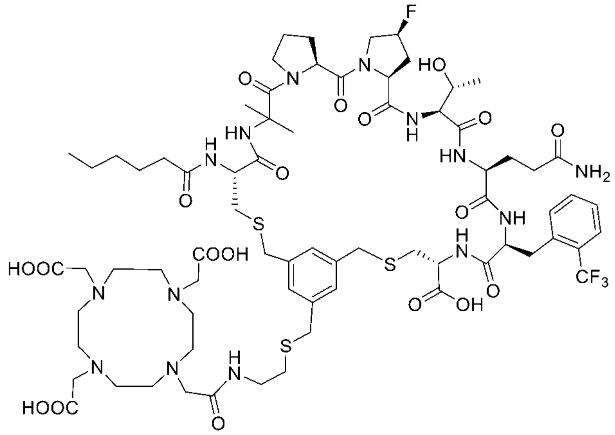

Hex-[Cys-(tMeBn(DOTA-AET))-Aib-Pro-((4S)-F-Pro)-Thr-Gln-(2-$CF_3$-Phe)-Cys]-OH,

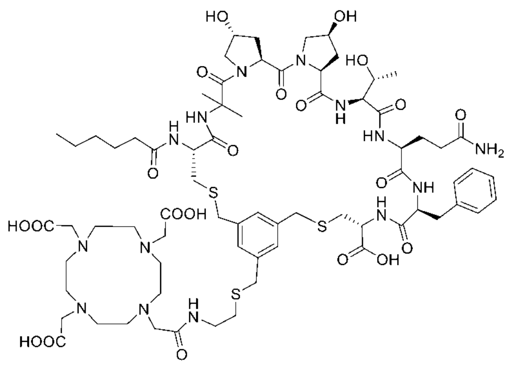

Hex-[Cys-(tMeBn(DOTA-AET))-Aib-((4R)-OH-Pro)-((4S)-OH-Pro)-Thr-Gln-Phe-Cys]-OH,

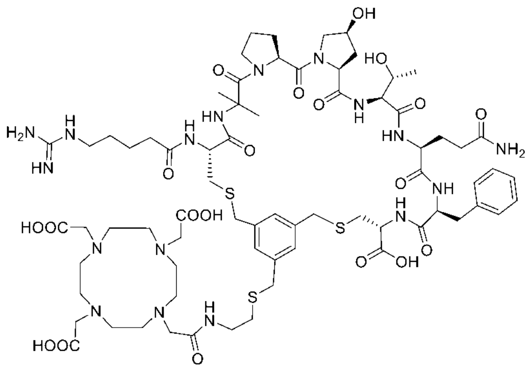

(5-guanidineValy)-[Cys-(tMeBn(DOTA-AET))-Aib-Pro-((4S)-OH-Pro)-Thr-Gln-Phe-Cys]-OH,

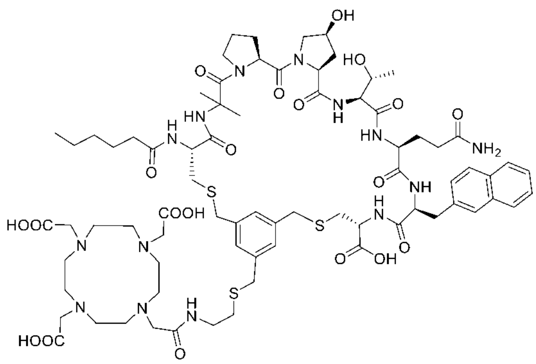

Hex-[Cys-(tMeBn(DOTA-AET))-Aib-Pro-((4S)-OH-Pro)-Thr-Gln-(2-Nal)-Cys]-OH,

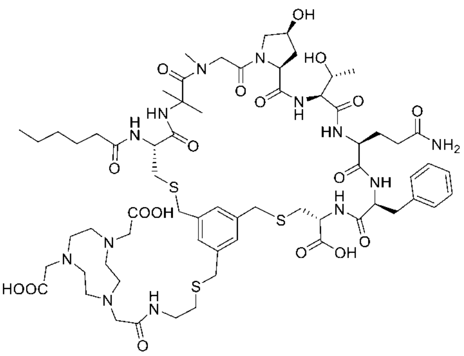

Hex-[Cys-(tMeBn(NOTA-AET))-Aib-Sar-((4S)-OH-Pro)-Thr-Gln-Phe-Cys]-OH,

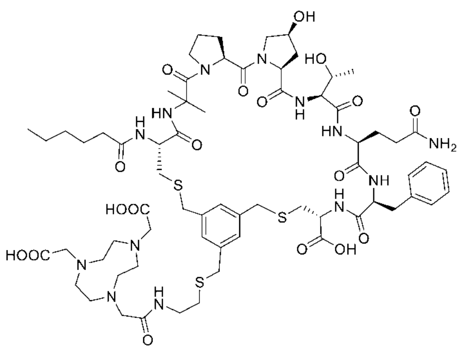

Hex-[Cys-(tMeBn(NOTA-AET))-Aib-Pro-((4S)-OH-Pro)-Thr-Gln-Phe-Cys]-OH,

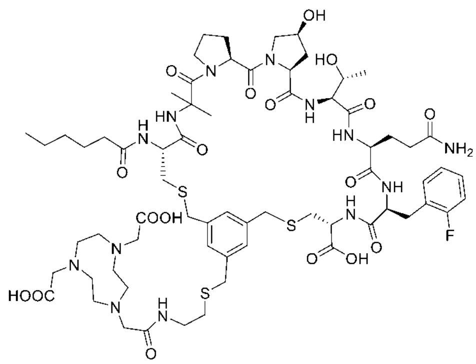

Hex-[Cys-(tMeBn(NOTA-AET))-Aib-Pro-((4S)-OH-Pro)-Thr-Gln-(2-F-Phe)-Cys]-OH,

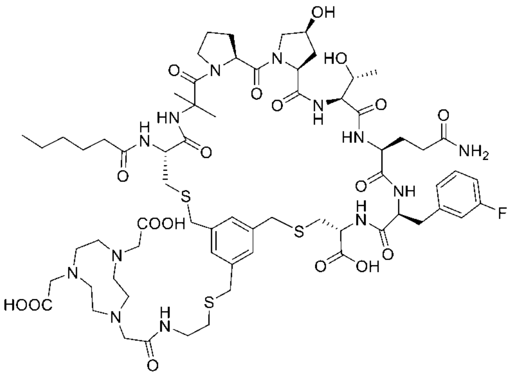

Hex-[Cys-(tMeBn(NOTA-AET))-Aib-Pro-((4S)-OH-Pro)-Thr-Gln-(3-F-Phe)-Cys]-OH,

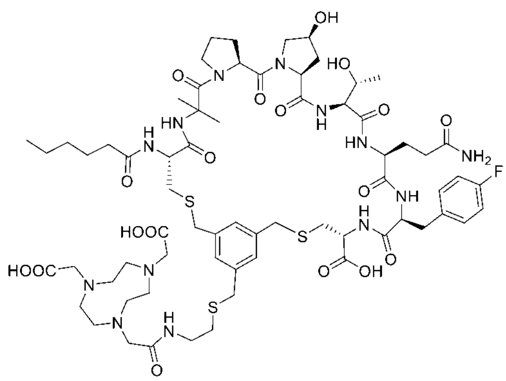

Hex-[Cys-(tMeBn(NOTA-AET))-Aib-Pro-((4S)-OH-Pro)-Thr-Gln-(4-F-Phe)-Cys]-OH,

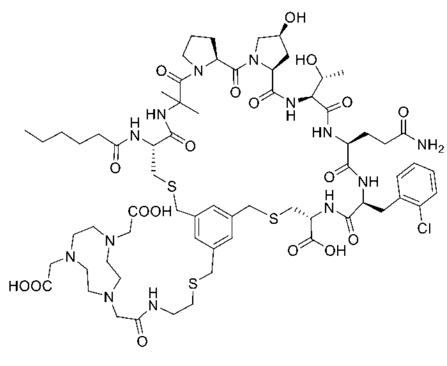

Hex-[Cys-(tMeBn(NOTA-AET))-Aib-Pro-((4S)-OH-Pro)-Thr-Gln-(2-Cl-Phe)-Cys]-OH,

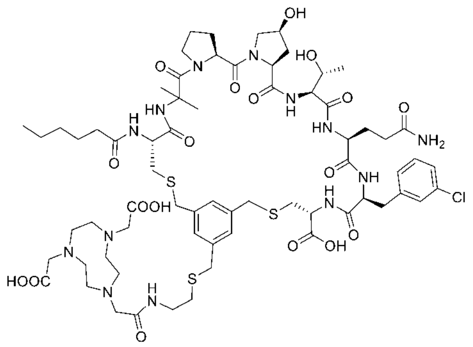

Hex-[Cys-(tMeBn(NOTA-AET))-Aib-Pro-((4S)-OH-Pro)-Thr-Gln-(3-Cl-Phe)-Cys]-OH,

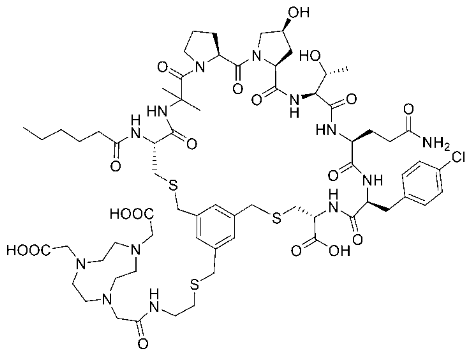

Hex-[Cys-(tMeBn(NOTA-AET))-Aib-Pro-((4S)-OH-Pro)-Thr-Gln-(4-Cl-Phe)-Cys]-OH,

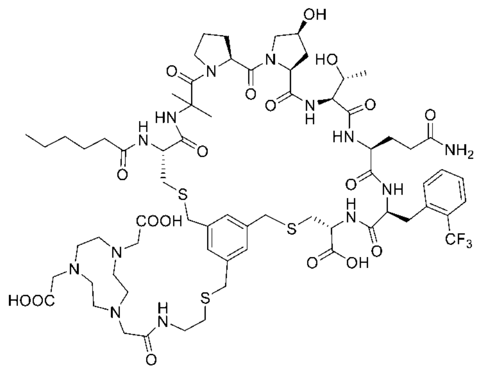

Hex-[Cys-(tMeBn(NOTA-AET))-Aib-Pro-((4S)-OH-Pro)-Thr-Gln-(2-$CF_3$-Phe)-Cys]-OH,

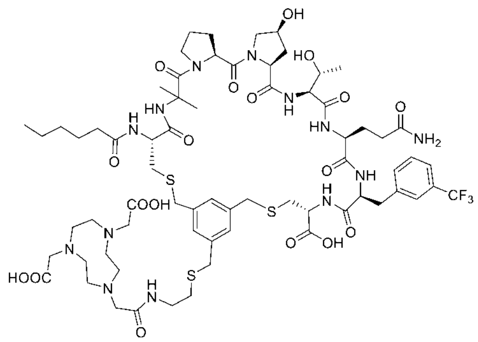

Hex-[Cys-(tMeBn(NOTA-AET))-Aib-Pro-((4S)-OH-Pro)-Thr-Gln-(3-$CF_3$-Phe)-Cys]-OH,

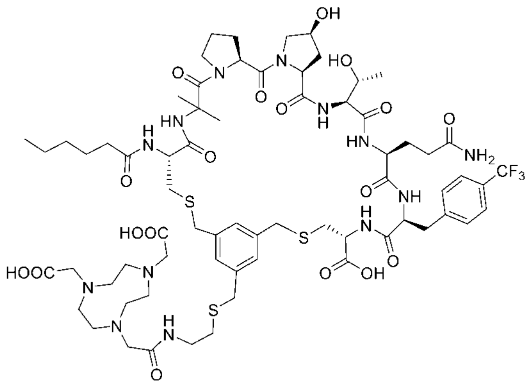

Hex-[Cys-(tMeBn(NOTA-AET))-Aib-Pro-((4S)-OH-Pro)-Thr-Gln-(4-$CF_3$-Phe)-Cys]-OH,

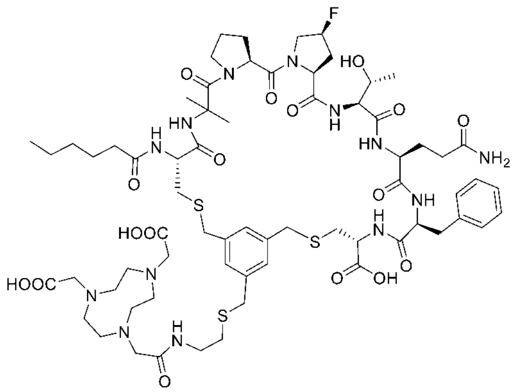

Hex-[Cys-(tMeBn(NOTA-AET))-Aib-Pro-((4S)-F-Pro)-Thr-Gln-Phe-Cys]-OH,

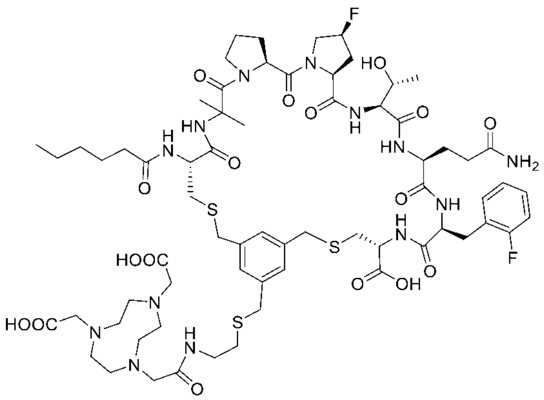

Hex-[Cys-(tMeBn(NOTA-AET))-Aib-Pro-((4S)-F-Pro)-Thr-Gln-(2-F-Phe)-Cys]-OH,

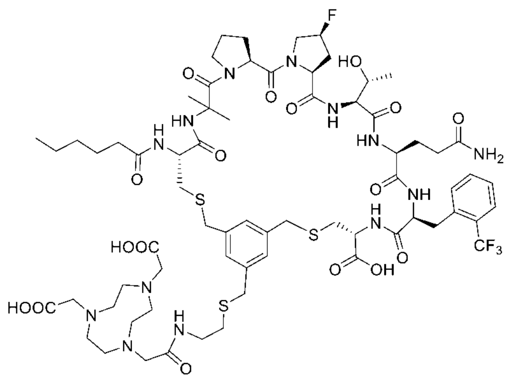

Hex-[Cys-(tMeBn(NOTA-AET))-Aib-Pro-((4S)-F-Pro)-Thr-Gln-(2-$CF_3$-Phe)-Cys]-OH.

In the compound or the pharmaceutically acceptable salt thereof provided by the present disclosure, the chelating agent complexes a nuclide, the nuclide is a diagnostically active nuclide, and the diagnostically active nuclide is selected from $^{43}$Sc, $^{44}$Sc, $^{51}$Mn, $^{52}$Mn, $^{64}$Cu, $^{67}$Ga, $^{68}$Ga, $^{86}$Y, $^{89}$Zr, $^{94m}$Tc, $^{99m}$Tc, $^{111}$In, $^{152}$Tb, $^{155}$Tb, $^{201}$Tl, $^{203}$Pb, $^{18}$F, $^{76}$Br, $^{77}$Br, $^{123}$I, $^{124}$I and $^{125}$I.

In some embodiments, the diagnostically active nuclide is selected from $^{43}$Sc, $^{44}$Sc, $^{64}$Cu, $^{67}$Ga, $^{68}$Ga, $^{86}$Y, $^{89}$Zr, $^{99m}$Tc, $^{111}$In, $^{152}$Tb, $^{155}$Tb, $^{203}$Pb, $^{18}$F, $^{76}$Br, $^{77}$Br, $^{123}$I, $^{124}$I and $^{125}$I.

In some embodiments, the diagnostically active nuclide is selected from $^{64}$Cu, $^{68}$Ga, $^{89}$Zr, $^{99m}$Tc, $^{111}$In, $^{18}$F, $^{123}$I and $^{124}$I.

In the compounds or the pharmaceutically acceptable salts thereof provided by the present disclosure, the chelating agent complexes a nuclide, and the nuclide is a therapeutically active nuclide selected from $^{47}$Sc, $^{67}$Cu, $^{89}$Sr, $^{90}$Y, $^{153}$Sm, $^{149}$Tb, $^{161}$Tb, $^{177}$Lu, $^{186}$Re, $^{188}$Re, $^{212}$Pb, $^{213}$Bi, $^{223}$Ra, $^{225}$Ac, $^{226}$Th, $^{227}$Th, $^{131}$I and $^{211}$A.

In an alternative embodiment, the therapeutically active radionuclide is selected from $^{47}$Sc, $^{67}$Cu, $^{90}$Y, $^{177}$Lu, $^{188}$Re, $^{212}$Pb, $^{213}$Bi, $^{225}$Ac, $^{227}$Th, $^{131}$I and $^{211}$At.

In an alternative embodiment, the therapeutically active radionuclide is selected from $^{90}$Y, $^{177}$Lu, $^{225}$Ac, $^{227}$Th, $^{131}$I and $^{211}$At.

The present disclosure provides compounds as shown below or pharmaceutically acceptable salts thereof:

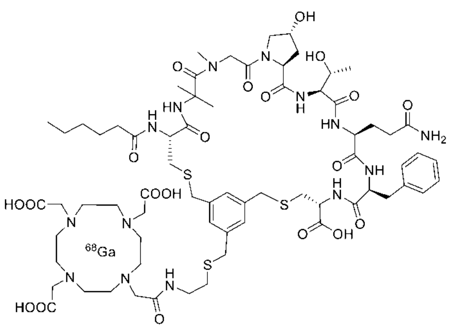
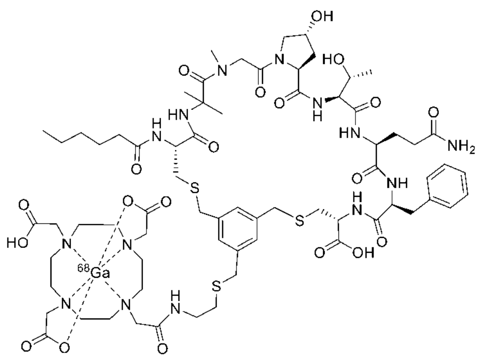

Hex-[Cys-(tMeBn($^{68}$Ga-DOTA-AET))-Aib-Sar-((4R)-OH-Pro)-Thr-Gln-Phe-Cys]-OH,

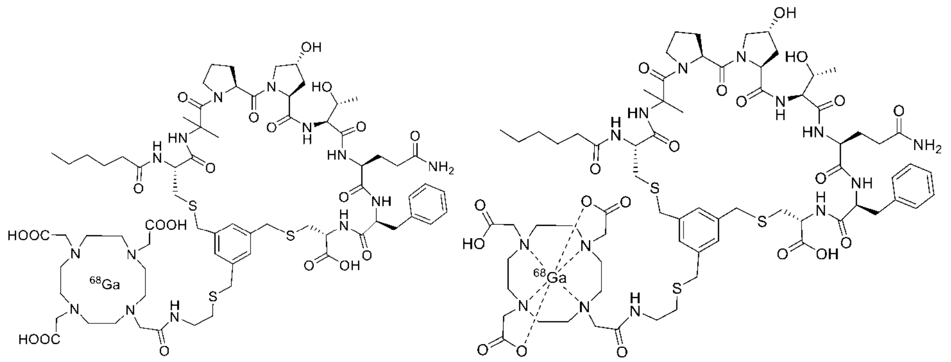

Hex-[Cys-(tMeBn($^{68}$Ga-DOTA-AET))-Aib-Pro-((4R)-OH-Pro)-Thr-Gln-Phe-Cys]-OH,

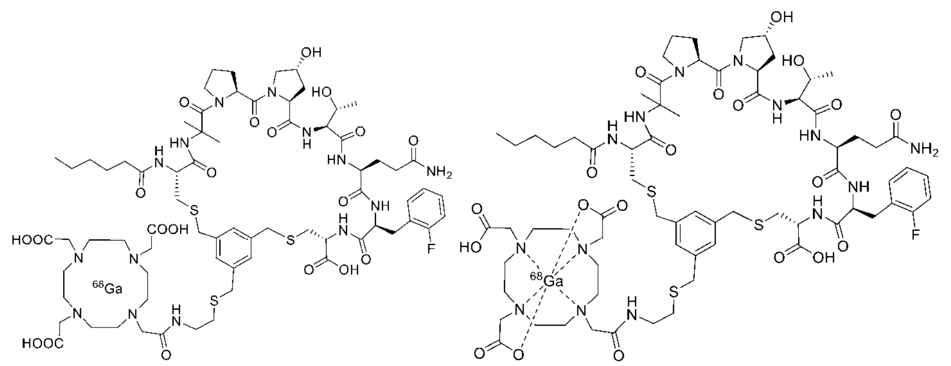

Hex-[Cys-(tMeBn($^{68}$Ga-DOTA-AET))-Aib-Pro-((4R)-OH-Pro)-Thr-Gln-(2-F-Phe)-Cys]-OH,

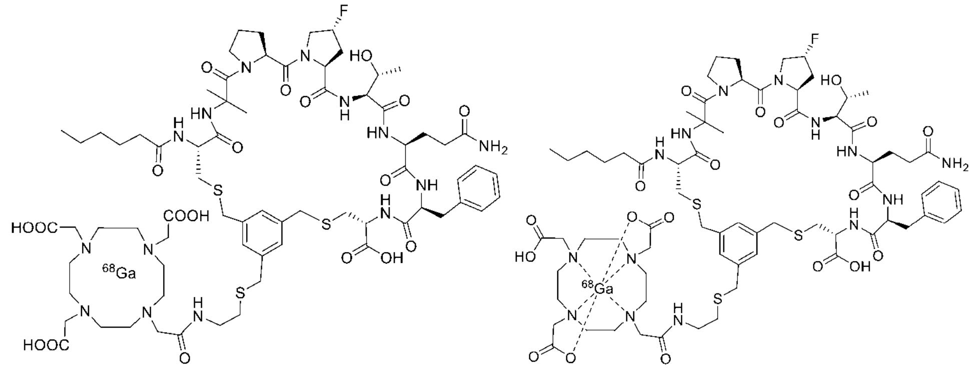

Hex-[Cys-(tMeBn($^{68}$Ga-DOTA-AET))-Aib-Pro-((4R)-F-Pro)-Thr-Gln-Phe-Cys]-OH,

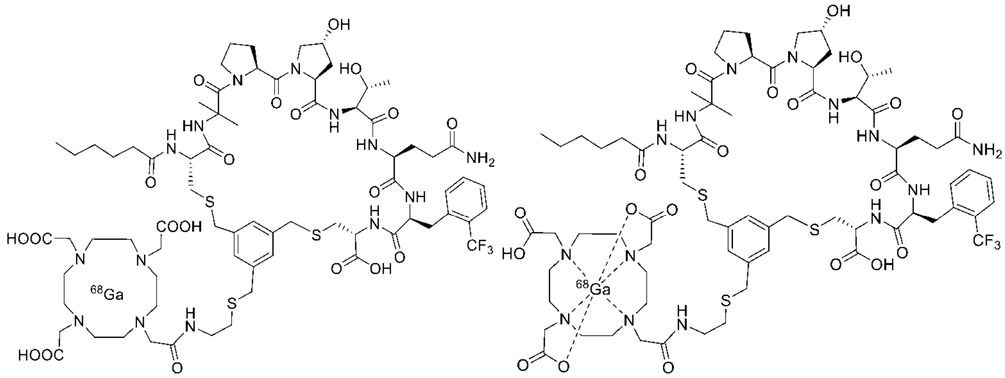

Hex-[Cys-(tMeBn($^{68}$Ga-DOTA-AET))-Aib-Pro-((4R)-OH-Pro)-Thr-Gln-(2-$CF_3$-Phe)-Cys]-OH,

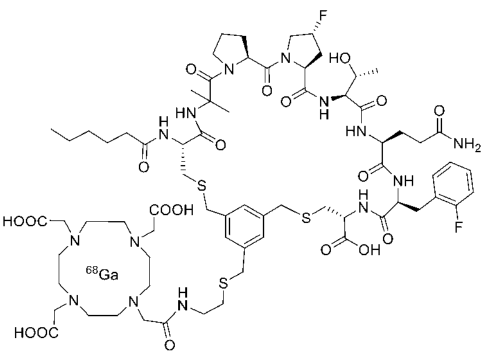
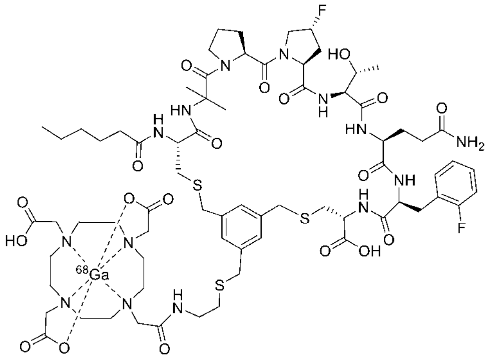

Hex-[Cys-(tMeBn($^{68}$Ga-DOTA-AET))-Aib-Pro-((4R)-F-Pro)-Thr-Gln-(2-F-Phe)-Cys]-OH,

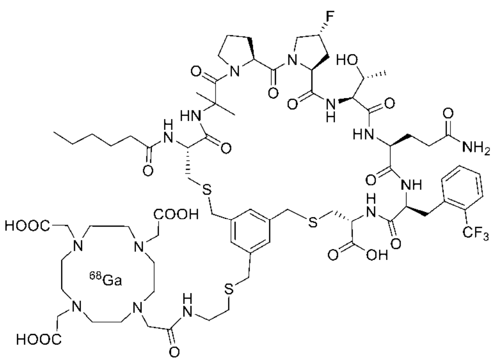
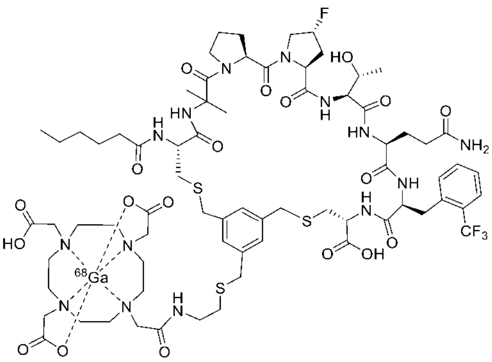

Hex-[Cys-(tMeBn($^{68}$Ga-DOTA-AET))-Aib-Pro-((4R)-F-Pro)-Thr-Gln-(2-F-Phe)-Cys]-OH,

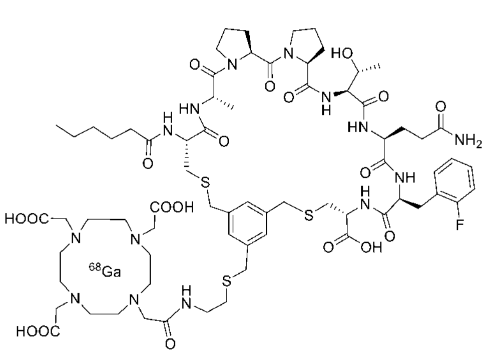
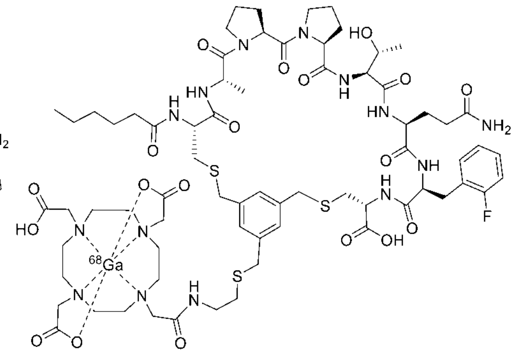

Hex-[Cys-(tMeBn($^{68}$Ga-DOTA-AET))-Ala-Pro-Pro-Thr-Gln-(2-F-Phe)-Cys]-OH,

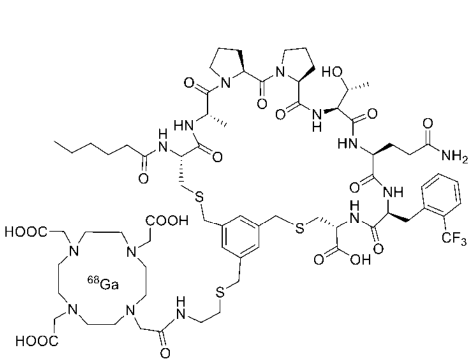
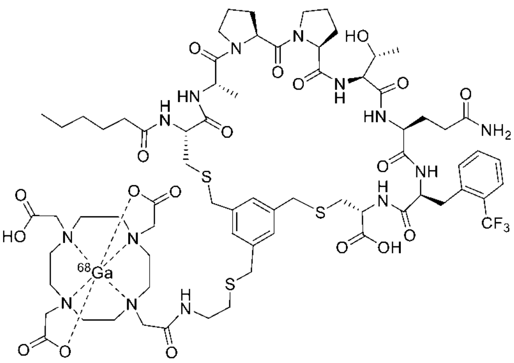

Hex-[Cys-(tMeBn($^{68}$Ga-DOTA-AET))-Ala-Pro-Pro-Thr-Gln-(2-$CF_3$-Phe)-Cys]-OH,

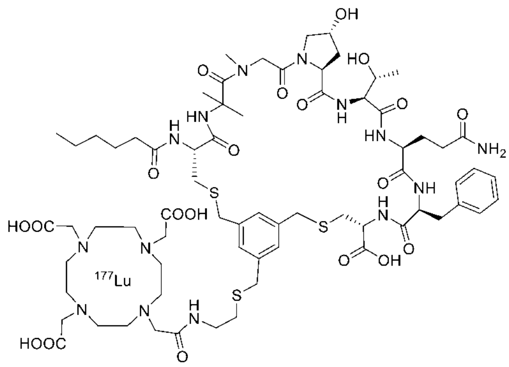

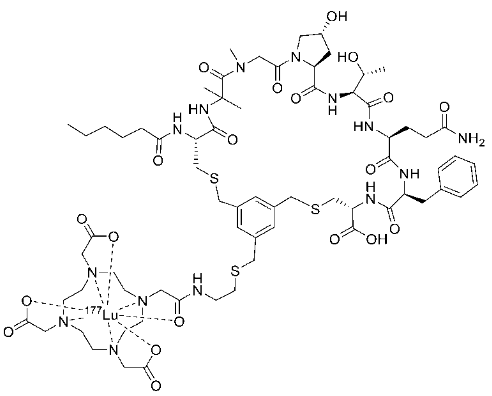

Hex-[Cys-(tMeBn($^{177}$Lu-DOTA-AET))-Aib-Sar-((4R)-OH-Pro)-Thr-Gln-Phe-Cys]-OH,

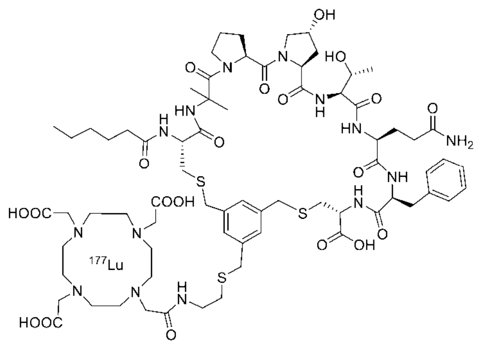

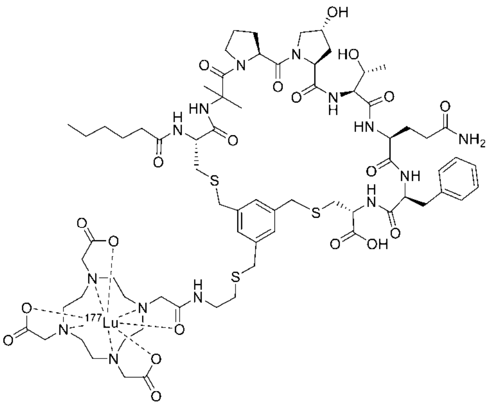

Hex-[Cys-(tMeBn($^{177}$Lu-DOTA-AET))-Aib-Pro-((4R)-OH-Pro)-Thr-Gln-Phe-Cys]-OH,

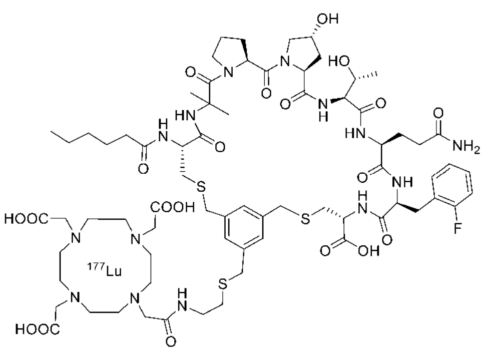

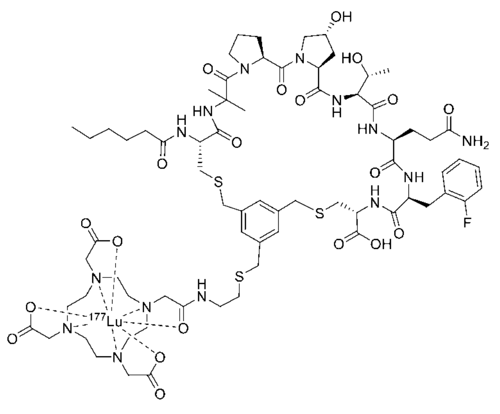

Hex-[Cys-(tMeBn($^{177}$Lu-DOTA-AET))-Aib-Pro-((4R)-OH-Pro)-Thr-Gln-(2-F-Phe)-Cys]-OH,

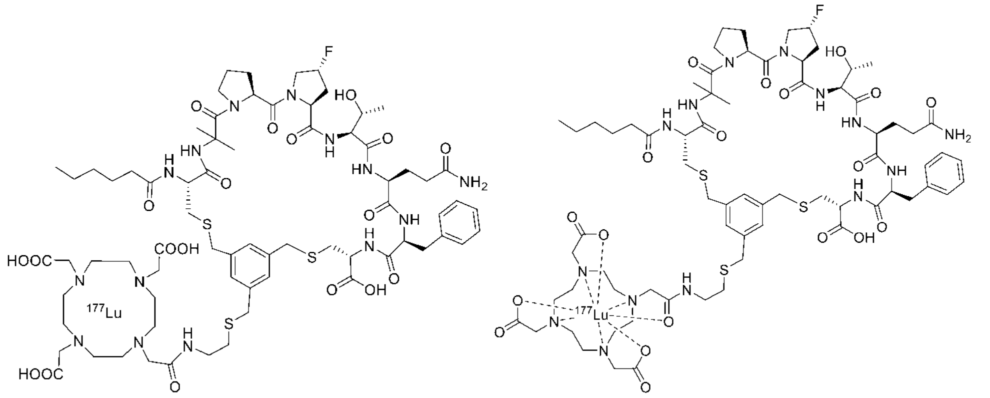

Hex-[Cys-(tMeBn($^{177}$Lu-DOTA-AET))-Aib-Pro-((4R)-F-Pro)-Thr-Gln-Phe-Cys]-OH,

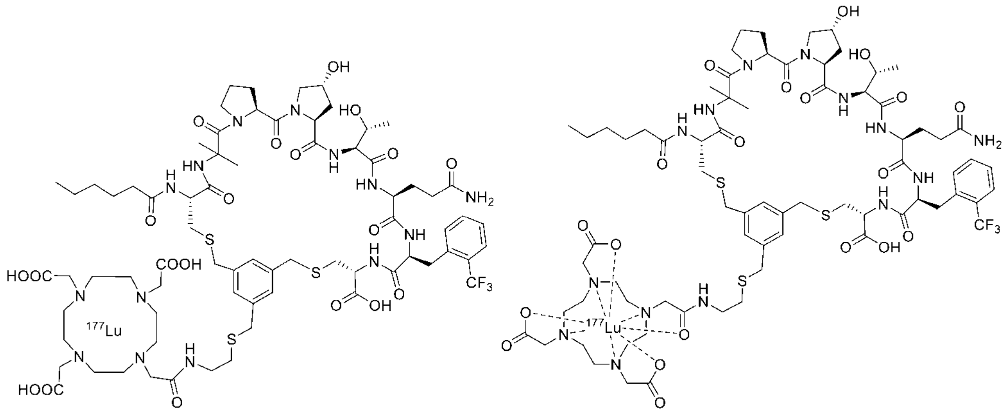

Hex-[Cys-(tMeBn($^{177}$Lu-DOTA-AET))-Aib-Pro-((4R)-OH-Pro)-Thr-Gln-(2-$CF_3$-Phe)-Cys]-OH,

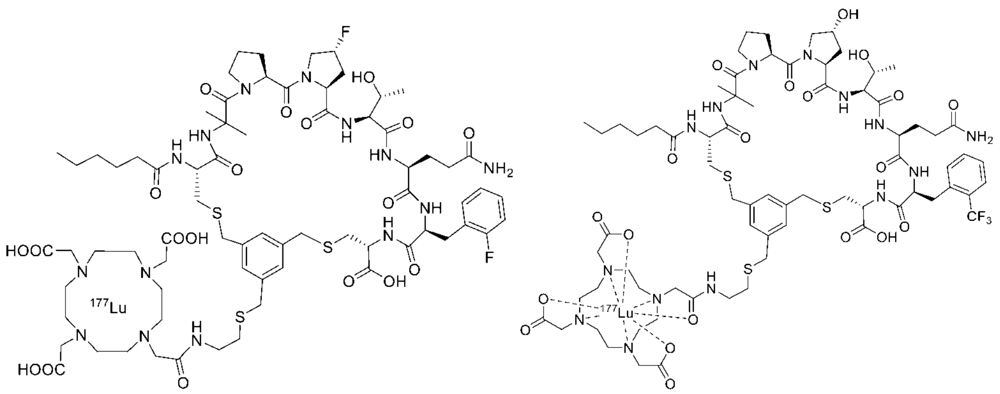

Hex-[Cys-(tMeBn($^{177}$Lu-DOTA-AET))-Aib-Pro-((4R)-F-Pro)-Thr-Gln-(2-F-Phe)-Cys]-OH,

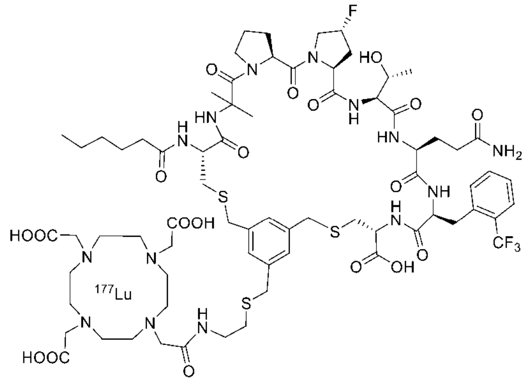

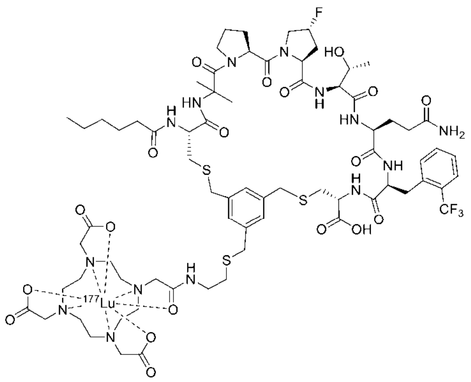

Hex-[Cys-(tMeBn($^{177}$Lu-DOTA-AET))-Aib-Pro-((4R)-F-Pro)-Thr-Gln-(2-$CF_3$-Phe)-Cys]-OH,

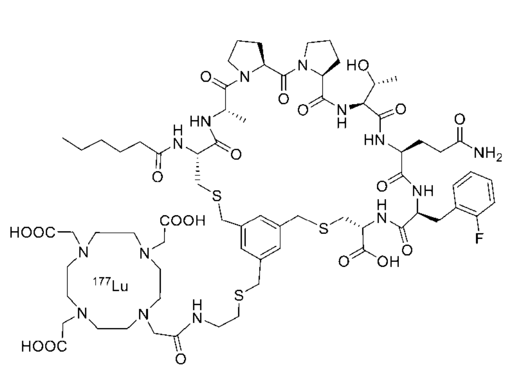

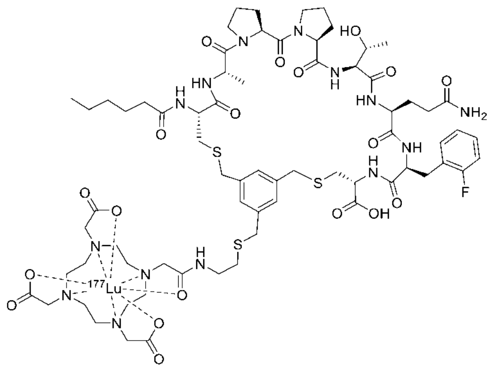

Hex-[Cys-(tMeBn($^{177}$Lu-DOTA-AET))-Ala-Pro-Pro-Thr-Gln-(2-F-Phe)-Cys]-OH,

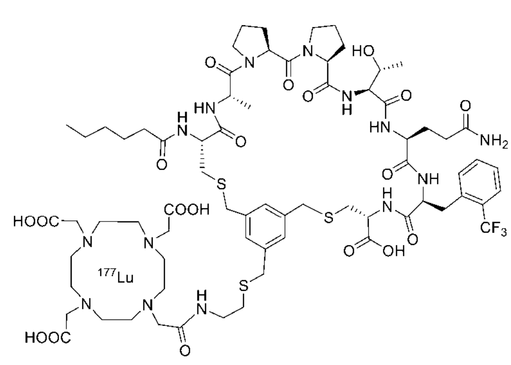

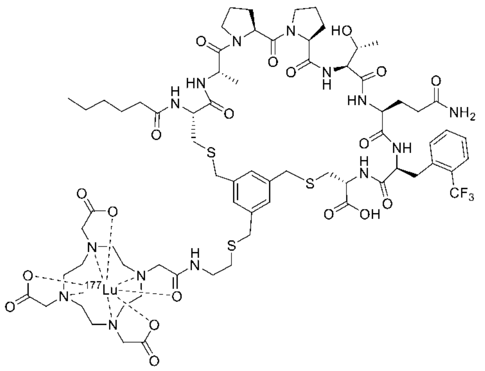

Hex-[Cys-(tMeBn($^{177}$Lu-DOTA-AET))-Ala-Pro-Pro-Thr-Gln-(2-$CF_3$-Phe)-Cys]-OH,

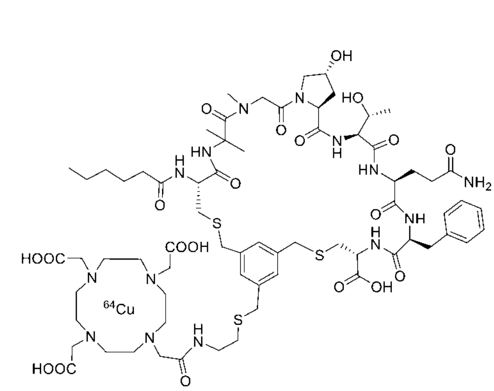

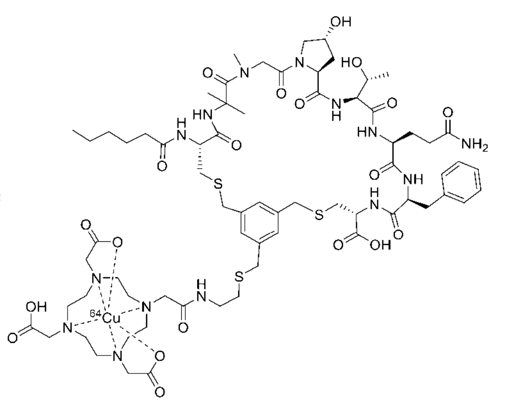

Hex-[Cys-(tMeBn($^{64}$Cu-DOTA-AET))-Aib-Sar-((4R)-OH-Pro)-Thr-Gln-Phe-Cys]-OH,

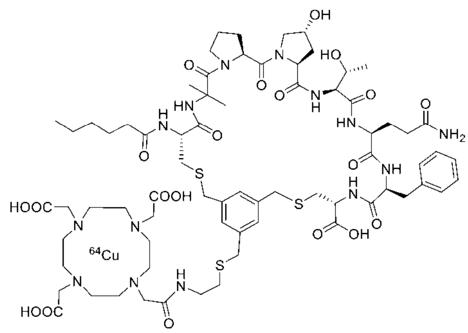

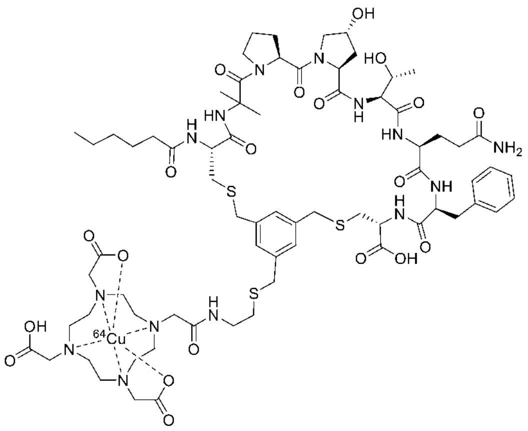

Hex-[Cys-(tMeBn($^{64}$Cu-DOTA-AET))-Aib-Pro-((4R)-OH-Pro)-Thr-Gln-Phe-Cys]-OH,

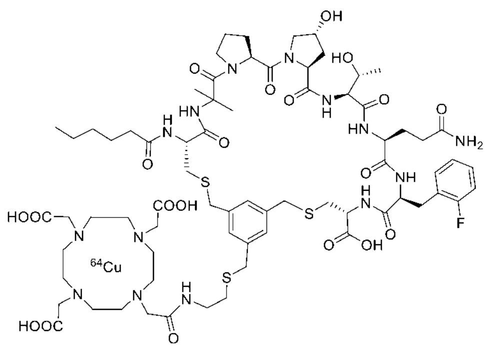

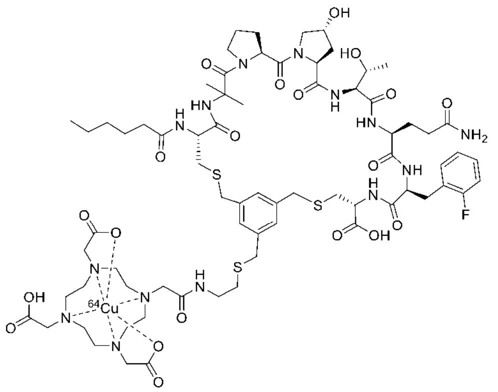

Hex-[Cys-(tMeBn($^{64}$Cu-DOTA-AET))-Aib-Pro-((4R)-OH-Pro)-Thr-Gln-(2-F-Phe)-Cys]-OH,

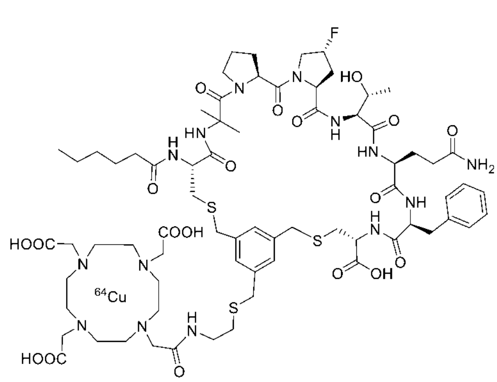

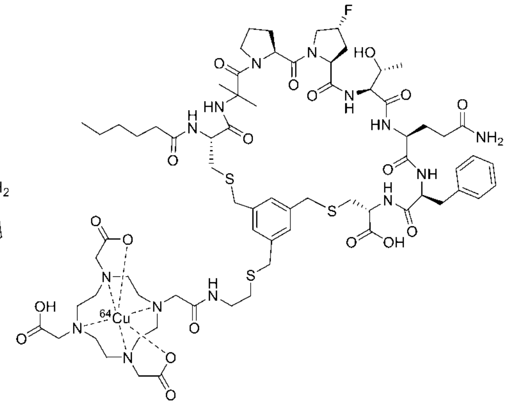

Hex-[Cys-(tMeBn($^{64}$Cu-DOTA-AET))-Aib-Pro-((4R)-F-Pro)-Thr-Gln-Phe-Cys]-OH,

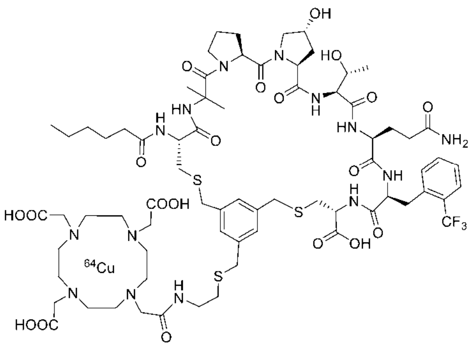
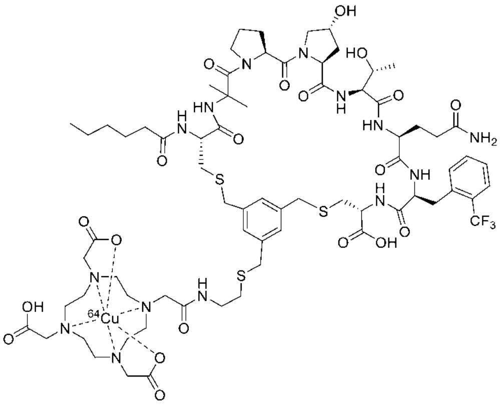

Hex-[Cys-(tMeBn($^{64}$Cu-DOTA-AET))-Aib-Pro-((4R)-OH-Pro)-Thr-Gln-(2-$CF_3$-Phe)-Cys]-OH,

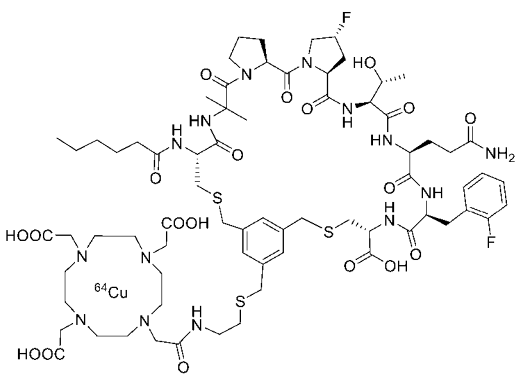
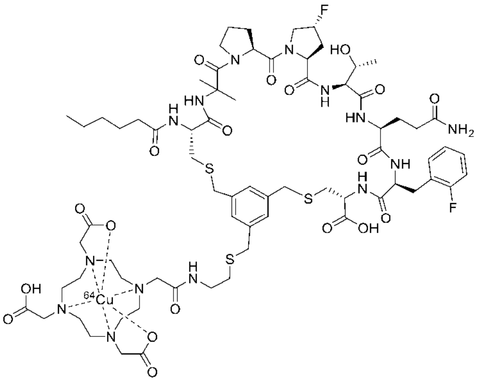

Hex-[Cys-(tMeBn($^{64}$Cu-DOTA-AET))-Aib-Pro-((4R)-F-Pro)-Thr-Gln-(2-F-Phe)-Cys]-OH,

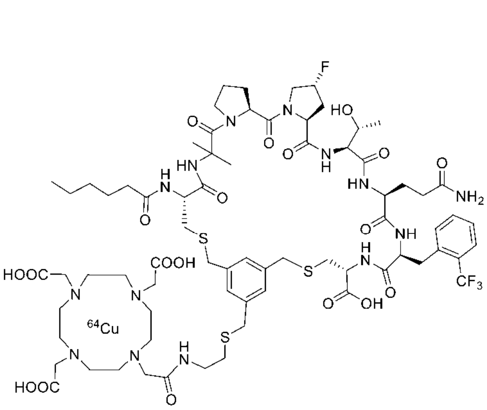
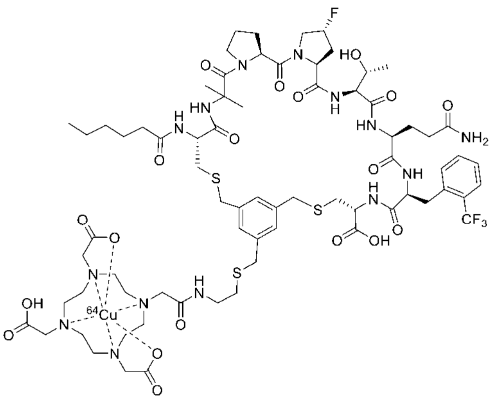

Hex-[Cys-(tMeBn($^{64}$Cu-DOTA-AET))-Aib-Pro-((4R)-F-Pro)-Thr-Gln-(2-$CF_3$-Phe)-Cys]-OH,

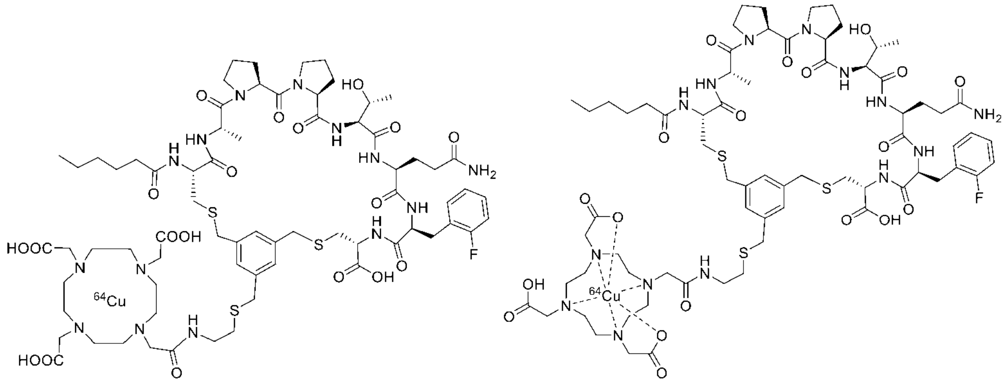

Hex-[Cys-(tMeBn($^{64}$Cu-DOTA-AET))-Ala-Pro-Pro-Thr-Gln-(2-F-Phe)-Cys]-OH,

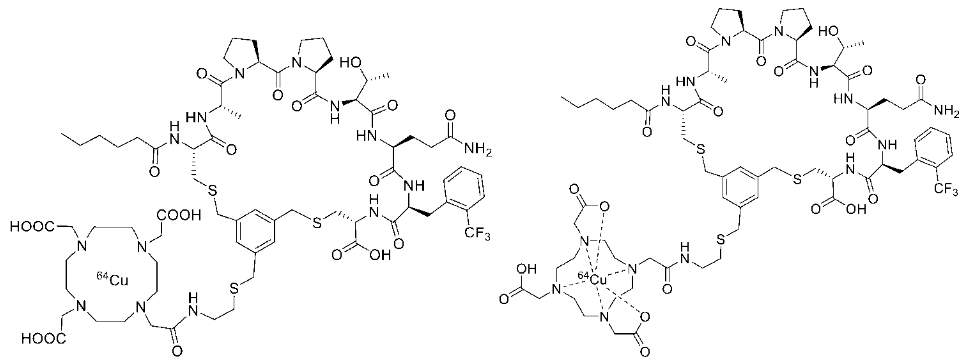

Hex-[Cys-(tMeBn($^{64}$Cu-DOTA-AET))-Ala-Pro-Pro-Thr-Gln-(2-$CF_3$-Phe)-Cys]-OH,

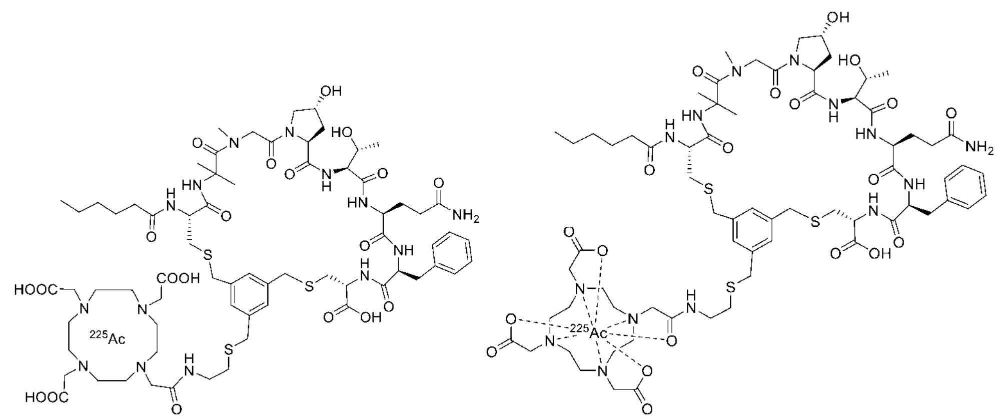

Hex-[Cys-(tMeBn($^{64}$Cu-DOTA-AET))-Aib-Sar-((4R)-OH-Pro)-Thr-Gln-Phe-Cys]-OH,

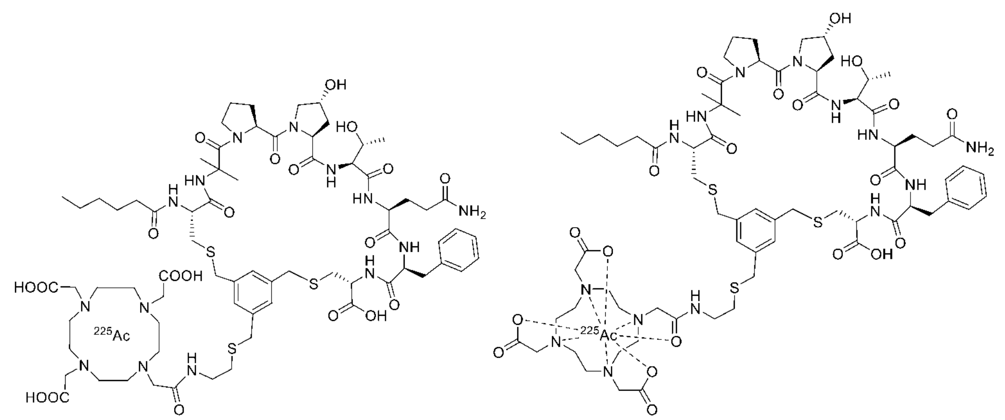

Hex-[Cys-(tMeBn($^{64}$Cu-DOTA-AET))-Aib-Pro-((4R)-OH-Pro)-Thr-Gln-Phe-Cys]-OH,

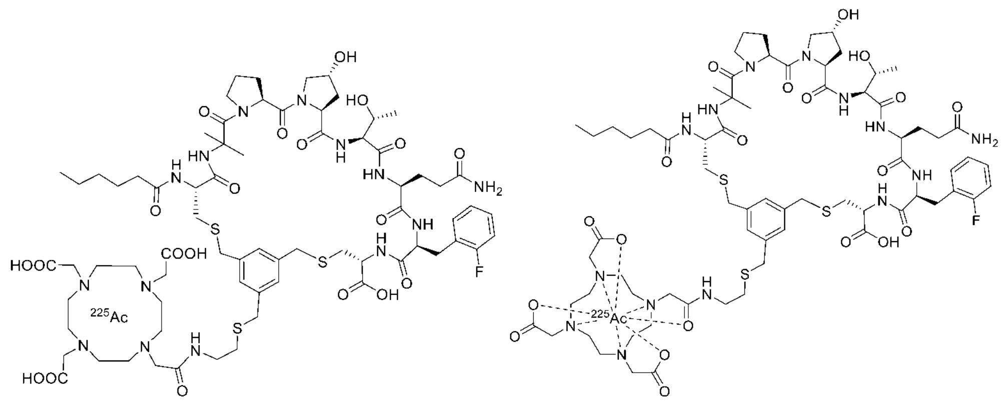

Hex-[Cys-(tMeBn($^{64}$Cu-DOTA-AET))-Aib-Pro-((4R)-OH-Pro)-Thr-Gln-(2-F-Phe)-Cys]-OH,

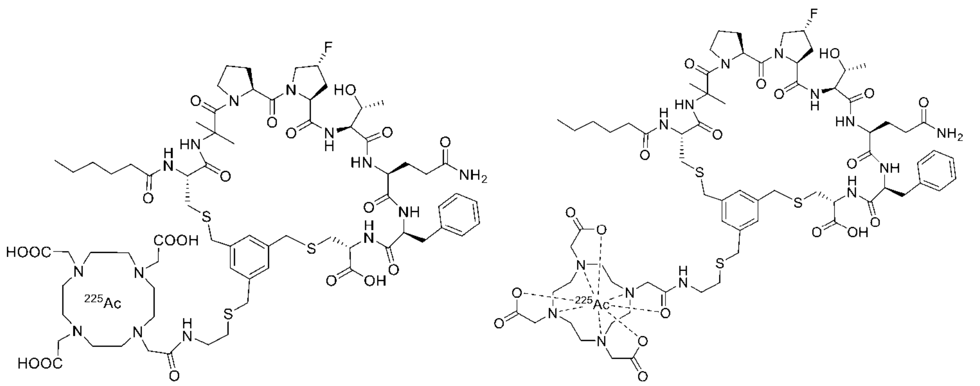

Hex-[Cys-(tMeBn($^{64}$Cu-DOTA-AET))-Aib-Pro-((4R)-F-Pro)-Thr-Gln-Phe-Cys]-OH,

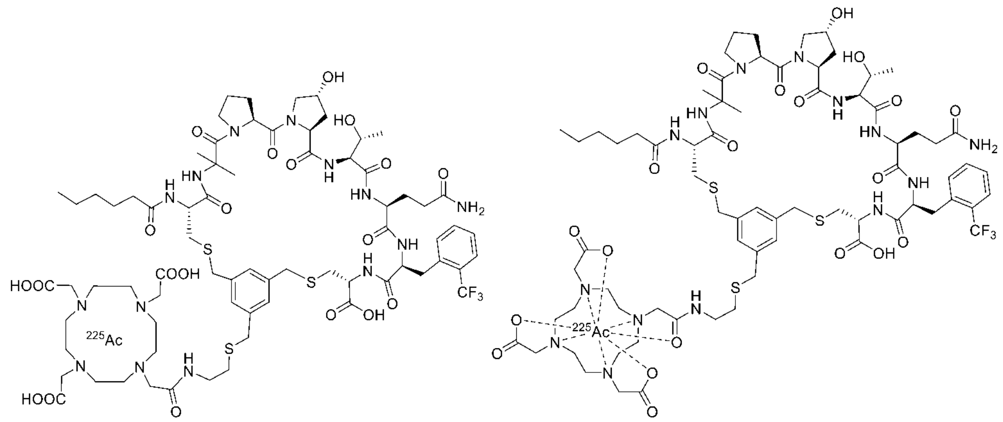

Hex-[Cys-(tMeBn($^{64}$Cu-DOTA-AET))-Aib-Pro-((4R)-OH-Pro)-Thr-Gln-(2-$CF_3$-Phe)-Cys]-OH,

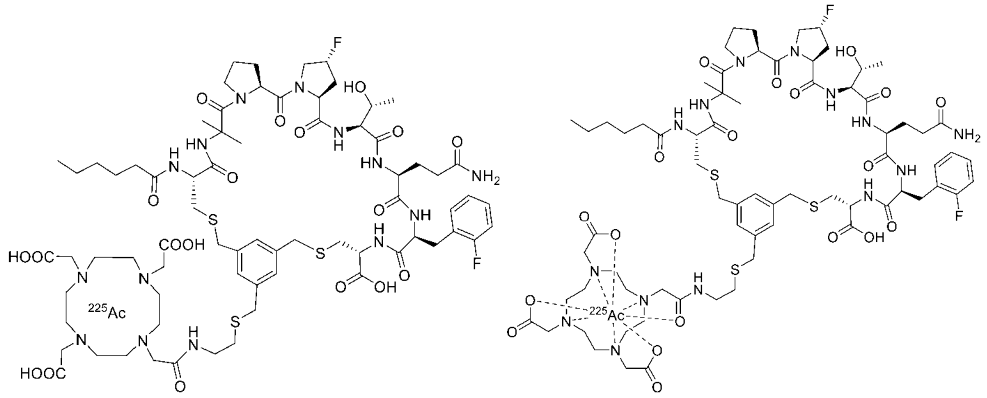

Hex-[Cys-(tMeBn($^{64}$Cu-DOTA-AET))-Aib-Pro-((4R)-F-Pro)-Thr-Gln-(2-F-Phe)-Cys]-OH,

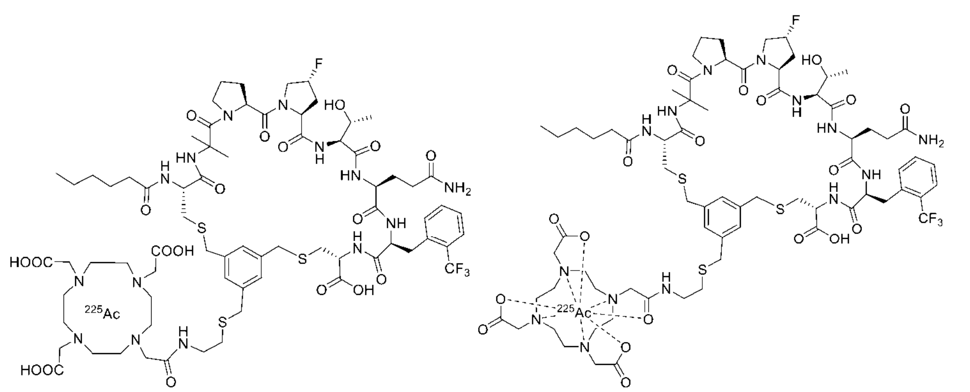

Hex-[Cys-(tMeBn($^{64}$Cu-DOTA-AET))-Aib-Pro-((4R)-F-Pro)-Thr-Gln-(2-$CF_3$-Phe)-Cys]-OH,

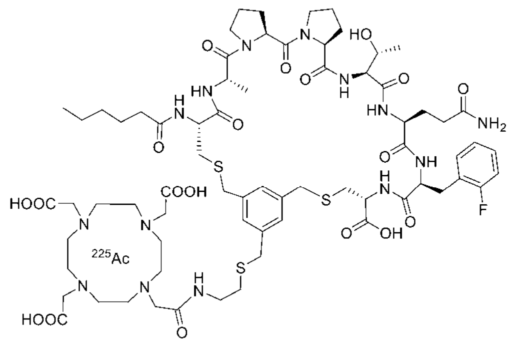

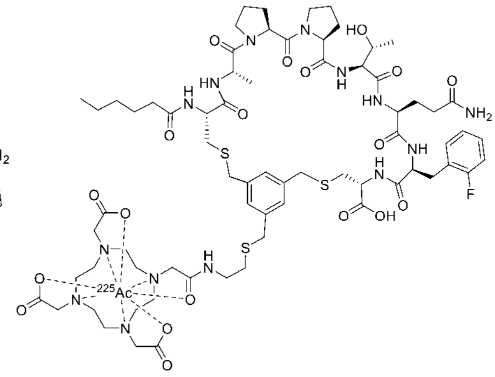

Hex-[Cys-(tMeBn($^{64}$Cu-DOTA-AET))-Ala-Pro-Pro-Thr-Gln-(2-F-Phe)-Cys]-OH,

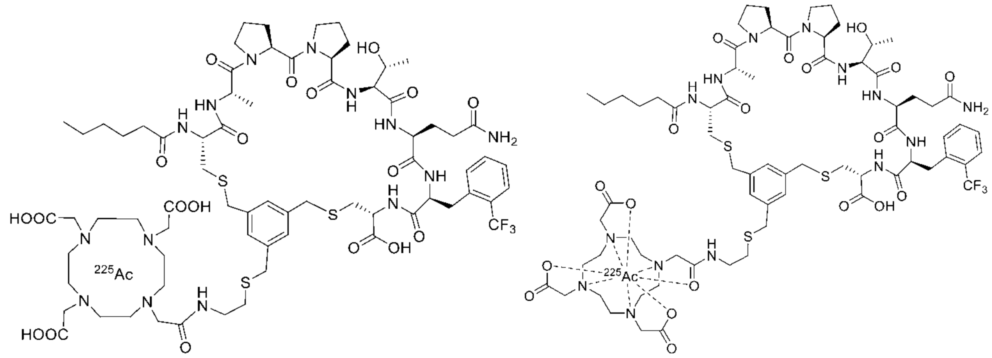

Hex-[Cys-(tMeBn($^{64}$Cu-DOTA-AET))-Ala-Pro-Pro-Thr-Gln-(2-$CF_3$-Phe)-Cys]-OH,

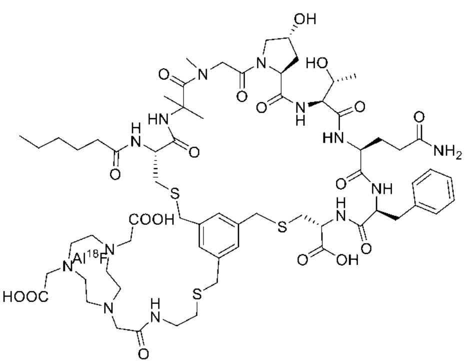

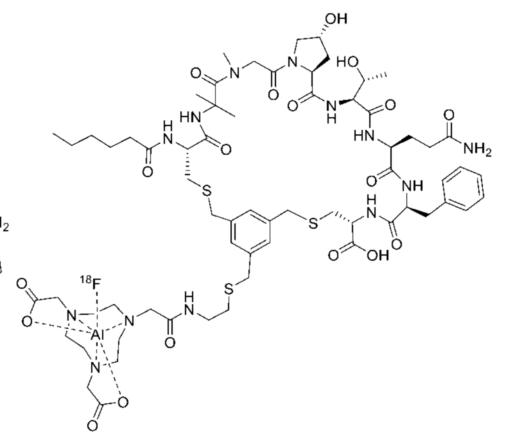

Hex-[Cys-(tMeBn(Al$^{18}$F-NOTA-AET))-Aib-Sar-((4R)-OH-Pro)-Thr-Gln-Phe-Cys]-OH,

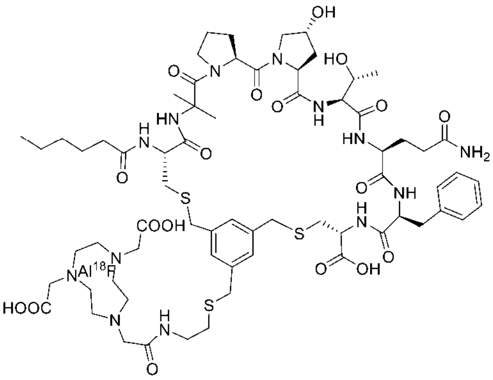

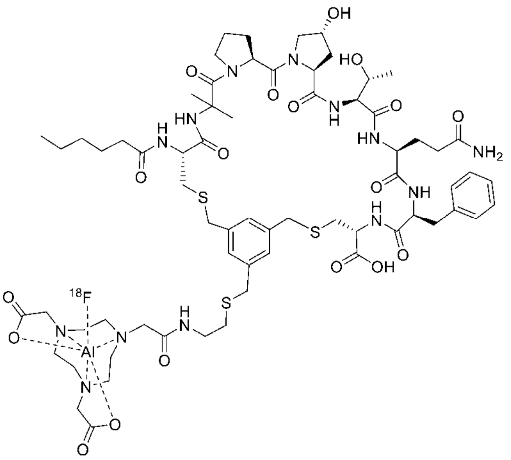

Hex-[Cys-(tMeBn(Al$^{18}$F-NOTA-AET))-Aib-Pro-((4R)-OH-Pro)-Thr-Gln-Phe-Cys]-OH,

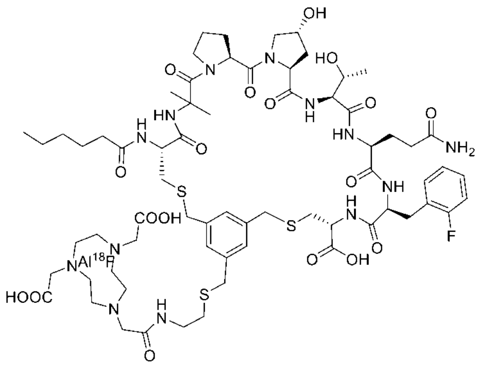

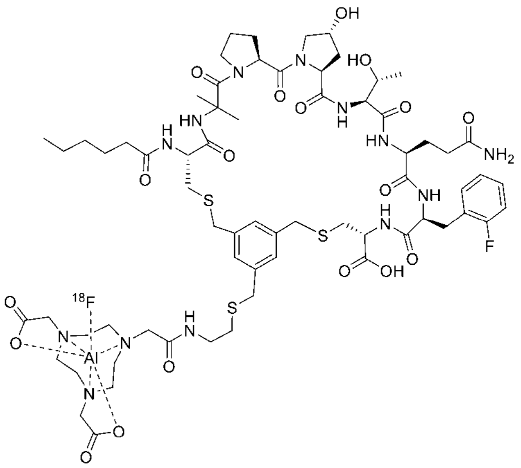

Hex-[Cys-(tMeBn(Al$^{18}$F-NOTA-AET))-Aib-Pro-((4R)-OH-Pro)-Thr-Gln-(2-F-Phe)-Cys]-OH,

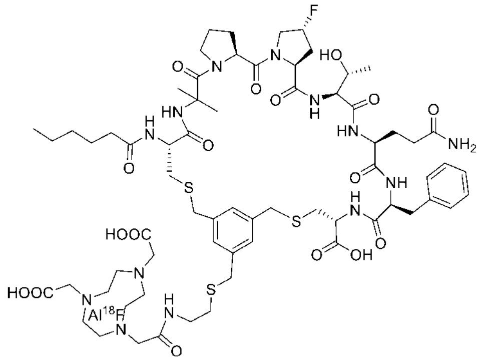

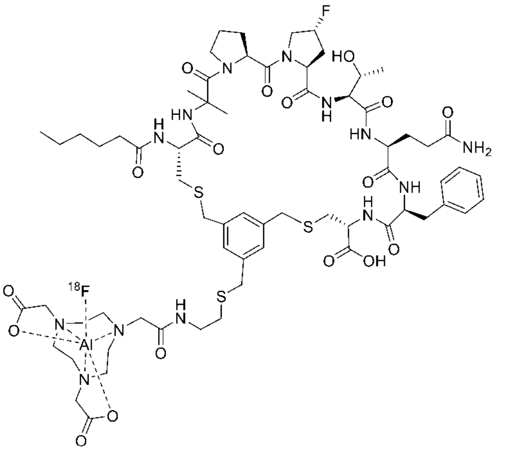

Hex-[Cys-(tMeBn(Al$^{18}$F-NOTA-AET))-Aib-Pro-((4R)-F-Pro)-Thr-Gln-Phe-Cys]-OH,

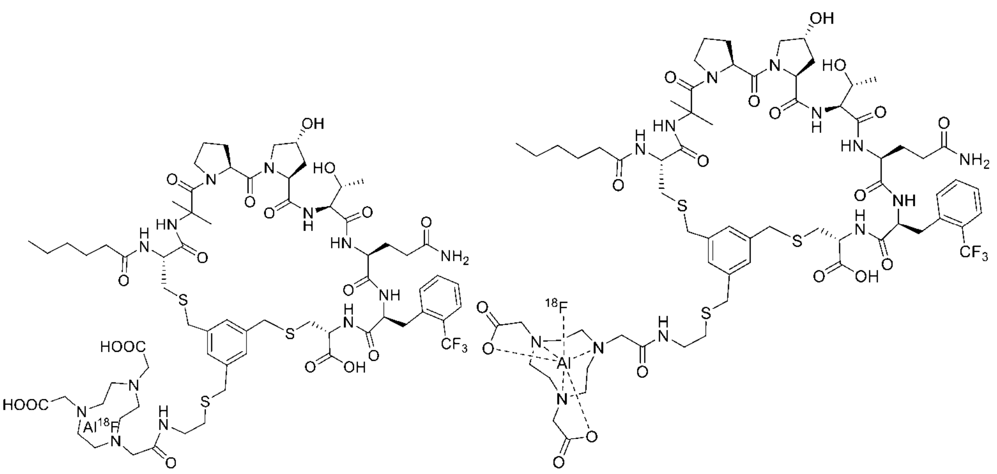

Hex-[Cys-(tMeBn(Al18F-NOTA-AET))-Aib-Pro-((4R)-OH-Pro)-Thr-Gln-(2-$CF_3$-Phe) -Cys]-OH,

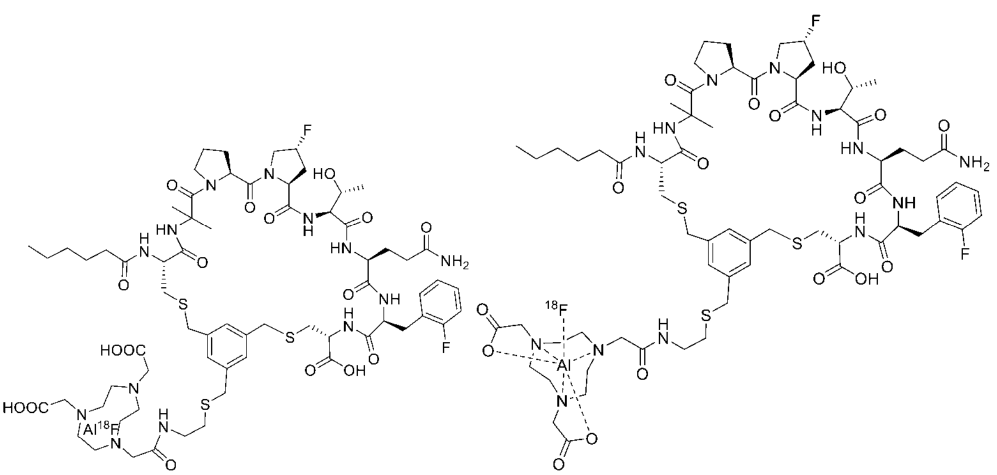

Hex-[Cys-(tMeBn(Al18F-NOTA-AET))-Aib-Pro-((4R)-F-Pro)-Thr-Gln-(2-F-Phe)-Cys]-OH,

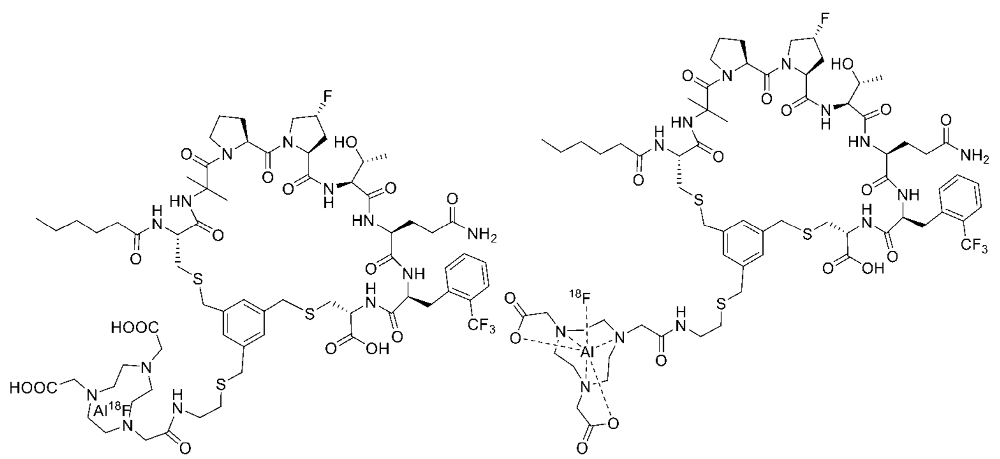

Hex-[Cys-(tMeBn(Al18F-NOTA-AET))-Aib-Pro-((4R)-F-Pro)-Thr-Gln-(2-$CF_3$-Phe)-Cys]-OH,

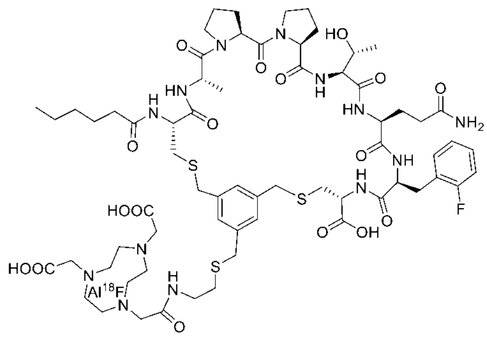
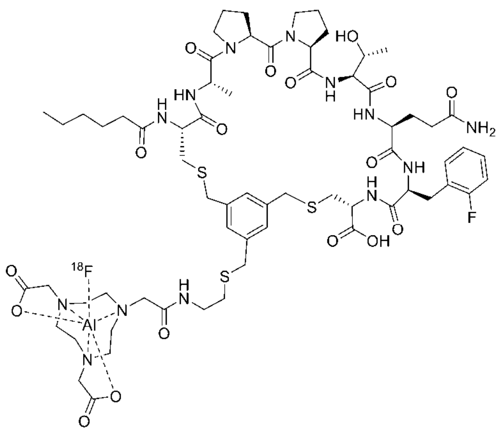

Hex-[Cys-(tMeBn(Al$^{18}$F-NOTA-AET))-Ala-Pro-Pro-Thr-Gln-(2-F-Phe)-Cys]-OH,

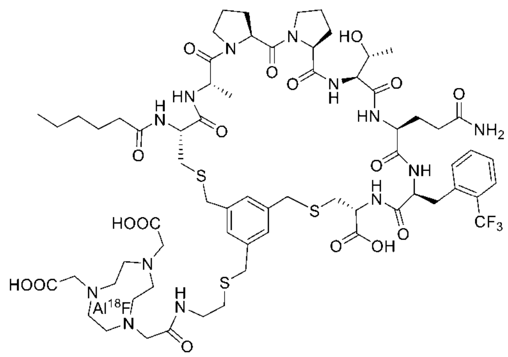
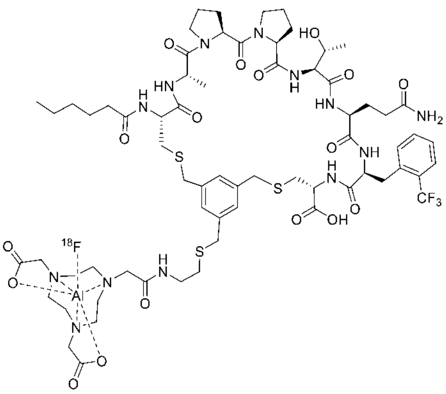

Hex-[Cys-(tMeBn(Al$^{18}$F-NOTA-AET))-Ala-Pro-Pro-Thr-Gln-(2-$CF_3$-Phe)-Cys]-OH,

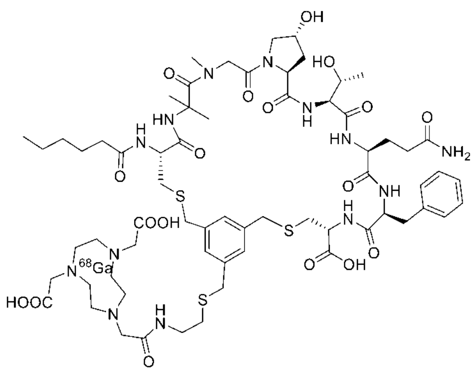
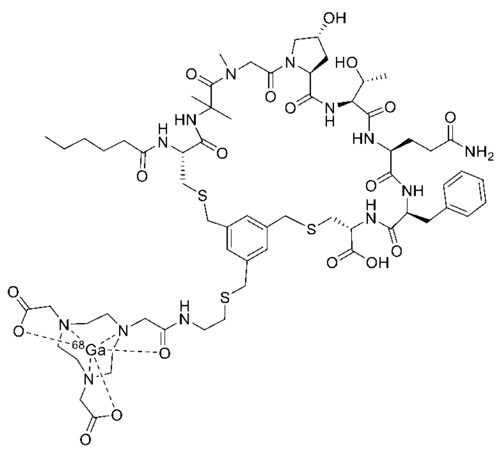

Hex-[Cys-(tMeBn($^{68}$Ga-NOTA-AET))-Aib-Sar-((4R)-OH-Pro)-Thr-Gln-Phe-Cys]-OH,

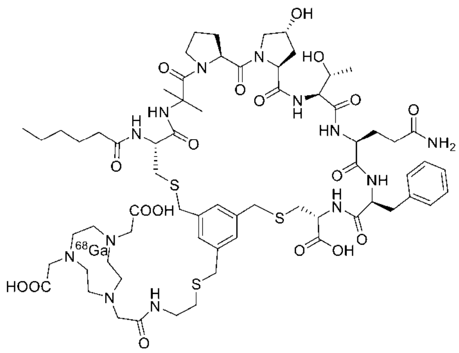

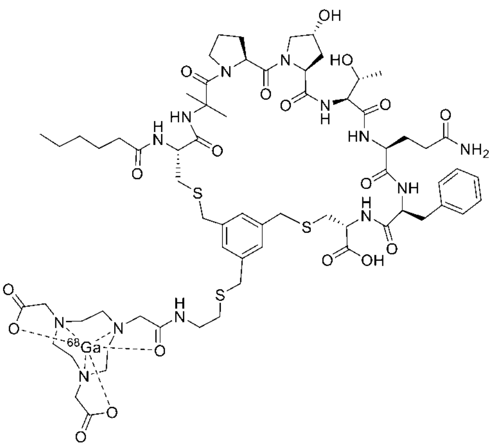

Hex-[Cys-(tMeBn($^{68}$Ga-NOTA-AET))-Aib-Pro-((4R)-OH-Pro)-Thr-Gln-Phe-Cys]-OH,

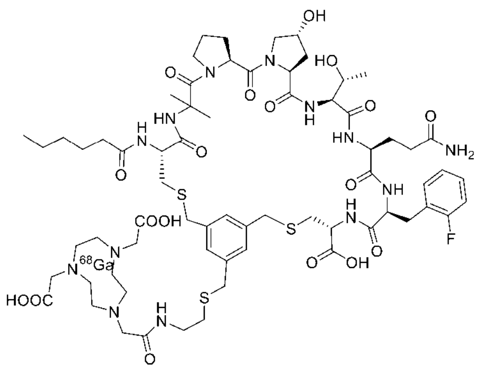

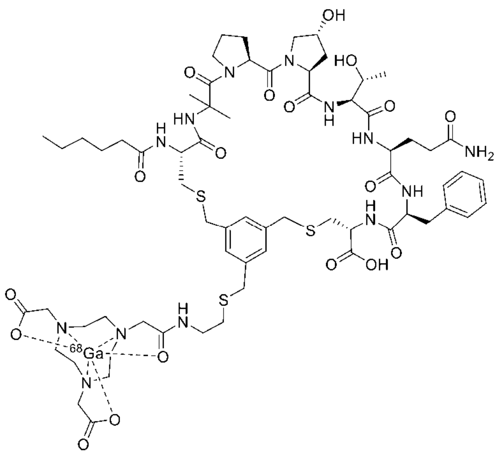

Hex-[Cys-(tMeBn($^{68}$Ga-NOTA-AET))-Aib-Pro-((4R)-OH-Pro)-Thr-Gln-(2-F-Phe)-Cys]-OH,

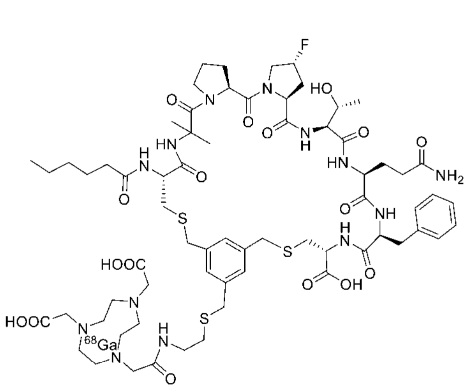

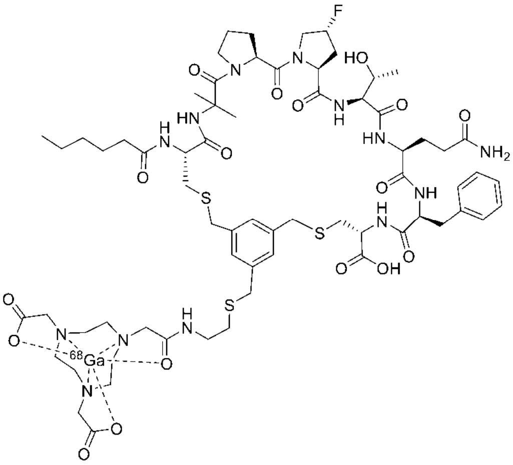

Hex-[Cys-(tMeBn($^{68}$Ga-NOTA-AET))-Aib-Pro-((4R)-F-Pro)-Thr-Gln-Phe-Cys]-OH,

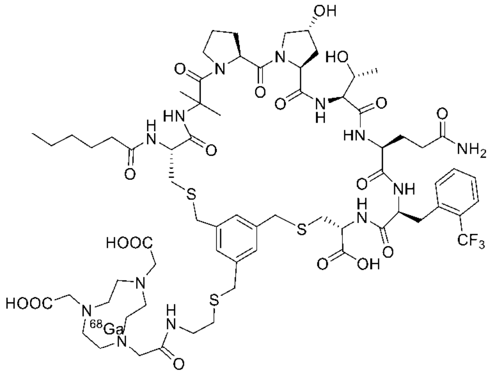

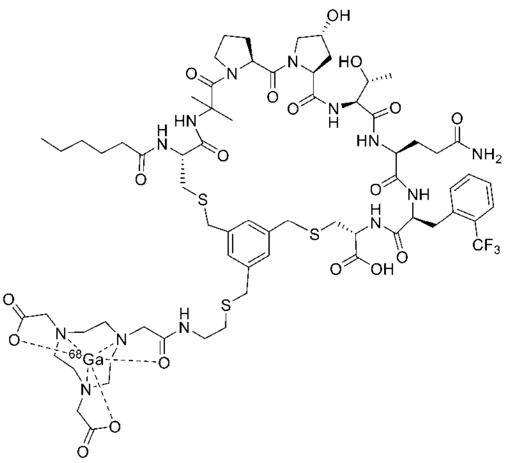

Hex-[Cys-(tMeBn($^{68}$Ga-NOTA-AET))-Aib-Pro-((4R)-OH-Pro)-Thr-Gln-(2-$CF_3$-Phe)-Cys]-OH,

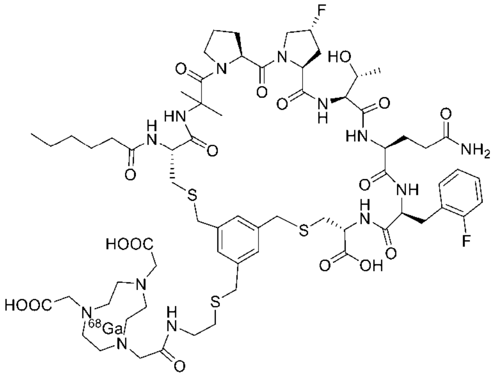

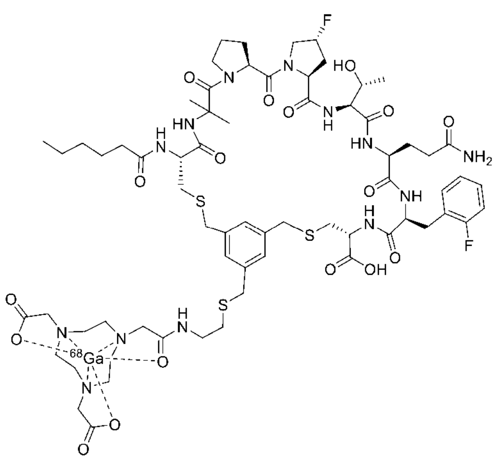

Hex-[Cys-(tMeBn($^{68}$Ga-NOTA-AET))-Aib-Pro-((4R)-F-Pro)-Thr-Gln-(2-F-Phe)-Cys]-OH,

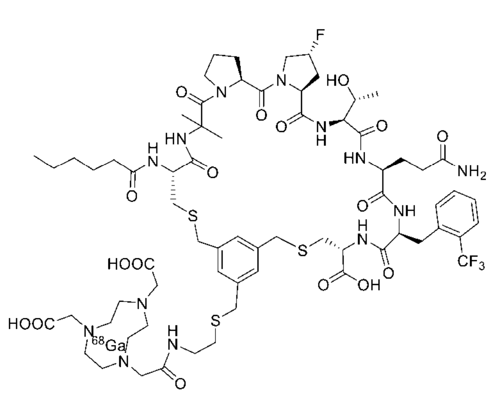

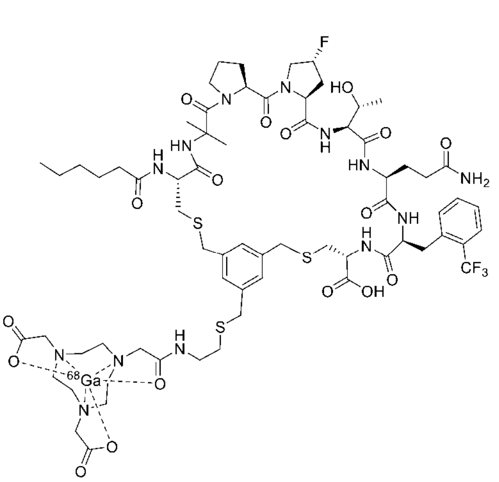

Hex-[Cys-(tMeBn($^{68}$Ga-NOTA-AET))-Aib-Pro-((4R)-F-Pro)-Thr-Gln-(2-$CF_3$-Phe)-Cys]-OH,

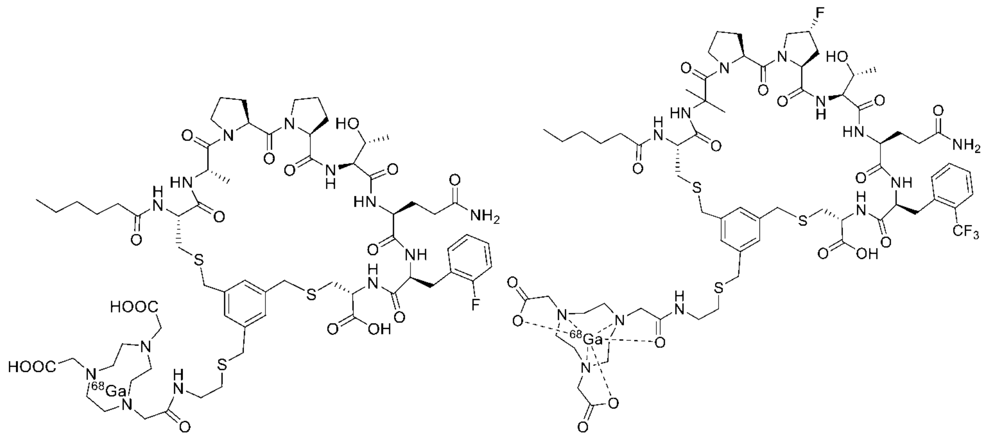

Hex-[Cys-(tMeBn($^{68}$Ga-NOTA-AET))-Ala-Pro-Pro-Thr-Gln-(2-F-Phe)-Cys]-OH,

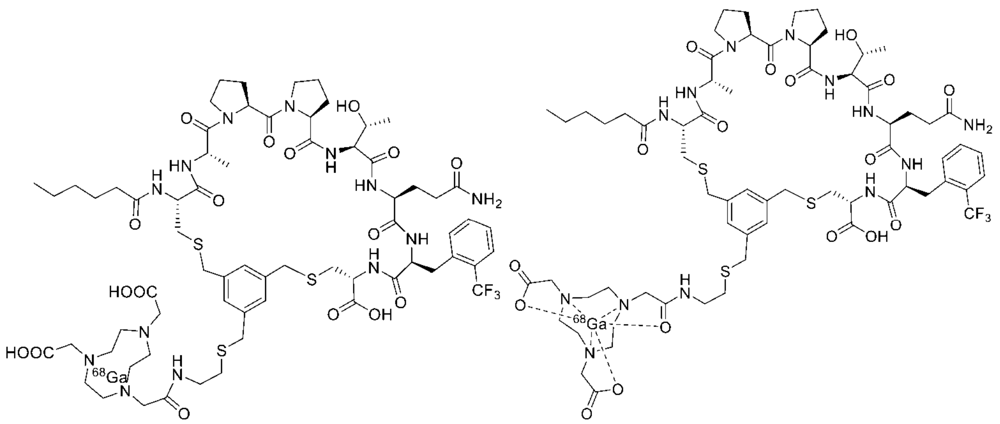

Hex-[Cys-(tMeBn($^{68}$Ga-NOTA-AET))-Ala-Pro-Pro-Thr-Gln-(2-$CF_3$-Phe)-Cys]-OH,

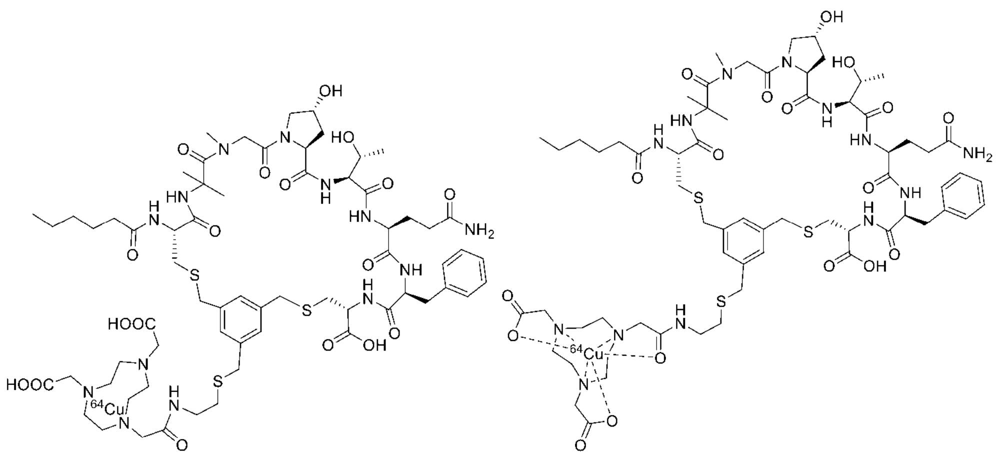

Hex-[Cys-(tMeBn($^{64}$Cu-NOTA-AET))-Aib-Sar-((4R)-OH-Pro)-Thr-Gln-Phe-Cys]-OH,

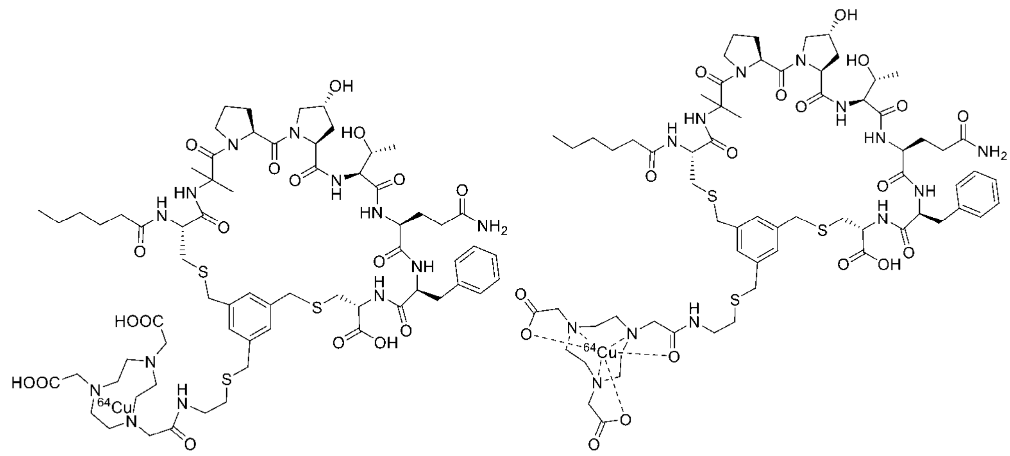

Hex-[Cys-(tMeBn($^{64}$Cu-NOTA-AET))-Aib-Pro-((4R)-OH-Pro)-Thr-Gln-Phe-Cys]-OH,

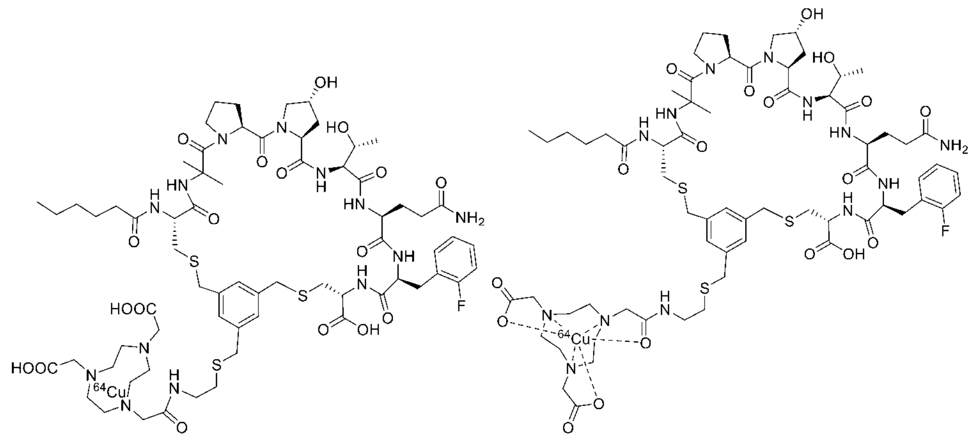

Hex-[Cys-(tMeBn($^{64}$Cu-NOTA-AET))-Aib-Pro-((4R)-OH-Pro)-Thr-Gln-(2-F-Phe)-Cys]-OH,

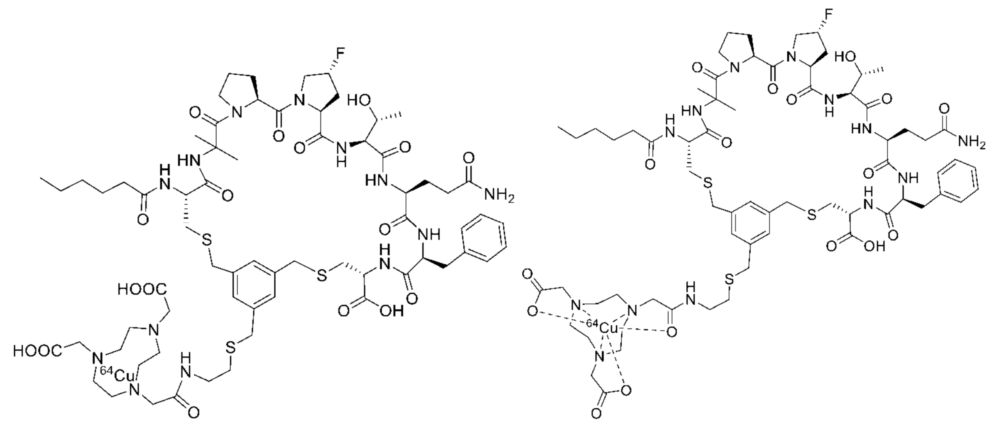

Hex-[Cys-(tMeBn($^{64}$Cu-NOTA-AET))-Aib-Pro-((4R)-F-Pro)-Thr-Gln-Phe-Cys]-OH,

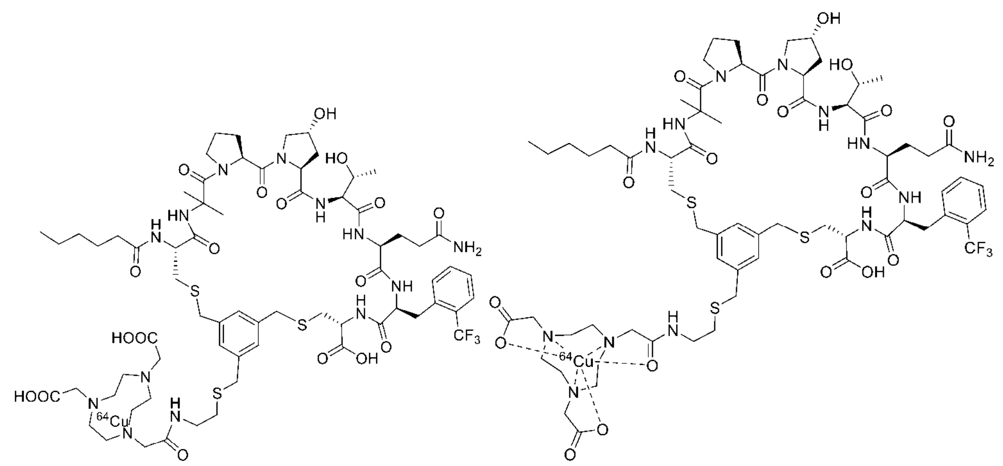

Hex-[Cys-(tMeBn($^{64}$Cu-NOTA-AET))-Aib-Pro-((4R)-OH-Pro)-Thr-Gln-(2-$CF_3$-Phe)-Cys]-OH,

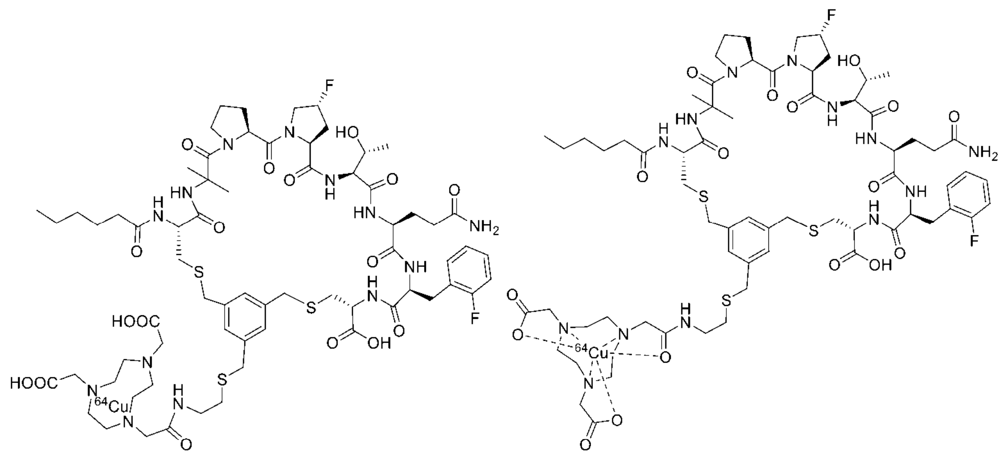

Hex-[Cys-(tMeBn($^{64}$Cu-NOTA-AET))-Aib-Pro-((4R)-F-Pro)-Thr-Gln-(2-F-Phe)-Cys]-OH,

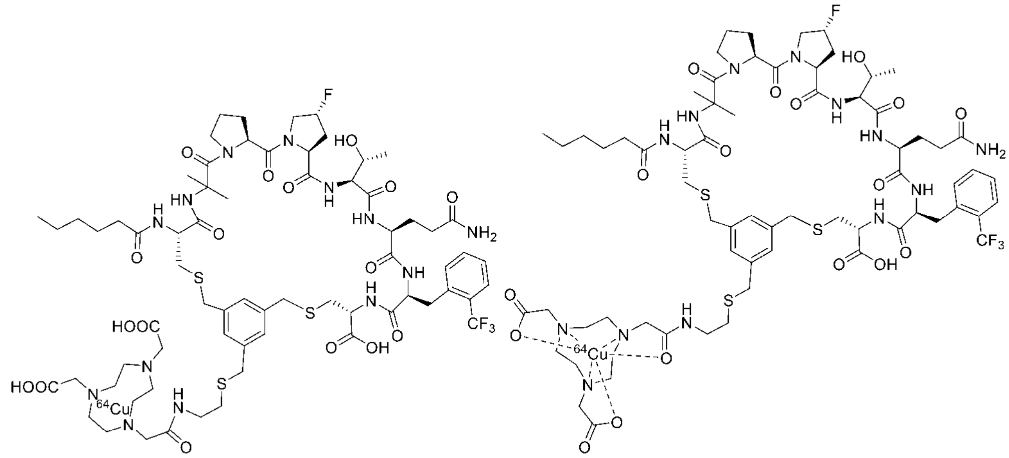

Hex-[Cys-(tMeBn($^{64}$Cu-NOTA-AET))-Aib-Pro-((4R)-F-Pro)-Thr-Gln-(2-$CF_3$-Phe)-Cys]-OH,

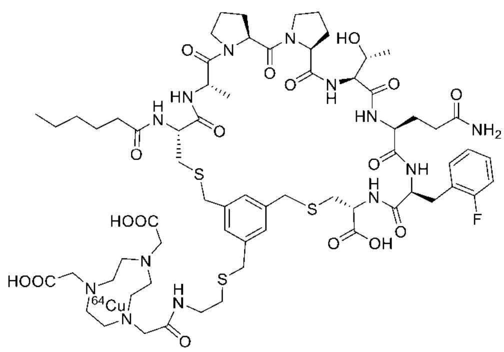
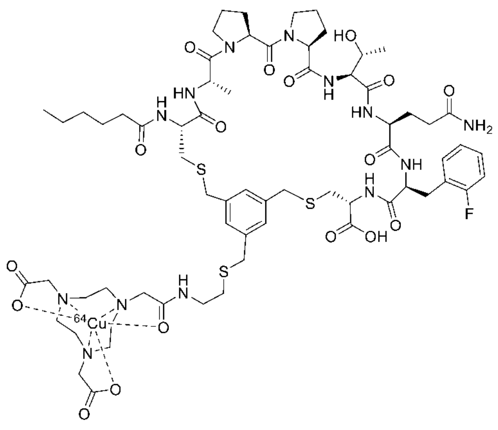

Hex-[Cys-(tMeBn($^{64}$Cu-NOTA-AET))-Ala-Pro-Pro-Thr-Gln-(2-F-Phe)-Cys]-OH, and

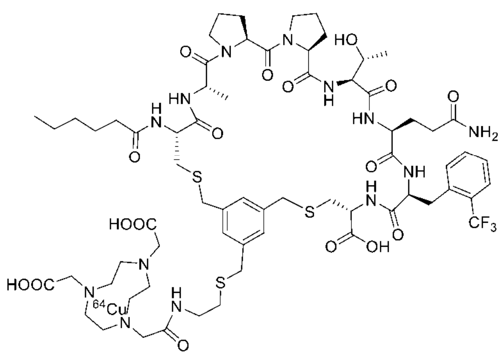
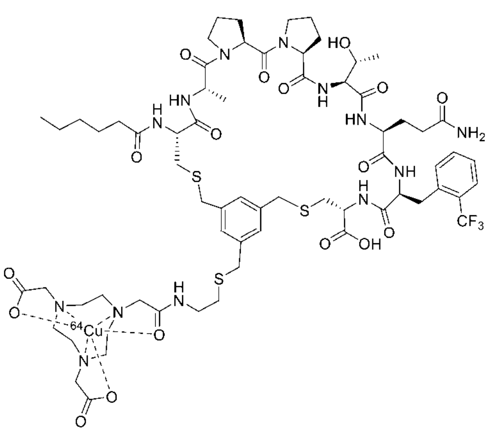

Hex-[Cys-(tMeBn($^{64}$Cu-NOTA-AET))-Ala-Pro-Pro-Thr-Gln-(2-$CF_3$-Phe)-Cys]-OH.

The aforementioned compounds provided by the present disclosure may be used as medicaments.

Another aspect of the present disclosure provides a pharmaceutical composition comprising the aforementioned compound or the pharmaceutically acceptable salt thereof, and one or more pharmaceutically acceptable carriers, diluents or excipients.

Another aspect of the present disclosure provides a method for preparing the aforementioned compound or the pharmaceutically acceptable salt thereof or the aforementioned pharmaceutical composition, which method comprises a step of complexing the compound or the pharmaceutically acceptable salt thereof with a nuclide.

In another aspect, the present disclosure provides the use of the aforementioned compound or the pharmaceutically acceptable salt thereof or the aforementioned pharmaceutical composition in the manufacture of a medicament for diagnosing or treating a disease, wherein the disease is a disease related to fibroblast activation protein (FAP).

In an alternative embodiment, the disease related to fibroblast activation protein (FAP) is a disease associated with upregulation of fibroblast activation protein (FAP) expression.

In an alternative embodiment, the disease is related to cells that exhibit upregulation of fibroblast activation protein (FAP) expression.

In an alternative embodiment, the disease is related to a diseased tissue containing cells that exhibit upregulation of fibroblast activation protein (FAP) expression.

In an alternative embodiment, the disease is a disease related to tumour-associated fibroblasts.

In another aspect, the present disclosure further provides the use of the aforementioned compound or the pharmaceutically acceptable salt thereof or the aforementioned pharmaceutical composition in the manufacture of a medicament for diagnosing or treating a disease, wherein the disease is a tumour or cancer.

In another aspect, the present disclosure provides the use of the aforementioned compound or the pharmaceutically acceptable salt thereof or the aforementioned pharmaceutical composition in the manufacture of a medicament for diagnosing or treating a disease, wherein the disease is selected from an inflammatory disease, a cardiovascular disease, an autoimmune disease and a fibrotic disease.

In an alternative embodiment, the disease is an inflammatory disease, e.g., atherosclerosis, arthritis or

rheumatoid arthritis.

In an alternative embodiment, the disease is a cardiovascular disease.

In an alternative embodiment, the disease is a cardiovascular disease related to atherosclerotic plaque.

In an alternative embodiment, the disease is atherosclerotic lesion, acute coronary syndrome, myocardial infarction, thrombosis, or vascular occlusion caused by plaque rupture.

In an alternative embodiment, the disease is a fibrotic disease, e.g., idiopathic pulmonary fibrosis, Crohn's disease and liver fibrosis.

The tumour or cancer described in the present disclosure is selected from solid tumour, epithelioma, bladder cancer, breast cancer, cervical cancer, colorectal cancer, cholangiocarcinoma, endometrial cancer, oesophageal cancer, gastric cancer, gastrointestinal stromal tumour, head and neck cancer, liver cancer, lung cancer, melanoma, mesothelioma, neuroendocrine tumour and carcinoma, ovarian cancer, pancreatic cancer, prostate cancer, renal cell carcinoma, salivary gland cancer, sarcoma, squamous cell carcinoma, glioma (e.g., human brain astroglioblastoma cells), and thyroid cancer (e.g., medullary thyroid carcinoma).

In an alternative embodiment, the tumour or cancer described in the present disclosure is selected from breast cancer, colorectal cancer, cholangiocarcinoma, head and neck cancer, lung cancer, mesothelioma, neuroendocrine tumour and carcinoma, ovarian cancer, pancreatic cancer, prostate cancer, sarcoma and squamous cell carcinoma.

The present disclosure further provides a method for diagnosing or treating a disease, which method comprises administering to a patient a diagnostically effective amount or a therapeutically effective amount of the aforementioned compound or the pharmaceutically acceptable salt thereof or the aforementioned pharmaceutical composition, wherein the disease is selected from a disease related to fibroblast activation protein (FAP), a tumour or cancer, an inflammatory disease, a cardiovascular disease, an autoimmune disease and a fibrotic disease.

The patient described in the present disclosure may be a mammal, e.g., human, dog, cat, horse and cow.

In some embodiments, the method for diagnosing a disease using the compound provided by the present disclosure is imaging, wherein the imaging method is selected from scintigraphy, single-photon emission computed tomography (SPECT) and positron emission tomography (PET).

In some embodiments, the unit dose of the pharmaceutical composition is 0.001 mg to 1000 mg.

In certain embodiments, the pharmaceutical composition comprises 0.01%-99.99% of the aforementioned compound or a pharmaceutically acceptable salt thereof, or an isotopic substitute thereof, on the basis of the total weight of the composition. In certain embodiments, the pharmaceutical composition comprises 0.1%-99.9% of the aforementioned compound or a pharmaceutically acceptable salt thereof, or an isotopic substitute thereof. In certain embodiments, the pharmaceutical composition comprises 0.5%-99.5% of the aforementioned compound or a pharmaceutically acceptable salt thereof, or an isotopic substitute thereof. In certain embodiments, the pharmaceutical composition comprises 1%-99% of the aforementioned compound or a pharmaceutically acceptable salt thereof, or an isotopic substitute thereof. In certain embodiments, the pharmaceutical composition comprises 2%-98% of the aforementioned compound or a pharmaceutically acceptable salt thereof, or an isotopic substitute thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure further provides a method for preparing the aforementioned compound or a pharmaceutically acceptable salt thereof or the aforementioned pharmaceutical composition, which method comprises a step of complexing the compound as shown in formula (I) or a pharmaceutically acceptable salt thereof with a nuclide,

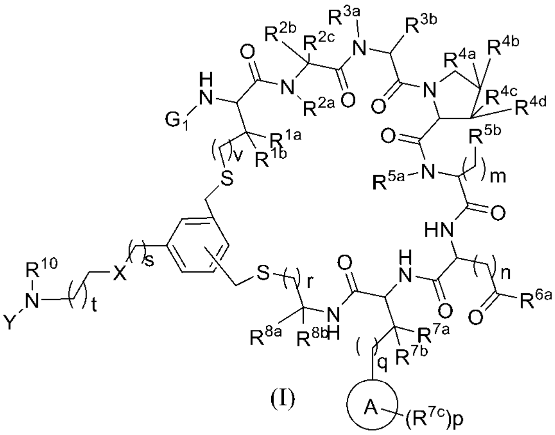

(I)

wherein the Y is a chelating agent, the chelating agent complexes a nuclide, and the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t and $R^{10}$ are as defined in formula (I).

The present disclosure further provides a method for preparing the aforementioned compound or a pharmaceutically acceptable salt thereof or the aforementioned pharmaceutical composition, which method comprises a step of reacting a compound as shown in formula (I-1) with a chelating agent,

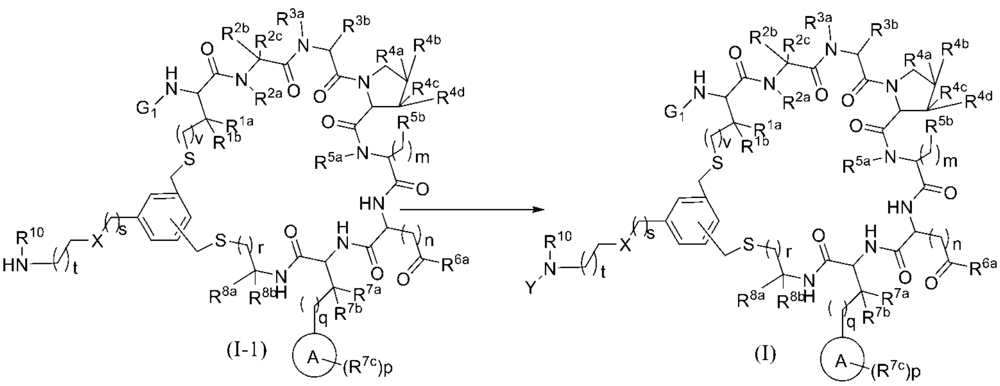

(I-1) (I)

wherein the $R^{1a}$, $R^{1b}$, v, $G_1$, $R^{2a}$, $R^{2b}$, $R^{2c}$, $R^{3a}$, $R^{3b}$, $R^{4a}$, $R^4$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t and $R^{10}$ are as defined in formula (I).

The present disclosure provides a compound as shown in formula (I-2) or a pharmaceutically acceptable salt thereof,

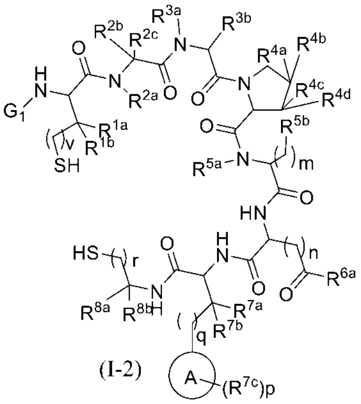

(I-2)

wherein the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$ and r are as defined in formula (I).

The present disclosure further provides a method for preparing the aforementioned compound as shown in formula (III) or a pharmaceutically acceptable salt thereof, which method comprises a step of reacting a compound as shown in formula (III-1) or a pharmaceutically acceptable salt thereof with 2-aminoethanethiol and 1,3,5-tris(bromomethyl)

benzene,

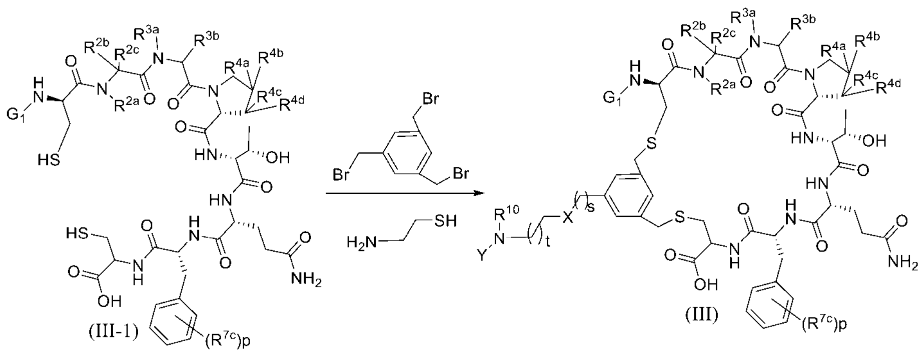

wherein the Y is hydrogen; $R^{10}$ is hydrogen; the t is 1; the X is a sulphur atom; the s is 1; the $R^{2a}$, $R^{2b}$, $R^{2c}$, $R^{3a}$, $R^{3b}$, $R^{4a}$, $R^{4b}$, $R^{4c}$, $R^{4d}$, $R^{7c}$, p and $G_1$ are as defined in formula (III).

Alternatively, the method for preparing the aforementioned compound as shown in formula (I) or a pharmaceutically acceptable salt thereof provided by the present disclosure further comprises the step in the aforementioned method for preparing the compound as shown in formula (III) or a pharmaceutically acceptable salt thereof.

The present disclosure provides a compound as shown in formula (III-1) or a pharmaceutically acceptable salt thereof,

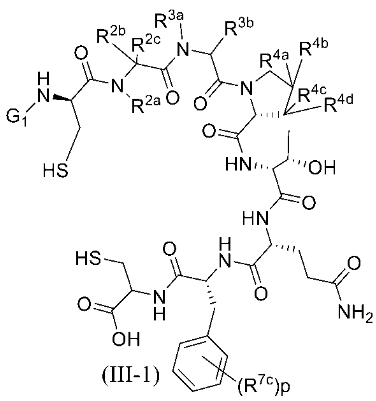

wherein the $R^{2a}$, $R^{2b}$, $R^{2c}$, $R^{3a}$, $R^{3b}$, $R^{4a}$, $R^{4b}$, $R^{4c}$, $R^{4d}$, $R^{7c}$, p and $G_1$ are as defined in formula (III).

In the present disclosure, the representation of the attachment between the chelating agent and the radioactive element in the structure of the radiolabelled compound is not limited to one type. Taking $^{177}$Lu-DOTA-09 as an example,

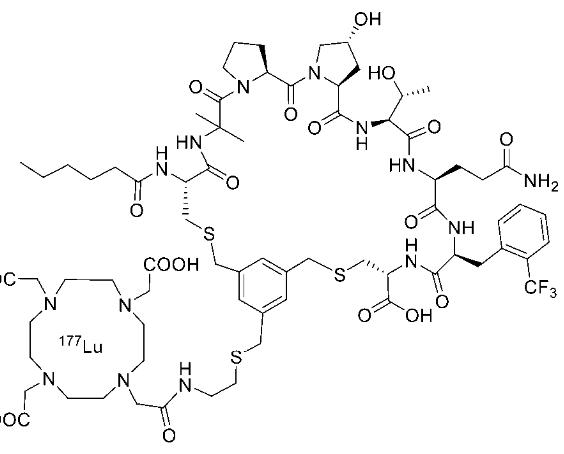

,

the structural formula may also be represented as:

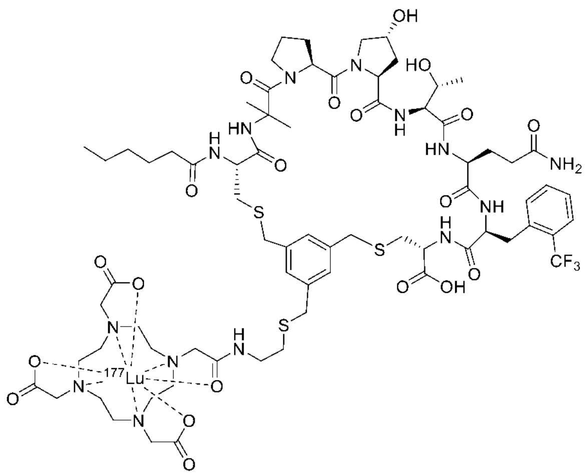

,

or

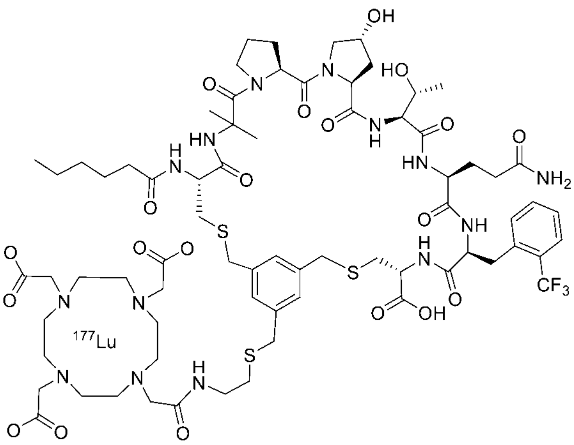

,

wherein

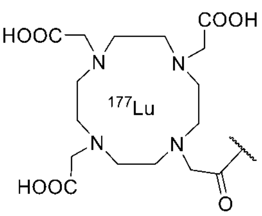

,

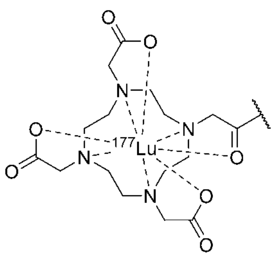

and

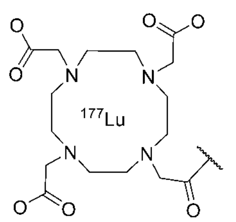

all indicate that a coordinate bond is formed between the chelating agent and $Al^{177}Lu$.

The pharmaceutically acceptable salts of the compounds described in the present disclosure may be selected from inorganic salts or organic salts.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in substituents such as alkyl. All these isomers and mixtures thereof are encompassed within the scope of the present disclosure. The compound comprising asymmetric carbon atoms of the present disclosure can be isolated in optically active-pure or racemic forms. The optically active-pure form can be resolved from the racemic mixture or synthesised by using chiral raw materials or chiral reagents.

Optically active (R)- and (S)-isomers and *D* and *L* isomers can be prepared by using chiral synthesis or chiral

reagents or other conventional techniques. If an enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art, and subsequent recovery of the pure enantiomers. In addition, separation of enantiomers and diastereomers is generally accomplished by using chromatography, which uses chiral stationary phases, optionally in combination with chemical derivatization methods (e.g., formation of carbamates from amines).

In the chemical structures of the compounds of the present disclosure, a bond "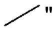" represents an unspecified configuration; that is, if chiral isomers exist in the chemical structures, the bond "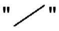" may be "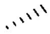" or "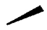", or both the configurations of "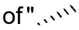" and "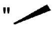" are included simultaneously. In the chemical structures of the compounds of the present disclosure, a bond "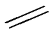" does not specify a configuration; that is, the configuration for the bond "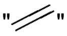" can be an E configuration or a Z configuration, or includes both the E configuration and the Z configuration.

The compounds and intermediates of the present disclosure may also exist in different tautomeric forms, and all such forms are encompassed within the scope of the present disclosure.

The present disclosure further includes isotopically labelled compounds of the present disclosure that are identical to those recited herein, but have one or more atoms replaced by an atom having an atomic mass or mass number different from the atomic mass or mass number usually found in nature. Examples of isotopes that can be incorporated into compounds of the present disclosure include isotopes of hydrogen, carbon, nitrogen, oxygen, phosphorus, sulphur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively.

Unless otherwise stated, when a position is specifically designated as deuterium (D), the position should be construed as having deuterium at an abundance at least 1000 times greater than the natural abundance of deuterium (which is 0.015%) (i.e., at least 10% incorporation of deuterium). The expression "having deuterium at an abundance greater than the natural abundance of deuterium" in the compounds in examples means having deuterium at an abundance at least 1000 times greater, at least 2000 times greater, at least 3000 times greater, at least 4000 times greater, at least 5000 times greater, at least 6000 times greater, or having deuterium at a higher abundance. The present disclosure further includes various deuterated forms of the compound of formula (I). Each available hydrogen atom attached to a carbon atom can be independently replaced with a deuterium atom. Those skilled in the art can synthesise deuterated forms of the compound of formula (I) by referring to relevant literatures. The deuterated forms of the compound of formula (I) can be prepared using commercially available deuterated starting materials, or can be synthesised using deuterated reagents using conventional techniques. The deuterated reagents include, but are not limited to, deuterated borane, solution of trideuterated borane in tetrahydrofuran, deuterated lithium aluminium hydride, deuterated iodoethane and deuterated iodomethane.

Description of Terminology

The term "optionally" or "optional" means that the event or circumstance subsequently described may but need not occur, and the description includes the case where the event or circumstance occurs or does not occur. For example, the term "$C_{1-6}$ alkyl optionally substituted with halogen or cyano" means that halogen or cyano may but need not be present. The description includes the case where the alkyl is substituted with halogen or cyano and the case where the alkyl is not substituted with halogen or cyano.

The term "pharmaceutical composition" denotes a mixture containing one or more compounds described herein or physiologically and pharmaceutically acceptable salts or prodrugs thereof and other chemical components, as well as other components, such as physiologically and pharmaceutically acceptable carriers and excipients. The purpose of pharmaceutical compositions is to facilitate administration to living organisms and facilitate the absorption of active ingredients to exert biological activity.

The term "pharmaceutically acceptable excipient" includes, but is not limited to, any adjuvant, carrier, glidant, sweetener, diluent, preservative, dye/colourant, flavouring agent, surfactant, wetting agent, dispersant, suspending agent, stabiliser, isotonic agent, solvent, or emulsifier that has been approved by the U.S. Food and Drug Administration (FDA) as acceptable for use in humans or livestock animals.

The term "effective amount" or "therapeutically effective amount" described in the present disclosure includes an amount sufficient to ameliorate or prevent a symptom or disorder of a medical disorder. An effective amount also means an amount sufficient to allow or facilitate diagnosis. An effective amount for a particular patient or veterinary subject may vary depending on factors such as the condition to be treated, the general health of the patient, the method, route and dose of administration, and the severity of side effects. An effective amount may be the maximum dosage or dosing regimen that

avoids significant side effects or toxic effects.

The term "alkyl" refers to a saturated aliphatic hydrocarbon group. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl and various branched-chain isomers thereof, etc.

The term "cycloalkyl" refers to a saturated or partially unsaturated monocyclic or polycyclic hydrocarbon. The cycloalkyl ring comprises 3 to 20 carbon atoms, preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc.

The term "heterocycloalkyl" refers to a saturated or partially unsaturated monocyclic or polycyclic hydrocarbon, which comprises 3 to 20 ring atoms, wherein one or more of the ring atoms are heteroatoms selected from nitrogen, oxygen or S(O)m (wherein m is an integer from 0 to 2), but excluding ring moieties of -O-O-, -O-S- or -S-S-, and the remaining ring atoms are carbon.

The term "alkoxy" refers to -O-(alkyl), wherein the alkyl is as defined above. Non-limiting examples of alkoxy include: methoxy, ethoxy, propoxy and butoxy.

The term "alkylene" refers to the moiety remaining after removal of two hydrogen atoms from an alkane molecule, including linear and branched -ylene groups comprising 1 to 20 carbon atoms. Non-limiting examples include methylene ($-CH_2-$) and ethylene (e.g., $-CH_2CH_2-$ or $-CH(CH_3)-$).

The term "aryl" refers to a 6- to 14-membered all-carbon monocyclic or fused polycyclic (i.e., rings sharing an adjacent pair of carbon atoms) group having a conjugated $\pi$ electron system, preferably 6- to 12-membered aryl, e.g., phenyl and naphthyl.

The aryl ring may be fused to a heteroaryl, heterocycloalkyl or cycloalkyl ring, wherein the ring attached to the parent structure is an aryl ring, and the non-limiting examples thereof include:

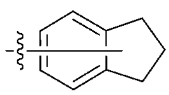, 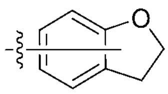, 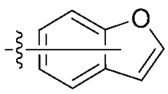, 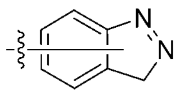, 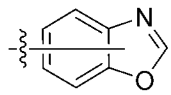,

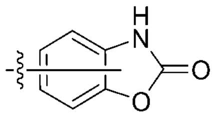, 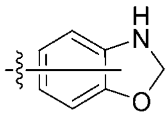, 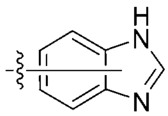, 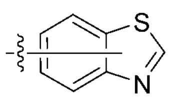, 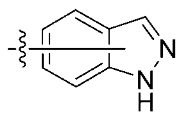,

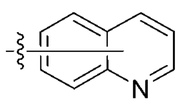, 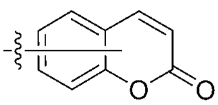 and 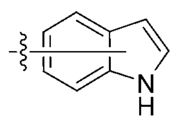.

The term "heteroaryl" refers to a heteroaromatic system containing 1 to 4 heteroatoms and 5 to 14 ring atoms, wherein the heteroatoms are selected from oxygen, sulphur and nitrogen. The heteroaryl is preferably 6- to 12-membered, and more preferably 5- to 6-membered. Non-limiting examples thereof include: imidazolyl, furyl, thienyl, thiazolyl, pyrazolyl, oxazolyl, isoxazolyl, pyrrolyl, tetrazolyl, pyridyl, pyrimidinyl, thiadiazolyl, pyrazinyl, triazolyl, indazolyl, benzimidazolyl,

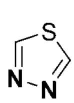, 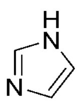, 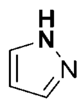,

etc.

The heteroaryl ring may be fused to an aryl, heterocycloalkyl or cycloalkyl ring, wherein the ring attached to the parent structure is a heteroaryl ring, and the non-limiting examples thereof include:

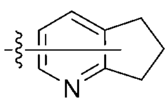, 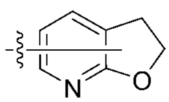, 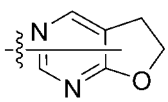, 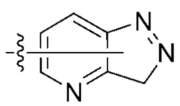, 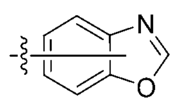,

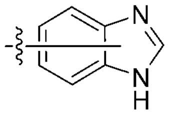, 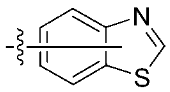, 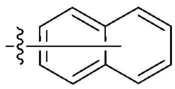 and 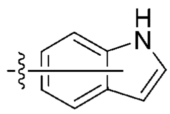.

The term "hydroxyl" refers to -OH.
The term "halogen" refers to fluorine, chlorine, bromine or iodine.
The term "cyano" refers to -CN.
The term "amino" refers to $-NH_2$.
The term "nitro" refers to $-NO_2$.
The term "oxo" refers to =O.
The term "substituted" refers to one or more hydrogen atoms in the group, preferably at most 5, more preferably 1 to 3 hydrogen atoms each independently substituted with a corresponding number of substituents. When the substituent is a ketone or oxo (i.e., =O), two (2) hydrogens on the atom are replaced.

Abbreviations, names and structures for unnatural amino acids and other chemical moieties

| Abbreviation | Name | Structure |
|---|---|---|
| Aib | 2-Aminoisobutyric acid | 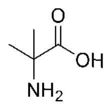 |
| Sar | Sarcosine | 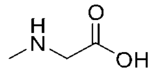 |
| 4-OH-Pro | 4-Hydroxy-L-proline | 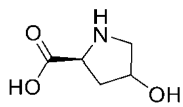 |
| (4R)-OH-Pro | (2S,4R)-4-Hydroxypyrrolidine-2-carboxylic acid | 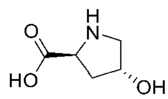 |
| (4S)-OH-Pro | (2S,4S)-4-Hydroxypyrrolidine-2-carboxylic acid | 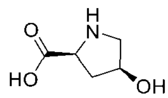 |
| 4-F-Pro | (2S)-4-Fluoropyrrolidine-2-carboxylic acid | 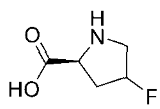 |
| (4R)-F-Pro | (2S,4R)-4-Fluoropyrrolidine-2-carboxylic acid | 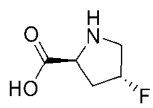 |
| (4S)-F-Pro | (2S,4S)-4-Fluoropyrrolidine-2-carboxylic acid | 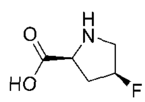 |
| 2-F-Phe | L-2-Fluorophenylalanine | 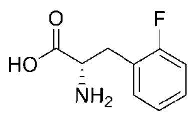 |

(continued)

| Abbreviation | Name | Structure |
|---|---|---|
| 3-F-Phe | L-3-Fluorophenylalanine | 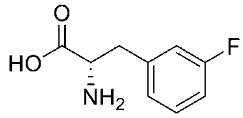 |
| 4-F-Phe | L-4-Fluorophenylalanine | 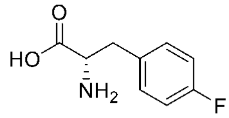 |
| 2-Cl-Phe | L-2-Chlorophenylalanine | 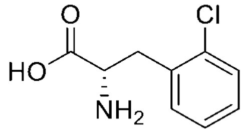 |
| 3-Cl-Phe | L-3-Chlorophenylalanine | 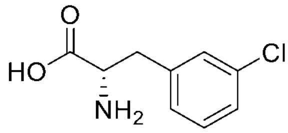 |
| 4-Cl-Phe | L-4-Chlorophenylalanine | 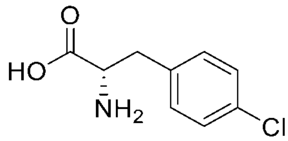 |
| 2-$CF_3$-Phe | L-2-Trifluoromethylphenylalanine | 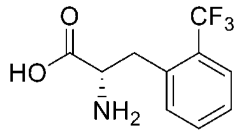 |
| 3-$CF_3$-Phe | L-3-Trifluoromethylphenylalanine | 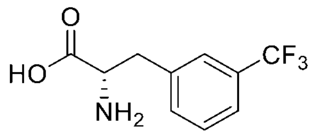 |
| 4-$CF_3$-Phe | L-4-Trifluoromethylphenylalanine | 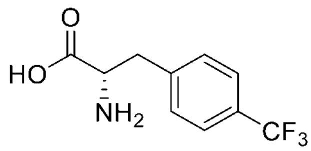 |
| 2-Nal | 3-(2-Naphthyl)-L-alanine | 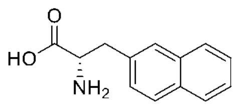 |
| Hex | Hexanoic acid | 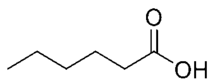 |
| Oct | Octanoic acid | 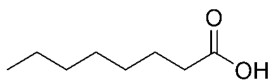 |
| Dec | Decanoic acid | 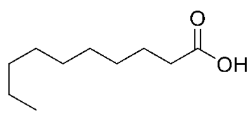 |

(continued)

| Abbreviation | Name | Structure |
|---|---|---|
| Dod | Dodecanoic acid | 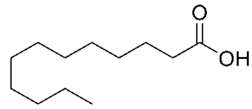 |
| Tet | Tetradecanoic acid | 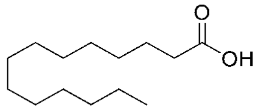 |
| Pal | Palmitic acid | 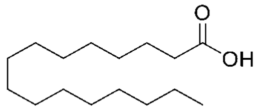 |
| 5-guanidine Valy | 5-Guanidinopentanoic acid | 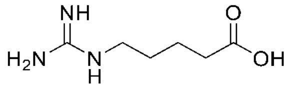 |
| 2-[2-(2-Methoxyethoxy)ethoxy]-Ace | 2-[2-(2-Methoxyethoxy)ethoxy]acetic acid | 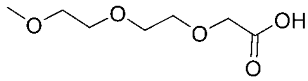 |
| 2-(2-Methoxyethoxy)-Ace | 2-(2-Methoxyethoxy) acetic acid | 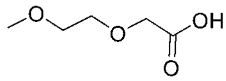 |
| tMeBn | 1,3,5-Trimethylbenzylidene | 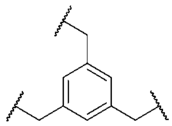 |
| AET | 2-Aminoethanethiol | 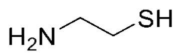 |
| DOTA | 1,4,7,10-Tetraazacyclododecane-1,4,7,10-tet-raacetic acid | 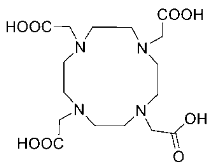 |
| NOTA | 1,4,7-Triazacyclononane-1,4,7-triacetic acid | 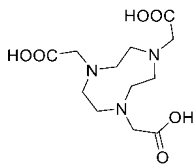 |

**Brief Description of the Drawings**

FIG. 1 shows the uptake of the compounds of the present disclosure in tumour and various tissues.
FIG. 2 shows the tumour/kidney uptake ratio curves of the compounds of the present disclosure and $^{68}$Ga-FAP-2286 (** indicates statistically significant difference).
FIG. 3 shows the tumour uptake of the compounds of the present disclosure and $^{68}$Ga-FAP-2286 at different time points.
FIG. 4 shows the tumour/kidney uptake ratios of the compounds of the present disclosure and $^{68}$Ga-FAP-2286 at different time points.
FIG. 5 shows the tumour uptake of the compounds of the present disclosure and $^{177}$Lu-FAP-2286 at different time

points.
FIG. 6 shows the tumour/kidney uptake ratios of the compounds of the present disclosure and $^{177}$Lu-FAP-2286 at different time points.
FIG. 7 shows the tumour uptake of the compounds of the present disclosure and $^{68}$Ga-FAP-2286 at different time points.
FIG. 8 shows the tumour/kidney uptake ratios of the compounds of the present disclosure and $^{68}$Ga-FAP-2286 at different time points.
FIG. 9 shows the tumour uptake of the compounds of the present disclosure and Al$^{18}$F-NOTA-2286 at different time points.
FIG. 10 shows the tumour/kidney uptake of the compounds of the present disclosure and Al$^{18}$F-NOTA-2286 at different time points.
FIG. 11 shows the tumour growth curves of U-87 MG model mice after administration of $^{177}$Lu-DOTA-09 at different radioactive doses (n = 8, mean ± standard deviation).
FIG. 12 shows the changes in body weight of mice during administration of $^{177}$Lu-DOTA-09 at different radioactive doses (n = 8, mean ± standard deviation).
FIG. 13 shows the survival time of U-87 MG model mice after administration of $^{177}$Lu-DOTA-09 at different radioactive doses.

## Detailed Description of Embodiments

## Example

The following synthetic descriptions or specific examples are for illustrative purposes only and shall not be construed as limiting, in any way, the preparation of the compounds of the present disclosure by other methods.

The chromatographic conditions for HPLC/MS analysis are as follows:
10 μl of each sample is injected automatically. Mobile phase: A: 0.1% formic acid in water, B: 0.1% formic acid in acetonitrile. Flow rate: 1.5 ml/min. Gradient: from 10% B to 95% B in 0-6 min, from 95% B to 100% B in 6-8 min, from 100% B to 10% B in 8-8.10 min, and 10% B maintained in 8.10-11.0 min.

Device model: Thermo Scientific ULTIMATE3000 ISQEM.

Chromatographic column: Eclipse Plus C18, 3.5 nm, 4.6 × 100 mm.

UV detection wavelength: 220 nM.

The purity data of the compounds is obtained by manual integration, and the molecular weight $[M+1]^+$ or $[M-1]^-$ is collected.

The chromatographic conditions for preparative liquid chromatography are as follows:
Mobile phase: A: 0.1% trifluoroacetic acid in water, B: 0.1% trifluoroacetic acid in acetonitrile. Flow rate: 60 ml/min. Gradient: from 25% B to 45% B in 0-20.0 min, from 45%B to 70% B in 20.0-30.0 min, and 70% B maintained in 30.0-40.0 min.

Device model: Agilent AGILENT1260II.

Chromatographic column: HPLCONE, 5.0 μm, 30 × 250 mm.

UV detection wavelength: 220 nM.

The chromatographic conditions for radio-HPLC analysis are as follows:
5-20 μl of each sample is injected automatically. Mobile phase: A: 0.1% formic acid in water, B: 0.1% formic acid in acetonitrile. Flow rate: 0.8 ml/min. Gradient: from 10% B to 50% B in 0-24 min, from 50% B to 10% B in 24-25 min, and 10% B maintained in 25-35 min.

Device model: Thermo Scientific ULTIMATE3000 ISQEM.

Chromatographic column: XBridge C18 3.5 μm, 4.6*150 mm.

UV detection wavelength: 225 nM.

Radiation detector: 30000 cps/V.

The radiochemical purity is obtained by manual integration.

Abbreviations and corresponding full names used in the examples:

| Abbreviation | Full name |
|---|---|
| Fmoc-Cys(Trt)-OH | N-Fluorenylmethoxycarbonyl-S-trityl-L-cysteine |
| Fmoc-Cys(Mmt)-OH | N-Fluorenylmethoxycarbonyl-S-(4-methoxytrityl)-L-cysteine |
| DMF | N,N-Dimethylformamide |
| DCM | Dichloromethane |

(continued)

| Abbreviation | Full name |
|---|---|
| DIPEA | N,N-Diisopropylethylamine |
| HOBT | 1-Hydroxybenzotriazole |
| DIC | N,N'-Diisopropylcarbodiimide |
| DMAP | 4-Dimethylaminopyridine |
| TFA | Trifluoroacetic acid |
| DTT | Dithiothreitol |
| Tips | Triisopropylsilane |
| DOTA | 1,4,7,10-Tetraazacyclododecane-1,4,7,10-tetraacetic acid |
| DOTA-NHS | 2,2',2"-(10-(2-((2,5-Dioxopyrrolidin-1-yl)oxy)-2-oxoethyl)-1,4,7,10-tetraazacyclododecane-1,4,7-triyl)triacetic acid |
| NOTA | 1,4,7-Triazacyclononane-1,4,7-triacetic acid |
| NOTA-NHS | 2,2'-(7-(2-((2,5-dioxopyrrolidin-1-yl)oxy)-2-oxoethyl)-1,4,7-triazacyclononane-1,4-diyl)diacetic acid |

Example 1. Preparation of DOTA-01

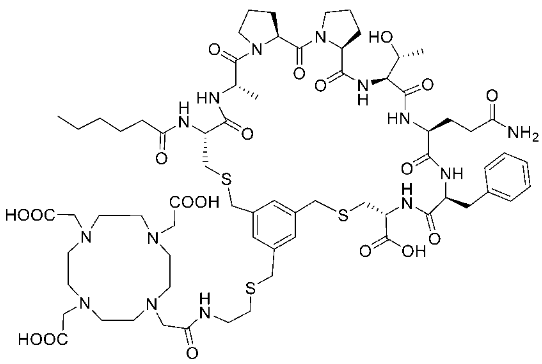

can be performed by either of two solid-phase synthetic schemes.

Scheme I

a. Loading of first amino acid onto solid-phase support

CTC resin (degree of substitution = 1.09 mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of

HOBT were dissolved in 8 Vol of DMF. 3.0 eq of DIC was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water = 1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, purification was performed by preparative liquid chromatography, followed by lyophilisation to obtain a cyclic peptide intermediate.

e. Conjugation with DOTA

The obtained lyophilised powder of the cyclic peptide was dissolved in DMF (10 times the mass of the lyophilised powder). 4.0 eq of DIPEA and 2.0 eq of DOTA-NHS were added. The reaction was monitored by LCMS until completion. Purification was performed by preparative liquid chromatography, followed by lyophilisation to obtain the target product. Purity: 98.2%, MS m/z (ESI): 1540.72 $[M-H]^-$.

Scheme II

1: Loading of first amino acid onto solid-phase support

Wang resin (degree of substitution = 1.08 mmol/g) was selected as a solid-phase support. The resin was swollen with DMF (8 times the mass of the resin) for 1 h. Subsequently, an amino acid solution was prepared: 3.00 eq of Fmoc-Cys(Mmt)-OH and 3.00 eq of HOBT were dissolved in DMF (8 times the mass of the resin). After uniform stirring, 3.00 eq of DIC was added for amino acid activation. After the activation was completed, the solution was added to the swollen resin. Bubbling was performed until uniform, 0.3 eq of DMAP was then added for catalysis. The reaction was performed for 3 hours. After the reaction was completed, the resin was washed with DMF (8 times the mass of the resin) 4 to 6 times. 3.00 eq of acetic anhydride and 6.00 DIPEA were dissolved in DMF (8 times the mass of the resin) for capping for 1 h. The capped resin was washed with DMF (8 times the mass of the resin) 4 to 6 times, and then shrunk with isopropyl ether, DCM and isopropyl ether (each 8 times the mass of the resin). After drying, the degree of substitution was measured for later use.

2: Amino acid coupling

In the synthesis, each amino acid was coupled using the general coupling method described below.

The Fmoc-Cys(Mmt)-Wang resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HOBT were dissolved in 8 Vol of DMF. 3.0 eq of DIC was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

3: Solid-phase cyclization

The side-chain protecting group Mmt for Cys was removed using 1% TFA/DCM (8 times the mass of the resin). The resin was washed with DMF (8 times the mass of the resin) 4 to 6 times, followed by addition of DMF (8 times the mass

of the resin) as a solvent. 7.2 eq of DIPEA was added, followed by the addition of 1.2 eq of 1,3,5-tris(bromomethyl) benzene. After the cyclization was performed, 3.6 eq of mercaptoethylamine was directly added to the system. After the reaction was completed, the resin was washed with DMF (8 times the mass of the resin) 4 to 6 times.

4: Conjugation with DOTA

4.0 eq of DIPEA and 2.0 eq of DOTA-NHS were dissolved in DMF (8 times the mass of the resin) to prepare a reaction solution. The reaction solution was added to a solid-phase column for reaction. After the reaction was completed, the resin was washed with DMF (8 times the mass of the resin) 4 to 6 times, and then shrunk with isopropyl ether, DCM and isopropyl ether (each 8 times the mass of the resin). The resin was dried for later use.

5: Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide. Purification was performed by preparative liquid chromatography, followed by lyophilisation to obtain a lyophilised powder. Purity: 96.7%, MS m/z (ESI): 1540.72 $[M-H]^-$.

Example 2. Preparation of DOTA-02

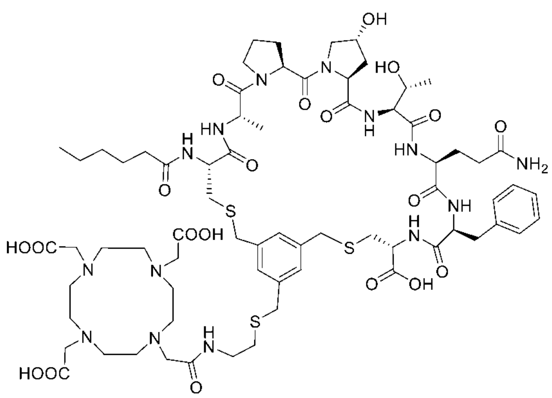

The target compound DOTA-02 was obtained in a manner similar to that of scheme I in Example 1. 97.7%, MS m/z (ESI): 1556.34 $[M-H]^-$.

Example 3. Preparation of DOTA-03

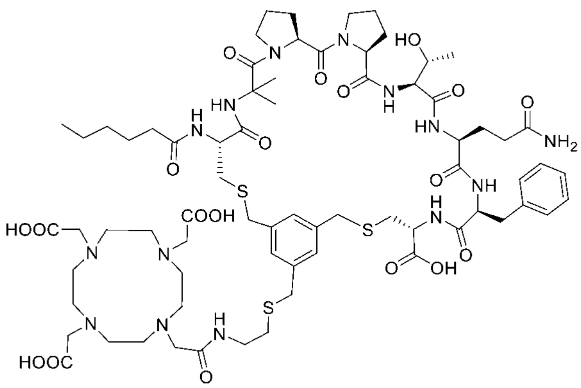

The target compound DOTA-03 was obtained in a manner similar to that of scheme I in Example 1. 96.3%, MS m/z (ESI): 1555.08 [M+H]+.

Example 4. Preparation of DOTA-04

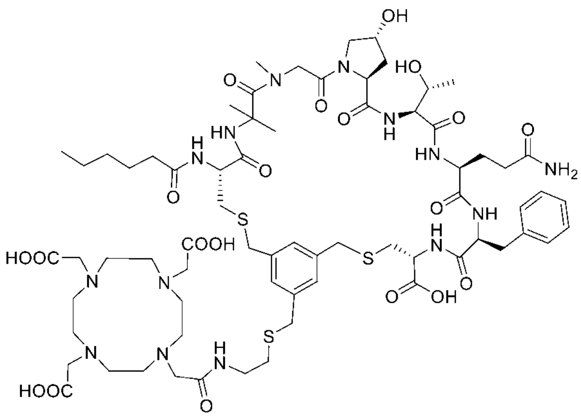

The target compound DOTA-04 was obtained in a manner similar to that of scheme I in Example 1. 95.2%, MS m/z (ESI): 1545.01 [M+H]+.

Example 5. Preparation of DOTA-05

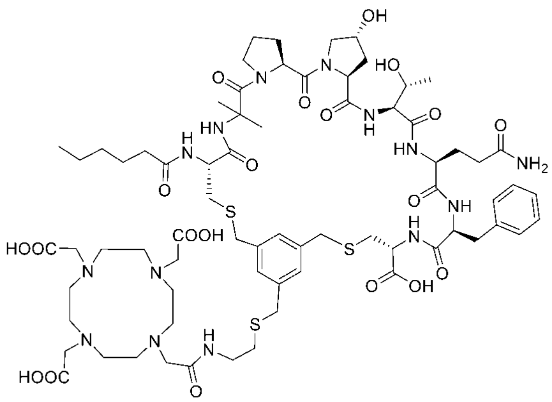

The target compound DOTA-05 was obtained in a manner similar to that of scheme I in Example 1;

a. Loading of first amino acid onto solid-phase support

CTC resin (degree of substitution = 1.09mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen in DMF (8 times the mass of the resin) for 1 h. The Fmoc protecting group was removed twice with a 20% piperidine/DMF solution (8 times the mass of the resin)

(first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HBTU were dissolved in 8 Vol of DMF. 3.0 eq of DEPEA was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water=1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, purification was performed by preparative liquid chromatography, followed by lyophilisation to obtain a cyclic peptide intermediate.

e. Conjugation with DOTA

The obtained lyophilised powder of the cyclic peptide was dissolved in DMF (10 times the mass of the lyophilised powder). 4.0 eq of DIPEA and 2.0 eq of DOTA-NHS were added. The reaction was monitored by LCMS until completion. Purification was performed by preparative liquid chromatography, followed by lyophilisation to obtain the target product.
Purity: 97.8%, MS m/z (ESI): 1571.10 $[M+H]^+$.

Example 6. Preparation of DOTA-06

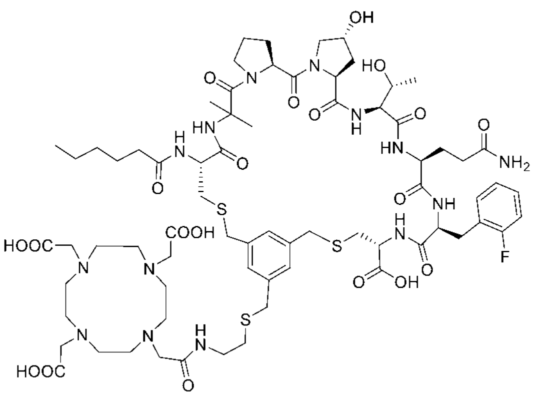

a. Loading of first amino acid onto solid-phase support

CTC resin (degree of substitution = 1.09mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HBTU were dissolved in 8 Vol of DMF. 3.0 eq of DIPEA was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water = 1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, the solvent was removed under reduced pressure, and the residue was purified by preparative liquid chromatography and lyophilised to obtain a cyclic peptide intermediate.

e. Conjugation with DOTA

The obtained lyophilised powder of the cyclic peptide was dissolved in DMF (10 times the mass of the lyophilised powder). 4.0 eq of DIPEA and 2.0 eq of DOTA-NHS were added. The reaction was monitored by LCMS until completion. Purification was performed by preparative liquid chromatography, followed by lyophilisation to obtain the target product.

Purity: 96.9%, MS m/z (ESI): 1587.88 $[M-H]^-$.

Example 7. Preparation of DOTA-07

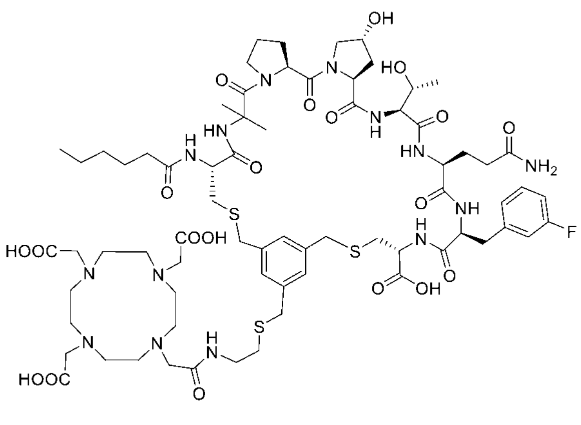

The target compound DOTA-07 was obtained in a manner similar to that of scheme I in Example 1. 96.9%, MS m/z (ESI): 1587.84 $[M-H]^-$.

Example 8. Preparation of DOTA-08

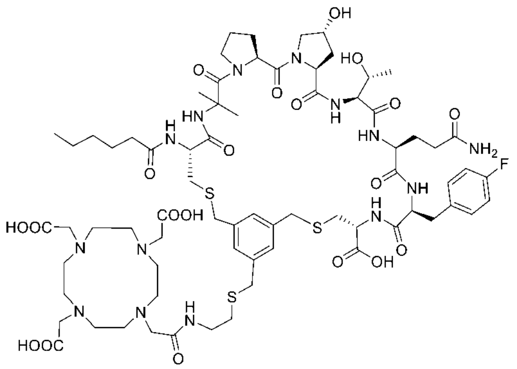

The target compound DOTA-08 was obtained in a manner similar to that of scheme I in Example 1. 96.9%, MS m/z (ESI): 1587.93 [M-H]$^-$.

Example 9. Preparation of DOTA-09

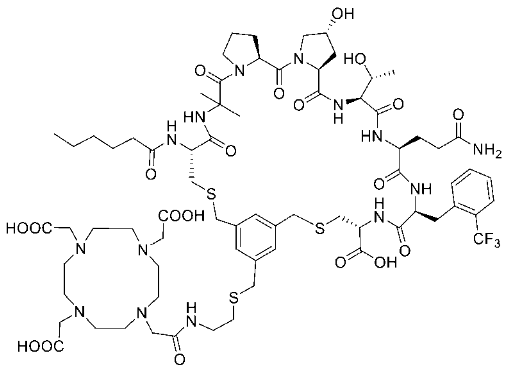

a. Loading of first amino acid onto solid-phase support

CTC resin (degree of substitution = 1.09mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HBTU were dissolved in 8 Vol of DMF. 3.0 eq of DIPEA was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water=1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, the solvent was removed under reduced pressure, and the residue was purified by preparative liquid chromatography and lyophilised to obtain a cyclic peptide intermediate.

e. Conjugation with DOTA

The obtained lyophilised powder of the cyclic peptide was dissolved in DMF (10 times the mass of the lyophilised powder). 4.0 eq of DIPEA and 2.0 eq of DOTA-NHS were added. The reaction was monitored by LCMS until completion. Purification was performed by preparative liquid chromatography, followed by lyophilisation to obtain the target product. Purity: 98.9%, MS m/z (ESI): 1637.78 [M-H]$^-$.

Example 10. Preparation of DOTA-10

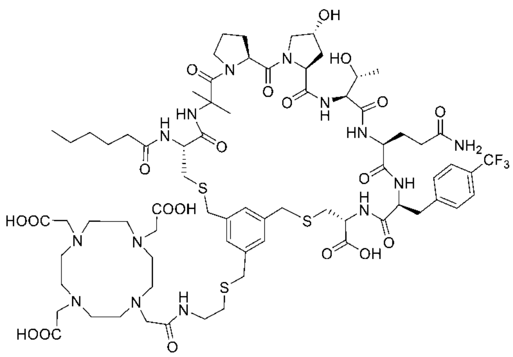

The target compound DOTA-10 was obtained in a manner similar to that of scheme I in Example 1. 99.3%, MS m/z (ESI): 1637.79 [M-H]$^-$.

Example 11. Preparation of DOTA-11

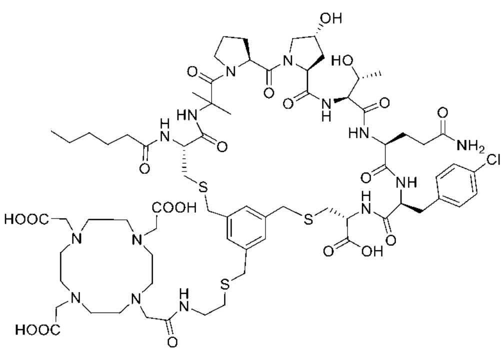

The target compound DOTA-11 was obtained in a manner similar to that of scheme I in Example 1. 99.4%, MS m/z (ESI): 1603.76 [M-H]-.

Example 12. Preparation of DOTA-12

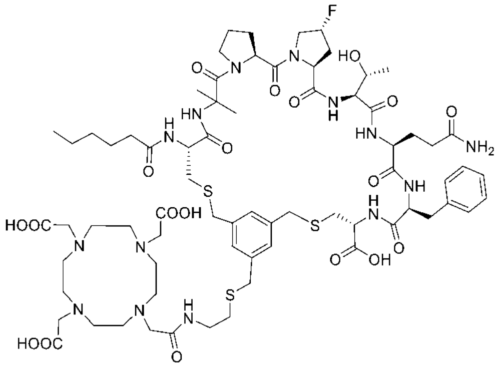

The target compound DOTA-12 was obtained in a manner similar to that of scheme I in Example 1. 99.1%, MS m/z (ESI): 1572.31 [M-H]-.

Example 13. Preparation of DOTA-13

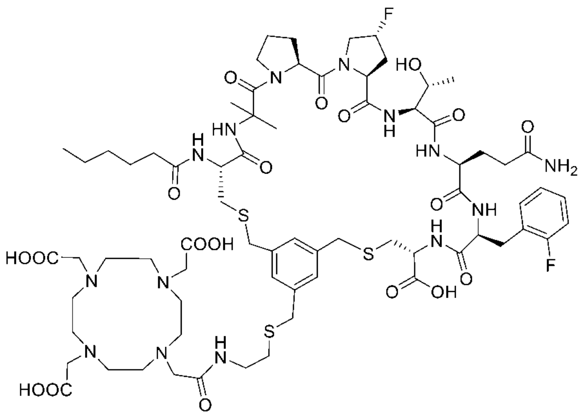

The target compound DOTA-13 was obtained in a manner similar to that of scheme I in Example 1. 98.9%, MS m/z (ESI): 1590.01 [M-H]-.

Example 14. Preparation of DOTA-14

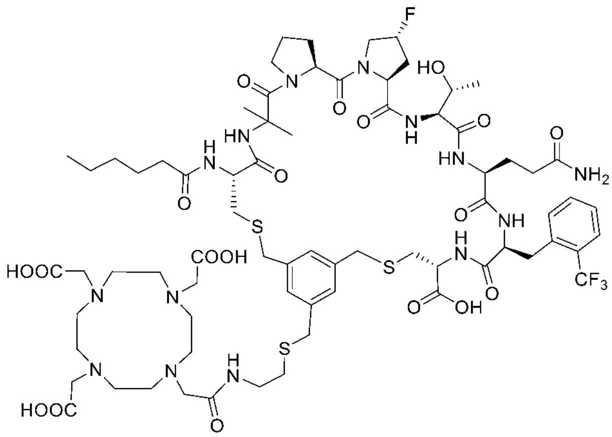

The target compound DOTA-14 was obtained in a manner similar to that of scheme I in Example 1. 97.4%, MS m/z (ESI): 1589.87 [M-H]$^-$.

Example 15. Preparation of NOTA-04

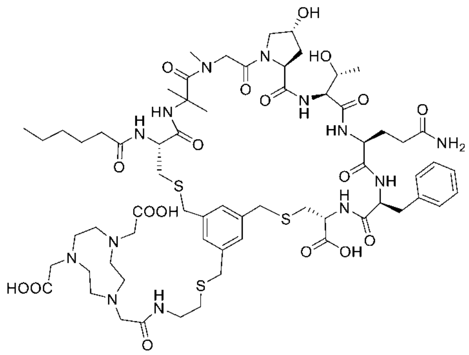

a. Loading of first amino acid onto solid-phase support

CTC resin (degree of substitution = 1.09mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HBTU were dissolved in 8 Vol of DMF. 3.0 eq of DEPEA was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water = 1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, the solvent was removed under reduced pressure, and the residue was purified by preparative liquid chromatography and lyophilised to obtain a cyclic peptide intermediate.

e. Conjugation with NOTA

The obtained lyophilised powder of the cyclic peptide was dissolved in DMF (10 times the mass of the lyophilised powder). 4.0 eq of DIPEA and 2.0 eq of NOTA-NHS were added. The reaction was monitored by LCMS until completion. Purification was performed by preparative liquid chromatography, followed by lyophilisation to obtain the target product.

Purity: 97.20%, MS m/z (ESI): 1442.89 $[M-H]^-$.

Example 16. Preparation of NOTA-05

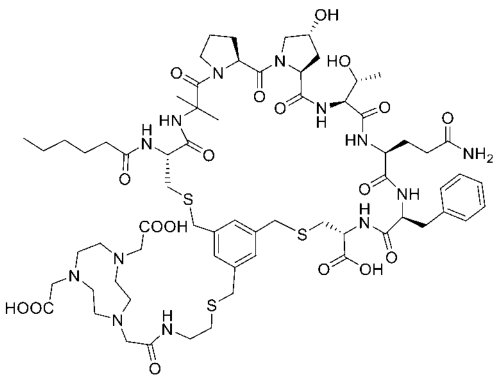

The target compound NOTA-05 was obtained in a manner similar to that in Example 15, purity: 97.20%, MS m/z (ESI): 1470.33 $[M+H]^+$.

Example 17. Preparation of NOTA-06

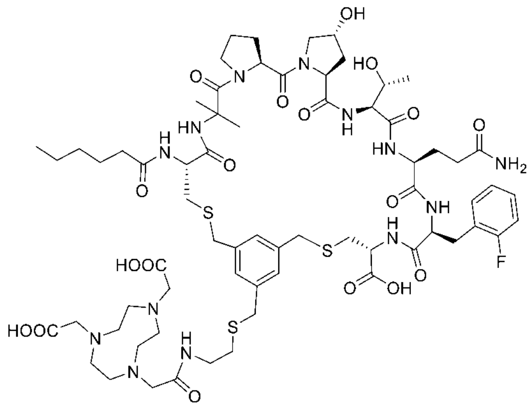

The target compound NOTA-06 was obtained in a manner similar to that in Example 15, purity: 99.2%, MS m/z (ESI): 1486.94 [M-H]-.

Example 18. Preparation of NOTA-07

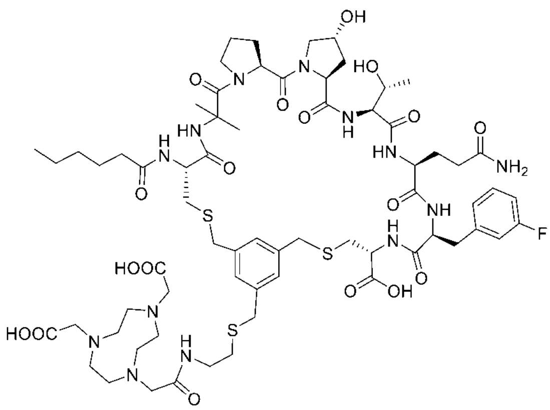

The target compound NOTA-07 was obtained in a manner similar to that in Example 15, purity: 99.0%, MS m/z (ESI): 1486.91 [M-H]-.

Example 19. Preparation of NOTA-08

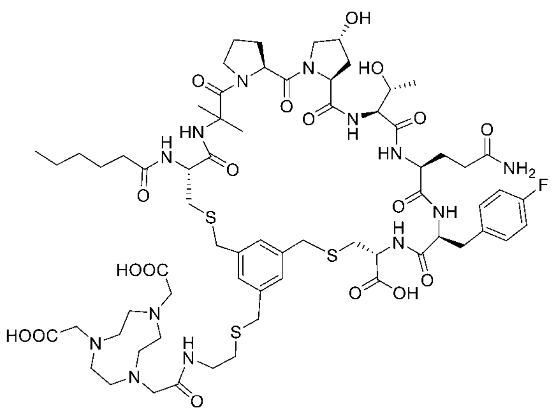

The target compound NOTA-08 was obtained in a manner similar to that in Example 15, purity: 99.6%, MS m/z (ESI): 1486.86 [M-H]-.

Example 20. Preparation of NOTA-09

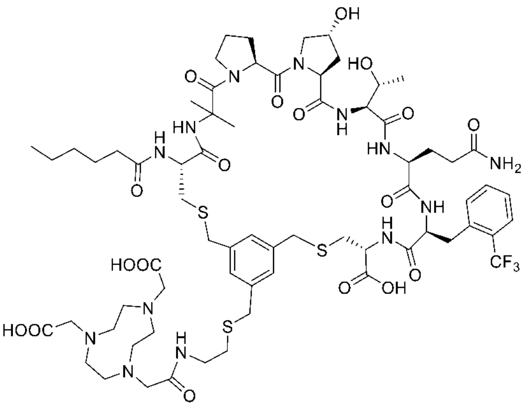

a. Loading of first amino acid onto solid-phase support

CTC resin (degree of substitution = 1.09mmol/g) was selected as a solid-phase support. The resin was swollen with DMF for 1 h. Subsequently, an amino acid solution was prepared: 3.00 eq of Fmoc-Cys(Trt)-OH and 6.00 eq of DIPEA were dissolved in DMF (8 times the mass of the resin). After uniform stirring, the solution was reacted with the resin for 3 h. The resin was washed, and then capped with methanol and DIPEA. The capped resin was washed with DMF (8 times the mass of the resin) 4 to 6 times, and then washed and shrunk sequentially with isopropyl ether, DCM and isopropyl ether (each 8 times the mass of the resin). After drying, the degree of substitution was measured for later use.

b. Amino acid coupling

In the synthesis, each amino acid was coupled using the general coupling method described below.

The Fmoc-Cys(Trt)-CTC resin obtained in the previous step was swollen with DMF (8 times the mass of the resin) for 1 h. The Fmoc protecting group was removed twice with a 20% piperidine/DMF solution (8 times the mass of the resin) (first time: 5 min, and second time: 20 min). After the deprotection was completed, the resin was washed with DMF (8 times the mass of the resin) 4 to 6 times. The activated amino acid solution was prepared: 3.0 eq of amino acid and 3.0 eq of HBTU were dissolved in 8 Vol of DMF. 3.0 eq of DEPEA was added for activation for 2-5 min, and then the reaction was performed. After the reaction was completed and the deprotection was finished, the resin was washed with DMF (8 times the mass of the resin) 4 to 6 times. After the coupling of the linear peptide resin was completed using the above method, the resin was washed, shrunk, and dried for later use.

c. Cleavage

The obtained peptide resin was cleaved using a cleavage solution with a ratio of TFA/DTT/$H_2O$/Tips of 94/2.5/2.5/1, wherein the cleavage solution was added in an amount 8 to 10 times the mass of the peptide resin. After 2 h of cleavage, filtration was performed to obtain the filtrate. The obtained filtrate was poured into isopropyl ether (80 to 100 times the mass of the peptide resin) for crystallisation. The resulting product was filtered, rinsed with isopropyl ether, and dried to obtain a linear crude peptide.

d. Cyclization

The obtained linear crude peptide was dissolved in a mixture of acetonitrile/purified water = 1/1 (400 times the mass of the crude peptide). First, 7.2 eq of DIPEA was added, followed by the addition of 1.2 eq of 1,3,5-tris(bromomethyl) benzene. After the reaction was completed, 3.6 eq of mercaptoethylamine was directly added to the system. After the reaction was completed, the solvent was removed under reduced pressure, and the residue was purified by preparative liquid chromatography and lyophilised to obtain a cyclic peptide intermediate.

e. Conjugation with NOTA

The obtained lyophilised powder of the cyclic peptide was dissolved in DMF (10 times the mass of the lyophilised

powder). 4.0 eq of DIPEA and 2.0 eq of NOTA-NHS were added. The reaction was monitored by LCMS until completion. Purification was performed by preparative liquid chromatography, followed by lyophilisation to obtain the target product. Purity: 99.7%, MS m/z (ESI): 1536.65 [M-H]-.

Example 21. Preparation of NOTA-12

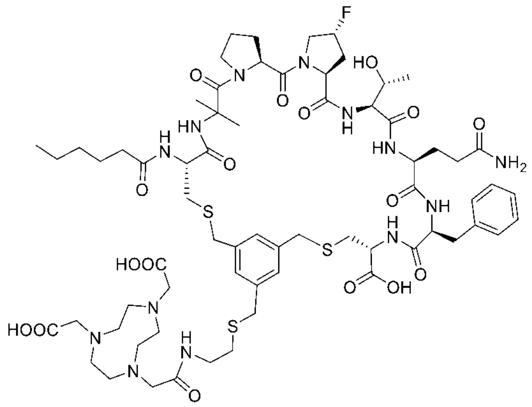

The target compound NOTA-12 was obtained in a manner similar to that in Example 15, purity: 98.8%, MS m/z (ESI): 1470.88 [M-H]-.

Example 22. Preparation of NOTA-13

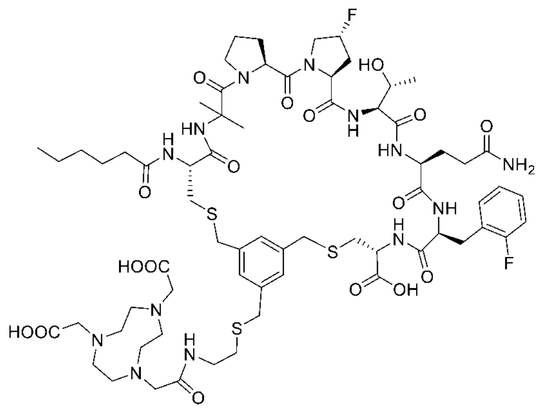

The target compound NOTA-13 was obtained in a manner similar to that in Example 15, purity: 98.6%, MS m/z (ESI): 1488.96 [M-H]-.

Example 23. Preparation of NOTA-14

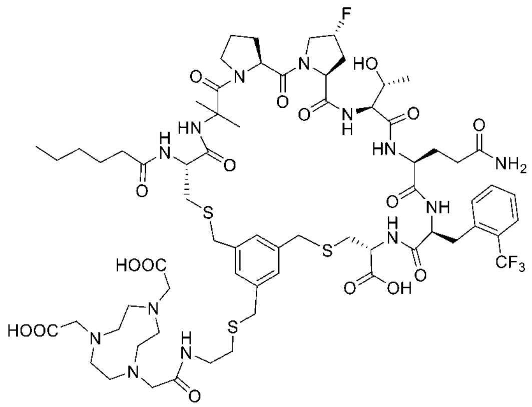

The target compound NOTA-14 was obtained in a manner similar to that in Example 15, purity: 97.9%, MS m/z (ESI): 1538.94 [M-H]-.

Example 24. Preparation of NOTA-15

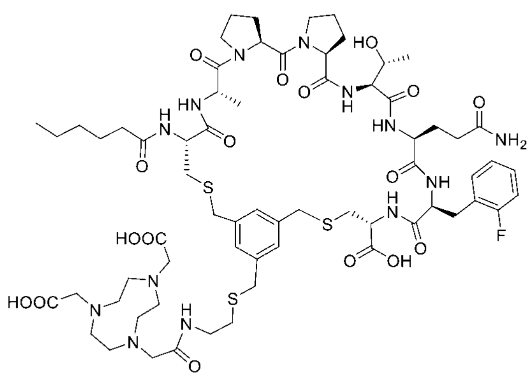

The target compound NOTA-15 was obtained in a manner similar to that in Example 15, purity: 97.3%, MS m/z (ESI): 1456.95 [M-H]-.

Example 25. Preparation of NOTA-16

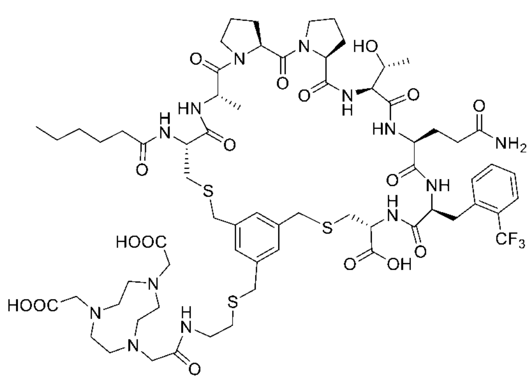

The target compound NOTA-16 was obtained in a manner similar to that in Example 15, purity: 97.8%, MS m/z (ESI): 1506.90 [M-H]-.

Example 26. Preparation of $^{68}$Ga-DOTA-05

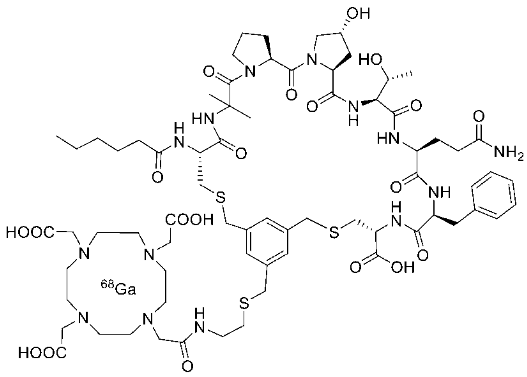

The $^{68}GaCl_3$ solution was rinsed with 0.1 N hydrochloric acid. 0.1 ml of a 1 mM aqueous solution of sodium acetate was added to a reaction vessel; 0.9 ml of the above $^{68}GaCl_3$ solution was then added. 5 μl of DOTA-05 (1 mg/ml) solution and 67 μl of gentisic acid solution (15 mg/ml) were added and uniformly mixed. The reaction solution was adjusted to pH 3.5-5.0, and heated at 95°C for 15 min. The radiochemical purity was 97.54% as detected by radio-HPLC.

If the radiochemical purity is not less than 90%, the product can be directly used. If the radiochemical purity is less than 90%, purification is performed using a C18 cartridge. Then, the cartridge is rinsed with ethanol, and the eluate is diluted with physiological saline until the ethanol content is below 10%.

Example 27. Preparation of $^{68}$Ga-DOTA-06

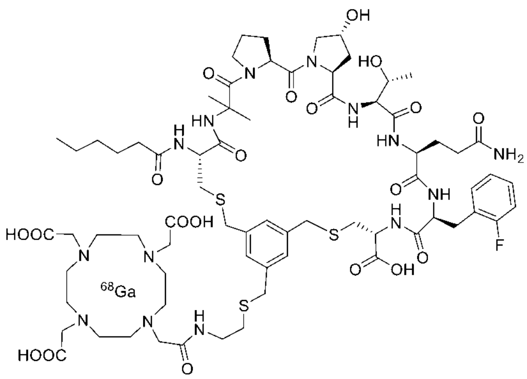

The target compound $^{68}$Ga-DOTA-06 was obtained in a manner similar to that in Example 26, radiochemical purity: 92.64%.

Example 28. Preparation of $^{68}$Ga-DOTA-07

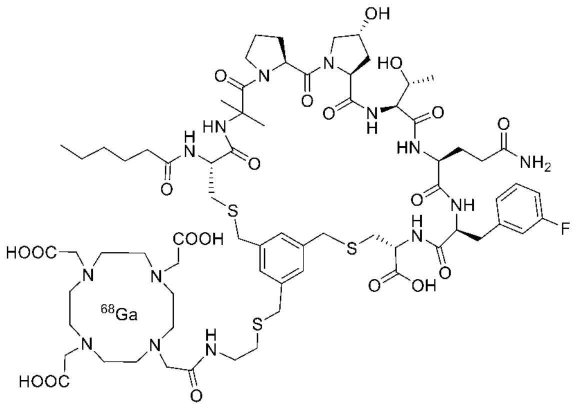

The target compound $^{68}$Ga-DOTA-07 was obtained in a manner similar to that in Example 26, radiochemical purity: 94.76%.

Example 29. Preparation of $^{68}$Ga-DOTA-08

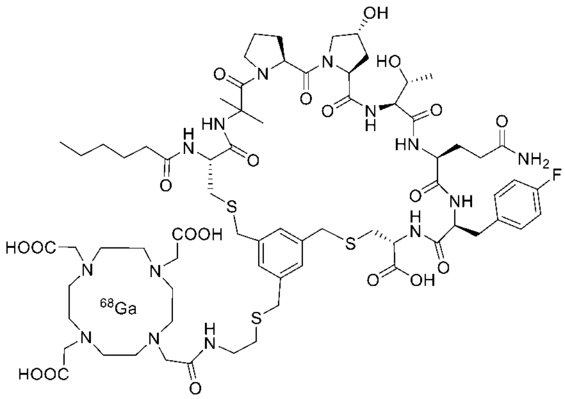

The target compound $^{68}$Ga-DOTA-08 was obtained in a manner similar to that in Example 26, radiochemical purity: 93.99%.

Example 30. Preparation of $^{68}$Ga-DOTA-09

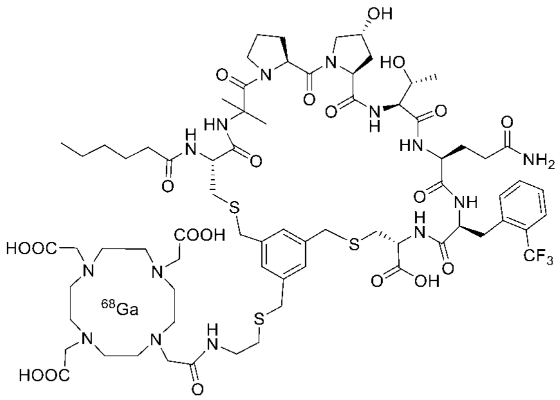

The $^{68}GaCl_3$ solution was rinsed with 0.1 N hydrochloric acid. 0.1 ml of a 1 mM aqueous solution of sodium acetate was added to a reaction vessel; 0.9 ml of the above $^{68}GaCl_3$ solution was then added. 5 μl of DOTA-09 (1 mg/ml)

solution and 67 μl of gentisic acid solution (15 mg/ml) were added and uniformly mixed. The reaction solution was adjusted to pH 3.5-5.0, and heated at 95°C for 15 min. The radiochemical purity was 93.38% as detected by radio-HPLC.

Example 31. Preparation of $^{68}$Ga-DOTA-12

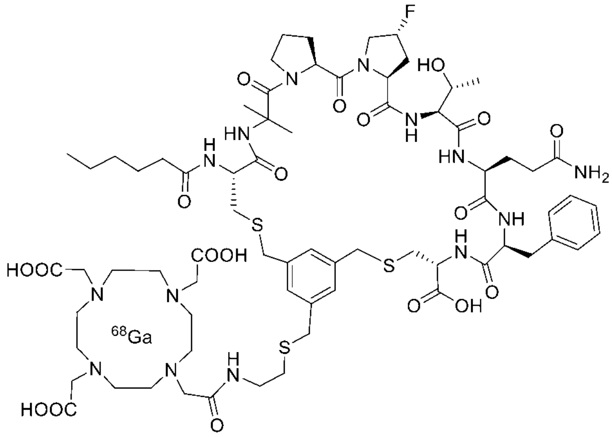

The target compound $^{68}$Ga-DOTA-12 was obtained in a manner similar to that in Example 26, radiochemical purity: 97.57%.

Example 32. Preparation of $^{68}$Ga-DOTA-13

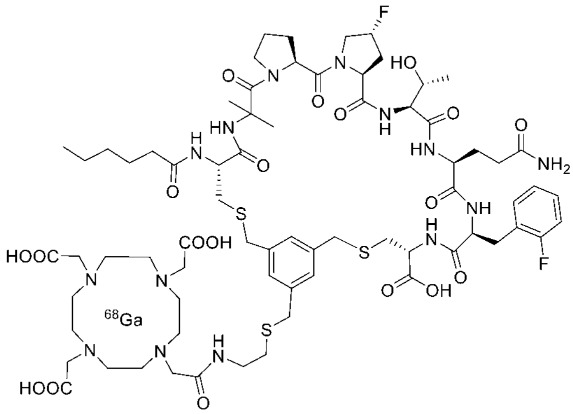

The target compound $^{68}$Ga-DOTA-13 was obtained in a manner similar to that in Example 26, radiochemical purity: 95.81%.

Example 33. Preparation of $^{177}$Lu-DOTA-05

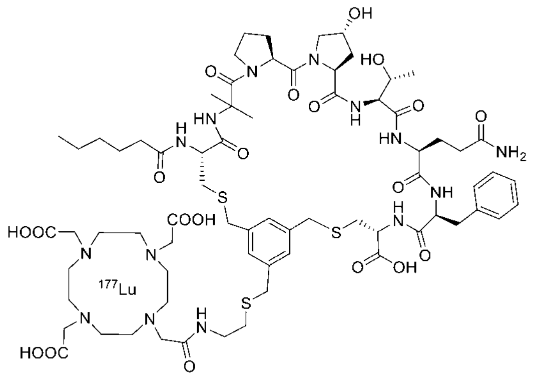

75 µl of 0.4 M acetic acid/sodium acetate buffer, 7.5 µl of **DOTA-05** solution (1 mg/ml), 31.5 µl (15 mg/ml) of gentisic acid solution and 7-15 mCi Lu-177 nuclide were sequentially added to a reaction vessel, and uniformly mixed. The reaction solution was adjusted to pH 4.0-4.5, and heated at 90°C-95°C for 15 min. The reaction solution was cooled to room temperature, and 1.4 ml of diluent (10 mg/ml vitamin C in physiological saline) was added. The radiochemical purity was 96.28% as detected by radio-HPLC.

Example 34. Preparation of $^{177}$Lu-DOTA-06

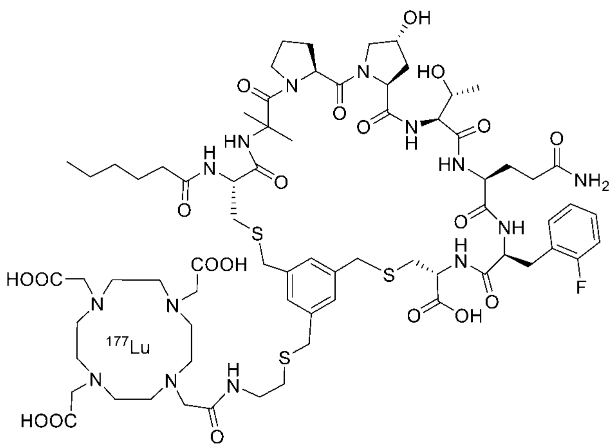

The target compound $^{177}$Lu-DOTA-06 was obtained in a manner similar to that in Example 33, radiochemical purity: 96.18%.

Example 35. Preparation of $^{177}$Lu-DOTA-09

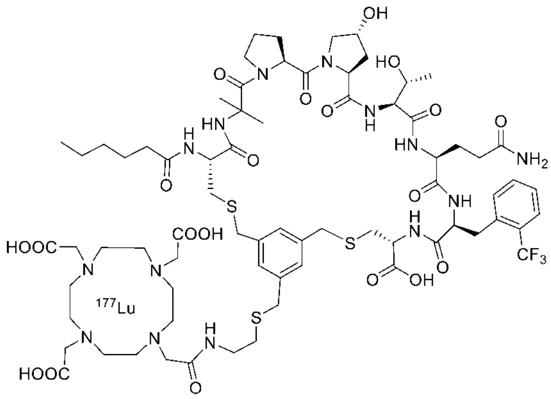

75 μl of 0.4 M acetic acid/sodium acetate buffer, 7.5 μl of **DOTA-09** solution (1 mg/ml), 31.5 μl (15 mg/ml) of gentisic acid solution and 7-15 mCi Lu-177 nuclide were sequentially added to a reaction vessel, and uniformly mixed. The reaction solution was adjusted to pH 4.0-4.5, and heated at 90°C-95°C for 15 min. The reaction solution was cooled to room temperature, and 1.4 ml of diluent (10 mg/ml vitamin C in physiological saline) was added. The radiochemical purity was 94.20% as detected by radio-HPLC.

Example 36. Preparation of $^{177}$Lu-DOTA-13

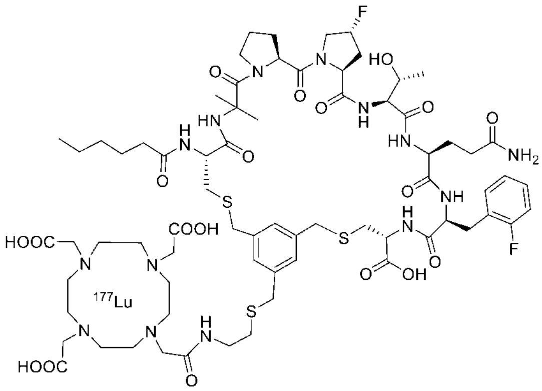

The target compound $^{177}$Lu-DOTA-13 was obtained in a manner similar to that in Example 33, radiochemical purity: 95.95%.

Example 37. Preparation of $^{177}$Lu-DOTA-14

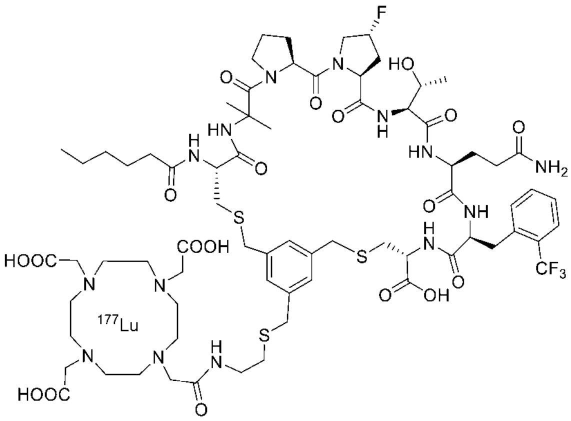

The target compound $^{177}$Lu-DOTA-14 was obtained in a manner similar to that in Example 33, radiochemical purity: 97.45%.

Example 38. Preparation of $^{68}$Ga-NOTA-05

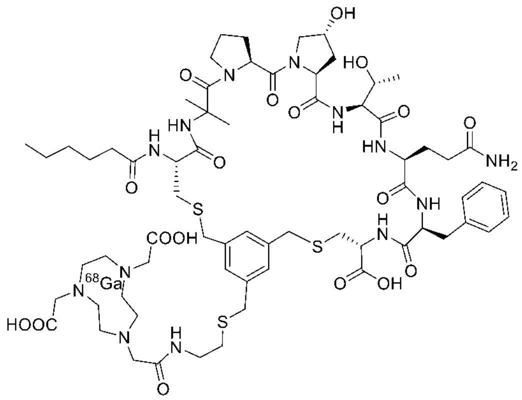

The $^{68}GaCl_3$ solution was rinsed with 0.1 N hydrochloric acid. 0.1 ml of a 1 mM aqueous solution of sodium acetate was added to a reaction vessel; 0.9 ml of the above $^{68}GaCl_3$ solution was then added. 5 μl of an aqueous solution of NOTA-05 (1 mg/ml) and 67 μl of an aqueous solution of gentisic acid (15 mg/ml) were added, and uniformly mixed. The reaction solution was reacted at room temperature for 15 min under a pH condition of 3.5-5.0. The radiochemical purity was 91.07% as detected by radio-HPLC.

If the radiochemical purity is not less than 90%, the product can be directly used. If the radiochemical purity is less than 90%, purification is performed using a C18 cartridge. Then, the cartridge is rinsed with ethanol, and the eluate is diluted with physiological saline until the ethanol content is below 10%.

Example 39. Preparation of $^{68}$Ga-NOTA-06

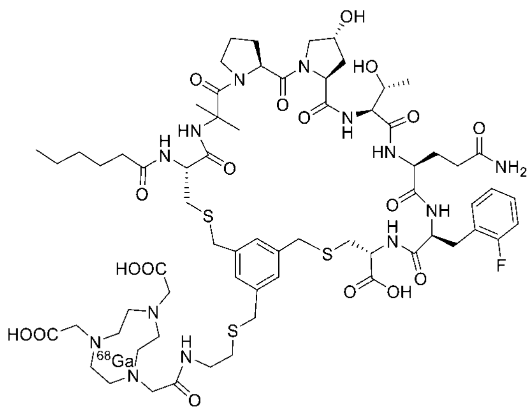

The target compound $^{68}$Ga-NOTA-06 was obtained in a manner similar to that in Example 38, radiochemical purity: 91.38%.

Example 40. Preparation of $^{68}$Ga-NOTA-07

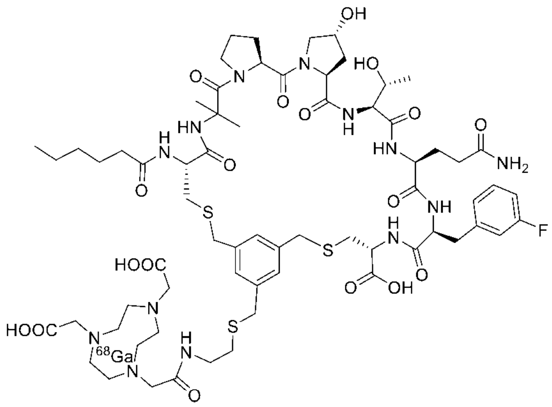

The target compound $^{68}$Ga-NOTA-07 was obtained in a manner similar to that in Example 38, radiochemical purity: 92.54%.

Example 41. Preparation of $^{68}$Ga-NOTA-09

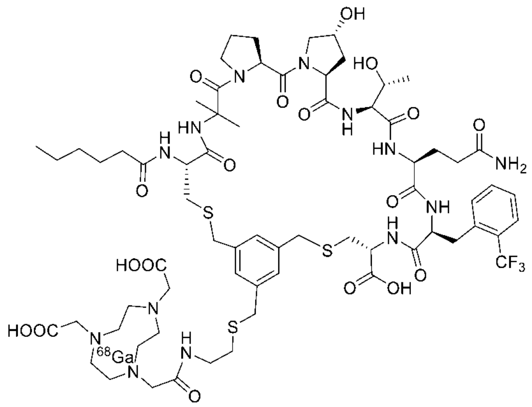

The $^{68}GaCl_3$ solution was rinsed with 0.1 N hydrochloric acid. 0.1 ml of a 1 mM aqueous solution of sodium acetate was added to a reaction vessel; 0.9 ml of the above $^{68}GaCl_3$ solution was then added. 5 μl of an aqueous solution of NOTA-09 (1 mg/ml) and 67 μl of an aqueous solution of gentisic acid (15 mg/ml) were added, and uniformly mixed. The reaction solution was reacted at room temperature for 15 min under a pH condition of 3.5-5.0. The radiochemical purity was 92.36% as detected by radio-HPLC.

Example 42. Preparation of Al$^{18}$F-NOTA-05

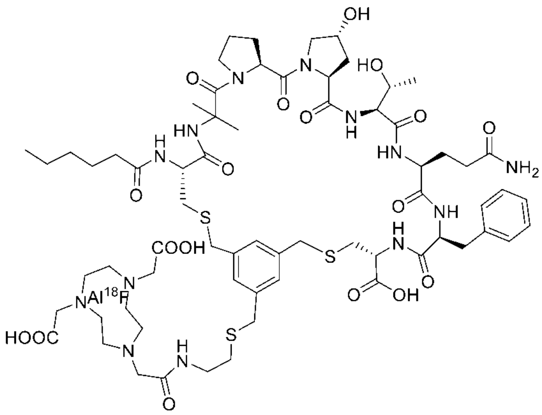

1 μl of a 10 mM aqueous solution of NOTA-05 was dissolved in 50-100 μl of 0.5 M acetic acid/sodium acetate buffer at pH 4.0-4.4. A 2 mM aqueous solution of $AlCl_3$ was then added in a molar ratio of 2 : 1, followed by the addition of 50 μl of 1850 MBq QMA-purified 18F⁻ ions. The mixture was reacted at 100°C for 10-15 min. Purification was performed using an HLB cartridge. The cartridge was rinsed with ethanol/water (1 : 1), and the eluate was diluted with physiological saline until the ethanol content was below 10%. The radiochemical purity was 92.37% as detected by radio-HPLC.

Example 43. Preparation of $Al^{18}F$-NOTA-09

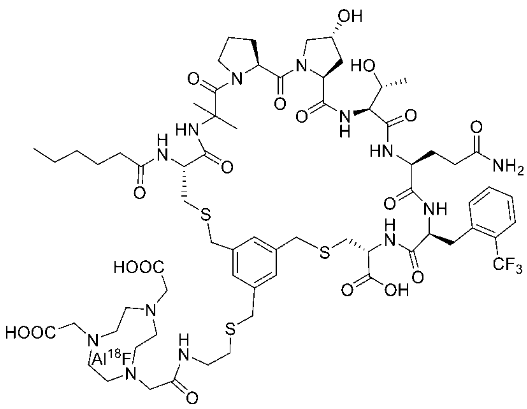

1 μl of a 10 mM aqueous solution of NOTA-09 was dissolved in 50-100 μl of 0.5 M acetic acid/sodium acetate buffer at pH 4.0-4.4. A 2 mM aqueous solution of $AlCl_3$ was then added in a molar ratio of 2 : 1, followed by the addition of 50 μl of 1850 MBq QMA-purified $18F^-$ ions. The mixture was reacted at 100°C for 10-15 min. Purification was performed using an HLB cartridge. The cartridge was rinsed with ethanol/water (1 : 1), and the eluate was diluted with physiological saline until the ethanol content was below 10%. The radiochemical purity was 91.57% as detected by radio-HPLC.

Example 44. Preparation of $Al^{18}F$-NOTA-14

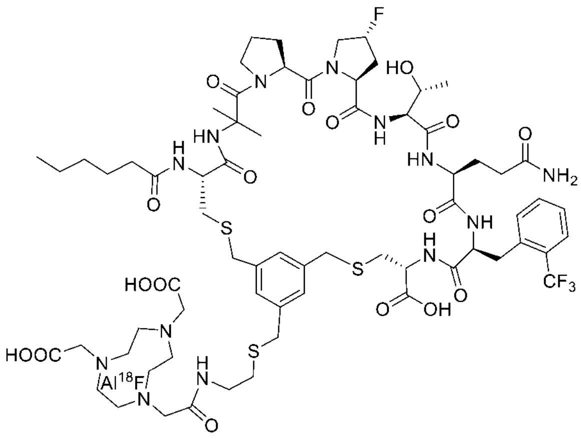

The target compound $Al^{18}F$-NOTA-14 was obtained in a manner similar to that in Example 42, radiochemical purity: 90.33%.

Example 45. Preparation of $Al^{18}F$-NOTA-15

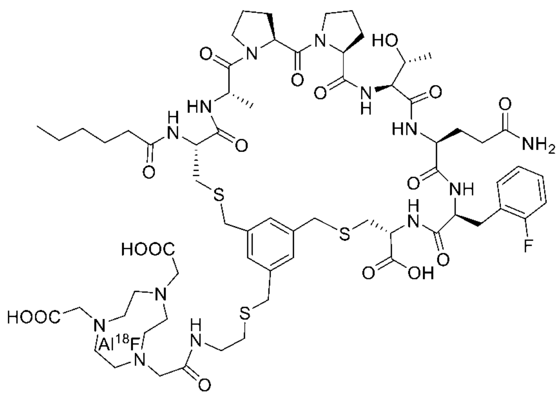

The target compound $Al^{18}F$-NOTA-15 was obtained in a manner similar to that in Example 42, radiochemical purity: 92.94%.

Example 46. Preparation of $Al^{18}F$-NOTA-16

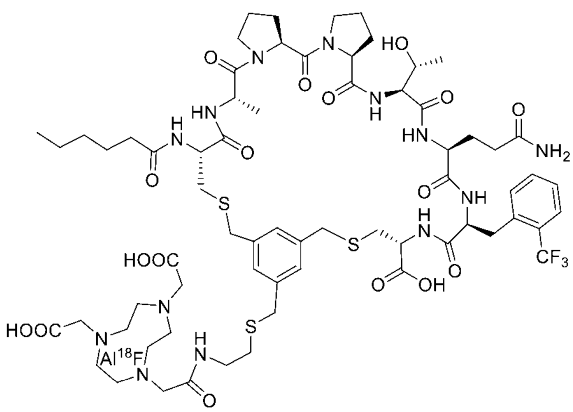

The target compound $Al^{18}F$-NOTA-16 was obtained in a manner similar to that in Example 42, radiochemical

purity: 91.60%.

Example 47. Preparation of $^{64}$Cu-DOTA-09

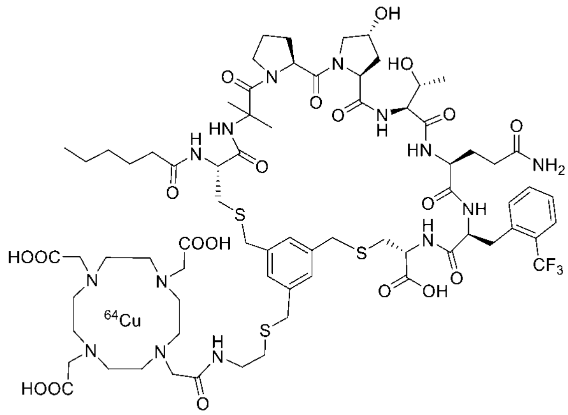

5 µl of a 10 mM aqueous solution of DOTA-09 was dissolved in 100 µl of sodium acetate solution at pH 3.5-6.5. Then, 50 µl of 1850 MBq $^{64}CuCl_2$ solution, which had been dissolved in 0.1 M hydrochloric acid, was added. The mixture was reacted at 80°C for 15 min. Purification was performed using a C18 cartridge, and the eluate was diluted with physiological saline until the ethanol content was below 10%. The radiochemical purity was 98.59% as detected by radio-HPLC.

Example 48. Preparation of $^{64}$Cu-NOTA-09

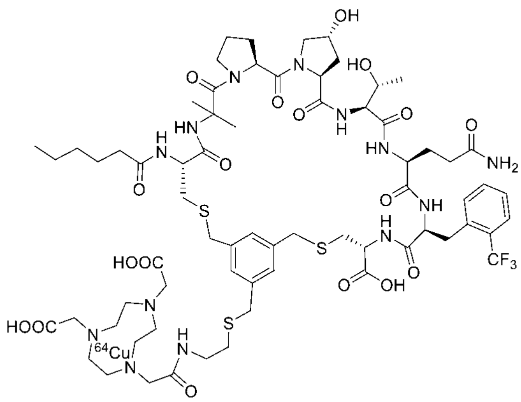

5 µl of a 10 mM aqueous solution of NOTA-09 was dissolved in 100 µl of sodium acetate solution at pH 3.5-6.5. Then, 50 µl of 1850 MBq $^{64}CuCl_2$ solution, which had been dissolved in 0.1 M hydrochloric acid, was added. The mixture was reacted at 80°C for 15 min. Purification was performed using a C18 cartridge, and the eluate was diluted with physiological saline until the ethanol content was below 10%. The radiochemical purity was 97.37% as detected by radio-HPLC.

Example 49. Preparation of $^{225}$Ac-DOTA-09

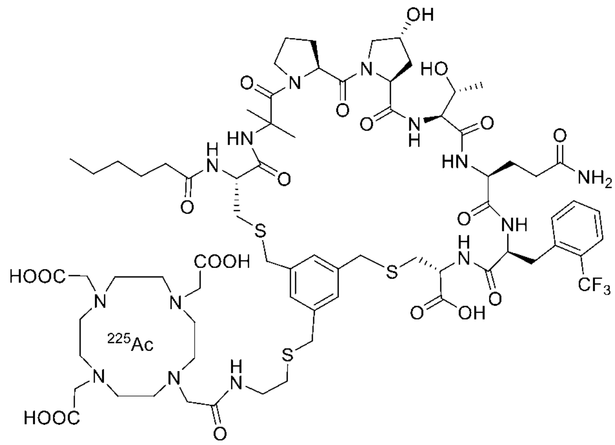

318 µl of tris(hydroxymethyl)aminomethane buffer (pH 8, 0.25 M), 5 µl of DOTA-09 solution (1 µmol/ml) and 20-50 µCi $^{225}$Ac nuclide were sequentially added to a reaction vessel, and uniformly mixed. An appropriate amount of 0.1 N hydrochloric acid was added to adjust the pH value of the reaction system to 6.0-8.0, and the reaction system was heated at 90°C-95°C for 10-20 min. After the heating was completed, the reaction solution was cooled to room temperature, and 0.65 ml of 10 mg/ml sodium ascorbate in physiological saline was added. The radiochemical purity was greater than 90% as detected by radio-HPLC.

Comparative Example 1. Preparation of NOTA-2286

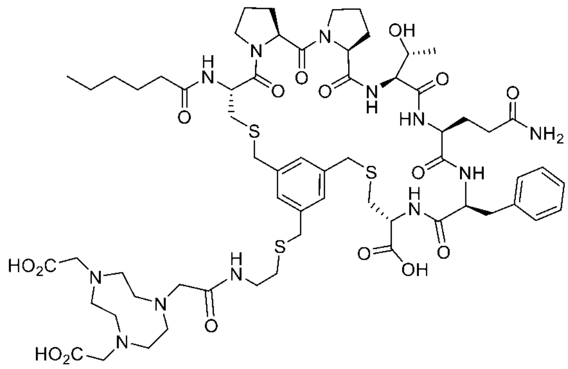

The target compound NOTA-2286 was obtained in a manner similar to that in Example 15, purity: 97.35%, MS m/z (ESI): 1367.78 [M-H]$^-$.

Comparative Example 2. Preparation of Al$^{18}$F-NOTA-2286

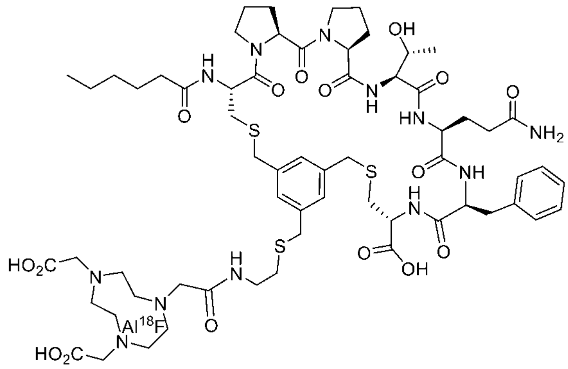

The target compound $Al^{18}F$-NOTA-2286 was obtained in a manner similar to that in Example 42, radiochemical purity: 95.37%.

Comparative Example 3. Preparation of $^{68}Ga$-NOTA-2286

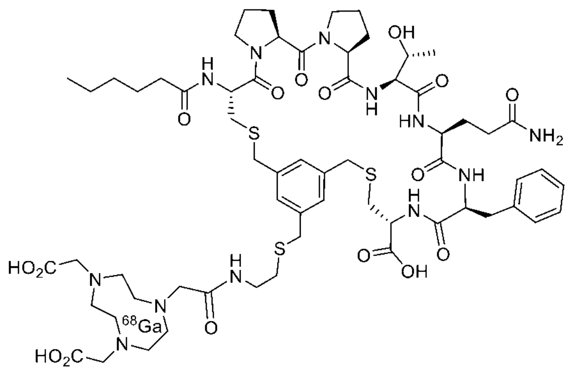

The target compound $^{68}Ga$-NOTA-2286 was obtained in a manner similar to that in Example 38, radiochemical purity: 96.29%.

Comparative Example 4. Preparation of $^{68}Ga$-FAP-2286

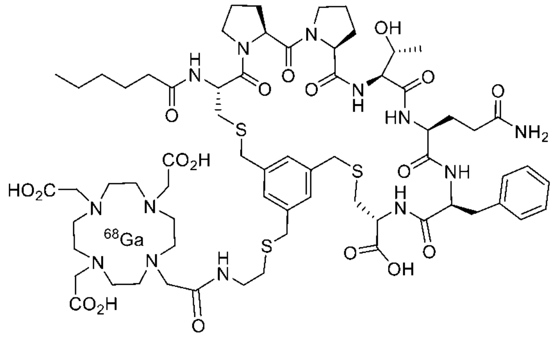

The target compound $^{68}Ga$-FAP-2286 was obtained in a manner similar to that in Example 26, radiochemical purity: 94.98%.

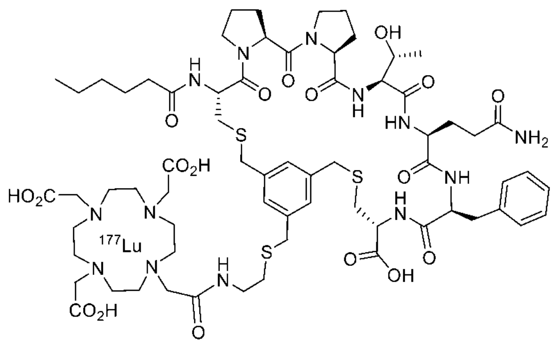

Comparative Example 5. Preparation of $^{177}Lu$-FAP-2286

The target compound $^{177}Lu$-FAP-2286 was obtained in a manner similar to that in Example 33, radiochemical purity: 97.76%.

## Biological evaluation

The present disclosure will be further described and explained below in conjunction with test examples, but these examples are not intended to limit the scope of the present disclosure.

Test Example 1. FAPα enzyme activity test

1.1 Experimental materials and instruments

Table 1. Source information of experimental materials and instruments

| Experimental materials | Source |
|---|---|
| FAPα protein | TANJIN HENGRUI MEDICINE CO., LTD. |
| Z-Gly-Pro-AMC | GLPBIO, GA23817 |
| PBS | Nanjing SenBeiJia Biological Technology Co., Ltd., BC-BPBS-01 |
| DMSO | sigma, D5879-500ML |
| Microplate | 675076 |
| Microplate reader | TECAN, Spark |

1.2 Experimental steps

The substrate (Z-Gly-Pro-AMC) was diluted with DMSO to prepare a 0.5 mM stock solution. Before each experiment, the 0.5 mM stock solution was diluted with PBS to 50 μM for later use. The FAPα protein was diluted with PBS to 0.5 ng/μl for later use. The test compounds and positive control were diluted with PBS to a concentration of 100 nM or 200 nM for the determination of single-point inhibition rate. For compounds with a single-point inhibition rate comparable to that of the positive compound, further determination of $IC_{50}$ values was performed. The test compounds and positive control were subjected to a 5-fold serial dilution with PBS to obtain 8 gradients for later use, with the highest concentration being 10 μM, and the lowest concentration being 0 nM. Test method: 85 μl of the FAPα dilution and 10 μl of the compound dilution were added to a microplate, uniformly mixed and incubated at 37°C for 10 min. Then, 5 μl of 50 μM substrate was added, uniformly mixed and incubated at 37°C for 10 min. The microplate was read on a microplate reader, with an excitation wavelength of 380 nm and an emission wavelength of 465 nm.

1.3 Experimental results

Table 2. $IC_{50}$ values of the compounds of the present disclosure against FAPα

| Compound No. | $IC_{50}$ (nM) | Compound No. | $IC_{50}$ (nM) |
|---|---|---|---|
| DOTA-01 | 13.91 | NOTA-04 | 0.47 |
| DOTA-02 | 10.31 | NOTA-05 | 0.45 |
| DOTA03 | 4.05 | NOTA-06 | 0.088 |
| DOTA-04 | 1.84 | NOTA-07 | 0.086 |
| DOTA-05 | 1.16 | NOTA-08 | 0.59 |
| DOTA-06 | 0.93 | NOTA-09 | 2.75 |
| DOTA-07 | 1.50 | NOTA-10 | / |
| DOTA-08 | 0.53 | NOTA-11 | / |
| DOTA-09 | 0.28 | NOTA-12 | 2.70 |
| DOTA-10 | 11.20 | NOTA-13 | 2.73 |

(continued)

| Compound No. | $IC_{50}$ (nM) | Compound No. | $IC_{50}$ (nM) |
|---|---|---|---|
| DOTA-11 | 4.04 | NOTA-14 | 3.40 |
| DOTA-12 | 1.08 | NOTA-15 | 2.72 |
| DOTA-13 | 1.09 | NOTA-16 | 3.59 |
| DOTA-14 | 0.53 | NOTA-2286 (Comparative Example 1) | 0.59 |
| FAP-2286 (positive) | 11.68 | | |
| Note: The structure of FAP-2286 is shown as follows: References: European Journal of Nuclear Medicine and Molecular Imaging (2022) 49:3651-3667 | | | |

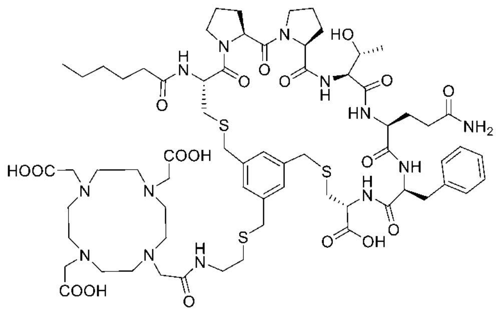

.

Test Example 2. PET/SPECT imaging test of labelled compounds

2.1 Experimental materials

Cell information: U-87 MG cells (Wuhan Pricella Biotechnology Co., Ltd.); Culture conditions: U-87 MG cell specific medium (MEM + 10% FBS + 1% P/S); Number of passages: 6-9.

Experimental animals: Germline: B-NDG (Biocytogen Jiangsu Co., Ltd.); Age: 5-8 weeks old; Body weight: 20-24 g.

Reagent: PBS (Solarbio, P1020); Matrigel (ABW, 0827045); Trypsin-EDTA (Gibco, 25200-072); U-87 MG specific medium (Pricella, CM-0238).

Instrument: Small animal PET/CT (ediso, nanoScan PET/CT 4heads); Activity meter (Capintec, activity meter); Electronic balance (Changzhou Shuangjie, DT100).

2.2 Experimental steps

Model construction

A sufficient quantity of U-87 MG cells was prepared and inoculated into the posterior position of the right forelimb of B-NDG mice in an inoculation volume of 100 μl, containing 50% Matrigel and $5 \times 10^6$ cells. After inoculation, the tumour volume and animal body weight were monitored twice a week. Model mice with a tumour volume between 200 and 500 $m^3$ were selected for enrolment in this experiment and randomly grouped on the basis of tumour volume.

Operation steps

1) The workbench surface was wiped with 75% medical alcohol and covered with a disposable sterile tablecloth.
2) 0.5 mL insulin syringes, alcohol swabs, cotton swabs and a marking pen were prepared in the injection room; and the mice were placed in a mouse restrainer, and the tails of the mice were disinfected with alcohol swabs.
3) The prepared test sample was injected into each mouse via the tail vein, with the time of each injection, the activity of the syringe and the activity of the empty syringe recorded.
4) Tumour-bearing mice were anesthetized with isoflurane, and then placed in a prone position on small animal PET/SPECT beds and fixed;

5) After administration, static PET/SPECT image acquisition was performed at different time points; prior to each static scan, a whole-body CT scan was performed to obtain whole-body distribution images of the labelled compound in the tumour-bearing mice, thereby obtaining PET/SPECT images for each experimental animal at different time points after administration; and major organs were selected and delineated, specifically including: parts such as tumour, muscle, bone, lung, brain, liver and kidney.

6) The radioactive accumulation and clearance of the labelled compounds in tumours and non-target tissues of tumour-bearing mice were observed.

2.3 Experimental results

Table 3. Uptake in tumours and various tissues and tumour/kidney ratios of the compounds of the present disclosure and $^{68}$Ga-FAP-2286

| Compound | $^{68}$Ga-FAP-2286 () | | $^{68}$Ga-DOTA-05 | | $^{68}$Ga-DOTA-06 | |
|---|---|---|---|---|---|---|
| **Time** | **1 h** | **3 h** | **1 h** | **3 h** | **1 h** | **3 h** |
| **Tumour** | 10.99±0.90 | 4.96±0.87 | 9.44±1.39 | 6.72±1.44 | 17.43±0.88 | 13.53±1.89 |
| **Kidney** | 7.04±0.42 | 5.67±0.11 | 4.06±0.56 | 2.52±0.22 | 3.66±0.32 | 2.14±0.18 |
| **Liver** | 1.14±0.26 | 0.30±0.09 | 0.81±0.13 | 0.42±0.08 | 1.48±0.01 | 0.77±0.06 |
| **Brain** | 0.32 ± 0.09 | 0.11±0.06 | 0.50±0.08 | 0.26±0.03 | 1.14±0.12 | 0.60±0.04 |
| **Lung** | 0.90±0.14 | 0.26±0.05 | 0.91±0.21 | 0.39±0.13 | 1.84±0.05 | 0.93±0.21 |
| **Muscle** | 0.22±0.06 | 0.05±0.01 | 0.48±0.21 | 0.14±0.07 | 1.94±0.60 | 0.89±0.10 |
| **Joint** | 0.94±0.20 | 0.29±0.07 | 1.40±0.70 | 0.58±0.10 | 3.70±0.65 | 1.95±0.79 |
| **Gallbladder** | 1.26 ± 0.17 | 0.38±0.09 | 0.89±0.14 | 0.41±0.10 | 1.46±0.08 | 0.82±0.18 |
| **Tumour/kidne y** | 1.56±0.11 | 0.87±0.14 | 2.37±0.62 | 2.65±0.34 | 4.78±0.18 | 6.31±0.34 |
| $^{68}$Ga-DOTA-05 and the positive control $^{68}$Ga-FAP-2286 exhibited comparable tumour uptake in model mice, while $^{68}$Ga-DOTA-06 showed significantly higher tumour uptake than that of $^{68}$Ga-FAP-2286. $^{68}$Ga-DOTA-05 and $^{68}$Ga-DOTA-06 showed lower kidney uptake and higher tumour/kidney uptake ratios (FIG. 1). Moreover, with the increase of time, the tumour/kidney uptake ratios of $^{68}$Ga-DOTA-05 and $^{68}$Ga-DOTA-06 increased, whereas the tumour/kidney uptake ratio of $^{68}$Ga-FAP-2286 decreased (FIG. 2). | | | | | | |

Table 4. Tumour uptake and tumour/kidney ratios of the compounds of the present disclosure and $^{68}$Ga-FAP-2286

| Tissue | Tumour | | Tumour/kidney | |
|---|---|---|---|---|
| **Time** | 1 h | 3h | 1h | 3h |
| **$^{68}$Ga-FAP-2286** | 10.99±0.90 | 4.96±0.87 | 1.56±0.11 | 0.87±0.14 |
| **$^{68}$Ga-DOTA-05** | 9.44±1.39 | 6.72±1.44 | 2.37±0.62 | 2.65±0.34 |
| **$^{68}$Ga-DOTA-06** | 17.43±0.88 | 13.53±1.89 | 4.78±0.18 | 6.31±0.34 |
| **$^{68}$Ga-DOTA-07** | 14.92±5.18 | 10.82±3.04 | 4.36±0.85 | 6.73±0.65 |
| **$^{68}$Ga-DOTA-08** | 19.22±0.92 | 14.29±1.47 | 4.75±0.16 | 7.32±0.92 |
| **$^{68}$Ga-DOTA-09** | / | 12.44±2.78 | / | 3.79±1.05 |
| **$^{68}$Ga-DOTA-12** | 13.91±3.64 | 8.74±2.59 | 3.74±0.58 | 4.72±0.48 |
| **$^{68}$Ga-DOTA-13** | / | 12.15±2.59 | / | 8.97±3.15 |

In the mouse model, the tumour uptake (FIG. 3) and tumour/kidney ratios (FIG. 4) of $^{68}$Ga-DOTA-06, $^{68}$Ga-DOTA-07, $^{68}$Ga-DOTA-08, $^{68}$Ga-DOTA-09, $^{68}$Ga-DOTA-12 and $^{68}$Ga-DOTA-13 were significantly higher than those of the control $^{68}$Ga-FAP-2286.

Table 5. Tumour uptake and tumour/kidney ratios of the compounds of the present disclosure and $^{177}$Lu-FAP-2286

| | Time | $^{177}$Lu-FAP-2286 | $^{177}$Lu-DOTA-06 | $^{177}$Lu-DOTA-09 | $^{177}$Lu-DOTA-13 | $^{177}$Lu-DOTA-14 |
|---|---|---|---|---|---|---|
| **Tumour** | 0.5 h | 29.84±3.52 | 27.50±3.74 | 29.52±2.98 | 26.57±3.35 | 25.61±3.20 |
| | 1.5 h | 28.49±3.49 | 28.71±2.44 | 30.88±3.14 | 26.96±1.84 | 26.71±2.44 |
| | 4.0 h | 23.66±3.01 | 27.77±4.60 | 31.09±3.88 | 26.03±3.98 | 28.74±2.76 |
| | 24h | 6.26±1.10 | 21.62±2.63 | 30.46±1.99 | 22.23±1.53 | 23.32±1.20 |
| | 48h | 6.40±1.24 | 14.11±2.03 | 24.10±2.21 | 12.58±1.42 | 19.73±1.72 |
| | 72h | 6.02±1.56 | 11.52±1.31 | 20.59±1.91 | 9.90±1.40 | 15.69±1.98 |
| | 168h | 6.30±2.32 | 9.16±1.49 | 12.81±0.90 | 8.46±0.98 | 10.40±2.25 |
| **Tumour/ kidney** | 0.5 h | 3.29±0.78 | 7.30±1.05 | 6.08±1.62 | 8.01±1.82 | 4.88±1.29 |
| | 1.5 h | 6.53±1.12 | 10.97±2.66 | 8.66±0.73 | 12.66±1.91 | 7.96±2.25 |
| | 4.0 h | 9.82±4.79 | 11.42±1.75 | 11.79±2.62 | 13.93±4.74 | 10.09±1.62 |
| | 24h | 2.75±0.90 | 15.45±3.12 | 19.38±5.84 | 15.46±3.17 | 15.89±3.71 |

In the mouse model, $^{177}$Lu-DOTA-09 and $^{177}$Lu-DOTA-14 decreased slowly in the tumour uptake, maintaining relatively stable intratumoral retention, whereas $^{177}$Lu-FAP-2286 showed a rapid decrease in intratumoral retention after 4 hours (FIG. 5). The tumour/kidney ratios (FIG. 6) of $^{177}$Lu-DOTA-09 and $^{177}$Lu-DOTA-14 were higher than that of the control $^{177}$Lu-FAP-2286.

Table 6. Tumour uptake and tumour/kidney ratios of the compounds of the present disclosure and $^{68}$Ga-NOTA-2286

| **Tissue** | **Tumour** | | | **Tumour/kidney** | | |
|---|---|---|---|---|---|---|
| **Time** | 0.5h | 1.5h | 3.0h | 0.5h | 1.5h | 3.0h |
| **$^{68}$Ga-NOTA-2286** | 6.27±1.03 | 4.37±1.03 | 2.85±0.83 | 1.59±0.55 | 1.77±0.31 | 2.43±1.04 |
| **$^{68}$Ga-NOTA-05** | 18.15±1.35 | 17.14±1.59 | 16.38±1.00 | 5.78±1.05 | 9.50±1.83 | 11.83±1.60 |
| **$^{68}$Ga-NOTA-06** | 24.98±6.33 | 23.33±4.71 | 21.71±4.88 | 6.81±2.29 | 9.79±3.33 | 18.51±10.73 |
| **$^{68}$Ga-NOTA-07** | 23.77±2.69 | 19.85±2.15 | 16.80±3.31 | 5.64±1.54 | 13.09±6.4 6 | 8.24±2.44 |
| **$^{68}$Ga-DOTA-09** | 15.32±1.14 | 15.09±0.53 | 13.24±1.21 | 2.81±0.21 | 5.34±0.92 | 10.17±2.53 |

In the mouse model, the tumour uptake (FIG. 7) and tumour/kidney ratios (FIG. 8) of $^{68}$Ga-NOTA-05, $^{68}$Ga-NOTA-06, $^{68}$Ga-NOTA-07 and $^{68}$Ga-DOTA-09 were significantly higher than those of the control $^{68}$Ga-NOTA-2286.

Table 7. Tumour uptake and tumour/kidney ratios of the compounds of the present disclosure and Al$^{18}$F-NOTA-2286

| **Tissue** | **Tumour** | | | **Tumour/kidney** | | |
|---|---|---|---|---|---|---|
| **Time** | 0.5h | 1.5h | 3.0h | 0.5h | 1.5h | 3.0h |
| **Al$^{18}$F-NOTA-2286** | 33.27±0.64 | 22.34±3.00 | 11.32±3.02 | 2.57±0.09 | 11.24±1.79 | 10.06±1.60 |
| **Al$^{18}$F-NOTA-09** | 48.94±4.56 | 38.13±3.50 | 28.72±3.12 | 7.06±0.80 | 16.97±1.74 | 20.65±5.55 |
| **Al$^{18}$F-NOTA-14** | 43.75±9.01 | 44.75±7.53 | 37.05±8.03 | 6.75±0.65 | 18.78±0.20 | 38.95±16.30 |
| **Al$^{18}$F-** | 26.50±6.07 | 23.11±7.02 | 22.82±4.71 | 6.59±0.72 | 9.84±3.70 | 15.03±3.02 |
| **NOTA-16** | | | | | | |

In the mouse model, the tumour uptake (FIG. 9) and tumour/kidney ratios (FIG. 10) of Al$^{18}$F-NOTA-09 and Al$^{18}$F-NOTA-14 were significantly higher than those of the control Al$^{18}$F-NOTA-2286.

Test Example 3. Plasma concentration experiment of $^{177}$Lu-labelled compound

3.1 Experimental materials

Experimental animals: Germline: SD rat (Beijing Vital River Laboratory Animal Technology Co., Ltd.); Age: 5-6 weeks old; Body weight: 100-150 g.
Reagent: Sodium Chloride Injection (SHANDONG QIDU PHARMACEUTICAL CO., LTD.).
Instrument: Activity meter (Capintec); Analytical balance with 0.1 mg readability (Sartorius Scientific Instruments Co., Ltd.); Low-energy γ-counter (PerkinElmer Instruments Co., Ltd, USA).

3.2 Experimental steps

1) Empty tubes for γ-counting were weighed and labelled in advance.
2) After the test sample was injected into the tail vein of rats, animal blood samples were collected from the orbit at 2 min, 5 min, 15 min, 30 min, 1 h, 2 h, 4 h, 6 h, 24 h, 48 h and 72 h. The sample tubes containing blood were weighed.
3) The net weight of the blood was calculated, and the radioactive count of the collected blood was determined using a γ-counter.
4) The distribution of the labelled compound in the blood of rats at different time points was determined. The test sample was accurately diluted 100-fold, and 0.5 ml of the dilution was added to a counting tube to serve as a 1% ID standard (i.e., one percent of the injected dose). The radioactive counts of both the 1% ID standard and the biological samples were simultaneously determined using the γ-counter.
5) The pharmacokinetic parameters were calculated using DAS software.

Data obtained from rat blood was expressed as the percentage of radioactive count per gram of blood relative to the total dose (radioactive count) (%ID/g). Specific calculation formula:

%ID/g = CPM organ or tissue/(CPM total administered dose × W organ or tissue) × 100%

Data for each sampling time point was expressed as mean ± standard deviation (mean±SD).
At the same time, the drug concentration in the blood was calculated on the basis of the radioactive count in the blood. Specific calculation formula:

$$\text{Plasma concentration}\ (\mu Ci/mL) = \frac{(CPM_{\text{Blood}}/CPM_{\text{Total administered dose}}) \times D\ (\mu Ci)_{\text{Administered dose}}}{W\ (g)\ \text{Blood}\ /1\ (g/mL)_{\text{Blood density}}}$$

3.3 Experimental results

Table 8. Pharmacokinetic parameters of the compounds of the present disclosure and $^{177}$Lu-FAP-2286

| Pharmacokine tic parameter | Unit | $^{177}$Lu-FAP-2286 | $^{177}$Lu-DOTA-09 | $^{177}$Lu-DOTA-06 | $^{177}$Lu-DOTA-13 | $^{177}$Lu-DOTA-14 |
|---|---|---|---|---|---|---|
| $t_{1/2}$ | h | 1.29±0.43 | 9.12±4.69 | 5.43±3.22 | 7.43±5.58 | 10.29±6.22 |
| $C_{max}$ | %ID/g | 0.73±0.27 | 0.89±0.21 | 1.42±0.18 | 1.42±0.18 | 0.61±0.06 |
| $AUC_{last}$ | h*%ID/g | 0.96±0.14 | 5.04±0.94 | 7.31±0.86 | 7.31±0.86 | 3.45±0.55 |
| $AUC_{INF_obs}$ | h*%ID/g | 0.97±0.14 | 5.28±1.07 | 7.53±0.85 | 7.53±0.85 | 3.67±0.61 |
| Vz_obs | μCi/%ID/g | 1.50±0.97 | 2.21±0.38 | 1.49±0.30 | 1.49±0.30 | 3.71±0.55 |
| Cl_obs | μCi/h*%ID/g | 0.53±0.08 | 0.10±0.02 | 0.07±0.01 | 0.07±0.01 | 0.14±0.03 |
| $MRT_{last}$ | h | 2.52±0.34 | 15.49±0.36 | 14.19±0.40 | 14.19±0.40 | 16.77±0.59 |

*In vivo* pharmacokinetic experiments showed that the half-life ($t_{1/2}$) and mean residence time (MRTlast) of $^{177}$Lu-DOTA-09, $^{177}$Lu-DOTA-06, $^{177}$Lu-DOTA-13, $^{177}$Lu-DOTA-14 in rats were significantly longer than those of $^{177}$Lu-FAP-2286, which was consistent with the longer intratumoral residence time observed in mouse imaging experiments.

Test Example 4. *In vivo* efficacy experiment of the compounds of the present disclosure in U-87 MG model mice

4.1. Cell information

Name: U-87 MG cells
Source: Wuhan Pricella Biotechnology Co., Ltd.
Cat. No.: CL-0238
Batch No.: YBMIL8BQH0
Culture conditions: U-87 MG cell specific medium (90% MEM + 10% FBS + 1% P/S)
Number of passages: 23-36

4.2. Experimental animals

Germline: B-NDG
Grade: SPF grade
Age: 5 weeks old
Body weight: 21-24 g
Gender and number: Male; 90 mice were introduced for U-87 MG model construction, among which 40 were selected for study enrolment.
Source: Biocytogen Jiangsu Co., Ltd.
Production license No.: SCXK (Jiangsu) 2021-0005.
Certificate No.: B202311200038

4.3. Experimental drug

Name: $^{177}$Lu-DOTA-09 (see Example 35 for preparation method)
Specification and composition: 31 mCi/2.5 mL/37.5 nmol
$^{177}$Lu-FAP-2286 (refer to Comparative Example 5)
Specification and composition: 15.87 mCi/1.25 mL/18.75 nmol

4.4. Instrument device information

Table 9. Instrument device information

| Device name | Model | No. | Manufacturer |
|---|---|---|---|
| Activity meter | CRC-55tR | IEB-008-02 | Capintec |
| Electronic balance | DT100 | IEB-003-01 | Changzhou Shuangjie |
| Electronic balance | DT100 | IEB-003-02 | Changzhou Shuangjie |
| Digital calliper | LR44 | IEB-012-01 | Shanghai Measuring & Cutting Tool Works Co., Ltd. |

4.5. Reagent information

Table 10. Reagent information

| Reagent name | Specification | Batch No. | Cat. No. | Storage condition | Manufacturer |
|---|---|---|---|---|---|
| PBS | 500 mL | BC20230406 | BC-BPBS-01 | Room temperature | Bio-Channel |
| Matrigel | 5 mL | 20223127PHH | 082704 | 2°C-8°C | ABW |
| Trypsin-EDTA | 500 mL | 2522739 | 25200-072 | 2°C-8°C | Gibco |
| U-87 MG cell specific medium | 125 mL | WH3723K018 | CM-0238 | 2°C-8°C | Procell |

4.6. Experimental design

A suspension containing about $4.5 \times 10^8$ U-87 MG cells was prepared and inoculated into the posterior position of the right forelimb of B-NDG mice in an inoculation volume of 100 μL, containing 50% Matrigel and $5 \times 10^6$ cells. A total of 90 mice were inoculated. When the tumour in the mice grew to about 150 $cm^3$, 40 mice with uniform tumour sizes were selected for study enrolment.

The administration regimen was as follows:

Route of administration: tail vein injection;
Administration volume: 0.03-0.07 mL/animal;
Administration frequency and dose: The control group was given PBS, the $^{177}$Lu-FAP-2286 group was given a single dose of 0.8 mCi/animal, the $^{177}$Lu-DOTA-09 0.4mCi group was given a single dose of 0.4 mCi/animal, the $^{177}$Lu-DOTA-09 0.8 mCi group was given a single dose of 0.8 mCi/animal, the $^{177}$Lu-DOTA-09 0.8mCi×2 group was given two doses of 0.8 mCi/animal, with an administration interval of 35 days. Each group included eight animals. The administration time and administration activity of each mouse were recorded in detail.

4.7. Operation steps

After tumour modelling, the tumour size was observed. On the day before administration or the day of administration, the tumour volume was measured using a digital calliper, and the animal body weight was recorded. The animals were evenly divided into five groups on the basis of tumour volume, as follows: control group, $^{177}$Lu-FAP-2286 0.8 mCi group, $^{177}$Lu-DOTA-09 0.4 mCi group, $^{177}$Lu-DOTA-09 0.8 mCi group, and $^{177}$Lu-DOTA-09 0.8 mCi*2 group, with eight animals in each group.

On the day of administration, the test sample was taken for preparation (the stock solution of the test sample was directly used), and the activity of the prepared solution was measured. The workbench surface was wiped with 75% medical alcohol and covered with a disposable sterile tablecloth. 0.5 mL insulin syringes, alcohol swabs, cotton swabs and a marking pen were prepared in the injection room. The mice were placed in a mouse restrainer, and the tails of the mice were disinfected with alcohol swabs. The prepared test sample was injected into each mouse via the tail vein, with the time of each injection recorded.

From the second day after administration, the stage of tumour and body weight measurement began. The tumour and body weight measurement was performed at least twice a week. During each measurement, the long and short diameters of the tumour were recorded. The measurements were kept at the same location as possible. The abnormalities and deaths of the animals were recorded in time. The animals were euthanized when the mean tumour diameter exceeded 20 mm, or when the tumours exhibited ulceration or necrosis. The tumour volume was calculated as follows: tumour volume ($mm^3$) = long diameter × short diameter × short diameter × 0.52.

4.8. Experimental results and discussion

**Tumour growth curve**

The experimental results showed that different doses of $^{177}$Lu-DOTA-09 exhibited certain tumour growth inhibitory effect in U-87 MG model mice (FIG. 11).

The U-87 MG tumour grew rapidly. On day 20 after administration, the tumour size in the control group reached 3057.58 ± 715.73 $mm^3$, the tumour size in the $^{177}$Lu-FAP-2286 0.8 mCi group was 1833.89 ± 338.00 $mm^3$, the tumour size in the $^{177}$Lu-DOTA-09 0.4 mCi group was 790.09 ± 618.40 $mm^3$, and the tumour sizes in the $^{177}$Lu-DOTA-09 0.8 mCi group and the $^{177}$Lu-DOTA-09 0.8 mCi*2 group were 314.25 ± 65.57 $mm^3$ and 326.04 ± 82.5 $mm^3$, respectively. On day 20 after administration, the tumour inhibition rates in the four administration groups were 40.02%, 74.16%, 89.72% and 89.34%, respectively.

On day 35 after the first administration, the $^{177}$Lu-DOTA-09 0.8 mCi*2 group was subjected to a second administration, which continued to inhibit tumour growth.

On day 46 after administration, the tumour size in the $^{177}$Lu-DOTA-09 0.8 mCi group was 3605.05 $\pm$ 803.40 mm$^3$, and the tumour size in the $^{177}$Lu-DOTA-09 0.8 mCi*2 group was 1735 $\pm$ 482.69 mm$^3$.

**Mouse body weight**

During the administration of $^{177}$Lu-DOTA-09, the body weights of the mice were monitored simultaneously. No significant body weight changes were observed in mice across all four dose groups, indicating that $^{177}$Lu-DOTA-09 did not cause toxic side effects leading to weight loss in the tumour-bearing animals (FIG. 12).

**Mouse survival time**

Statistical analysis showed that $^{177}$Lu-DOTA-09 significantly improved the survival rate of U-87 MG model mice (FIG. 13). In the control group, excessive tumour burden appeared from day 20 after administration, and all mice died due to the tumour burden by day 42, with a median survival of 35 days, while in the $^{177}$Lu-DOTA-09 0.8 mCi*2 group, about half of the mice remained alive by day 59 after administration, indicating that $^{177}$Lu-DOTA-09 showed a significant improvement in the survival of tumour-bearing mice. Statistical analysis showed that the median survival times of the control group, the $^{177}$Lu-FAP-2286 0.8 mCi group, the $^{177}$Lu-DOTA-09 0.4 mCi group, the $^{177}$Lu-DOTA-09 0.8 mCi group and the $^{177}$Lu-DOTA-09 0.8 mCi*2 group were 35 days, 39 days, 42 days, 49 days and 57.5 days, respectively.

**Experimental conclusion**

$^{177}$Lu-DOTA-09, as a $^{177}$Lu-labelled radioactive targeted drug, can significantly inhibit the growth of FAP-positive tumours in U-87 MG model mice and prolong the survival time of the mice.

## Claims

1. A compound as shown in formula (I) or a pharmaceutically acceptable salt thereof,

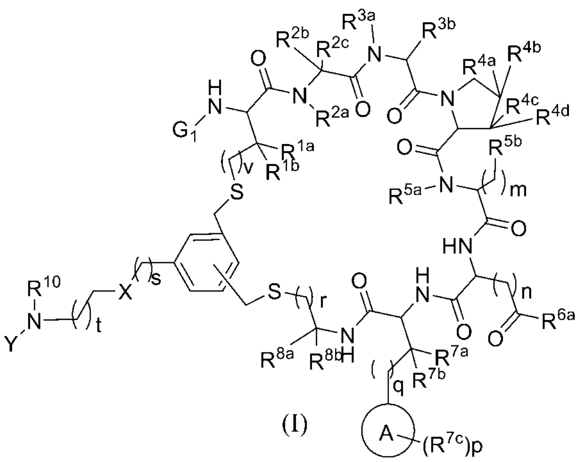

(I)

**characterised in that** the $R^{1a}$ and $R^{1b}$ are each independently selected from hydrogen or methyl;
the v is selected from 0 or 1;
the $G_1$ is selected from -(CO)-L-$R^{1c}$, the L is selected from $C_{3-20}$ alkylene, the $R^{1c}$ is selected from methyl or -NH-(C=NH)-$NR^{1d}R^{1f}$, and the $R^{1d}$ and $R^{1f}$ are each independently selected from hydrogen or methyl; or the $G_1$ is selected from

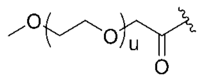,

and the u is selected from an integer from 1 to 5;

the $R^{2a}$, $R^{2b}$ and $R^{2c}$ are each independently selected from hydrogen or $C_{1-3}$ alkyl;
the $R^{3a}$ and $R^{3b}$ are each independently selected from hydrogen and $C_{1-3}$ alkyl, or the $R^{3a}$ and $R^{3b}$ together with the atom to which each is attached form 5- to 6-membered nitrogen-containing heterocycloalkyl, the $C_{1-3}$ alkyl or 5- to 6-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl;
the $R^{4a}$, $R^{4b}$, $R^{4c}$ and $R^{4d}$ are each independently selected from hydrogen, hydroxyl, amino, halogen, $C_{1-3}$ alkyl or $C_{1-3}$ haloalkyl;
the $R^{5a}$ is selected from hydrogen or methyl;
the $R^{5b}$ is selected from $C_{1-6}$ alkyl, 6- to 10-membered aryl or 5- to 6-membered heteroaryl, the $C_{1-6}$ alkyl, 6- to 10-membered aryl or 5- to 6-membered heteroaryl is optionally substituted with 1 or 2 $R^B$, and the $R^B$ groups are the same or different, and are selected from hydroxyl, amino, halogen and $-CONH_2$;
the m is selected from 1, 2 or 3; the hydrogen on $CH_2$ is optionally substituted with one or more groups selected from hydroxyl, halogen, $C_{1-3}$ alkyl, 6- to 10-membered aryl or 5- to 6-membered heteroaryl;
the $R^{6a}$ is selected from hydroxyl or amino;
the n is selected from 1, 2 or 3;
the $R^{7a}$ and $R^{7b}$ are each independently selected from hydrogen and $C_{1-3}$ alkyl;
the q is selected from 0 or 1;
the ring A is selected from phenyl, pyridyl, naphthyl or thienyl;
each $R^{7c}$ is independently selected from halogen, nitro, amino, cyano, hydroxyl, $C_{1-6}$ alkyl, $C_{1-6}$ alkoxy, hydroxy $C_{1-6}$ alkyl, trifluoromethyl, difluoromethyl and trifluoroethyl;
the p is selected from 0, 1, 2 or 3;
the $R^{8a}$ and $R^{8b}$ are each independently selected from hydrogen, -COOH, - $CONH_2$, $CH_2$-OH, $-CONHR^{8c}$ and $-(CO)-(NR^{8c})-R^{8d}$, and the $R^{8c}$ and $R^{8d}$ are each independently selected from $C_{1-6}$ alkyl;
the r is selected from 0 or 1;
the s is selected from 0 or 1;
the X is selected from O, S or $NR^9$, and the $R^9$ is selected from hydrogen or $C_{1-6}$ alkyl;
the t is selected from 1, 2, 3, 4, 5 or 6;
the $R^{10}$ is selected from hydrogen or $C_{1-6}$ alkyl;
the Y is a chelating agent or hydrogen, and the chelating agent optionally complexes a nuclide; preferably, the Y is a chelating agent.

2. The compound as shown in formula (I) or the pharmaceutically acceptable salt thereof according to claim 1, **characterised in that** the compound is a compound as shown in formula (II) or a pharmaceutically acceptable salt thereof,

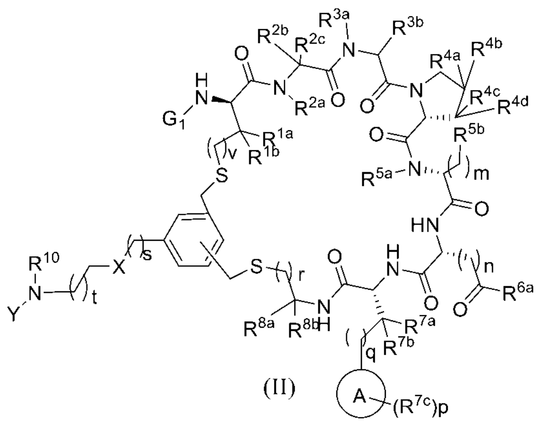

(II)

wherein the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t, $R^{10}$ and Y are as defined in claim 1.

3. The compound as shown in formula (I) or the pharmaceutically acceptable salt thereof according to claim 1 or 2, **characterised in that** the $R^{2a}$ is hydrogen or methyl, and the $R^{2b}$ and $R^{2c}$ are each independently selected from hydrogen or methyl; preferably, the $R^{2b}$ is hydrogen, the $R^{2c}$ is methyl, or the $R^{2b}$ and $R^{2c}$ are both methyl.

4. The compound as shown in formula (I) or the pharmaceutically acceptable salt thereof according to any one of claims 1

to 3, **characterised in that** the $R^{4a}$ and $R^{4b}$ are each independently selected from hydrogen and hydroxyl; preferably, the $R^{4a}$ is hydrogen, and the $R^{4b}$ is hydroxyl.

**5.** The compound as shown in formula (I) or the pharmaceutically acceptable salt thereof according to either claim 3 or claim 4, **characterised in that** the $R^{2a}$ is hydrogen or methyl; the $R^{2b}$ and $R^{2c}$ are each independently selected from hydrogen or methyl, with at least one being methyl; the $R^{4a}$ is hydrogen, and the $R^{4b}$ is hydroxyl; the $R^{4c}$ and $R^{4d}$ are each independently selected from hydrogen.

**6.** The compound as shown in formula (I) or the pharmaceutically acceptable salt thereof according to any one of claims 1 to 5, **characterised in that** the $R^{3a}$ and $R^{3b}$ are each independently selected from hydrogen and $C_{1-3}$ alkyl, the $C_{1-3}$ alkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl; preferably, the $R^{3a}$ is methyl, and the $R^{3b}$ is hydrogen.

**7.** The compound as shown in formula (I) or the pharmaceutically acceptable salt thereof according to any one of claims 1 to 5, **characterised in that** the $R^{3a}$ and $R^{3b}$ together with the atom to which each is attached form 5-membered nitrogen-containing heterocycloalkyl, the 5-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl; preferably, the 5-membered nitrogen-containing heterocycloalkyl is unsubstituted.

**8.** The compound as shown in formula (I) or the pharmaceutically acceptable salt thereof according to any one of claims 1 to 7, **characterised in that** the $R^{7a}$ and $R^{7b}$ are each independently selected from hydrogen and $C_{1-3}$ alkyl;

the q is selected from 0 or 1;
the ring A is selected from phenyl or naphthyl;
each $R^{7c}$ is independently selected from halogen, nitro, amino, cyano, hydroxyl, $C_{1-6}$ alkyl, $C_{1-6}$ alkoxy, hydroxy $C_{1-6}$ alkyl, trifluoromethyl, difluoromethyl and trifluoroethyl;
the p is selected from 0, 1, 2 or 3;
preferably,
the $R^{7a}$ and $R^{7b}$ are each independently selected from hydrogen;
the q is 0;
the ring A is selected from phenyl;
each $R^{7c}$ is independently selected from fluorine, chlorine, bromine and iodine, or trifluoromethyl, difluoromethyl and trifluoroethyl;
the p is selected from 0, 1 or 2.

**9.** The compound as shown in formula (I) or the pharmaceutically acceptable salt thereof according to any one of claims 1 to 8, **characterised in that** the $R^{1a}$ and $R^{1b}$ are each independently selected from hydrogen or methyl;

the v is selected from 0 or 1;
the $G_1$ is selected from -(CO)-L-$R^{1c}$, the L is selected from $C_{3-20}$ alkylene, and the $R^{1c}$ is selected from methyl;
preferably, the $R^{1a}$ and $R^{1b}$ are each independently selected from hydrogen;
the v is selected from 0;
the $G_1$ is selected from -(CO)-L-$R^{1c}$, the L is selected from $C_{4-14}$ alkylene, and the $R^{1c}$ is selected from methyl.

**10.** The compound as shown in formula (I) or the pharmaceutically acceptable salt thereof according to any one of claims 1 to 8, **characterised in that** the $R^{1a}$ and $R^{1b}$ are each independently selected from hydrogen or methyl;

the v is selected from 0 or 1;
the $G_1$ is selected from -(CO)-L-$R^{1c}$, the L is selected from $C_{3-20}$ alkylene, the $R^{1c}$ is selected from -NH-(C=NH)-$NR^{1d}$ $R^{1f}$, and the $R^{1d}$ and $R^{1f}$ are each independently selected from hydrogen or methyl;
preferably,
the $R^{1a}$ and $R^{1b}$ are each independently selected from hydrogen;
the v is selected from 0;
the $G_1$ is selected from -(CO)-L-$R^{1c}$, the L is selected from $C_{4-14}$ alkylene, the $R^{1c}$ is selected from -NH-(C=NH)-$NR^{1d}R^{1f}$; and the $R^{1d}$ and $R^{1f}$ are each independently selected from hydrogen or methyl.

**11.** The compound as shown in formula (I) or the pharmaceutically acceptable salt thereof according to any one of claims 1 to 8, **characterised in that** the $R^{1a}$ and $R^{1b}$ are each independently selected from hydrogen or methyl;

the v is selected from 0 or 1;
the $G_1$ is selected from

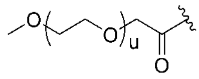

,

and the u is selected from an integer from 1 to 5;
preferably,
the $R^{1a}$ and $R^{1b}$ are each independently selected from hydrogen;
the $G_1$ is selected from

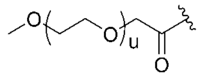

;

the v is selected from 0;
the u is selected from 1 or 2.

**12.** The compound as shown in formula (I) or the pharmaceutically acceptable salt thereof according to any one of claims 1 to 11, **characterised in that**

the $R^{5a}$ is selected from hydrogen;
the $R^{5b}$ is selected from $C_{1-6}$ alkyl, the $C_{1-6}$ alkyl is optionally substituted with 1 or 2 $R^B$, and the $R^B$ groups are the same or different, and are selected from hydroxyl or halogen;
the m is selected from 0 or 1; the hydrogen on $CH_2$ is optionally substituted with one or more groups selected from hydroxyl, halogen and $C_{1-3}$ alkyl;
the n is 2;
the $R^{6a}$ is amino;
the $R^{8a}$ and $R^{8b}$ are each independently selected from hydrogen, -COOH and - $CONH_2$, with at least one being -COOH or -$CONH_2$, and the r is 1.

**13.** The compound as shown in formula (I) or the pharmaceutically acceptable salt thereof according to claim 1, **characterised in that** the compound is a compound as shown in formula (III) or a pharmaceutically acceptable salt thereof,

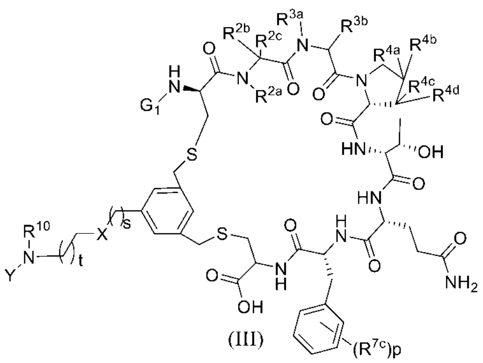

(III)

wherein the $R^{2a}$ is hydrogen or methyl;
the $R^{2b}$ and $R^{2c}$ are each independently selected from hydrogen or methyl, with at least one being methyl;
the $R^{3a}$ and $R^{3b}$ are each independently selected from hydrogen and $C_{1-3}$ alkyl, the $C_{1-3}$ alkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl; or the $R^{3a}$ and $R^{3b}$ together with the atom to which each is attached form 5-membered nitrogen-containing heterocycloalkyl, the 5-membered nitrogen-containing heterocycloalkyl is optionally substituted with 1, 2 or 3 $R^A$, and the $R^A$ groups are the same or different, and are selected from hydroxyl, amino, halogen, $C_{1-3}$ alkyl or $C_{1-3}$ haloalkyl;
the $R^{4a}$ is hydrogen, fluorine or trifluoromethyl, and the $R^{4b}$ is fluorine or hydroxyl;

the $R^{4c}$ and $R^{4d}$ are each independently selected from hydrogen;
each $R^{7c}$ is independently selected from fluorine, chlorine, bromine and iodine, or trifluoromethyl, difluoromethyl and trifluoroethyl;
the p is selected from 0, 1 or 2;
the $G_1$, s, X, t, $R^{10}$ and Y are as defined in claim 1.

**14.** The compound as shown in formula (I) or the pharmaceutically acceptable salt thereof according to claim 13, **characterised in that** the $R^{4a}$ is hydrogen, and the $R^{4b}$ is hydroxyl.

**15.** The compound as shown in formula (I) or the pharmaceutically acceptable salt thereof according to claim 14, **characterised in that** the $R^{3a}$ is methyl, and the $R^{3b}$ is hydrogen.

**16.** The compound as shown in formula (I) or the pharmaceutically acceptable salt thereof according to claim 14, **characterised in that** the $R^{3a}$ and $R^{3b}$ together with the atom to which each is attached form 5-membered nitrogen-containing heterocycloalkyl, and the 5-membered nitrogen-containing heterocycloalkyl is unsubstituted.

**17.** The compound as shown in formula (I) or the pharmaceutically acceptable salt thereof according to any one of claims 1 to 16, **characterised in that** the s is 1; X is S; $R^{10}$ is hydrogen.

**18.** The compound as shown in formula (I) or the pharmaceutically acceptable salt thereof according to 17, **characterised in that** the $G_1$ is selected from -(CO)-L-$R^{1c}$, the L is selected from $C_{4-14}$ alkylene, and the $R^{1c}$ is selected from methyl; preferably, the L is selected from $C_4$ alkylene, $C_6$ alkylene, $C_8$ alkylene, $C_{10}$ alkylene, $C_{12}$ alkylene or $C_{14}$ alkylene.

**19.** The compound as shown in formula (I) or the pharmaceutically acceptable salt thereof according to 17, **characterised in that** the $G_1$ is selected from -(CO)-L-$R^{1c}$, the L is selected from -(CO)-L-$R^{1c}$, the L is selected from $C_{4-14}$ alkylene, the $R^{1c}$ is selected from -NH(C=NH)-$NR^{1d}R^{1f}$, and the $R^{1d}$ and $R^{1f}$ are each independently selected from hydrogen or methyl; preferably, the L is selected from $C_4$ alkylene, $C_5$ alkylene or $C_6$ alkylene.

**20.** The compound as shown in formula (I) or the pharmaceutically acceptable salt thereof according to claim 17, **characterised in that** the $G_1$ is selected from

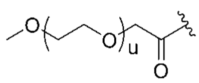,

and the u is selected from 1 or 2.

**21.** The compound as shown in formula (I) or the pharmaceutically acceptable salt thereof according to any one of claims 1 to 20, **characterised in that** the fragment attached to Y is selected from group 1:

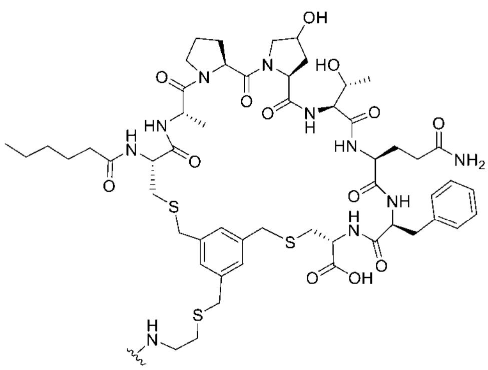, 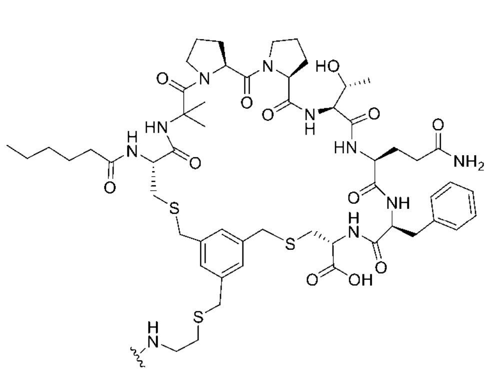,

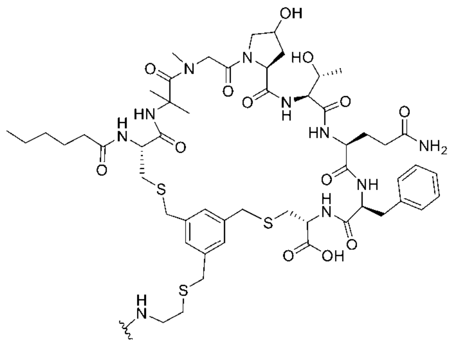
,
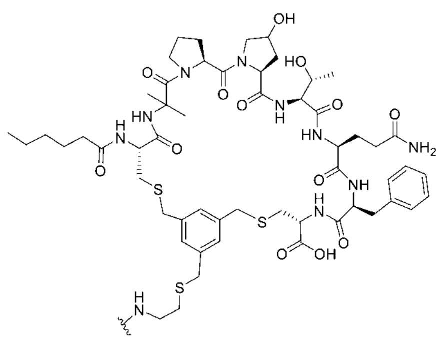
,
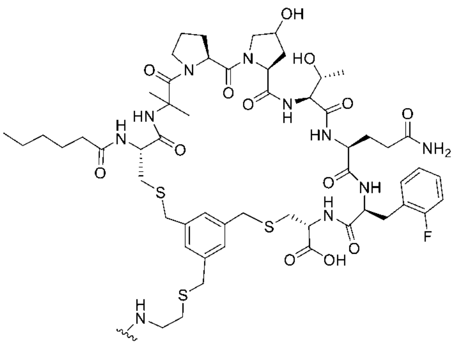
,
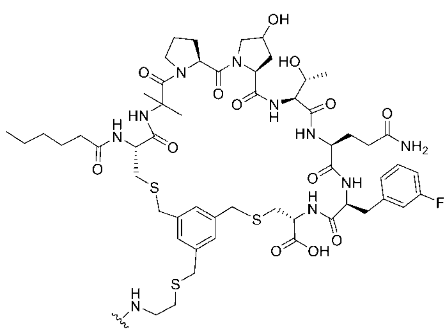
,
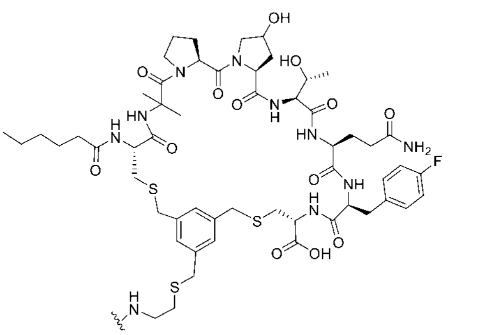
,
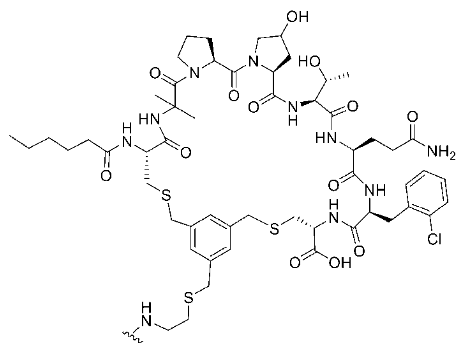
,
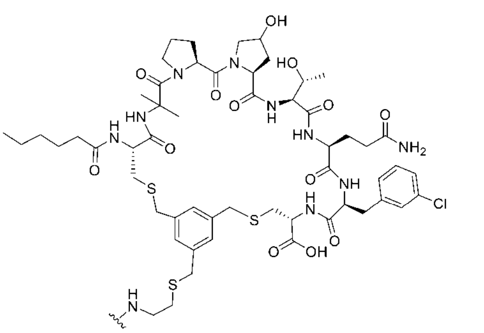
,
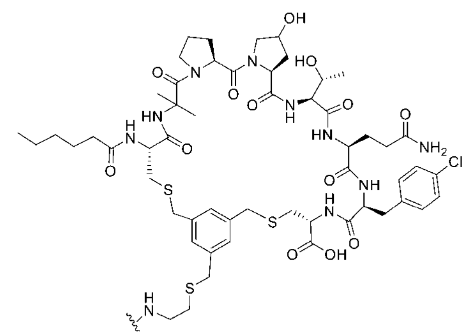
,

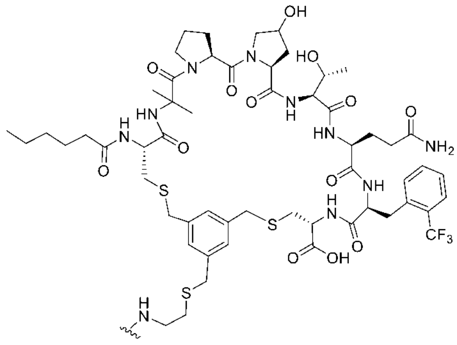
,
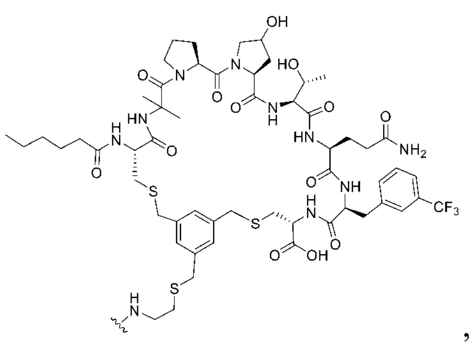
,

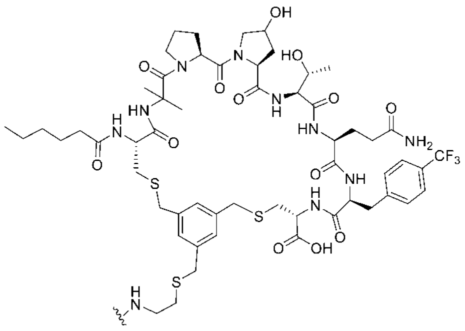
,
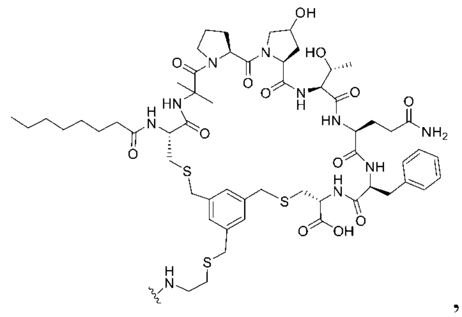
,

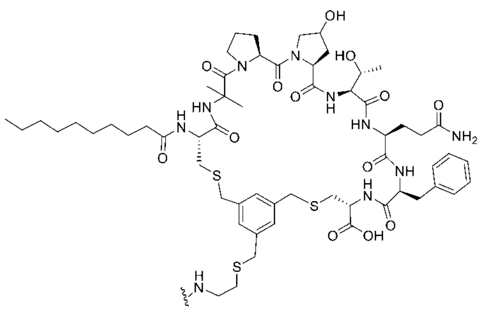
,
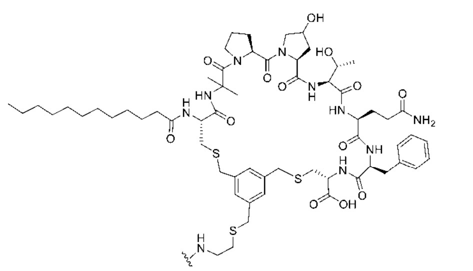
,

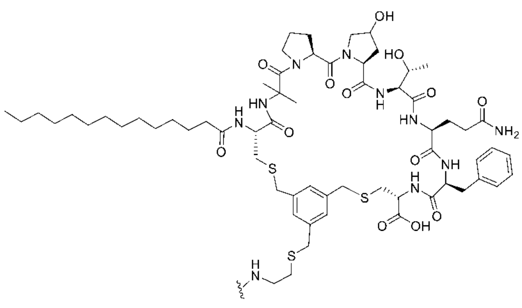
,
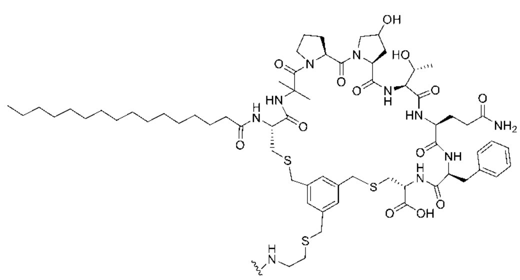
,

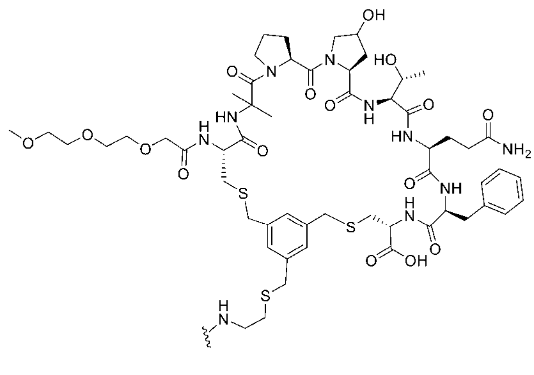

,

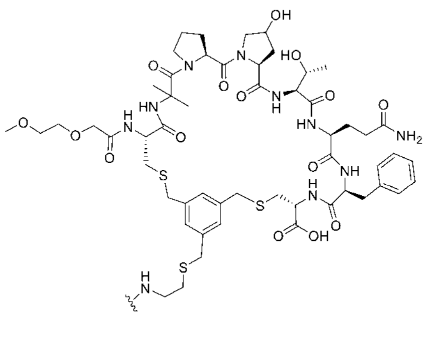

,

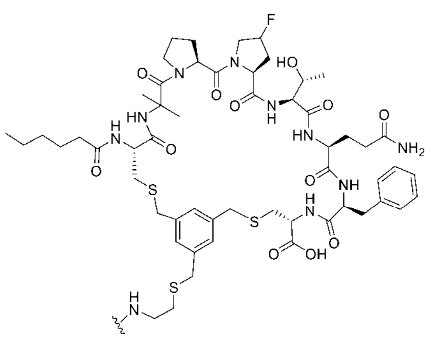

,

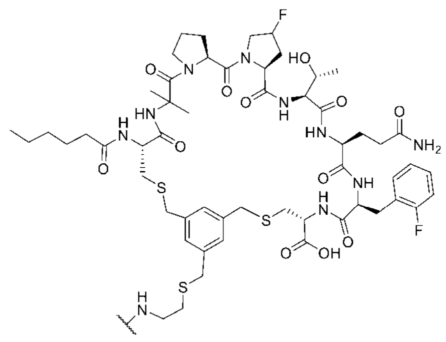

,

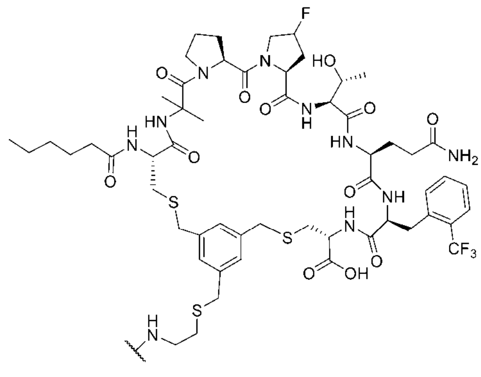

,

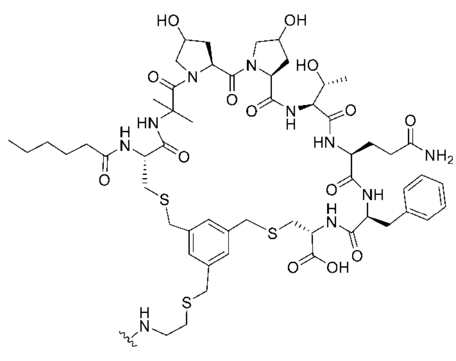

,

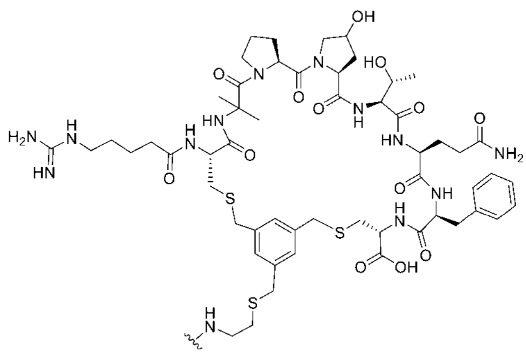

,

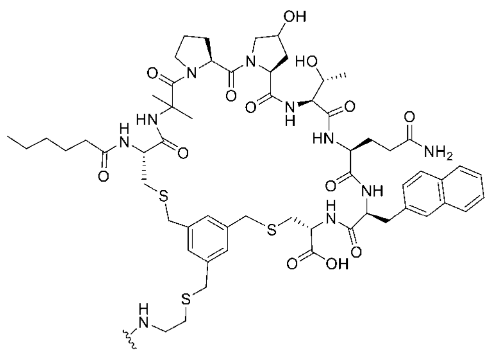

,

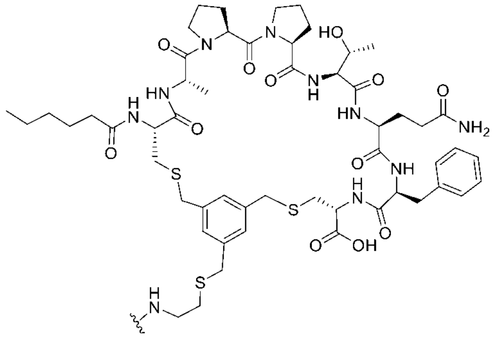

,

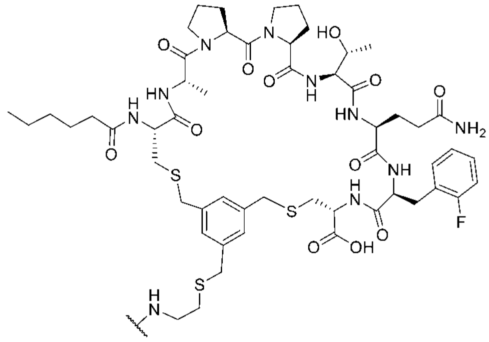

or

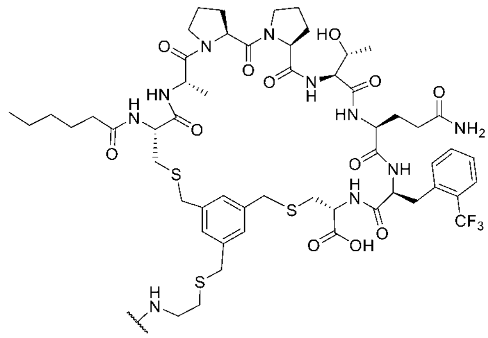

;

group 2:

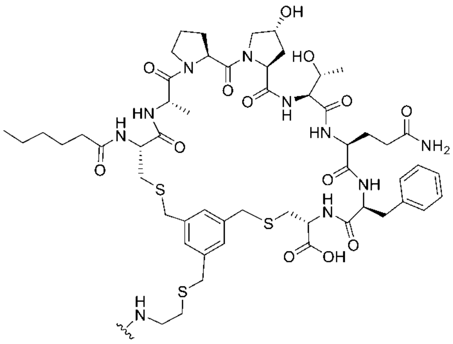

,

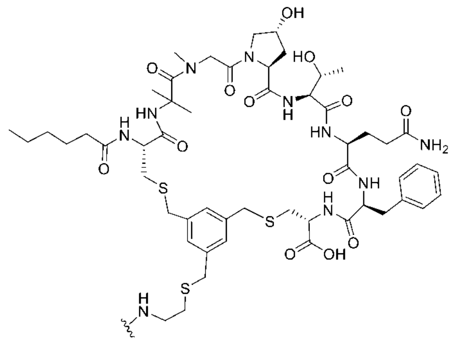

,

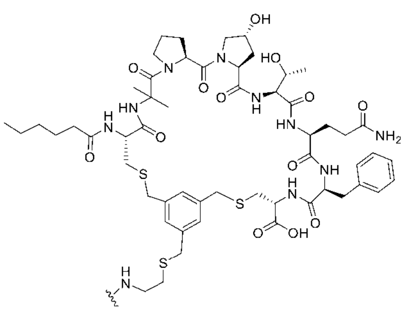

,

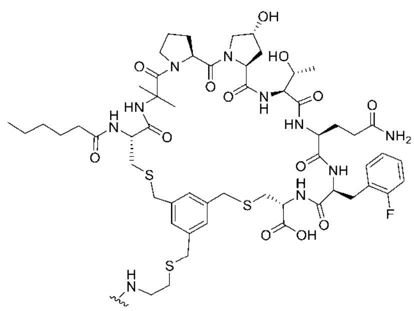

,

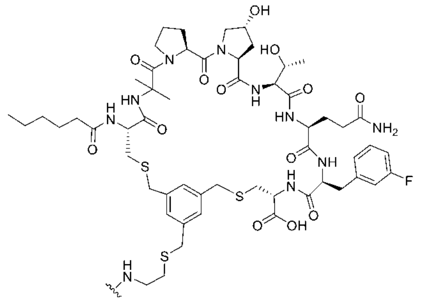
,
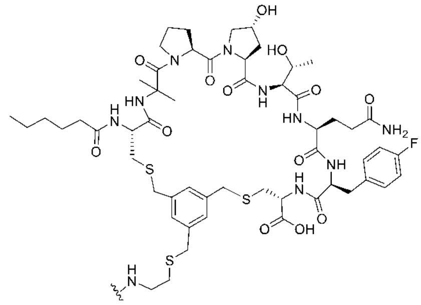
,
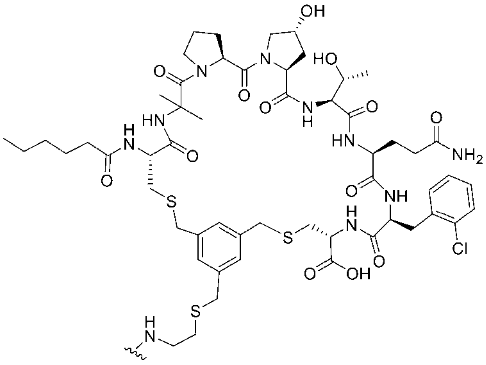
,
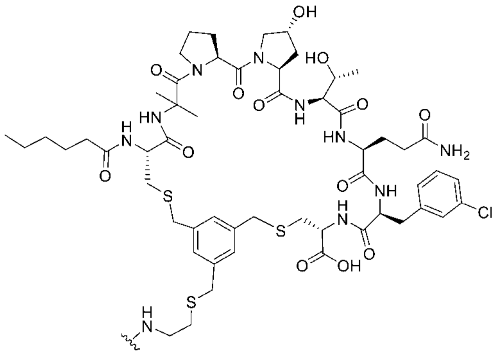
,
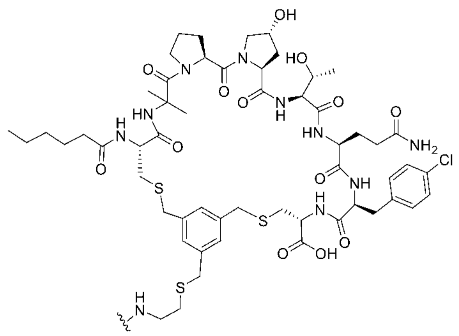
,
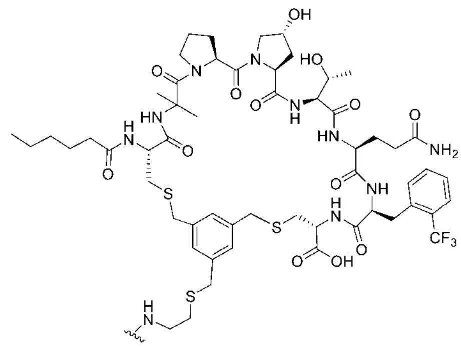
,
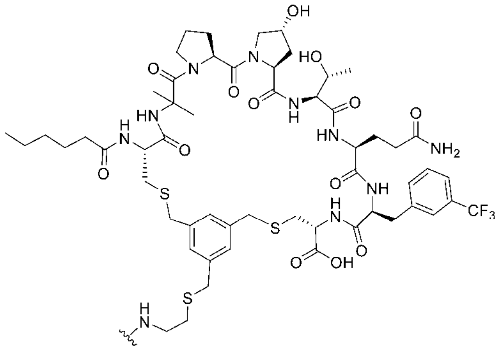
,
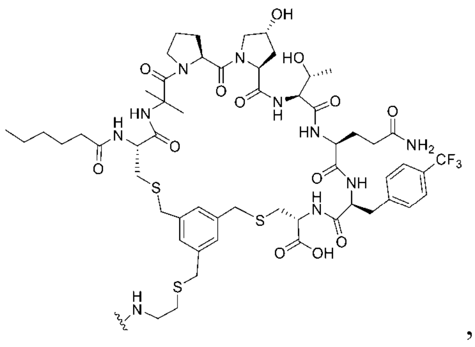
,

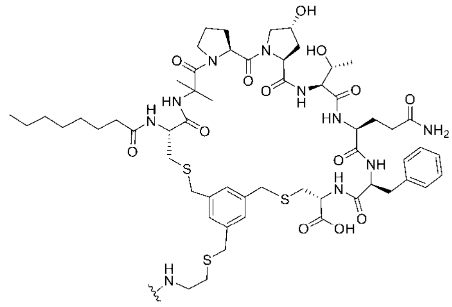
,
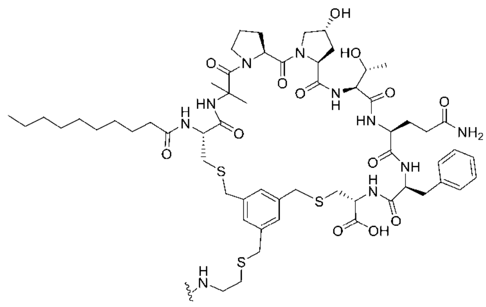
,
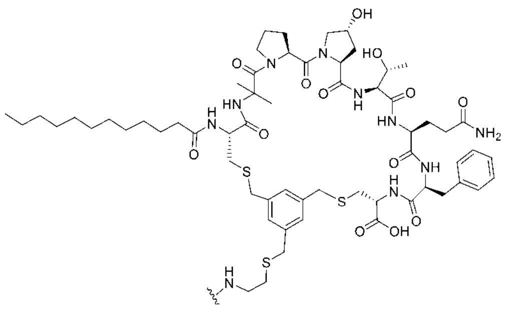
,
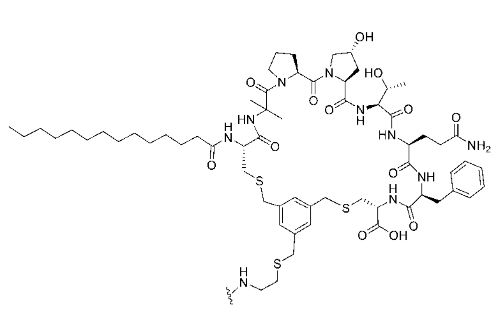
,
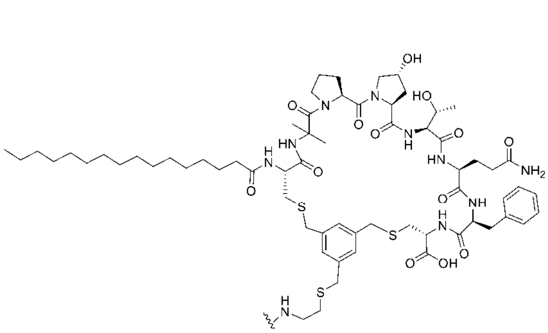
,
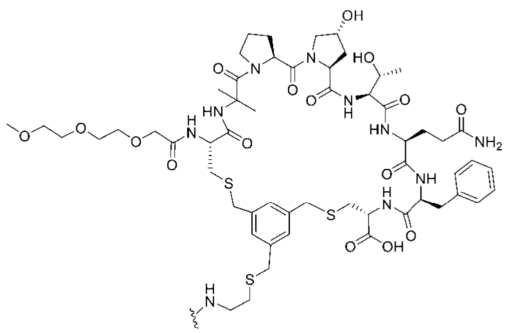
,
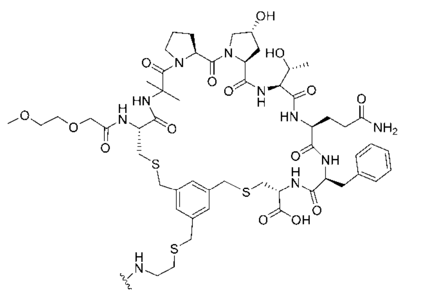
,
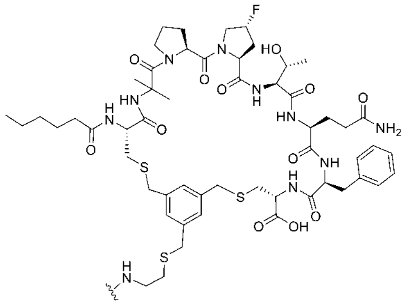
,
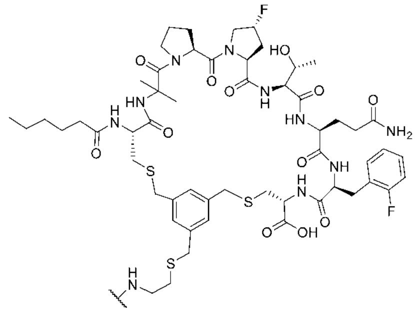
,
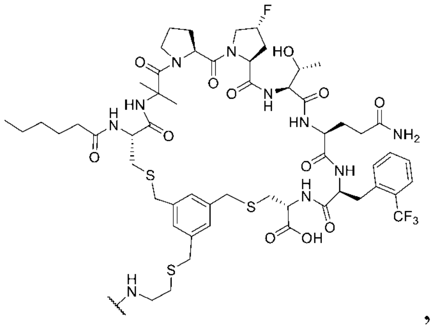
,

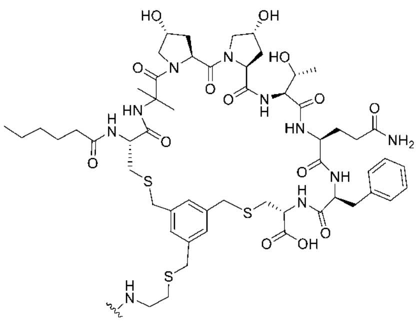,

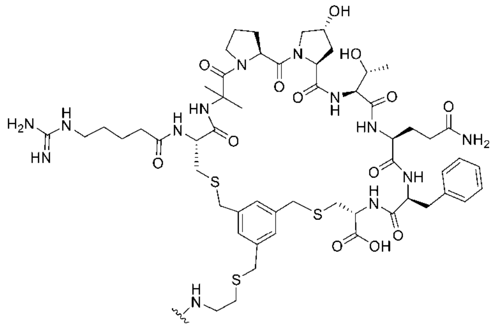

or

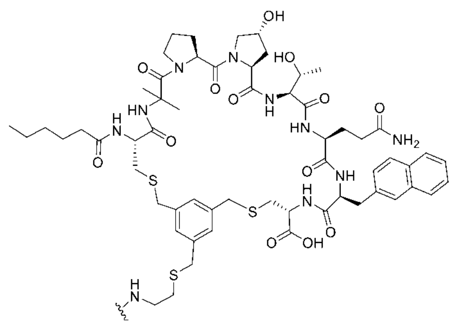;

group 3:

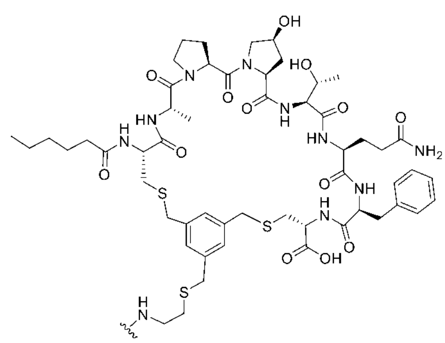,

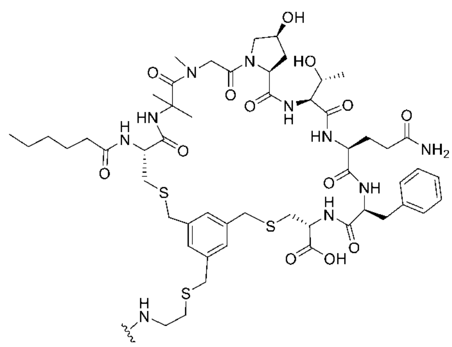,

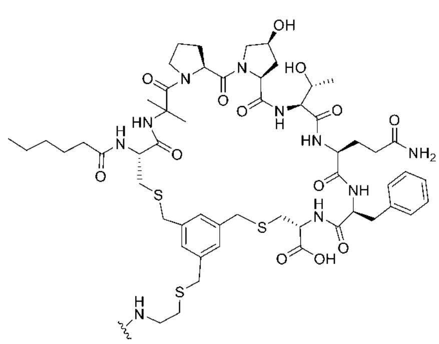,

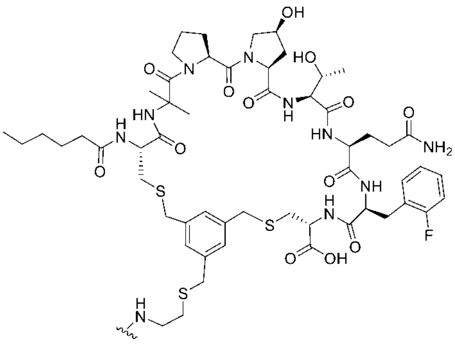,

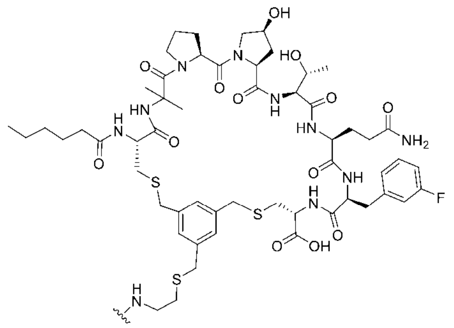
,
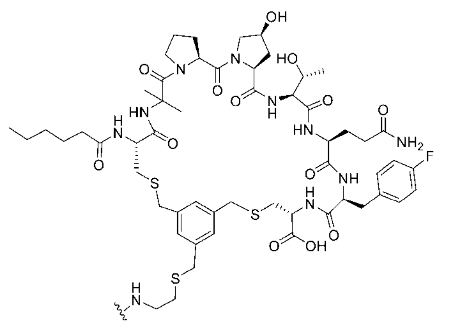
,
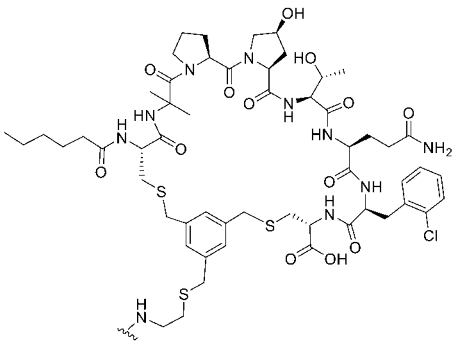
,
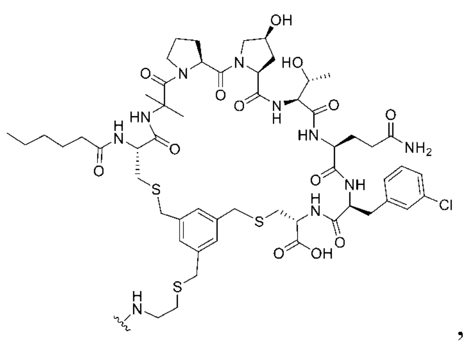
,
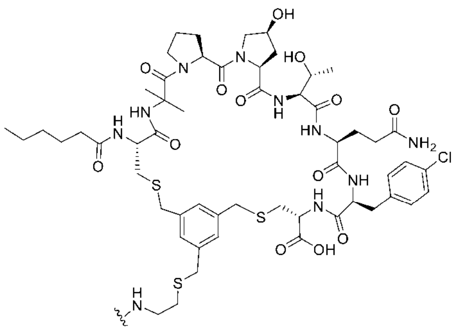
,
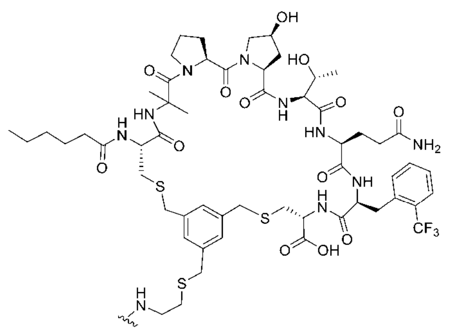
,
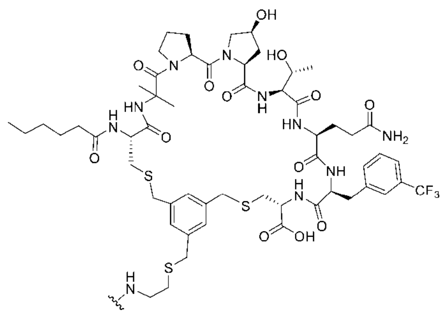
,
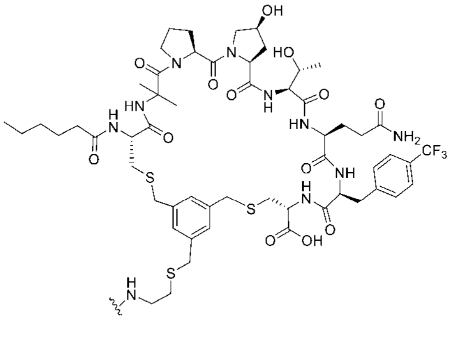
,

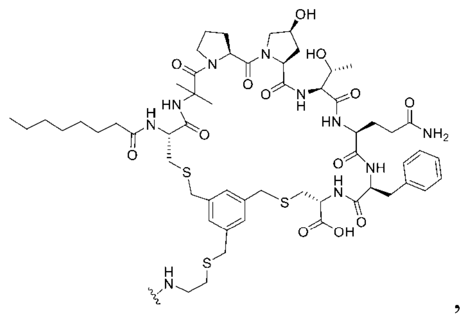

,

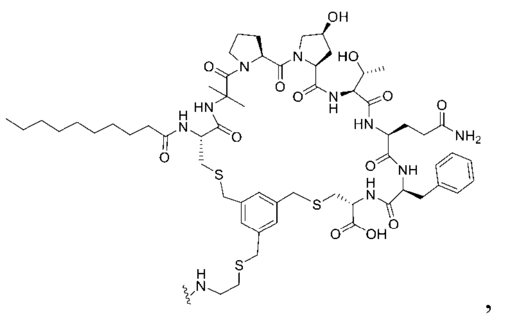

,

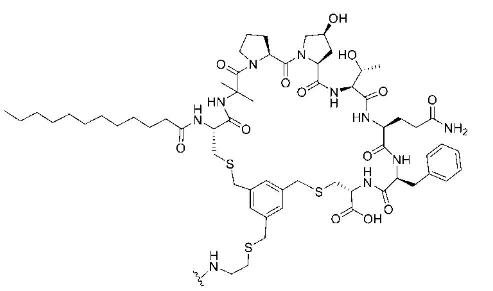

,

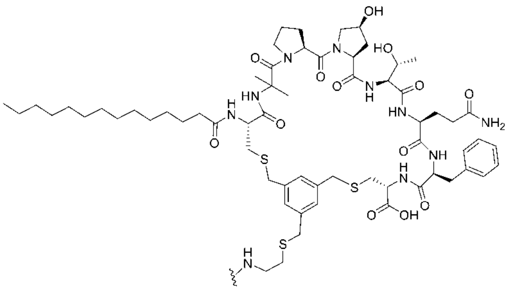

,

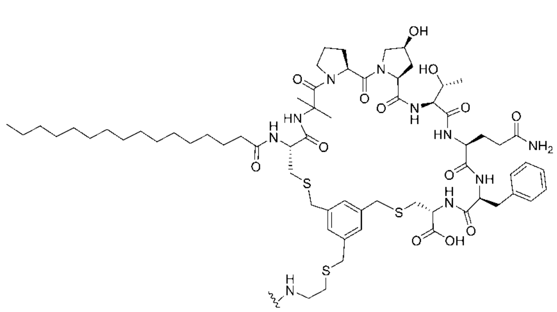

,

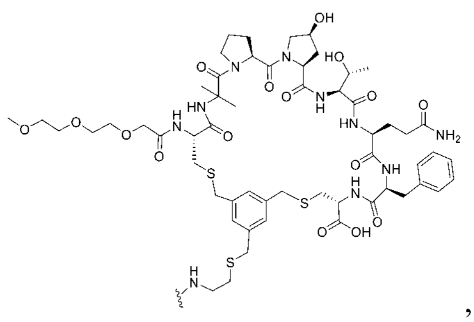

,

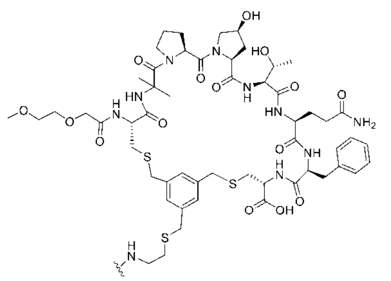

,

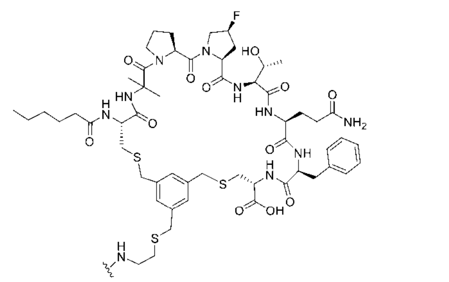

,

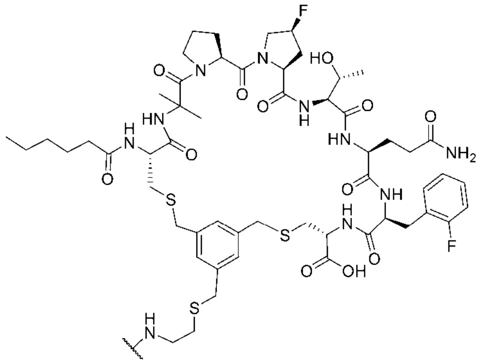

,

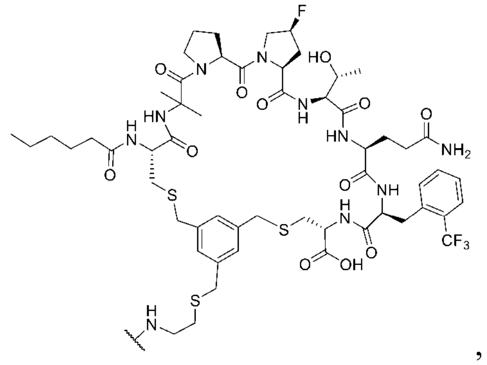

,

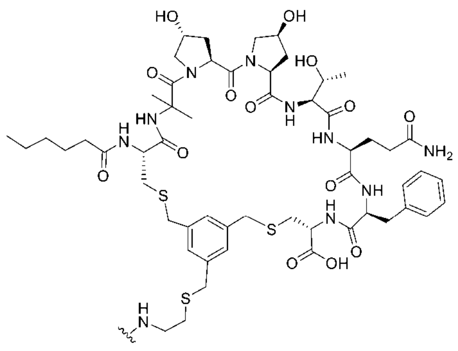,
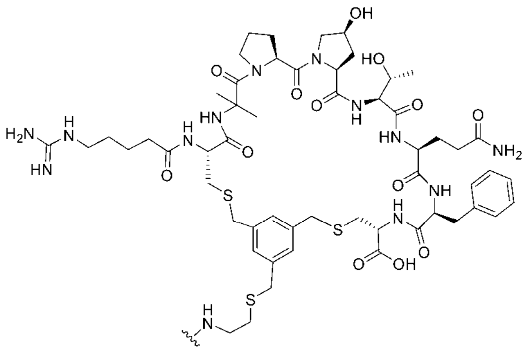

or

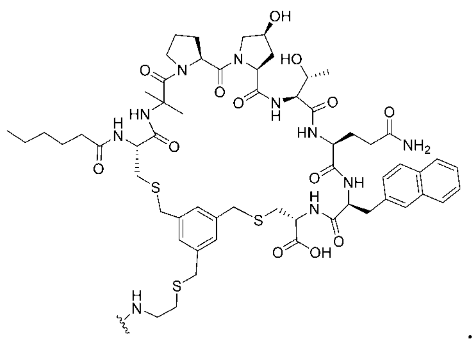.

**22.** The compound as shown in formula (I) or the pharmaceutically acceptable salt thereof according to any one of claims 1 to 21, **characterised in that** the chelating agent is selected from DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NxS$_4$-x(N$_4$, N$_2$S$_2$, N$_3$S), Hynic, 99mTc(CO)$_3$-chelating agent,

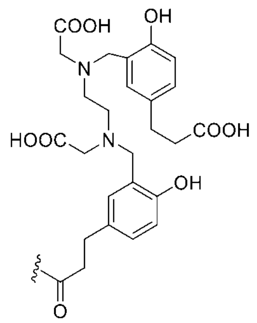,

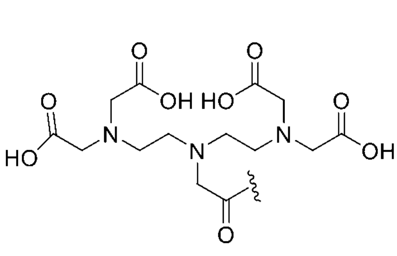, 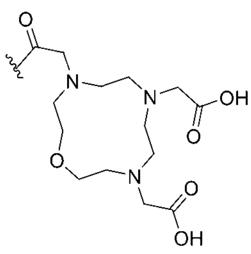, 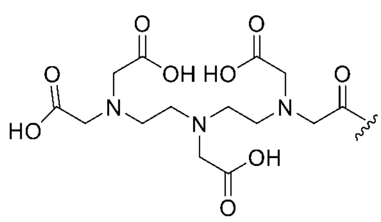,

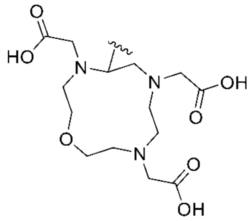 , 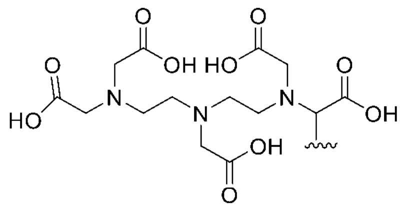 , 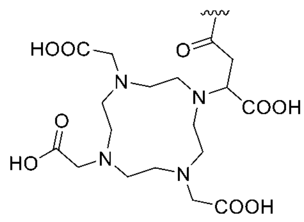 ,

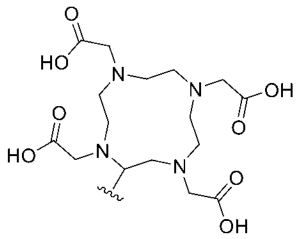 , 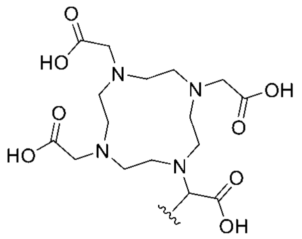 , 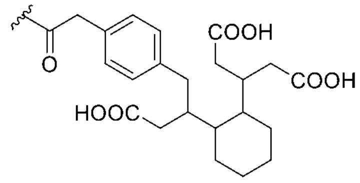 ,

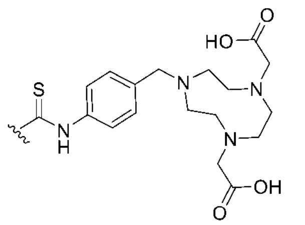 , 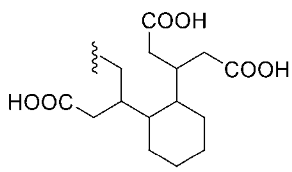 , 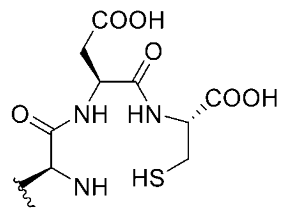 ,

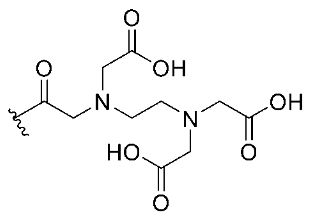 , 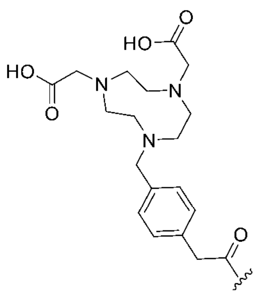 , 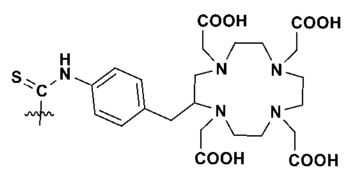 ,

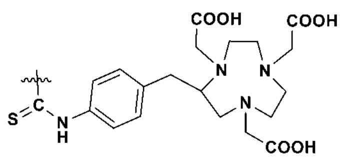 , 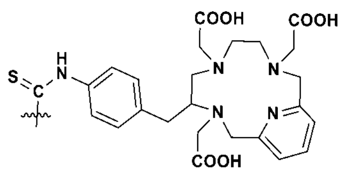 , 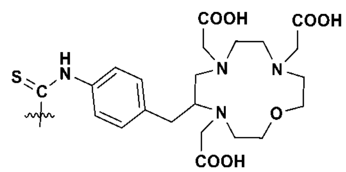 ,

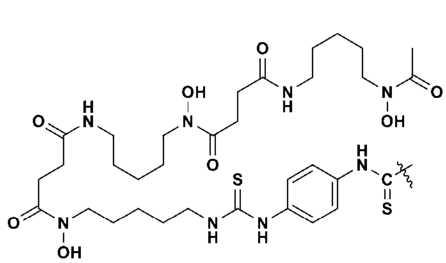 , 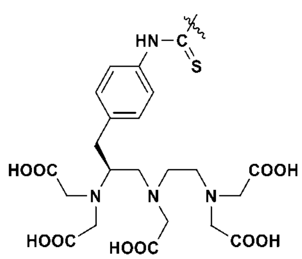 , 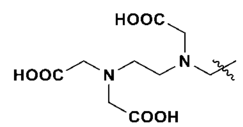 ,

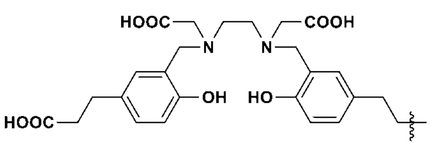 and 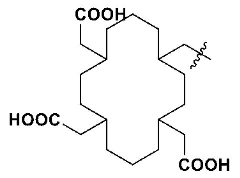 ,

preferably DOTA, DOTAGA, NOTA, NODAGA, NODA-MPAA, HBED, CB-TE2A, DFO, THP and $N_4$, and most preferably DOTA, DOTAGA, NOTA and NODAGA.

**23.** The compound as shown in formula (I) or the pharmaceutically acceptable salt thereof according to any one of claims 1 to 22, **characterised in that** the compound is selected from: group 4:

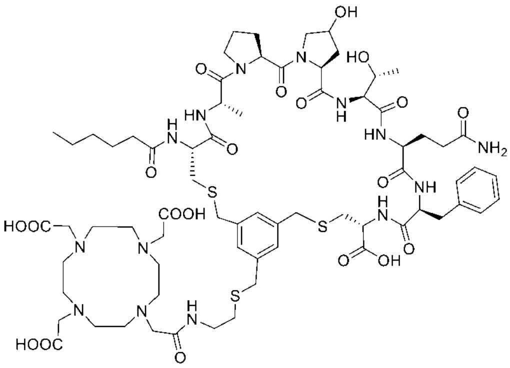

Hex-[Cys-(tMeBn(DOTA-AET))-Ala-Pro-(4-OH-Pro)-Thr-Gln-Phe-Cys]-OH,

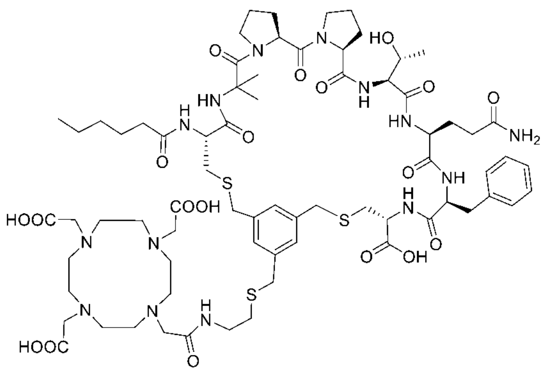

Hex-[Cys-(tMeBn(DOTA-AET))-Aib-Pro-Pro-Thr-Gln-Phe-Cys]-OH,

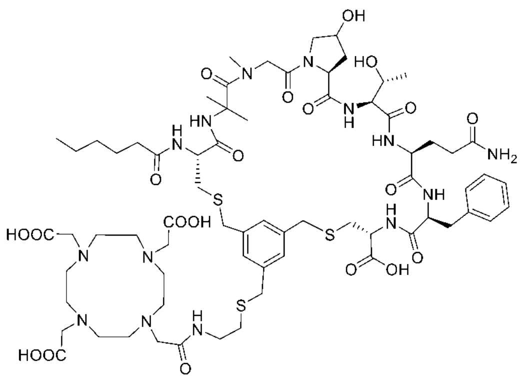

Hex-[Cys-(tMeBn(DOTA-AET))-Aib-Sar-(4-OH-Pro)-Thr-Gln-Phe-Cys]-OH,

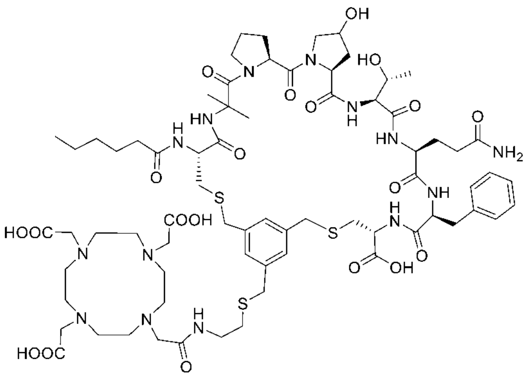

Hex-[Cys-(tMeBn(DOTA-AET))-Aib-Pro-(4-OH-Pro)-Thr-Gln-Phe-Cys]-OH,

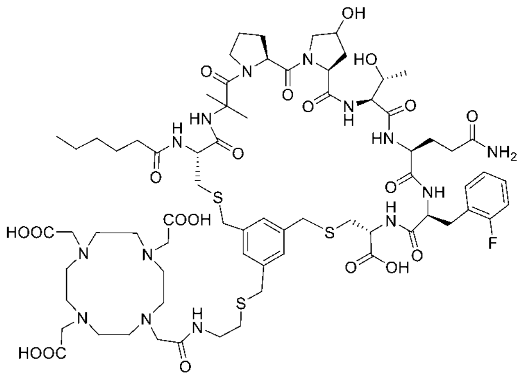

Hex-[Cys-(tMeBn(DOTA-AET))-Aib-Pro-(4-OH-Pro)-Thr-Gln-(2-F-Phe)-Cys]-OH,

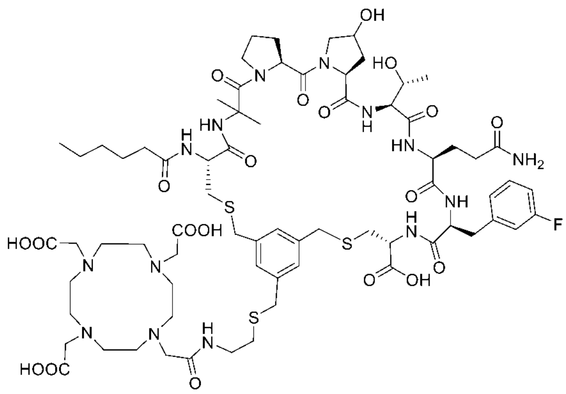

Hex-[Cys-(tMeBn(DOTA-AET))-Aib-Pro-(4-OH-Pro)-Thr-Gln-(3-F-Phe)-Cys]-OH,

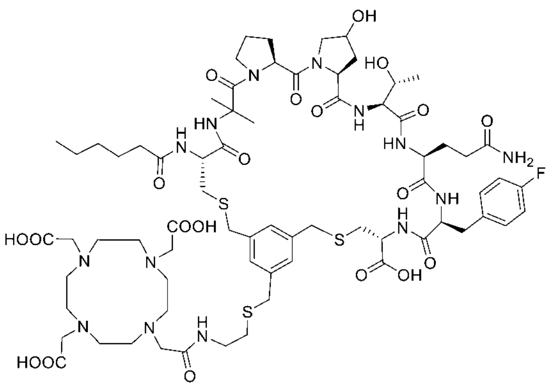

Hex-[Cys-(tMeBn(DOTA-AET))-Aib-Pro-(4-OH-Pro)-Thr-Gln-(4-F-Phe)-Cys]-OH,

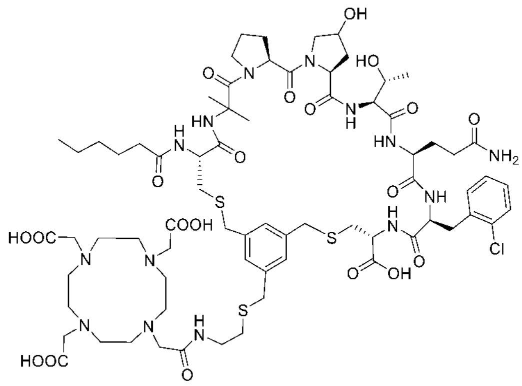

Hex-[Cys-(tMeBn(DOTA-AET))-Aib-Pro-(4-OH-Pro)-Thr-Gln-(2-Cl-Phe)-Cys]-OH,

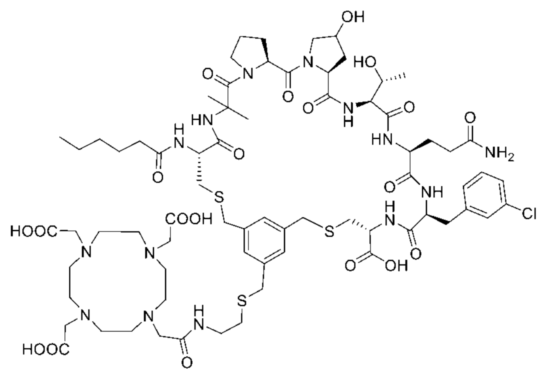

Hex-[Cys-(tMeBn(DOTA-AET))-Aib-Pro-(4-OH-Pro)-Thr-Gln-(3-Cl-Phe)-Cys]-OH,

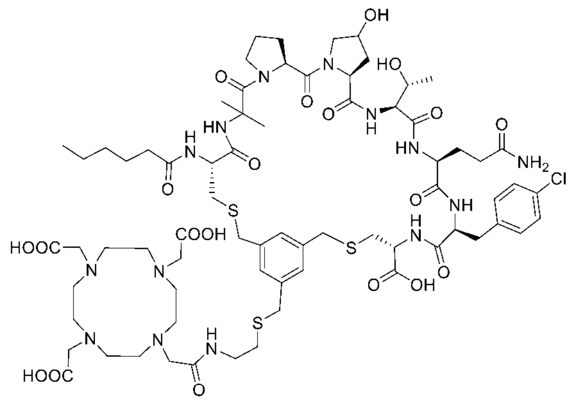

Hex-[Cys-(tMeBn(DOTA-AET))-Aib-Pro-(4-OH-Pro)-Thr-Gln-(4-Cl-Phe)-Cys]-OH,

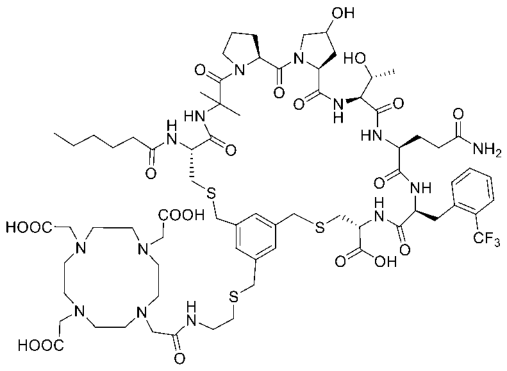

Hex-[Cys-(tMeBn(DOTA-AET))-Aib-Pro-(4-OH-Pro)-Thr-Gln-(2-$CF_3$-Phe)-Cys]-OH,

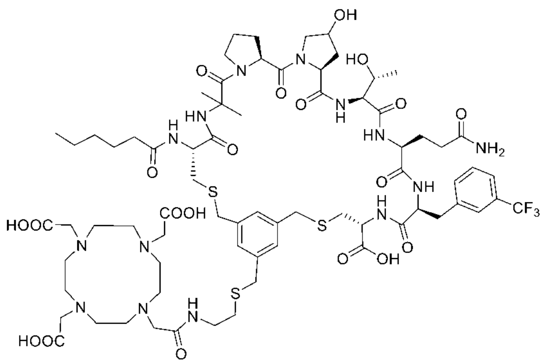

Hex-[Cys-(tMeBn(DOTA-AET))-Aib-Pro-(4-OH-Pro)-Thr-Gln-(3-$CF_3$-Phe)-Cys]-OH,

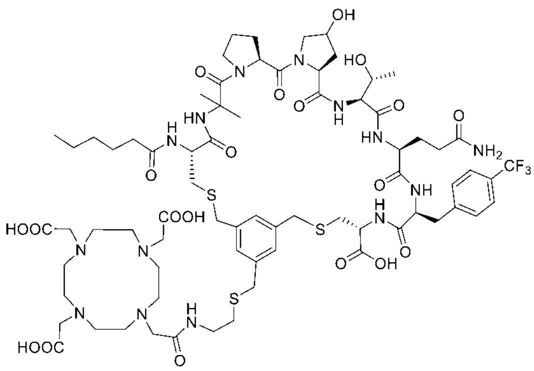

Hex-[Cys-(tMeBn(DOTA-AET))-Aib-Pro-(4-OH-Pro)-Thr-Gln-(4-$CF_3$-Phe)-Cys]-OH,

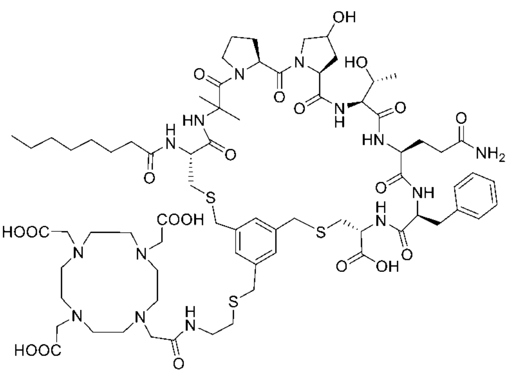

Oct-[Cys-(tMeBn(DOTA-AET))-Aib-Pro-(4-OH-Pro)-Thr-Gln-Phe-Cys]-OH,

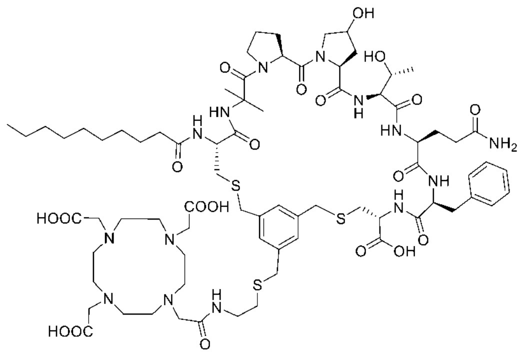

Dec-[Cys-(tMeBn(DOTA-AET))-Aib-Pro-(4-OH-Pro)-Thr-Gln-Phe-Cys]-OH,

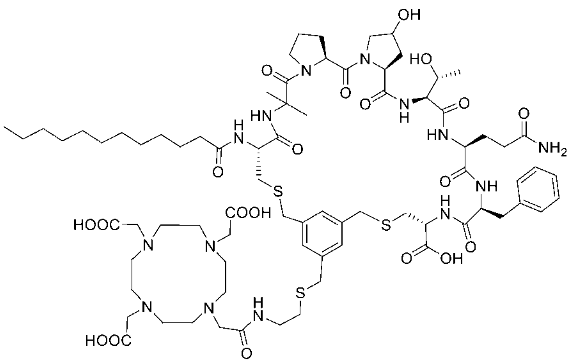

Dod-[Cys-(tMeBn(DOTA-AET))-Aib-Pro-(4-OH-Pro)-Thr-Gln-Phe-Cys]-OH,

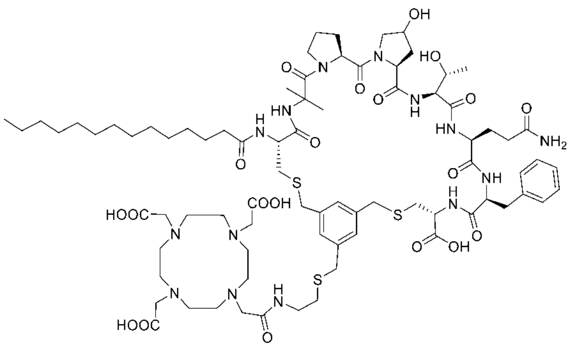

Tet-[Cys-(tMeBn(DOTA-AET))-Aib-Pro-(4-OH-Pro)-Thr-Gln-Phe-Cys]-OH,

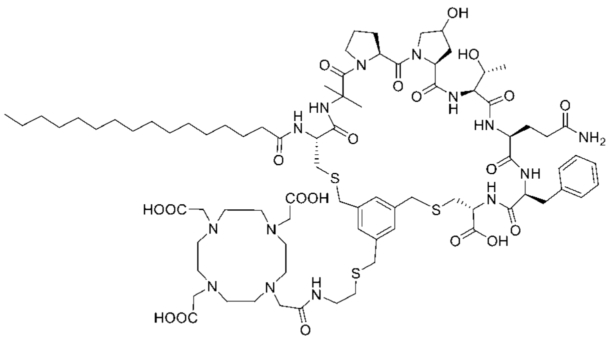

Pal-[Cys-(tMeBn(DOTA-AET))-Aib-Pro-(4-OH-Pro)-Thr-Gln-Phe-Cys]-OH,

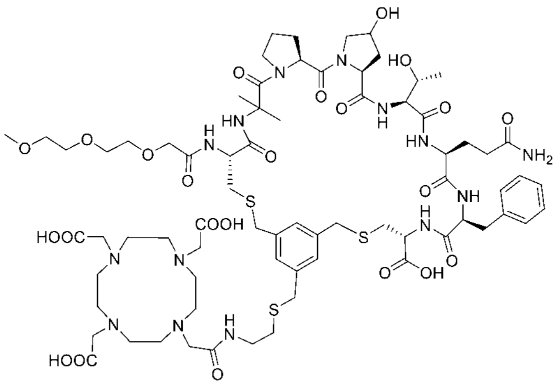

12-[2-(2-Methoxyethoxy)ethoxy]-Ace l -[Cys-(tMeBn(DOTA-AET))-Aib-Pro-(4-OH-Pro)-Thr-Gln-Phe-Cys]-OH,

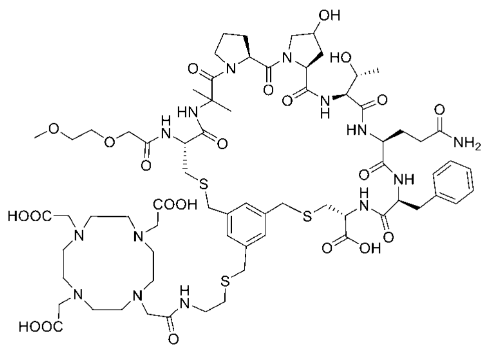

[2-(2-Methoxyethoxy)-Ace]-[Cys-(tMeBn(DOTA-AET))-Aib-Pro-(4-OH-Pro)-Thr-Gln-Phe-Cys]-OH,

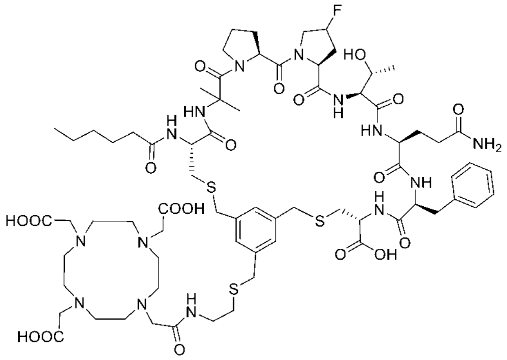

Hex-[Cys-(tMeBn(DOTA-AET))-Aib-Pro-(4-F-Pro)-Thr-Gln-Phe-Cys]-OH,

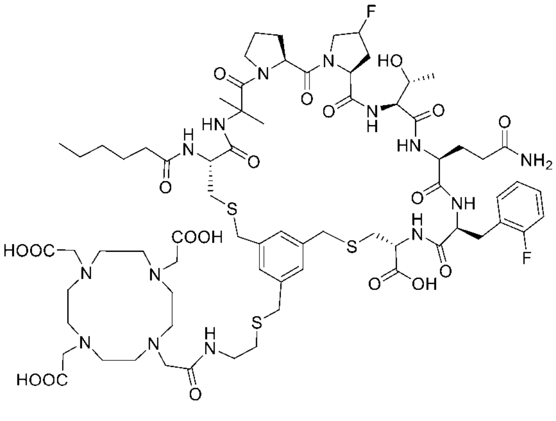

Hex-[Cys-(tMeBn(DOTA-AET))-Aib-Pro-(4-F-Pro)-Thr-Gln-(2-F-Phe)-Cys]-OH,

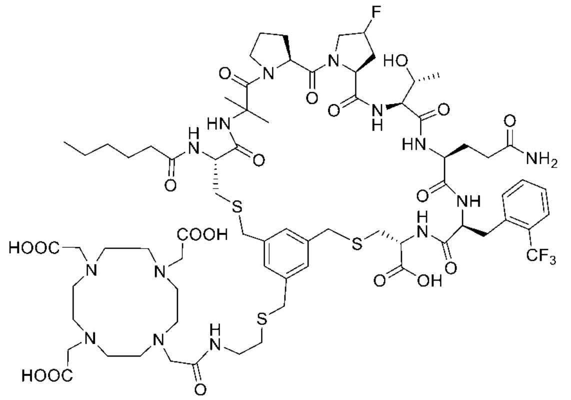

Hex-[Cys-(tMeBn(DOTA-AET))-Aib-Pro-(4-F-Pro)-Thr-Gln-(2-$CF_3$-Phe)-Cys]-OH,

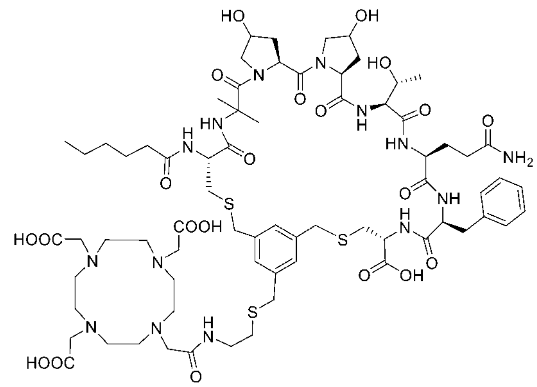

Hex-[Cys-(tMeBn(DOTA-AET))-Aib-(4-OH-Pro)-(4-OH-Pro)-Thr-Gln-Phe-Cys]-OH,

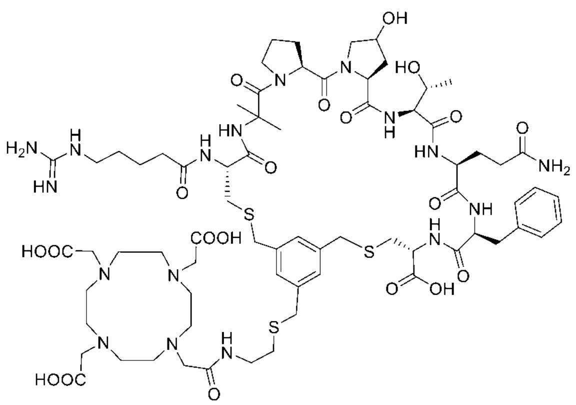

(5-guanidineValy)-[Cys-(tMeBn(DOTA-AET))-Aib-Pro-(4-OH-Pro)-Thr-Gln-Phe-Cys]-OH,

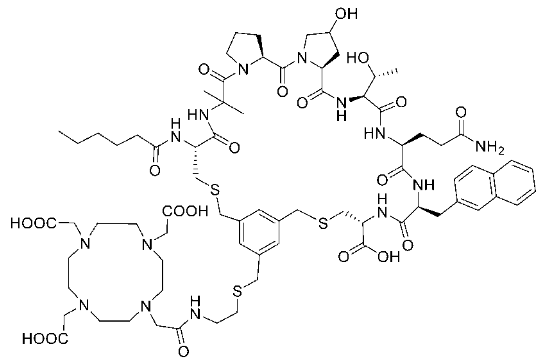

Hex-[Cys-(tMeBn(DOTA-AET))-Aib-Pro-(4-OH-Pro)-Thr-Gln-(2-Nal)-Cys]-OH,

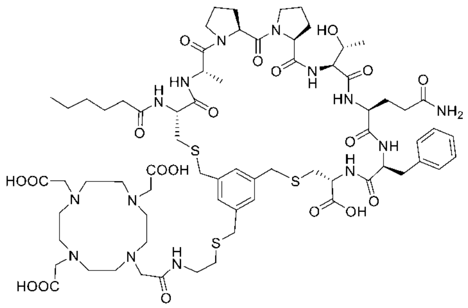

Hex-[Cys-(tMeBn(DOTA-AET))-Ala-Pro-Pro-Thr-Gln-Phe-Cys]-OH,

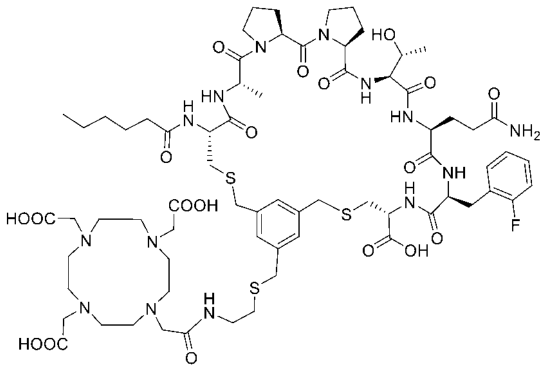

Hex-[Cys-(tMeBn(DOTA-AET))-Ala-Pro-Pro-Thr-Gln-(2-F-Phe)-Cys]-OH,

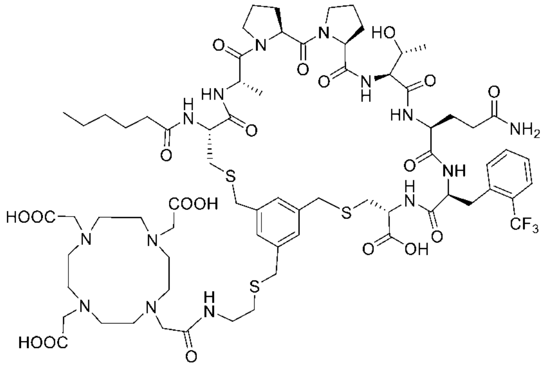

Hex-[Cys-(tMeBn(DOTA-AET))-Ala-Pro-Pro-Thr-Gln-(2-$CF_3$-Phe)-Cys]-OH,

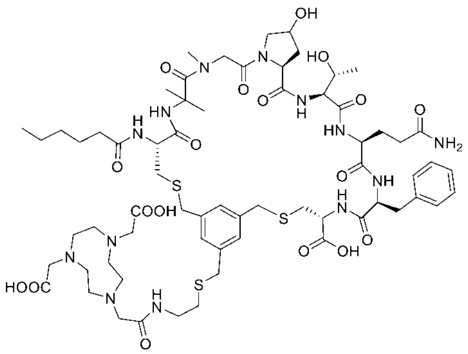

Hex-[Cys-(tMeBn(NOTA-AET))-Aib-Sar-(4-OH-Pro)-Thr-Gln-Phe-Cys]-OH,

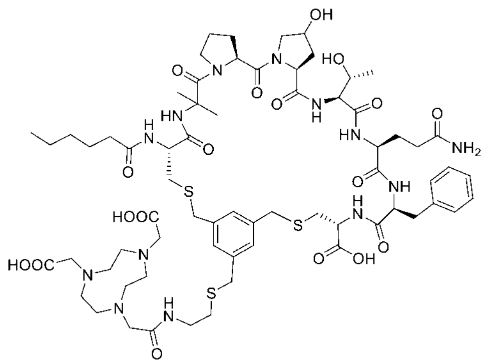

Hex-[Cys-(tMeBn(NOTA-AET))-Aib-Pro-(4-OH-Pro)-Thr-Gln-Phe-Cys]-OH,

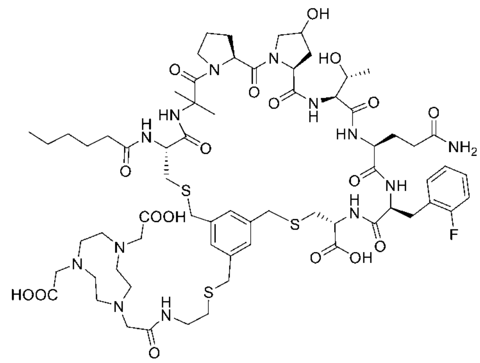

Hex-[Cys-(tMeBn(NOTA-AET))-Aib-Pro-(4-OH-Pro)-Thr-Gln-(2-F-Phe)-Cys]-OH,

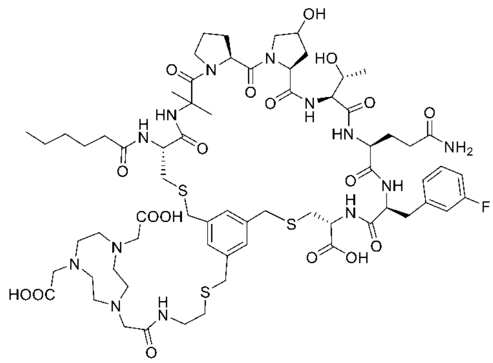

Hex-[Cys-(tMeBn(NOTA-AET))-Aib-Pro-(4-OH-Pro)-Thr-Gln-(3-F-Phe)-Cys]-OH,

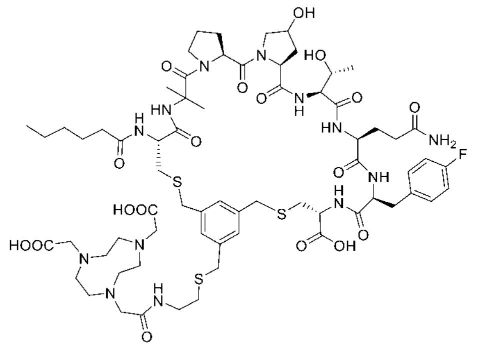

Hex-[Cys-(tMeBn(NOTA-AET))-Aib-Pro-(4-OH-Pro)-Thr-Gln-(4-F-Phe)-Cys]-OH,

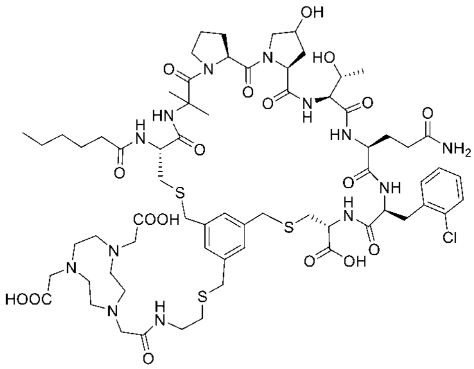

Hex-[Cys-(tMeBn(NOTA-AET))-Aib-Pro-(4-OH-Pro)-Thr-Gln-(2-Cl-Phe)-Cys]-OH,

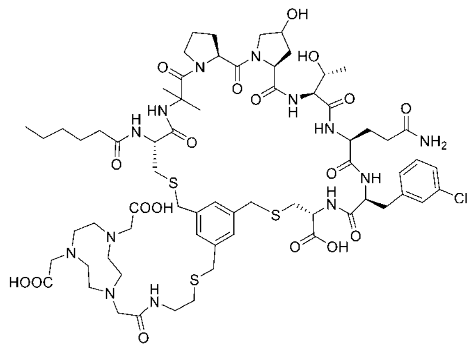

Hex-[Cys-(tMeBn(NOTA-AET))-Aib-Pro-(4-OH-Pro)-Thr-Gln-(3-Cl-Phe)-Cys]-OH,

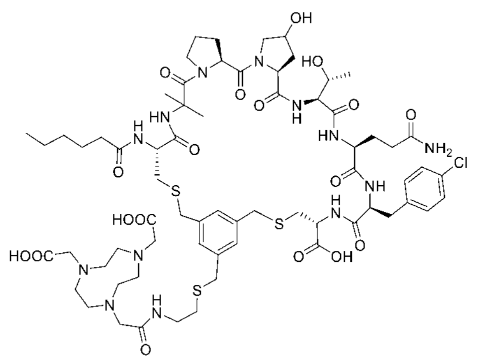

Hex-[Cys-(tMeBn(NOTA-AET))-Aib-Pro-(4-OH-Pro)-Thr-Gln-(4-Cl-Phe)-Cys]-OH,

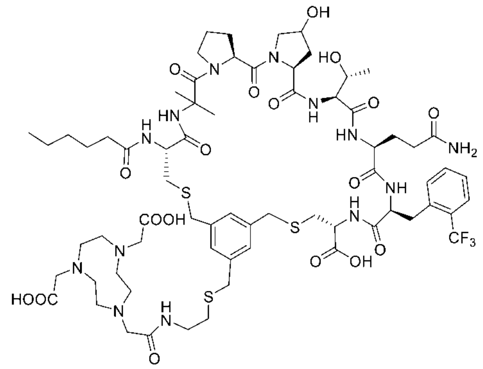

Hex-[Cys-(tMeBn(NOTA-AET))-Aib-Pro-(4-OH-Pro)-Thr-Gln-(2-$CF_3$-Phe)-Cys]-OH,

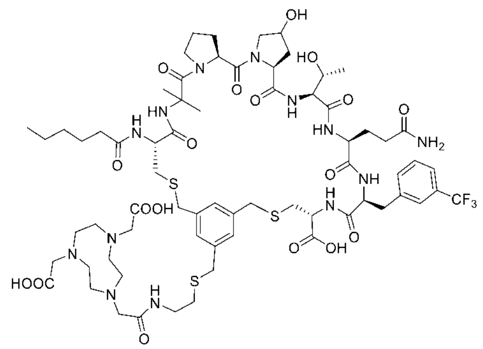

Hex-[Cys-(tMeBn(NOTA-AET))-Aib-Pro-(4-OH-Pro)-Thr-Gln-(3-$CF_3$-Phe)-Cys]-OH,

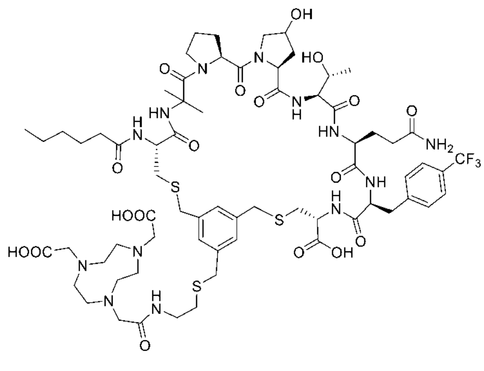

Hex-[Cys-(tMeBn(NOTA-AET))-Aib-Pro-(4-OH-Pro)-Thr-Gln-(4-$CF_3$-Phe)-Cys]-OH,

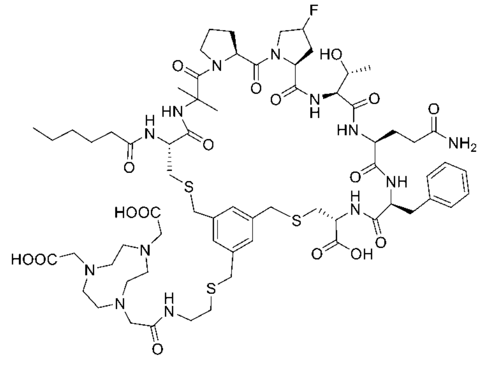

Hex-[Cys-(tMeBn(NOTA-AET))-Aib-Pro-(4-F-Pro)-Thr-Gln-Phe-Cys]-OH,

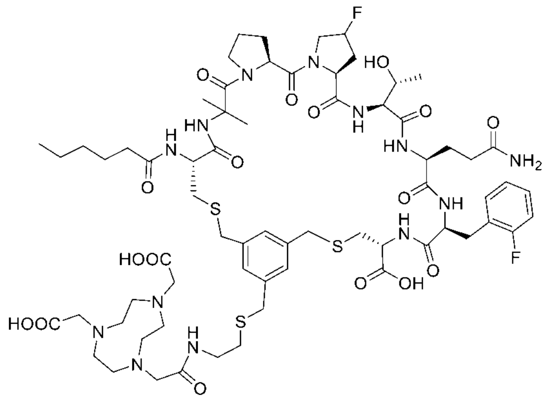

Hex-[Cys-(tMeBn(NOTA-AET))-Aib-Pro-(4-F-Pro)-Thr-Gln-(2-F-Phe)-Cys]-OH,

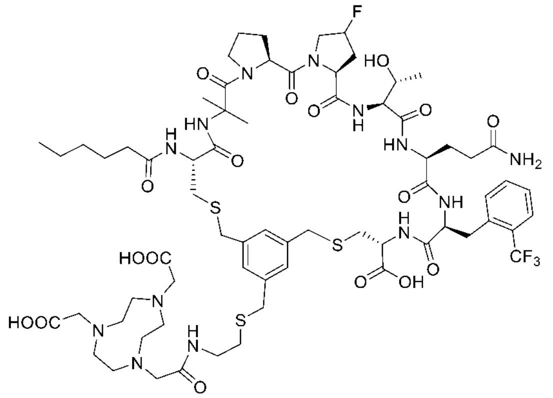

Hex-[Cys-(tMeBn(NOTA-AET))-Aib-Pro-(4-F-Pro)-Thr-Gln-(2-$CF_3$-Phe)-Cys]-OH,

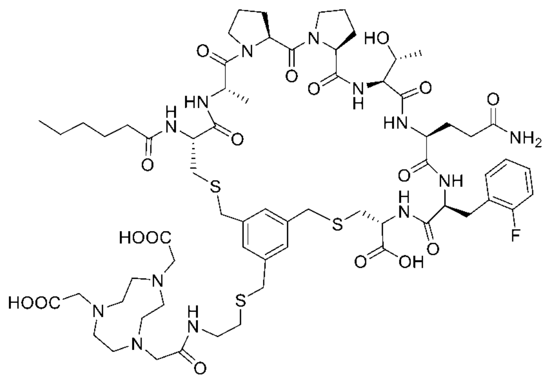

Hex-[Cys-(tMeBn(NOTA-AET))-Ala-Pro-Pro-Thr-Gln-(2-F-Phe)-Cys]-OH, and

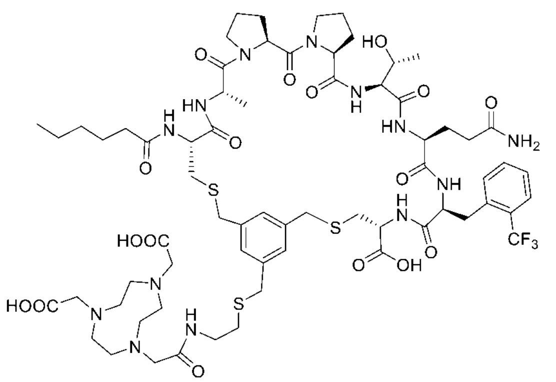

Hex-[Cys-(tMeBn(NOTA-AET))-Ala-Pro-Pro-Thr-Gln-(2-$CF_3$-Phe)-Cys]-OH; group 5:

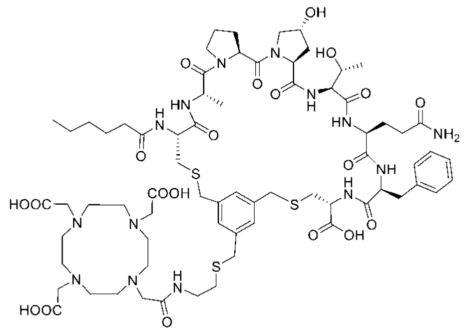

Hex-[Cys-(tMeBn(DOTA-AET))-Ala-Pro-((4R)-OH-Pro)-Thr-Gln-Phe-Cys]-OH,

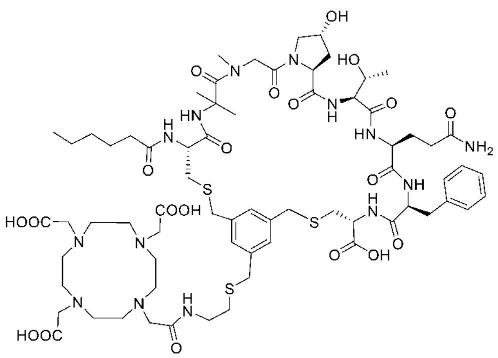

Hex-[Cys-(tMeBn(DOTA-AET))-Aib-Sar-((4R)-OH-Pro)-Thr-Gln-Phe-Cys]-OH,

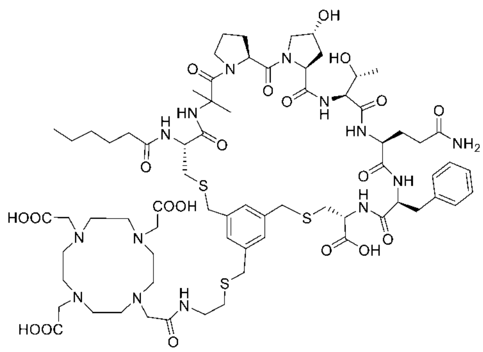

Hex-[Cys-(tMeBn(DOTA-AET))-Aib-Pro-((4R)-OH-Pro)-Thr-Gln-Phe-Cys]-OH,

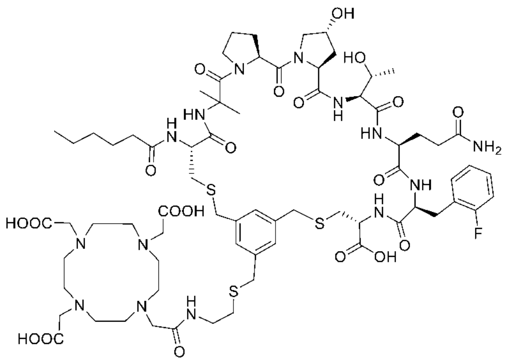

Hex-[Cys-(tMeBn(DOTA-AET))-Aib-Pro-((4R)-OH-Pro)-Thr-Gln-(2-F-Phe)-Cys]-OH,

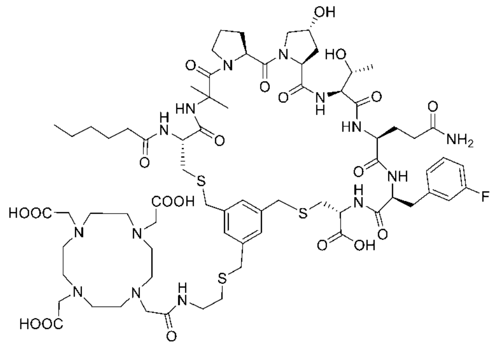

Hex-[Cys-(tMeBn(DOTA-AET))-Aib-Pro-((4R)-OH-Pro)-Thr-Gln-(3-F-Phe)-Cys]-OH,

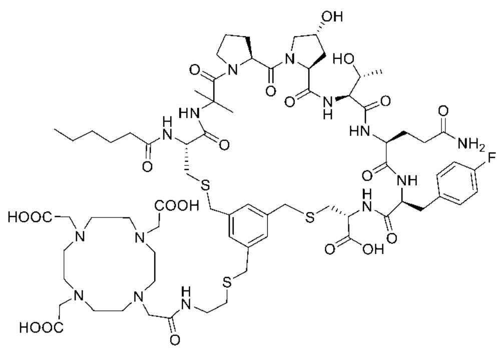

Hex-[Cys-(tMeBn(DOTA-AET))-Aib-Pro-((4R)-OH-Pro)-Thr-Gln-(4-F-Phe)-Cys]-OH,

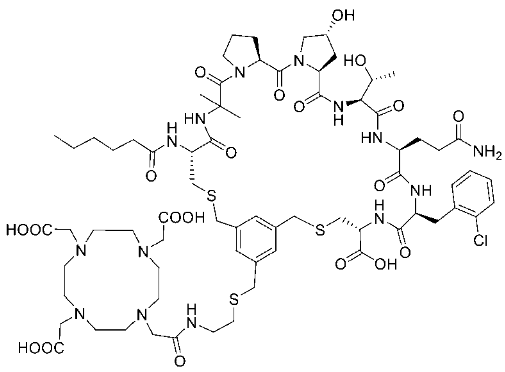

Hex-[Cys-(tMeBn(DOTA-AET))-Aib-Pro-((4R)-OH-Pro)-Thr-Gln-(2-Cl-Phe)-Cys]-OH,

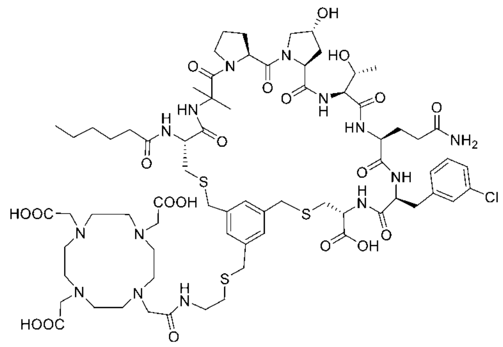

Hex-[Cys-(tMeBn(DOTA-AET))-Aib-Pro-((4R)-OH-Pro)-Thr-Gln-(3-Cl-Phe)-Cys]-OH,

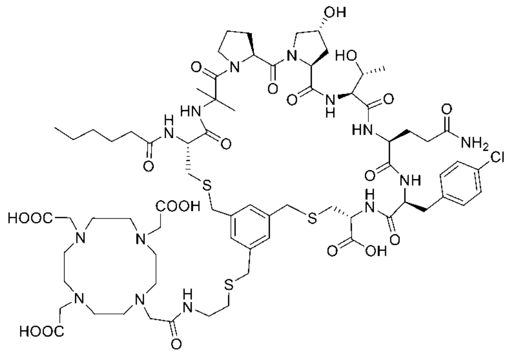

Hex-[Cys-(tMeBn(DOTA-AET))-Aib-Pro-((4R)-OH-Pro)-Thr-Gln-(4-Cl-Phe)-Cys]-OH,

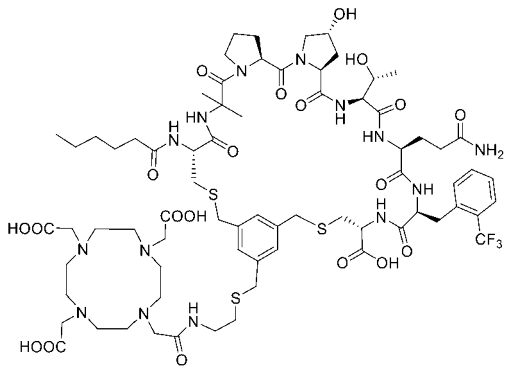

Hex-[Cys-(tMeBn(DOTA-AET))-Aib-Pro-((4R)-OH-Pro)-Thr-Gln-(2-$CF_3$-Phe)-Cys]-OH,

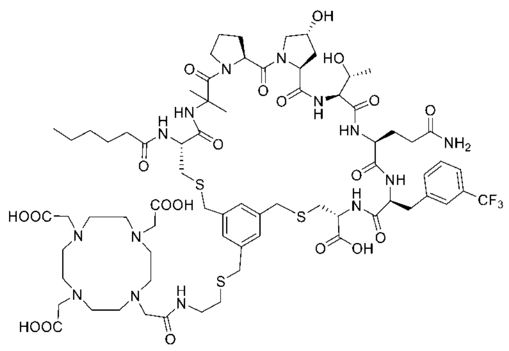

Hex-[Cys-(tMeBn(DOTA-AET))-Aib-Pro-((4R)-OH-Pro)-Thr-Gln-(3-$CF_3$-Phe)-Cys]-OH,

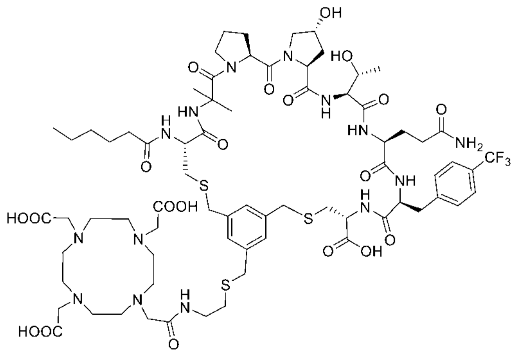

Hex-[Cys-(tMeBn(DOTA-AET))-Aib-Pro-((4R)-OH-Pro)-Thr-Gln-(4-$CF_3$-Phe)-Cys]-OH,

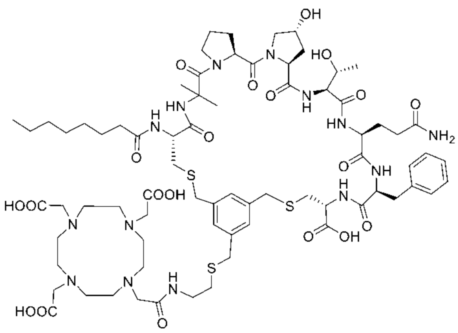

Oct-[Cys-(tMeBn(DOTA-AET))-Aib-Pro-((4R)-OH-Pro)-Thr-Gln-Phe-Cys]-OH,

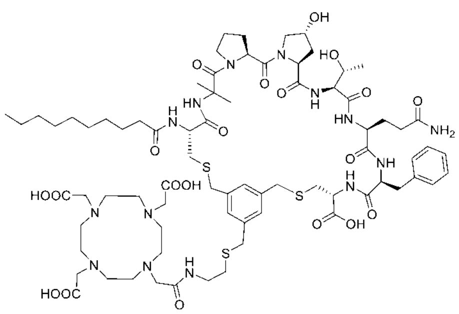

Dec-[Cys-(tMeBn(DOTA-AET))-Aib-Pro-((4R)-OH-Pro)-Thr-Gln-Phe-Cys]-OH,

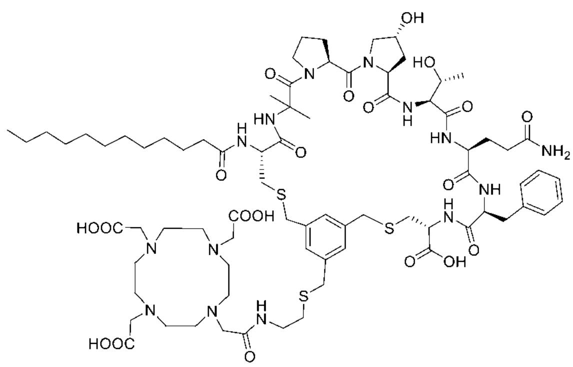

Dod-[Cys-(tMeBn(DOTA-AET))-Aib-Pro-((4R)-OH-Pro)-Thr-Gln-Phe-Cys]-OH,

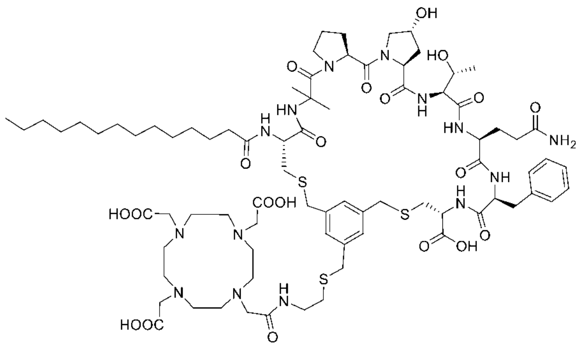

Tet-[Cys-(tMeBn(DOTA-AET))-Aib-Pro-((4R)-OH-Pro)-Thr-Gln-Phe-Cys]-OH,

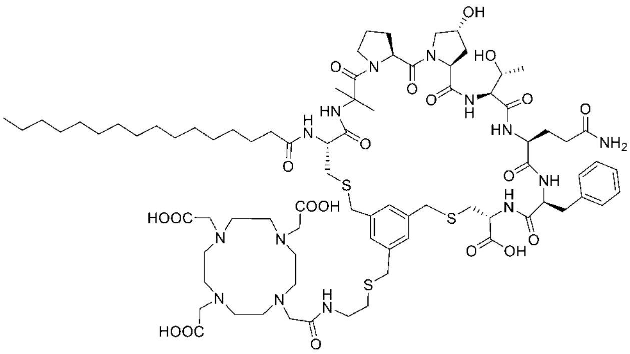

Pal-[Cys-(tMeBn(DOTA-AET))-Aib-Pro-((4R)-OH-Pro)-Thr-Gln-Phe-Cys]-OH,

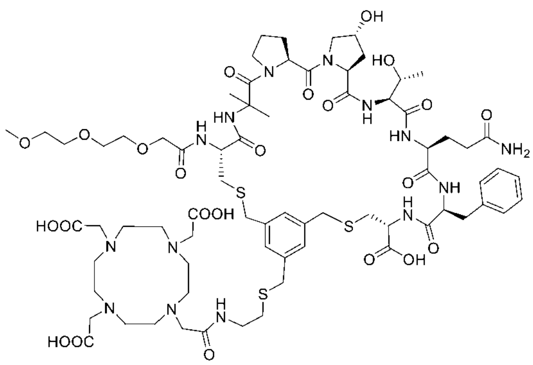

{2-[2-(2-Methoxyethoxy)ethoxy]-Ace}-[Cys-(tMeBn(DOTA-AET))-Aib-Pro-((4R)-OH-Pro)-Thr-Gln-Phe-Cys]-OH,

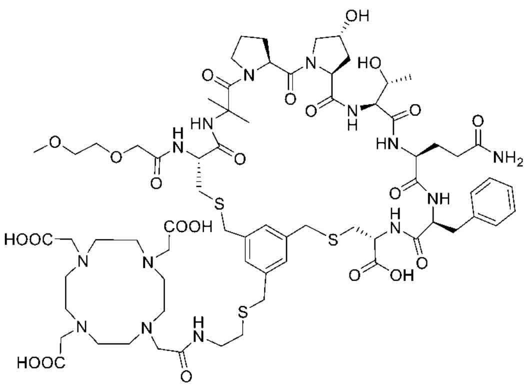

[2-(2-Methoxyethoxy)-Ace]-[Cys-(tMeBn(DOTA-AET))-Aib-Pro-((4R)-OH-Pro)-Thr-Gln-Phe-Cys]-OH,

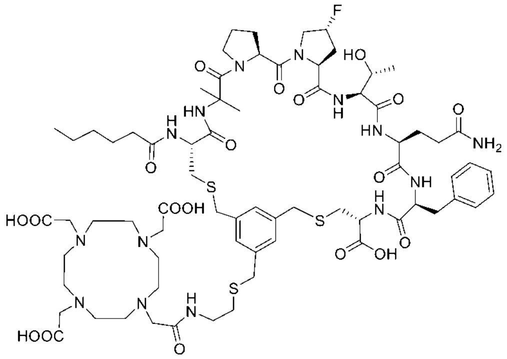

Hex-[Cys-(tMeBn(DOTA-AET))-Aib-Pro-((4R)-F-Pro)-Thr-Gln-Phe-Cys]-OH,

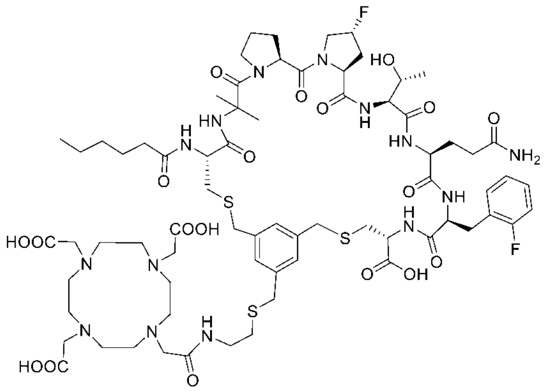

Hex-[Cys-(tMeBn(DOTA-AET))-Aib-Pro-((4R)-F-Pro)-Thr-Gln-(2-F-Phe)-Cys]-OH,

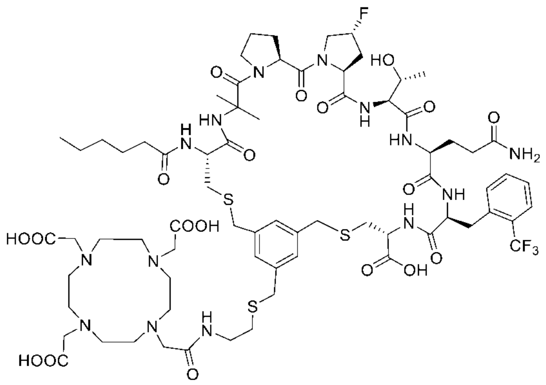

Hex-[Cys-(tMeBn(DOTA-AET))-Aib-Pro-((4R)-F-Pro)-Thr-Gln-(2-$CF_3$-Phe)-Cys]-OH,

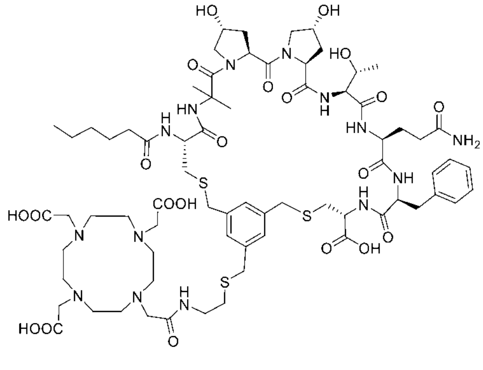

Hex-[Cys-(tMeBn(DOTA-AET))-Aib-((4R)-OH-Pro)-((4R)-OH-Pro)-Thr-Gln-Phe-Cys]-OH,

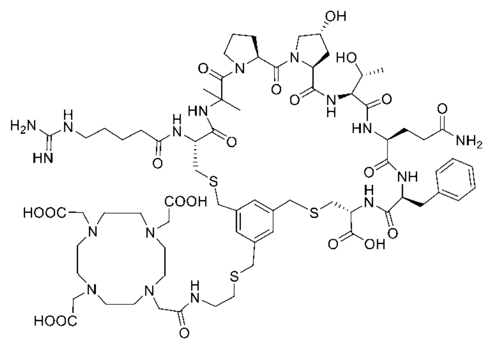

(5-guanidineValy)-[Cys-(tMeBn(DOTA-AET))-Aib-Pro-((4R)-OH-Pro)-Thr-Gln-Phe-Cys]-OH,

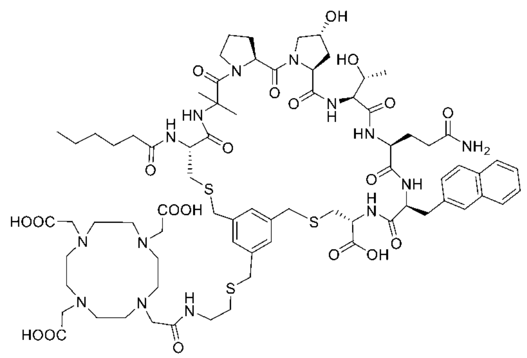

Hex-[Cys-(tMeBn(DOTA-AET))-Aib-Pro-((4R)-OH-Pro)-Thr-Gln-(2-Nal)-Cys]-OH,

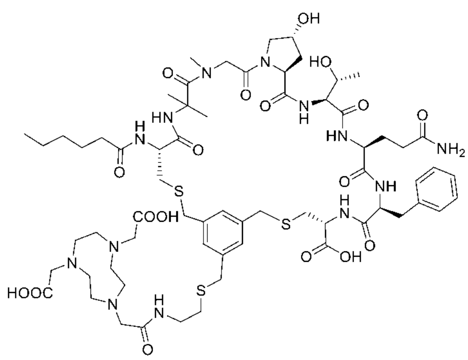

Hex-[Cys-(tMeBn(NOTA-AET))-Aib-Sar-((4R)-OH-Pro)-Thr-Gln-Phe-Cys]-OH,

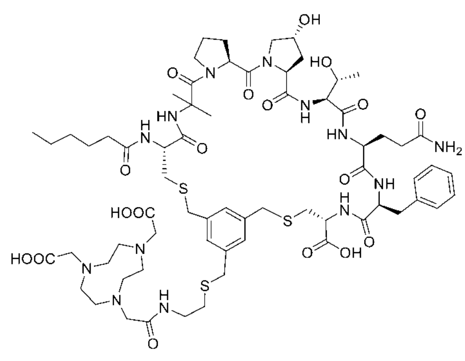

Hex-[Cys-(tMeBn(NOTA-AET))-Aib-Pro-((4R)-OH-Pro)-Thr-Gln-Phe-Cys]-OH,

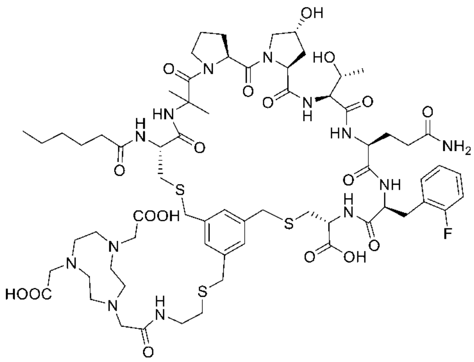

Hex-[Cys-(tMeBn(NOTA-AET))-Aib-Pro-((4R)-OH-Pro)-Thr-Gln-(2-F-Phe)-Cys]-OH,

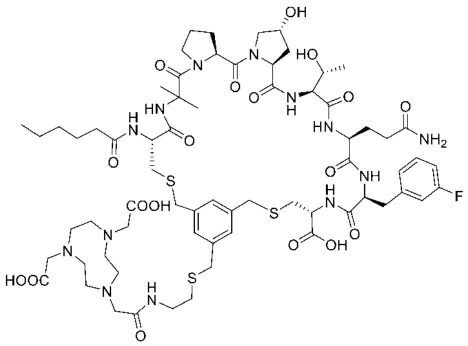

Hex-[Cys-(tMeBn(NOTA-AET))-Aib-Pro-((4R)-OH-Pro)-Thr-Gln-(3-F-Phe)-Cys]-OH,

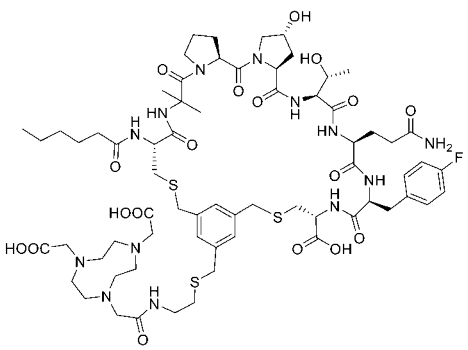

Hex-[Cys-(tMeBn(NOTA-AET))-Aib-Pro-((4R)-OH-Pro)-Thr-Gln-(4-F-Phe)-Cys]-OH,

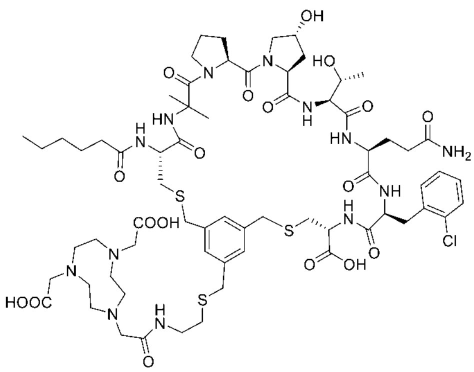

Hex-[Cys-(tMeBn(NOTA-AET))-Aib-Pro-((4R)-OH-Pro)-Thr-Gln-(2-Cl-Phe)-Cys]-OH,

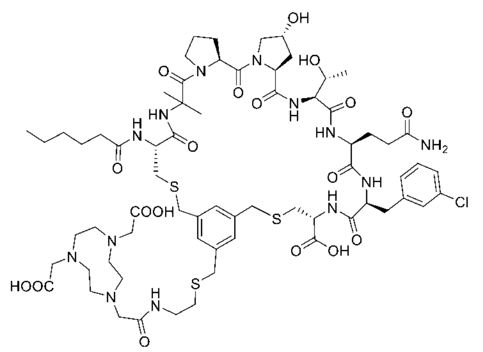

Hex-[Cys-(tMeBn(NOTA-AET))-Aib-Pro-((4R)-OH-Pro)-Thr-Gln-(3-Cl-Phe)-Cys]-OH,

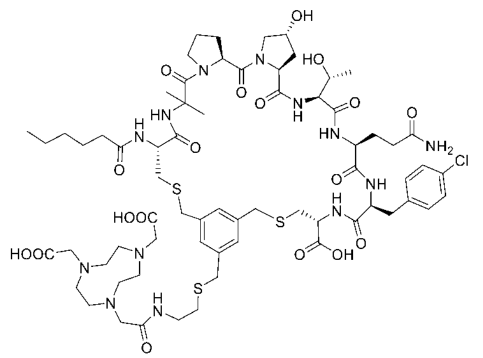

Hex-[Cys-(tMeBn(NOTA-AET))-Aib-Pro-((4R)-OH-Pro)-Thr-Gln-(4-Cl-Phe)-Cys]-OH,

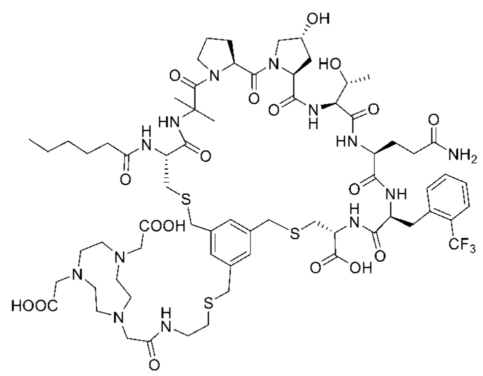

Hex-[Cys-(tMeBn(NOTA-AET))-Aib-Pro-((4R)-OH-Pro)-Thr-Gln-(2-$CF_3$-Phe)-Cys]-OH,

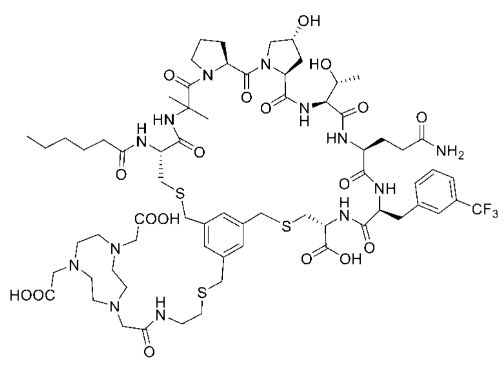

Hex-[Cys-(tMeBn(NOTA-AET))-Aib-Pro-((4R)-OH-Pro)-Thr-Gln-(3-$CF_3$-Phe)-Cys]-OH,

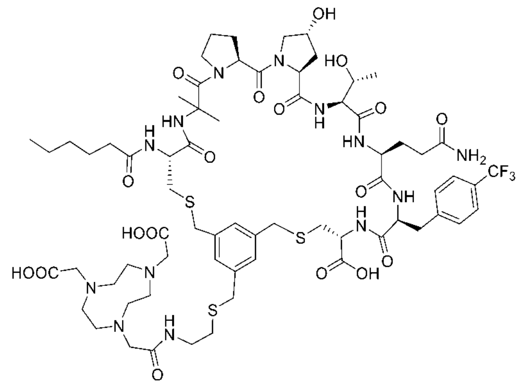

Hex-[Cys-(tMeBn(NOTA-AET))-Aib-Pro-((4R)-OH-Pro)-Thr-Gln-(4-$CF_3$-Phe)-Cys]-OH,

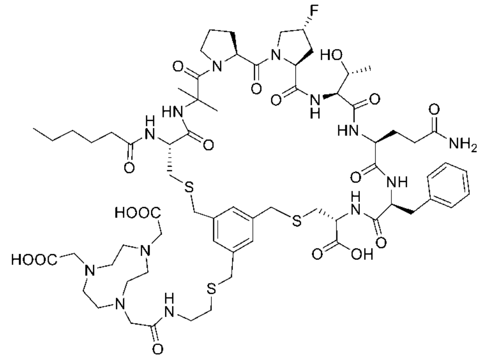

Hex-[Cys-(tMeBn(NOTA-AET))-Aib-Pro-((4R)-F-Pro)-Thr-Gln-Phe-Cys]-OH,

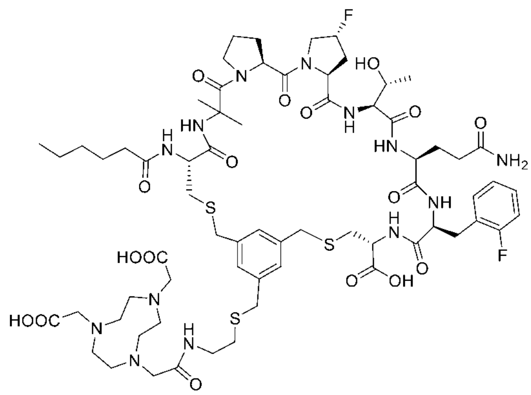

Hex-[Cys-(tMeBn(NOTA-AET))-Aib-Pro-((4R)-F-Pro)-Thr-Gln-(2-F-Phe)-Cys]-OH,

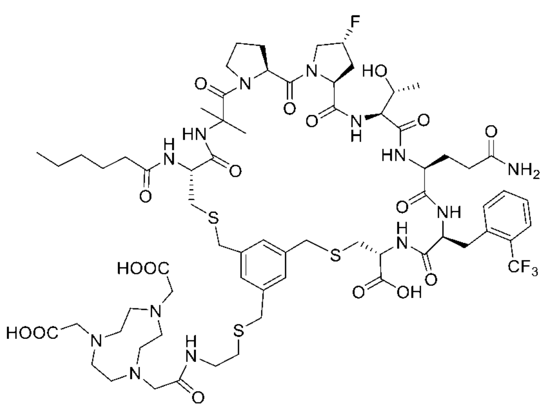

Hex-[Cys-(tMeBn(NOTA-AET))-Aib-Pro-((4R)-F-Pro)-Thr-Gln-(2-$CF_3$-Phe)-Cys]-OH; group 6:

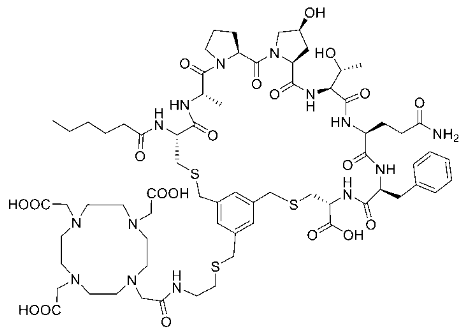

Hex-[Cys-(tMeBn(DOTA-AET))-Ala-Pro-((4S)-OH-Pro)-Thr-Gln-Phe-Cys]-OH,

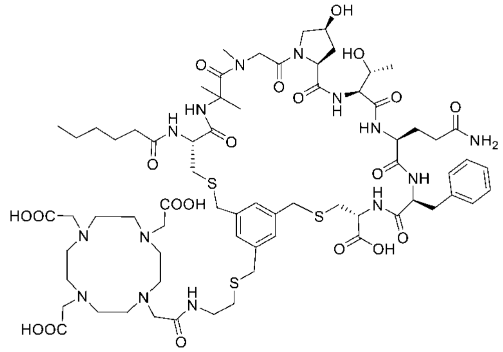

Hex-[Cys-(tMeBn(DOTA-AET))-Aib-Sar-((4S)-OH-Pro)-Thr-Gln-Phe-Cys]-OH,

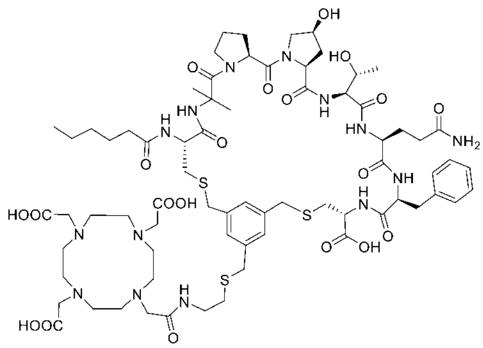

Hex-[Cys-(tMeBn(DOTA-AET))-Aib-Pro-((4S)-OH-Pro)-Thr-Gln-Phe-Cys]-OH,

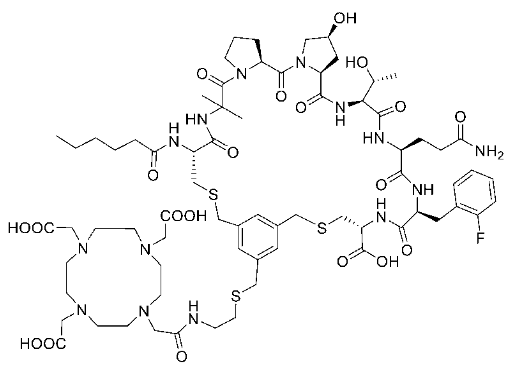

Hex-[Cys-(tMeBn(DOTA-AET))-Aib-Pro-((4S)-OH-Pro)-Thr-Gln-(2-F-Phe)-Cys]-OH,

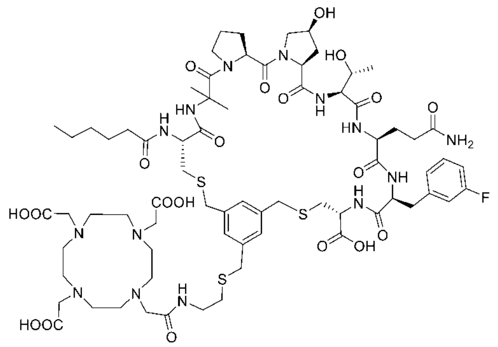

Hex-[Cys-(tMeBn(DOTA-AET))-Aib-Pro-((4S)-OH-Pro)-Thr-Gln-(3-F-Phe)-Cys]-OH,

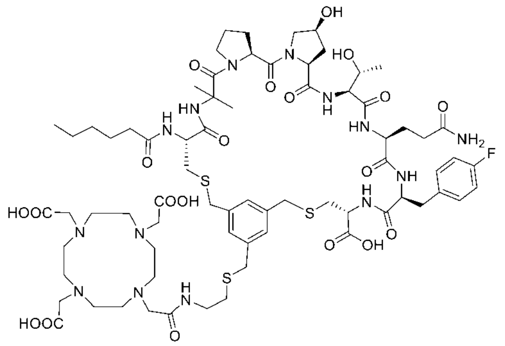

Hex-[Cys-(tMeBn(DOTA-AET))-Aib-Pro-((4S)-OH-Pro)-Thr-Gln-(4-F-Phe)-Cys]-OH,

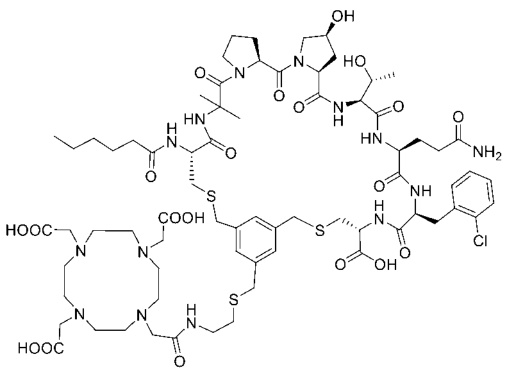

Hex-[Cys-(tMeBn(DOTA-AET))-Aib-Pro-((4S)-OH-Pro)-Thr-Gln-(2-Cl-Phe)-Cys]-OH,

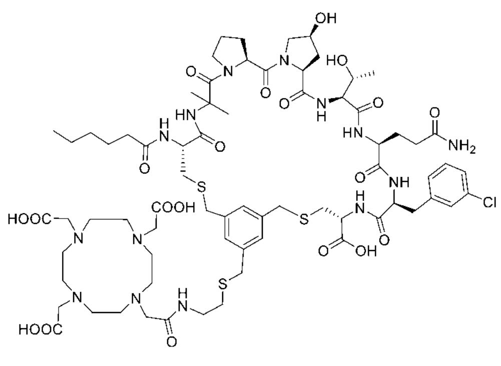

Hex-[Cys-(tMeBn(DOTA-AET))-Aib-Pro-((4S)-OH-Pro)-Thr-Gln-(3-Cl-Phe)-Cys]-OH,

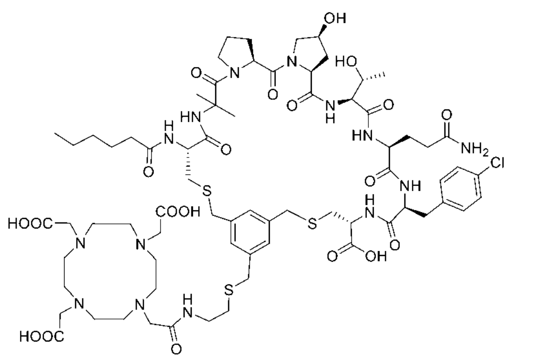

Hex-[Cys-(tMeBn(DOTA-AET))-Aib-Pro-((4S)-OH-Pro)-Thr-Gln-(4-Cl-Phe)-Cys]-OH,

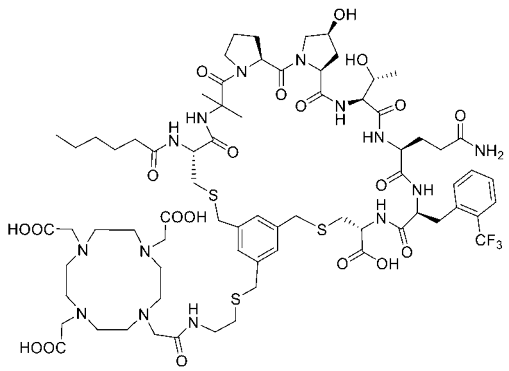

Hex-[Cys-(tMeBn(DOTA-AET))-Aib-Pro-((4S)-OH-Pro)-Thr-Gln-(2-$CF_3$-Phe)-Cys]-OH,

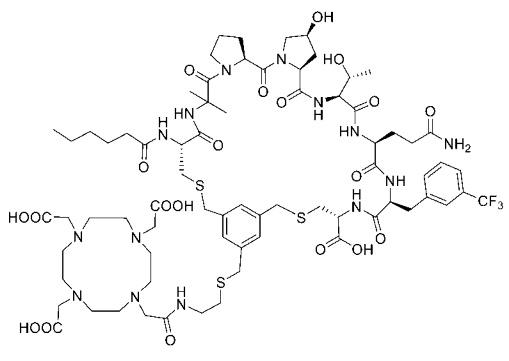

Hex-[Cys-(tMeBn(DOTA-AET))-Aib-Pro-((4S)-OH-Pro)-Thr-Gln-(3-$CF_3$-Phe)-Cys]-OH,

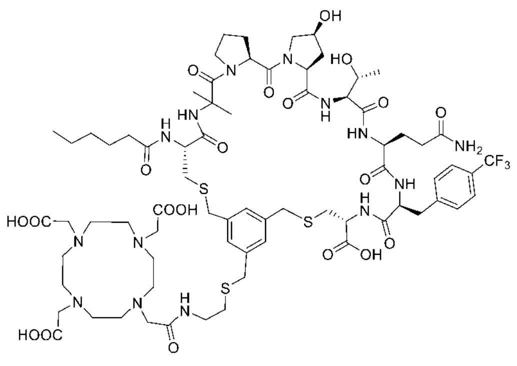

Hex-[Cys-(tMeBn(DOTA-AET))-Aib-Pro-((4S)-OH-Pro)-Thr-Gln-(4-$CF_3$-Phe)-Cys]-OH,

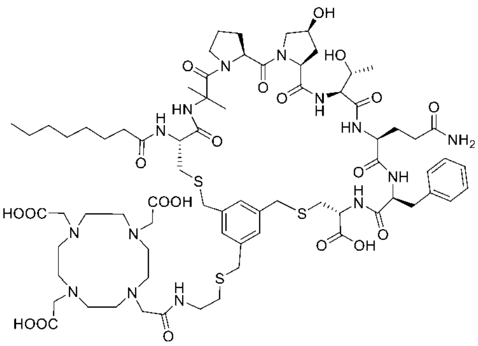

Oct-[Cys-(tMeBn(DOTA-AET))-Aib-Pro-((4S)-OH-Pro)-Thr-Gln-Phe-Cys]-OH,

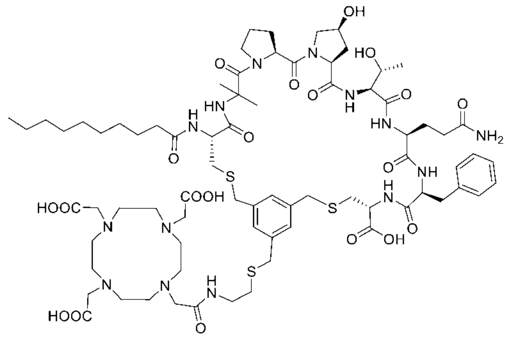

Dec-[Cys-(tMeBn(DOTA-AET))-Aib-Pro-((4S)-OH-Pro)-Thr-Gln-Phe-Cys]-OH,

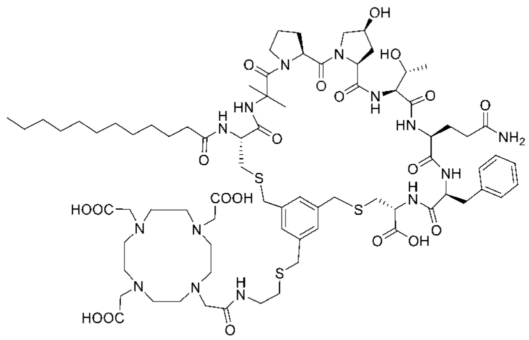

Dod-[Cys-(tMeBn(DOTA-AET))-Aib-Pro-((4S)-OH-Pro)-Thr-Gln-Phe-Cys]-OH,

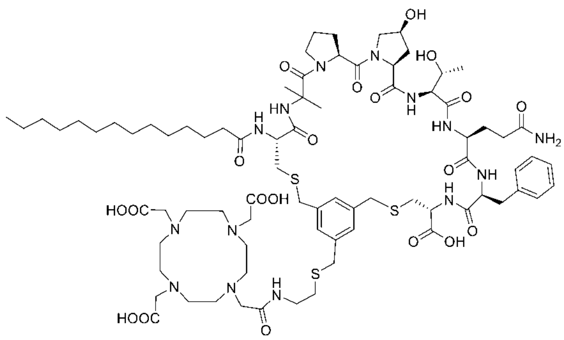

Tet-[Cys-(tMeBn(DOTA-AET))-Aib-Pro-((4S)-OH-Pro)-Thr-Gln-Phe-Cys]-OH,

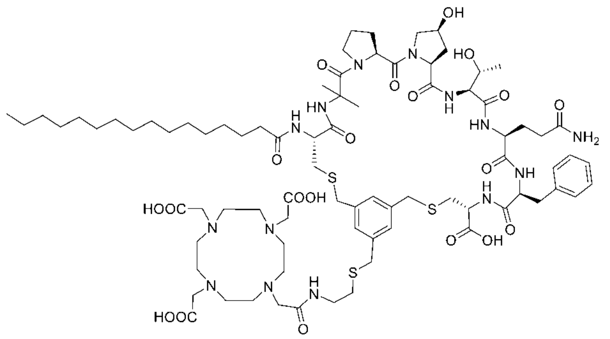

Pal-[Cys-(tMeBn(DOTA-AET))-Aib-Pro-((4S)-OH-Pro)-Thr-Gln-Phe-Cys]-OH,

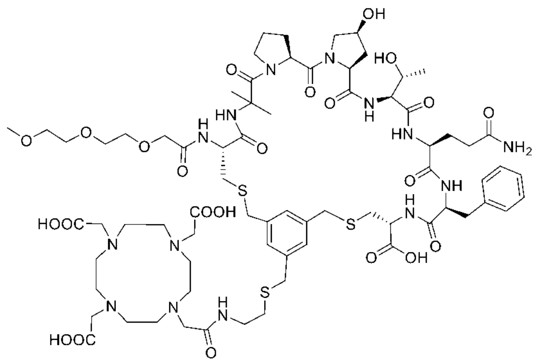

{2-[2-(2-Methoxyethoxy)ethoxy]-Ace}-[Cys-(tMeBn(DOTA-AET))-Aib-Pro-((4S)-OH-Pro)-Thr-Gln-Phe-Cys]-OH,

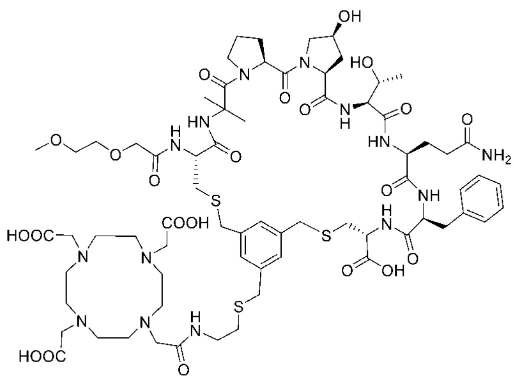

[2-(2-Methoxyethoxy)-Ace]-[Cys-(tMeBn(DOTA-AET))-Aib-Pro-((4S)-OH-Pro)-Thr-Gln-Phe-Cys]-OH,

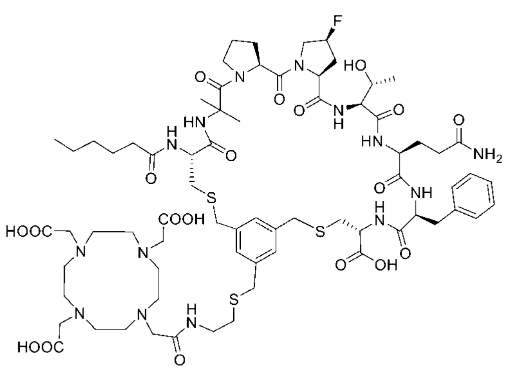

Hex-[Cys-(tMeBn(DOTA-AET))-Aib-Pro-((4S)-F-Pro)-Thr-Gln-Phe-Cys]-OH,

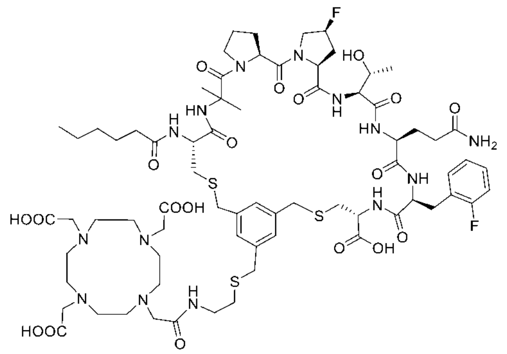

Hex-[Cys-(tMeBn(DOTA-AET))-Aib-Pro-((4S)-F-Pro)-Thr-Gln-(2-F-Phe)-Cys]-OH,

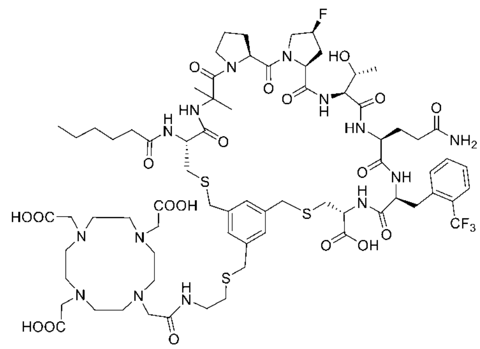

Hex-[Cys-(tMeBn(DOTA-AET))-Aib-Pro-((4S)-F-Pro)-Thr-Gln-(2-$CF_3$-Phe)-Cys]-OH,

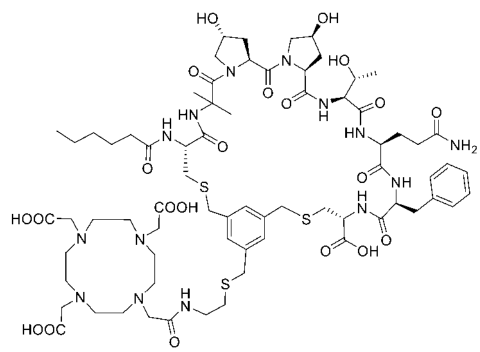

Hex-[Cys-(tMeBn(DOTA-AET))-Aib-((4R)-OH-Pro)-((4S)-OH-Pro)-Thr-Gln-Phe-Cys]-OH,

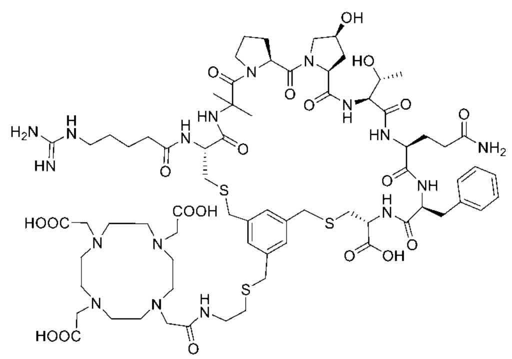

(5-guanidineValy)-[Cys-(tMeBn(DOTA-AET))-Aib-Pro-((4S)-OH-Pro)-Thr-Gln-Phe-Cys]-OH,

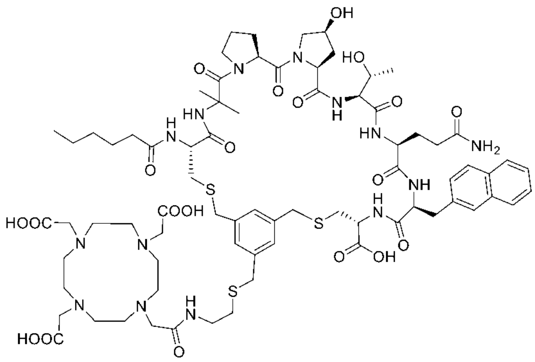

Hex-[Cys-(tMeBn(DOTA-AET))-Aib-Pro-((4S)-OH-Pro)-Thr-Gln-(2-Nal)-Cys]-OH,

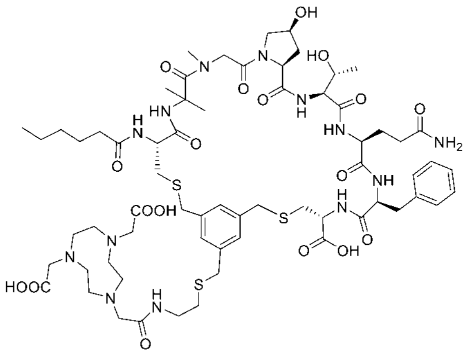

Hex-[Cys-(tMeBn(NOTA-AET))-Aib-Sar-((4S)-OH-Pro)-Thr-Gln-Phe-Cys]-OH,

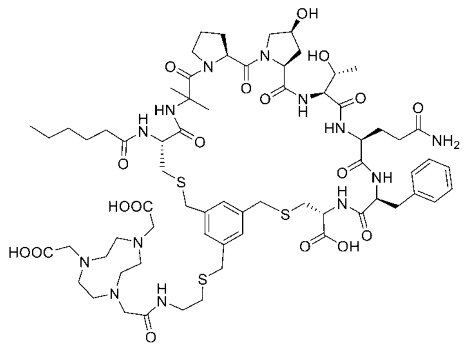

Hex-[Cys-(tMeBn(NOTA-AET))-Aib-Pro-((4S)-OH-Pro)-Thr-Gln-Phe-Cys]-OH,

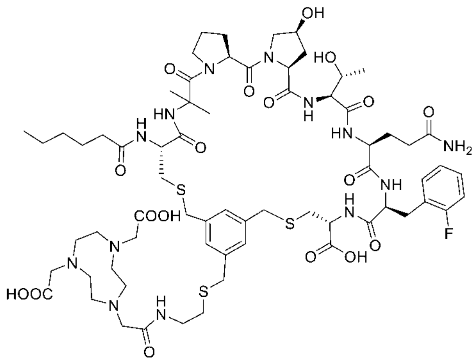

Hex-[Cys-(tMeBn(NOTA-AET))-Aib-Pro-((4S)-OH-Pro)-Thr-Gln-(2-F-Phe)-Cys]-OH,

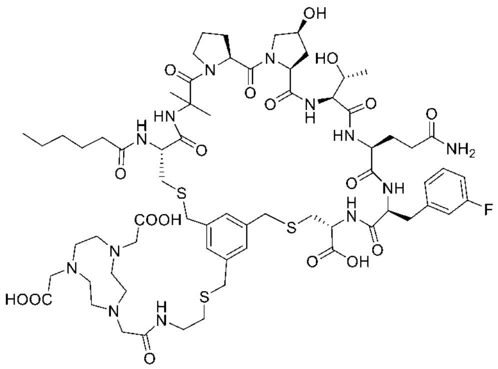

Hex-[Cys-(tMeBn(NOTA-AET))-Aib-Pro-((4S)-OH-Pro)-Thr-Gln-(3-F-Phe)-Cys]-OH,

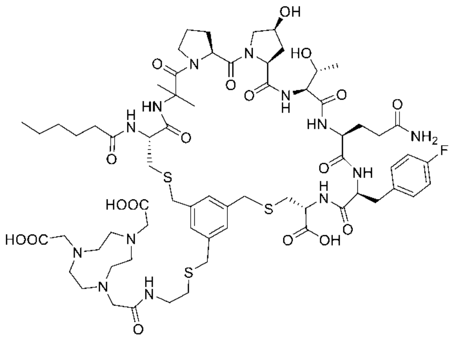

Hex-[Cys-(tMeBn(NOTA-AET))-Aib-Pro-((4S)-OH-Pro)-Thr-Gln-(4-F-Phe)-Cys]-OH,

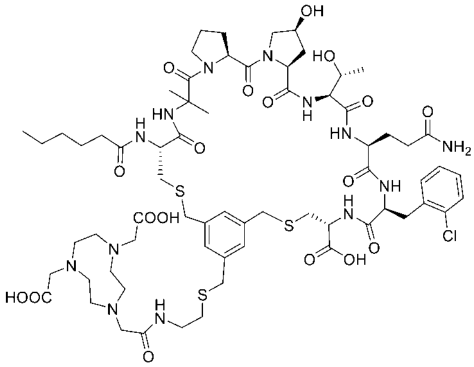

Hex-[Cys-(tMeBn(NOTA-AET))-Aib-Pro-((4S)-OH-Pro)-Thr-Gln-(2-Cl-Phe)-Cys]-OH,

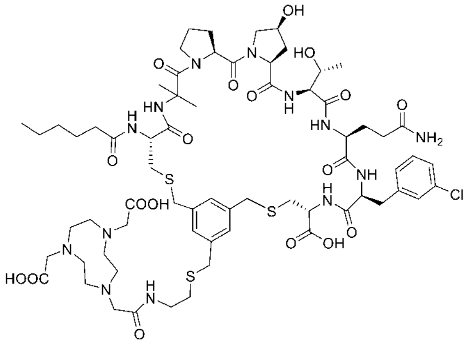

Hex-[Cys-(tMeBn(NOTA-AET))-Aib-Pro-((4S)-OH-Pro)-Thr-Gln-(3-Cl-Phe)-Cys]-OH,

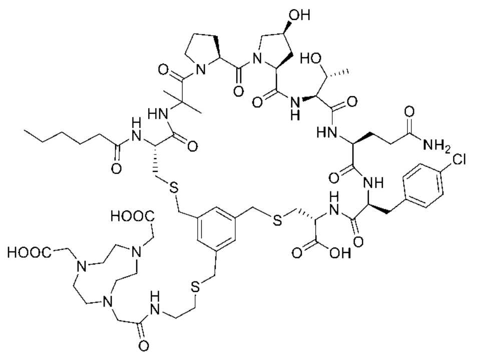

Hex-[Cys-(tMeBn(NOTA-AET))-Aib-Pro-((4S)-OH-Pro)-Thr-Gln-(4-Cl-Phe)-Cys]-OH,

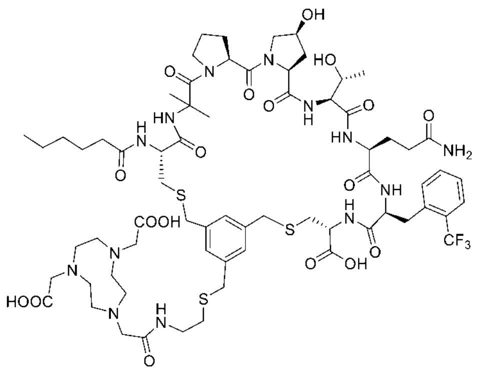

Hex-[Cys-(tMeBn(NOTA-AET))-Aib-Pro-((4S)-OH-Pro)-Thr-Gln-(2-$CF_3$-Phe)-Cys]-OH,

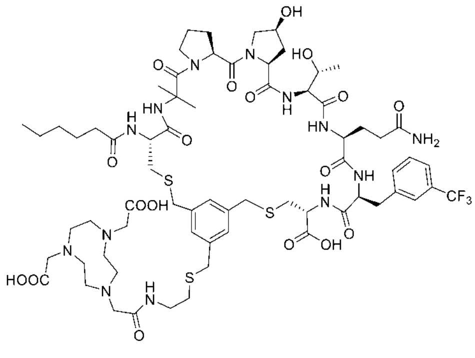

Hex-[Cys-(tMeBn(NOTA-AET))-Aib-Pro-((4S)-OH-Pro)-Thr-Gln-(3-$CF_3$-Phe)-Cys]-OH,

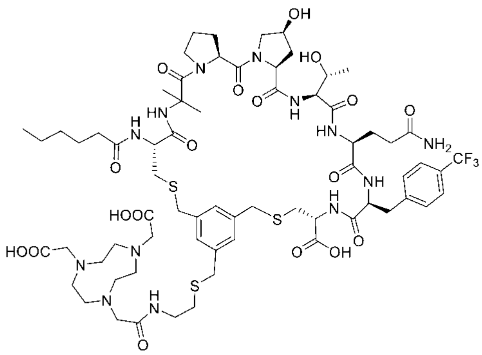

Hex-[Cys-(tMeBn(NOTA-AET))-Aib-Pro-((4S)-OH-Pro)-Thr-Gln-(4-$CF_3$-Phe)-Cys]-OH,

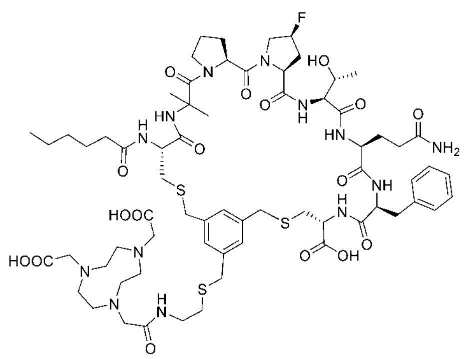

Hex-[Cys-(tMeBn(NOTA-AET))-Aib-Pro-((4S)-F-Pro)-Thr-Gln-Phe-Cys]-OH,

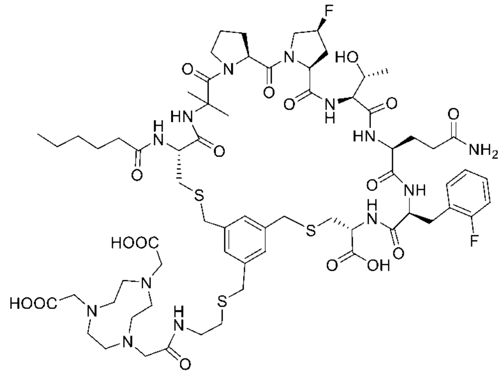

Hex-[Cys-(tMeBn(NOTA-AET))-Aib-Pro-((4S)-F-Pro)-Thr-Gln-(2-F-Phe)-Cys]-OH,

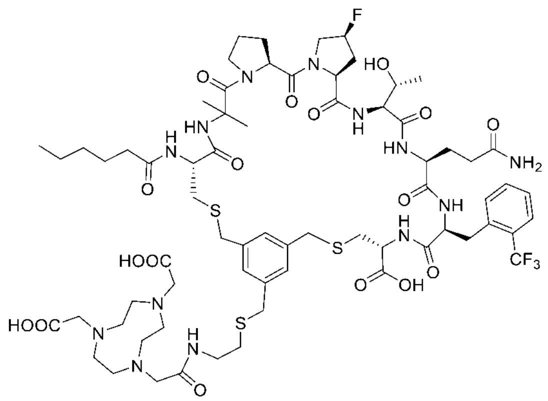

Hex-[Cys-(tMeBn(NOTA-AET))-Aib-Pro-((4S)-F-Pro)-Thr-Gln-(2-$CF_3$-Phe)-Cys]-OH.

**24.** The compound as shown in formula (I) or the pharmaceutically acceptable salt thereof according to any one of claims 1 to 23, **characterised in that** the chelating agent complexes a nuclide, the nuclide comprises a diagnostically active nuclide or a therapeutically active nuclide, and the diagnostically active nuclide is selected from $^{43}$Sc, $^{44}$Sc, $^{51}$Mn, $^{52}$Mn, $^{64}$Cu, $^{67}$Ga, $^{68}$Ga, $^{86}$Y, $^{89}$Zr, $^{94m}$Tc, $^{99m}$Tc, $^{111}$In, $^{152}$Tb, $^{155}$Tb, $^{201}$Tl, $^{203}$Pb, $^{18}$F, $^{76}$Br, $^{77}$Br, $^{123}$I, $^{124}$I and $^{125}$I, preferably $^{43}$Sc, $^{44}$Sc, 6$^{4}$Cu, $^{67}$Ga, $^{63}$Ga, $^{36}$Y, $^{81}$Zr, $^{99m}$Tc, $^{111}$In, $^{152}$Tb, $^{155}$Tb, $^{203}$Pb, $^{18}$F, $^{76}$Br, $^{77}$Br $^{123}$I, $^{124}$I and $^{125}$I, and most preferably $^{64}$Cu, $^{68}$Ga, $^{89}$Zr, $^{99m}$Tc, $^{111}$In, $^{18}$ F, $^{123}$I and $^{124}$I, the therapeutically active nuclide is selected from $^{47}$Sc, $^{67}$Cu, $^{89}$Sr, $^{90}$Y, $^{153}$Sm, $^{149}$Tb, $^{161}$Tb, $^{177}$Lu, $^{186}$Re, $^{188}$Re, $^{212}$Pb, $^{213}$Bi, $^{223}$Ra, $^{225}$Ac, $^{226}$Th, $^{227}$Th, $^{131}$I and $^{211}$At, preferably $^{47}$Sc, $^{67}$Cu, $^{90}$Y, $^{177}$Lu, $^{188}$Re, $^{212}$Pb, $^{213}$Bi, $^{225}$Ac, $^{227}$Th, $^{131}$I and $^{211}$At, and most preferably $^{90}$Y, $^{177}$Lu, $^{225}$Ac, $^{227}$Th, $^{131}$I and $^{211}$At.

**25.** The compound as shown in formula (I) or the pharmaceutically acceptable salt thereof according to any one of claims 1 to 24, **characterised in that** the compound is selected from:

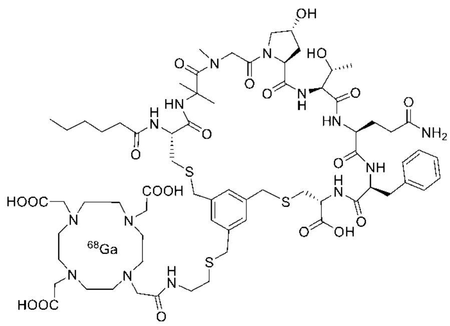 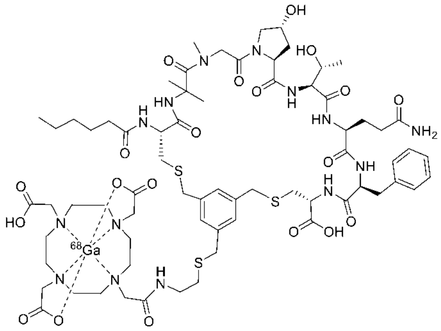

Hex-[Cys-(tMeBn($^{68}$Ga-DOTA-AET))-Aib-Sar-((4R)-OH-Pro)-Thr-Gln-Phe-Cys]-OH,

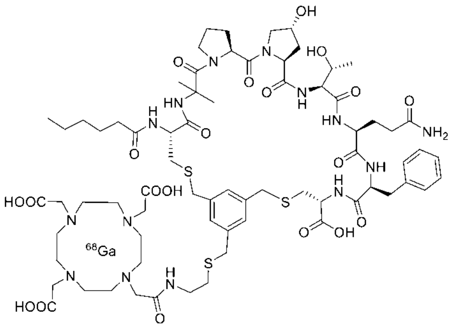 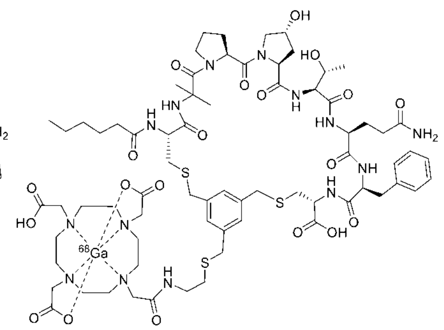

Hex-[Cys-(tMeBn($^{68}$Ga-DOTA-AET))-Aib-Pro-((4R)-OH-Pro)-Thr-Gln-Phe-Cys]-OH,

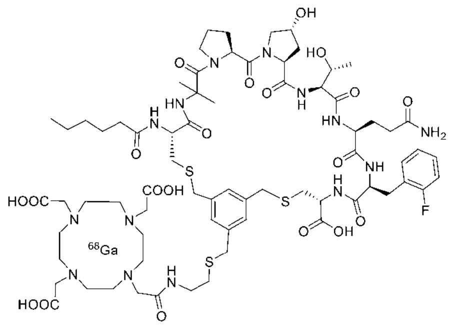 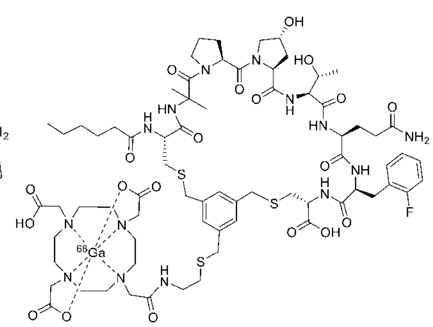

Hex-[Cys-(tMeBn($^{68}$Ga-DOTA-AET))-Aib-Pro-((4R)-OH-Pro)-Thr-Gln-(2-F-Phe)-Cys]-OH,

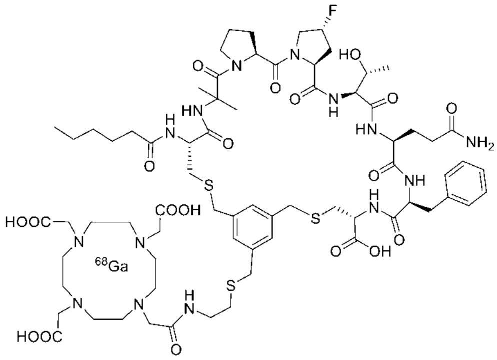 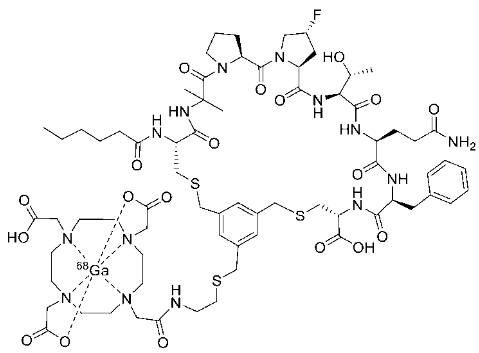

Hex-[Cys-(tMeBn($^{68}$Ga-DOTA-AET))-Aib-Pro-((4R)-F-Pro)-Thr-Gln-Phe-Cys]-OH,

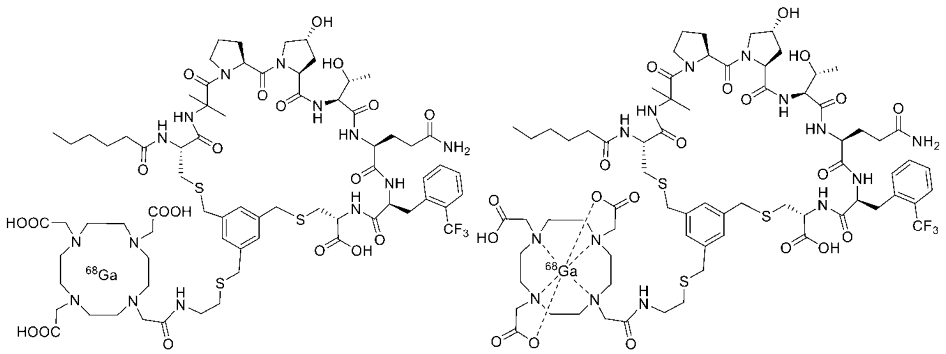

Hex-[Cys-(tMeBn($^{68}$Ga-DOTA-AET))-Aib-Pro-((4R)-OH-Pro)-Thr-Gln-(2-$CF_3$-Phe)-Cys]-OH,

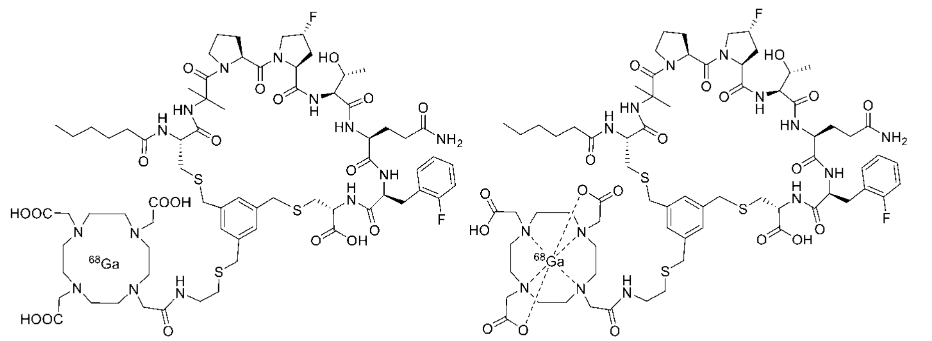

Hex-[Cys-(tMeBn($^{68}$Ga-DOTA-AET))-Aib-Pro-((4R)-F-Pro)-Thr-Gln-(2-F-Phe)-Cys]-OH,

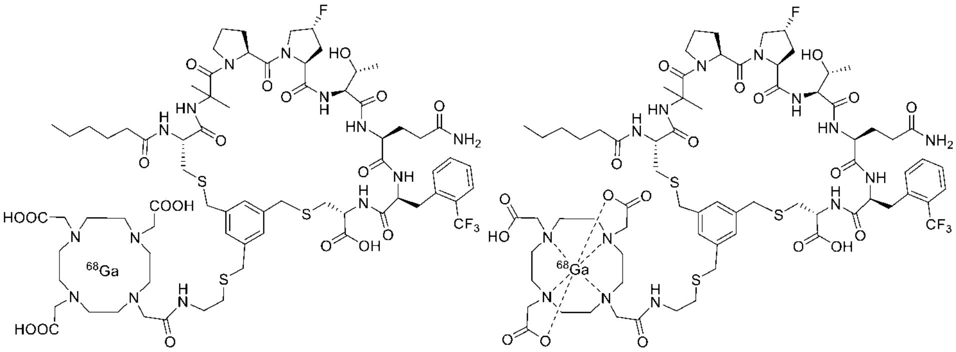

Hex-[Cys-(tMeBn($^{68}$Ga-DOTA-AET))-Aib-Pro-((4R)-F-Pro)-Thr-Gln-(2-$CF_3$-Phe)-Cys]-OH,

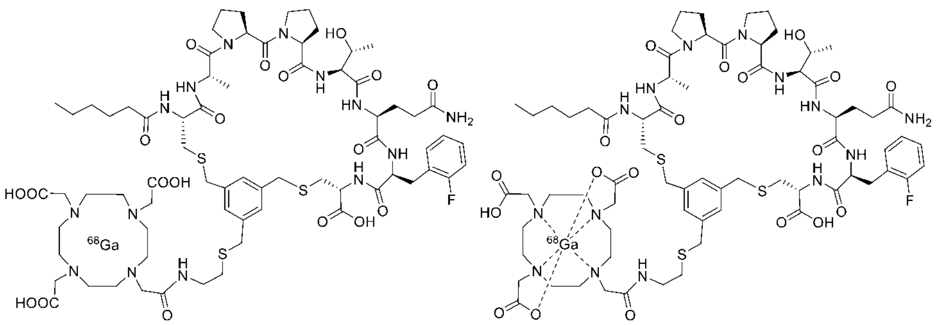

Hex-[Cys-(tMeBn($^{68}$Ga-DOTA-AET))-Ala-Pro-Pro-Thr-Gln-(2-F-Phe)-Cys]-OH,

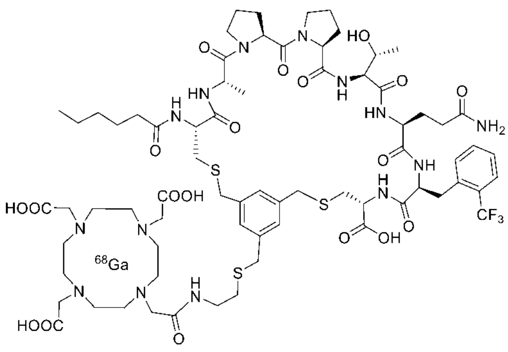
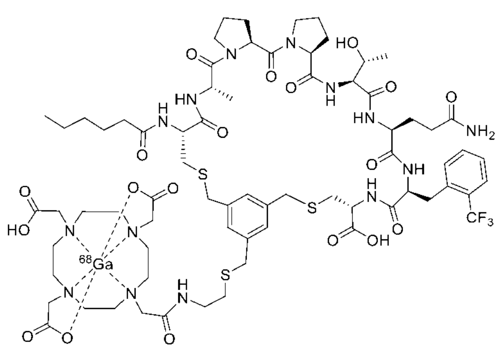

Hex-[Cys-(tMeBn($^{68}$Ga-DOTA-AET))-Ala-Pro-Pro-Thr-Gln-(2-$CF_3$-Phe)-Cys]-OH,

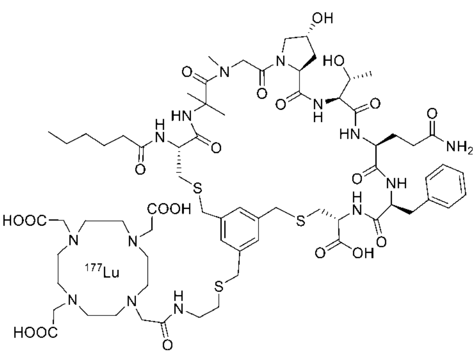
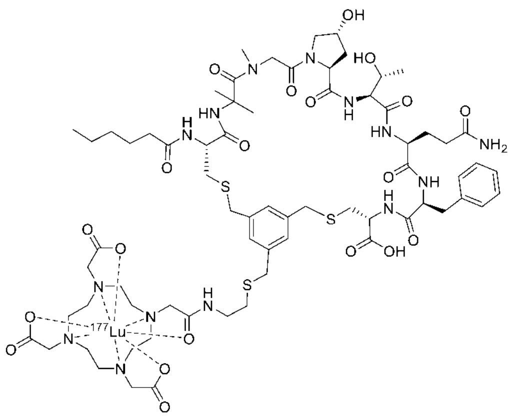

Hex-[Cys-(tMeBn($^{177}$Lu-DOTA-AET))-Aib-Sar-((4R)-OH-Pro)-Thr-Gln-Phe-Cys]-OH,

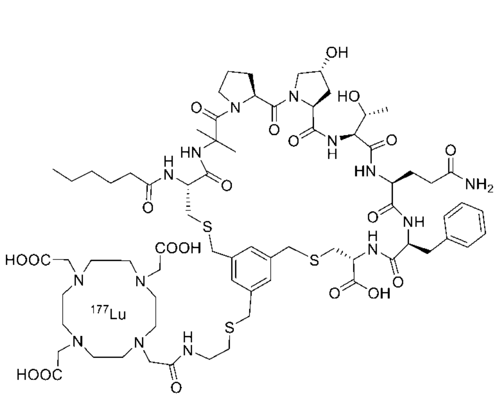
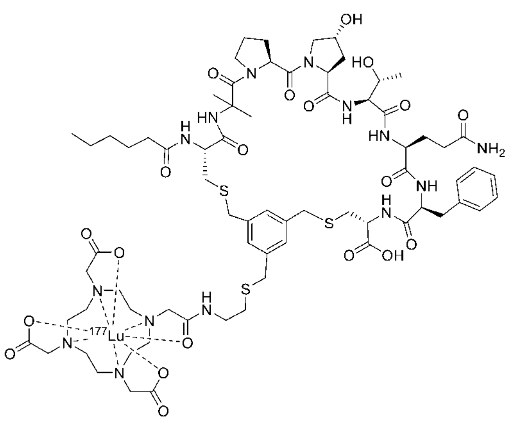

Hex-[Cys-(tMeBn($^{177}$Lu-DOTA-AET))-Aib-Pro-((4R)-OH-Pro)-Thr-Gln-Phe-Cys]-OH,

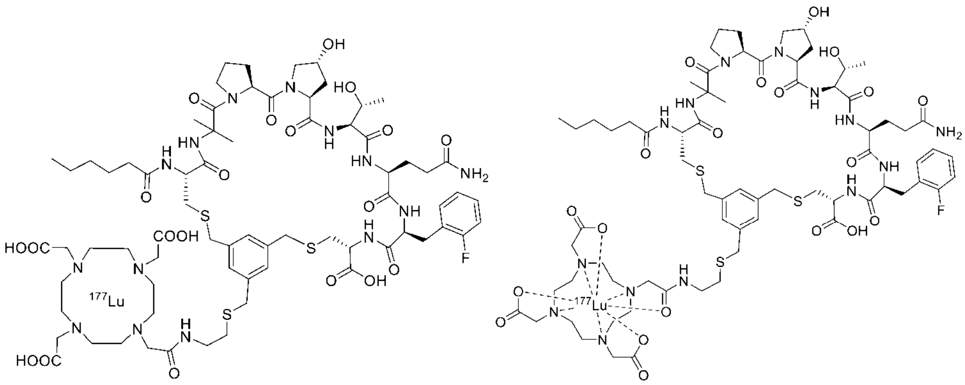

Hex-[Cys-(tMeBn($^{177}$Lu-DOTA-AET))-Aib-Pro-((4R)-OH-Pro)-Thr-Gln-(2-F-Phe)-Cys]-OH,

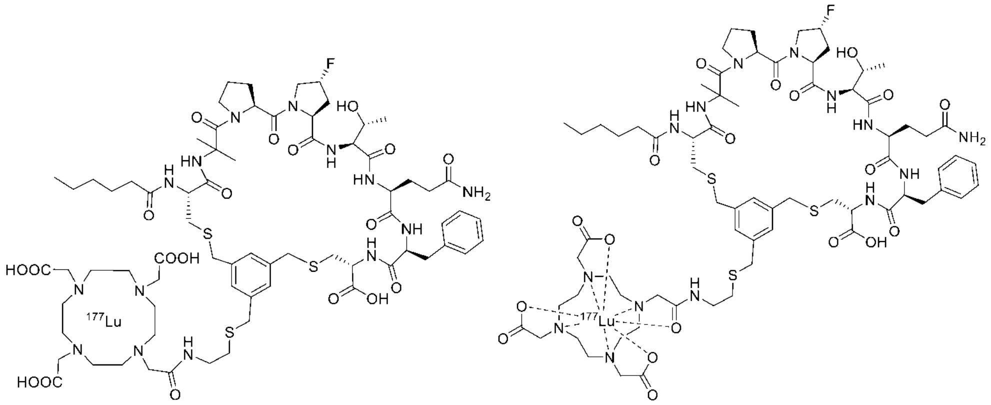

Hex-[Cys-(tMeBn($^{177}$Lu-DOTA-AET))-Aib-Pro-((4R)-F-Pro)-Thr-Gln-Phe-Cys]-OH,

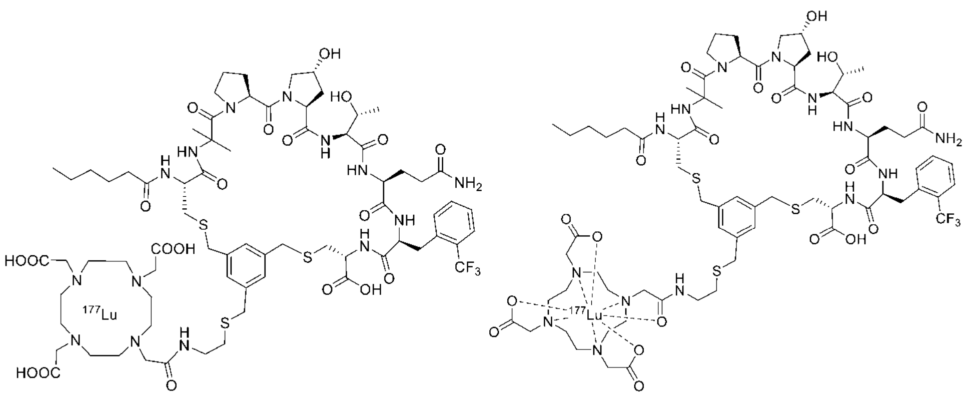

Hex-[Cys-(tMeBn($^{177}$Lu-DOTA-AET))-Aib-Pro-((4R)-OH-Pro)-Thr-Gln-(2-$CF_3$-Phe)-Cys]-OH,

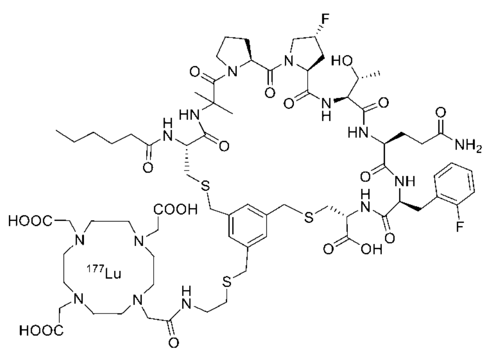
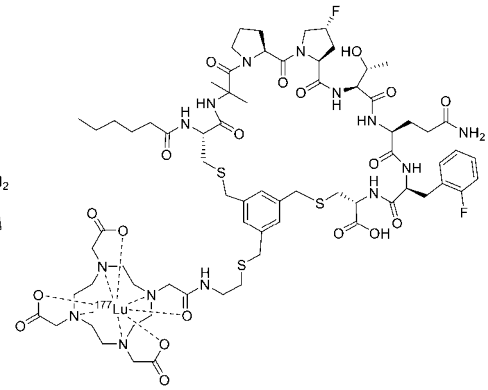

Hex-[Cys-(tMeBn($^{177}$Lu-DOTA-AET))-Aib-Pro-((4R)-F-Pro)-Thr-Gln-(2-F-Phe)-Cys]-OH,

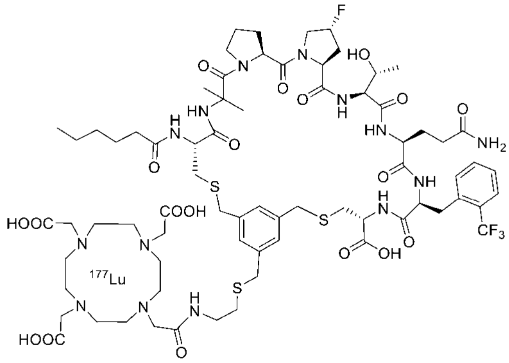
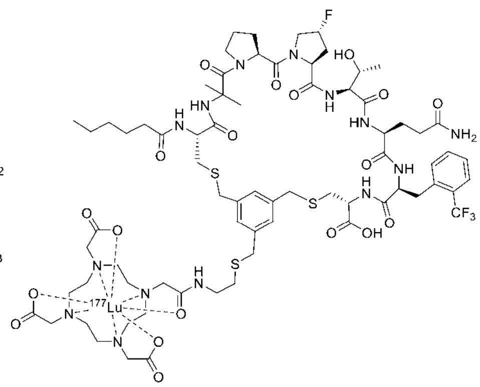

Hex-[Cys-(tMeBn($^{177}$Lu-DOTA-AET))-Aib-Pro-((4R)-F-Pro)-Thr-Gln-(2-$CF_3$-Phe)-Cys]-OH,

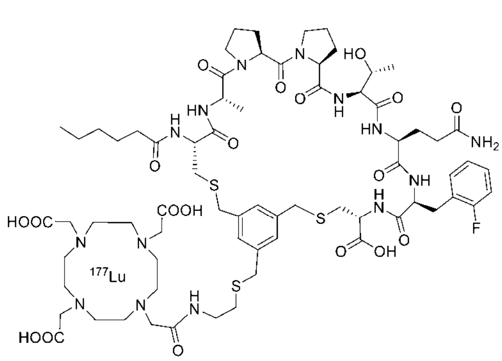
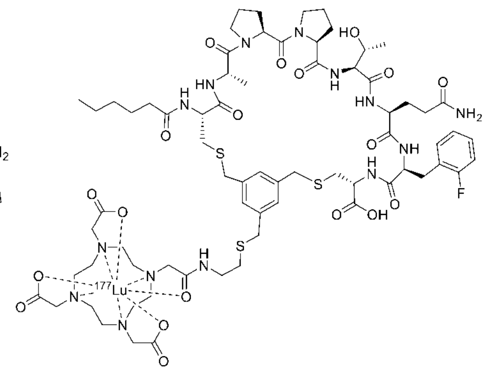

Hex-[Cys-(tMeBn($^{177}$Lu-DOTA-AET))-Ala-Pro-Pro-Thr-Gln-(2-F-Phe)-Cys]-OH,

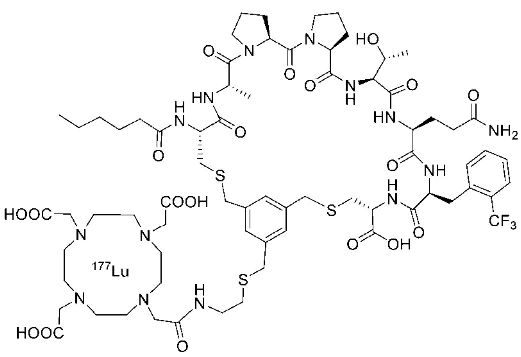
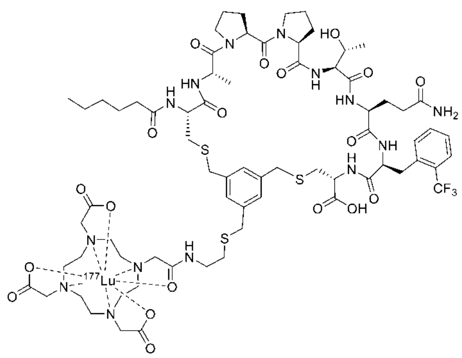

Hex-[Cys-(tMeBn($^{177}$Lu-DOTA-AET))-Ala-Pro-Pro-Thr-Gln-(2-$CF_3$-Phe)-Cys]-OH,

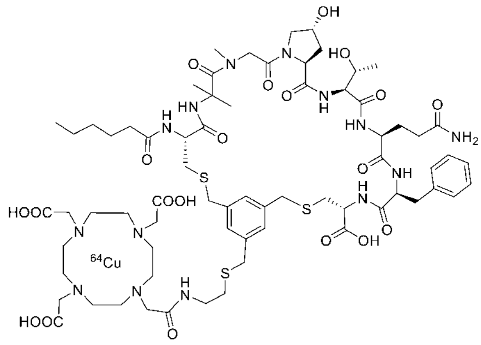
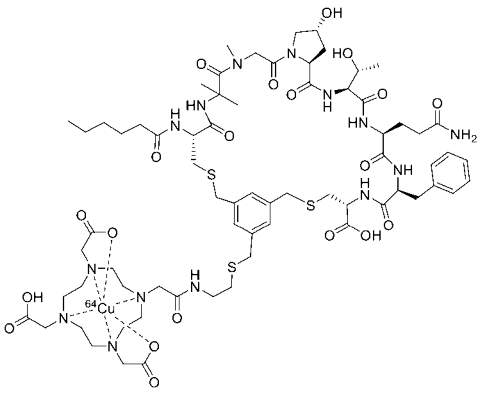

Hex-[Cys-(tMeBn($^{64}$Cu-DOTA-AET))-Aib-Sar-((4R)-OH-Pro)-Thr-Gln-Phe-Cys]-OH,

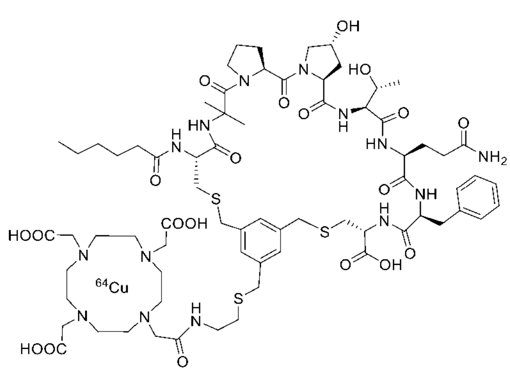
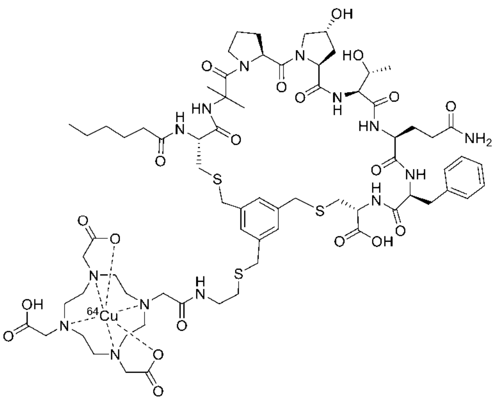

Hex-[Cys-(tMeBn($^{64}$Cu-DOTA-AET))-Aib-Pro-((4R)-OH-Pro)-Thr-Gln-Phe-Cys]-OH,

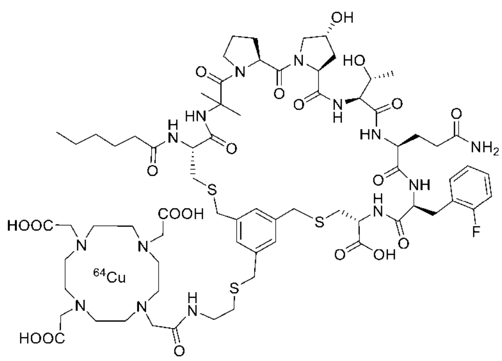
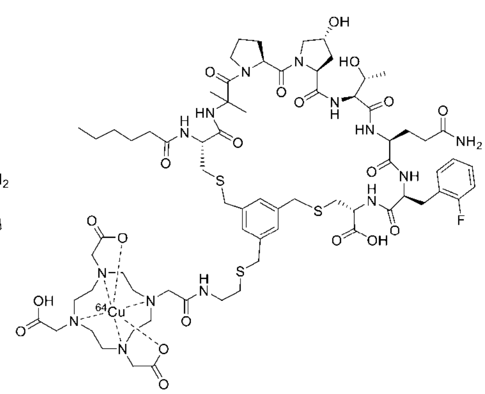

Hex-[Cys-(tMeBn($^{64}$Cu-DOTA-AET))-Aib-Pro-((4R)-OH-Pro)-Thr-Gln-(2-F-Phe)-Cys]-OH,

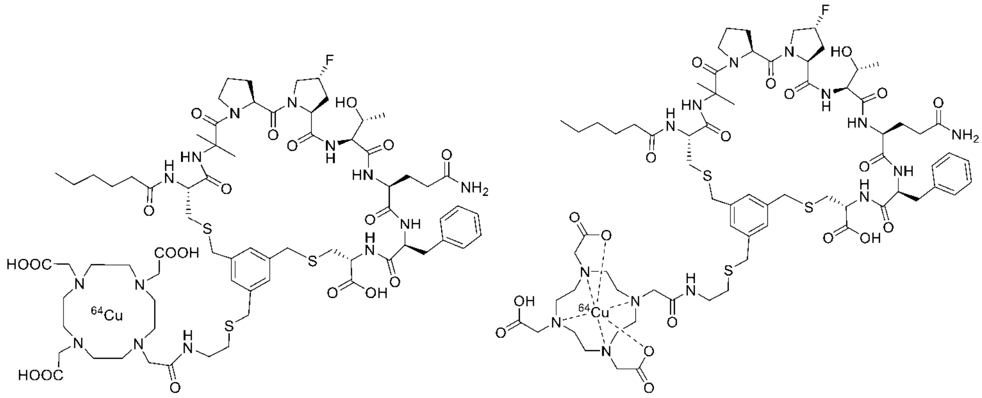

Hex-[Cys-(tMeBn($^{64}$Cu-DOTA-AET))-Aib-Pro-((4R)-F-Pro)-Thr-Gln-Phe-Cys]-OH,

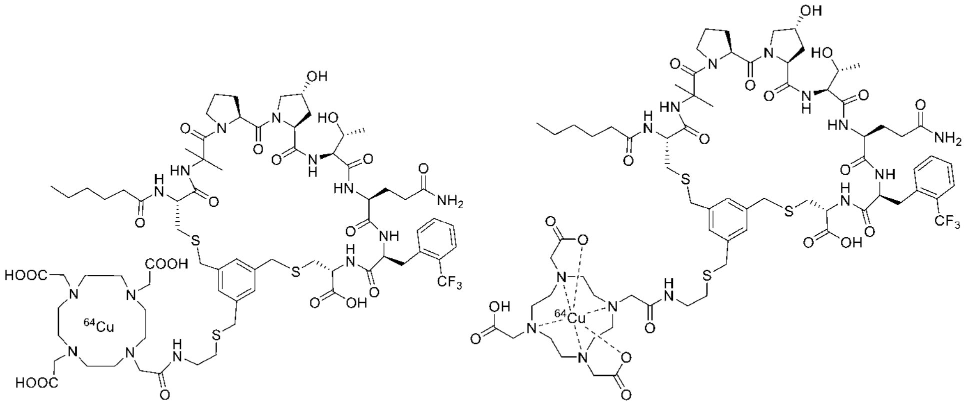

Hex-[Cys-(tMeBn($^{64}$Cu-DOTA-AET))-Aib-Pro-((4R)-OH-Pro)-Thr-Gln-(2-$CF_3$-Phe)-Cys]-OH,

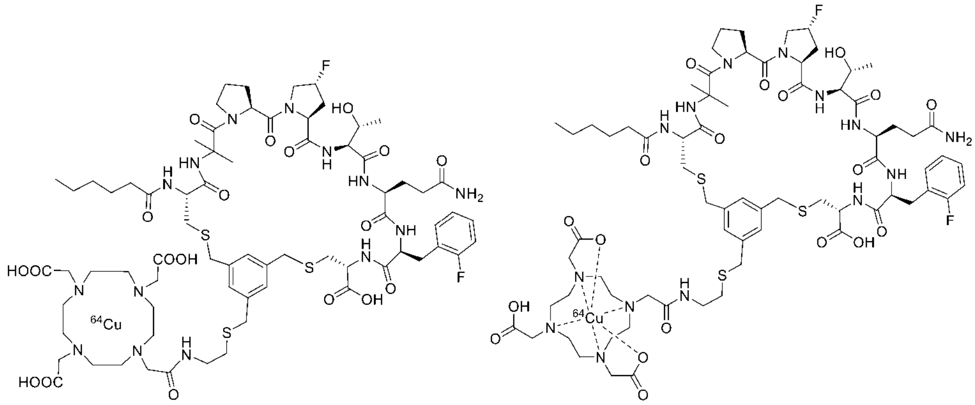

Hex-[Cys-(tMeBn($^{64}$Cu-DOTA-AET))-Aib-Pro-((4R)-F-Pro)-Thr-Gln-(2-F-Phe)-Cys]-OH,

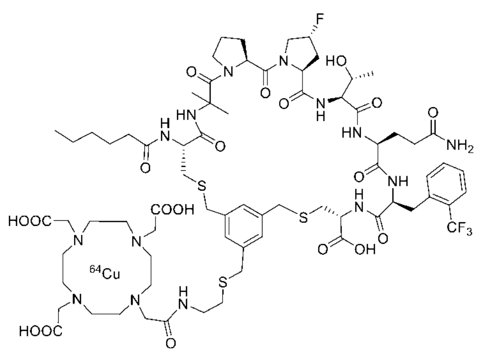
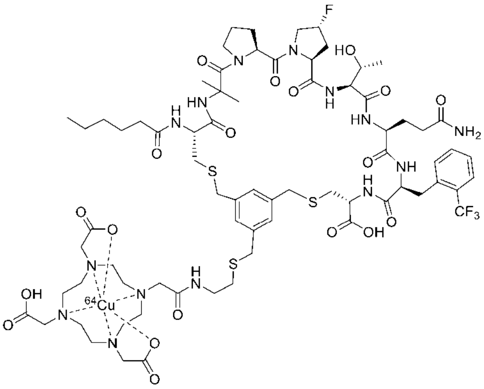

Hex-[Cys-(tMeBn($^{64}$Cu-DOTA-AET))-Aib-Pro-((4R)-F-Pro)-Thr-Gln-(2-$CF_3$-Phe)-Cys]-OH,

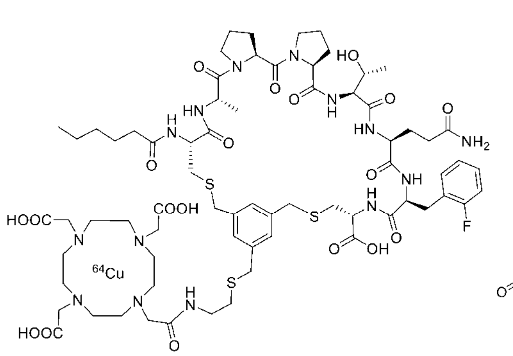
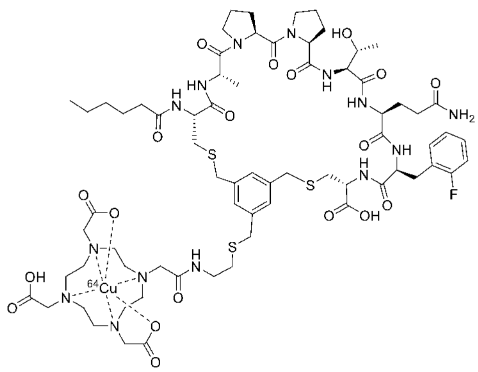

Hex-[Cys-(tMeBn($^{64}$Cu-DOTA-AET))-Ala-Pro-Pro-Thr-Gln-(2-F-Phe)-Cys]-OH,

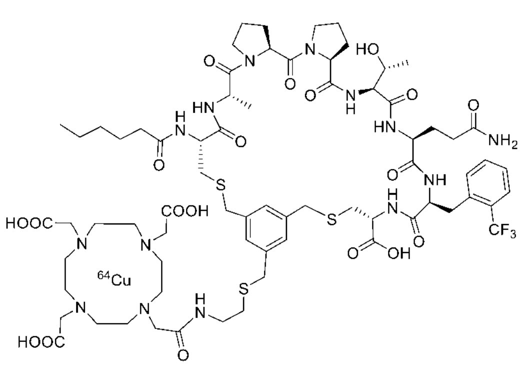
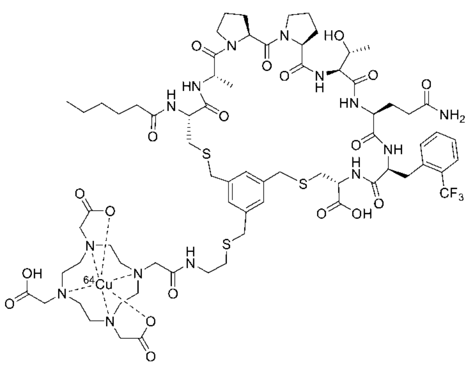

Hex-[Cys-(tMeBn($^{64}$Cu-DOTA-AET))-Ala-Pro-Pro-Thr-Gln-(2-$CF_3$-Phe)-Cys]-OH,

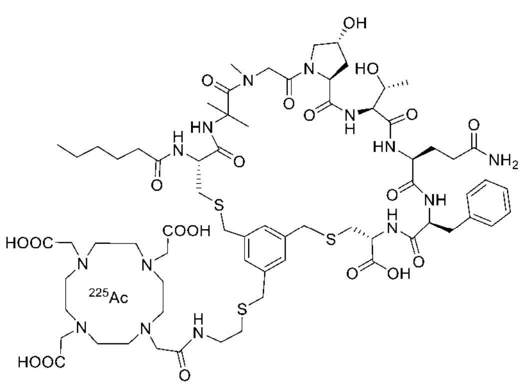
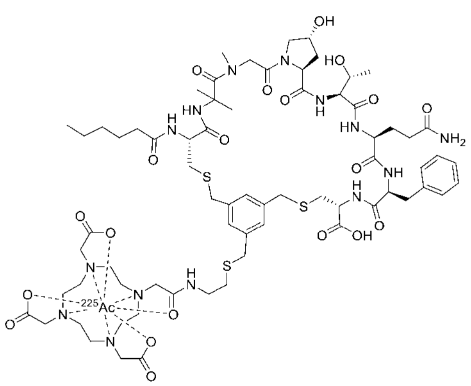

Hex-[Cys-(tMeBn($^{225}$Ac-DOTA-AET))-Aib-Sar-((4R)-OH-Pro)-Thr-Gln-Phe-Cys]-OH,

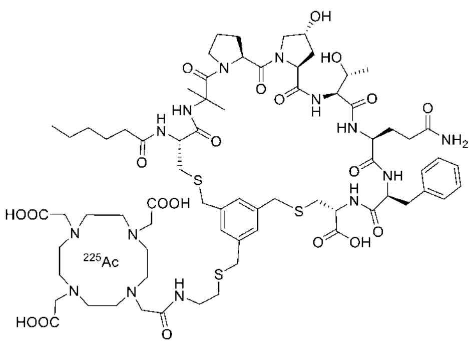

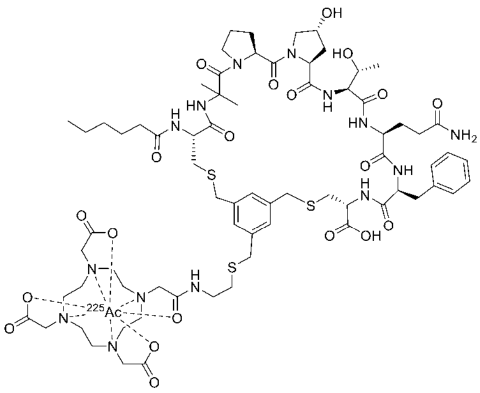

Hex-[Cys-(tMeBn($^{225}$Ac-DOTA-AET))-Aib-Pro-((4R)-OH-Pro)-Thr-Gln-Phe-Cys]-OH,

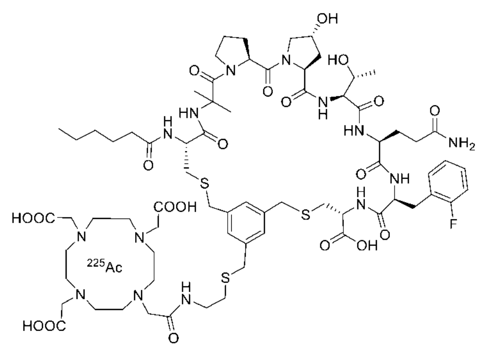

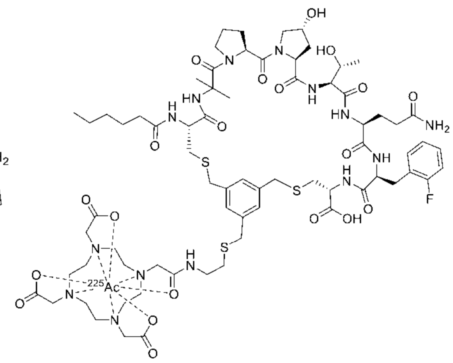

Hex-[Cys-(tMeBn($^{225}$Ac-DOTA-AET))-Aib-Pro-((4R)-OH-Pro)-Thr-Gln-(2-F-Phe)-Cys]-OH,

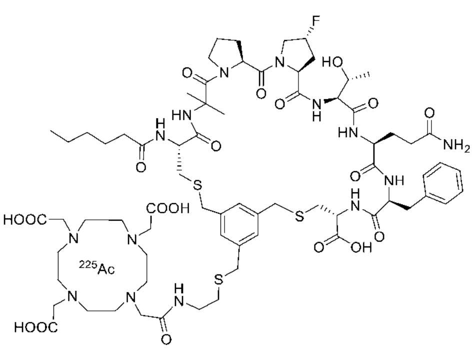

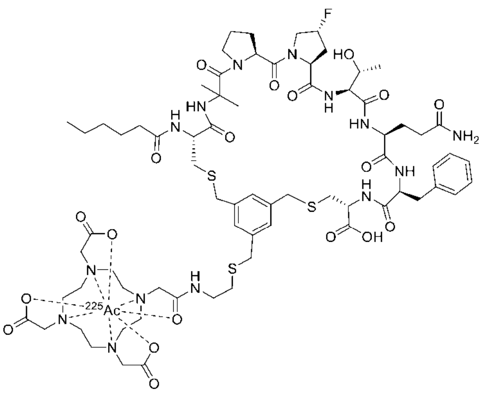

Hex-[Cys-(tMeBn($^{225}$Ac-DOTA-AET))-Aib-Pro-((4R)-F-Pro)-Thr-Gln-Phe - Cys]-OH,

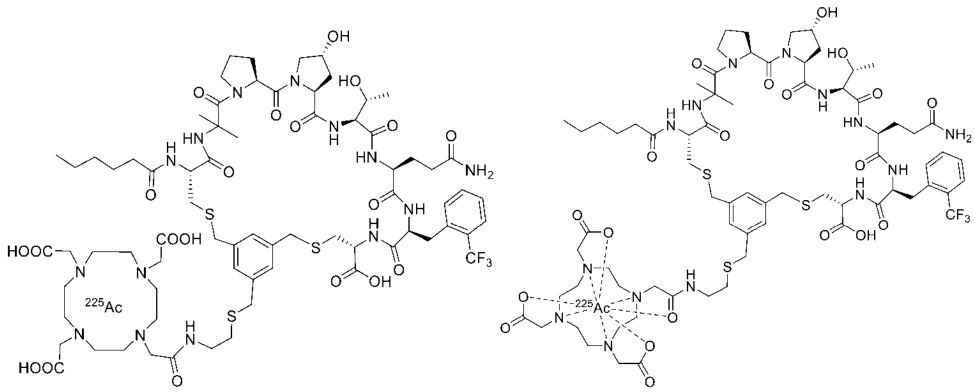

Hex-[Cys-(tMeBn($^{225}$Ac-DOTA-AET))-Aib-Pro-((4R)-OH-Pro)-Thr-Gln-(2-$CF_3$-Phe) -Cys]-OH,

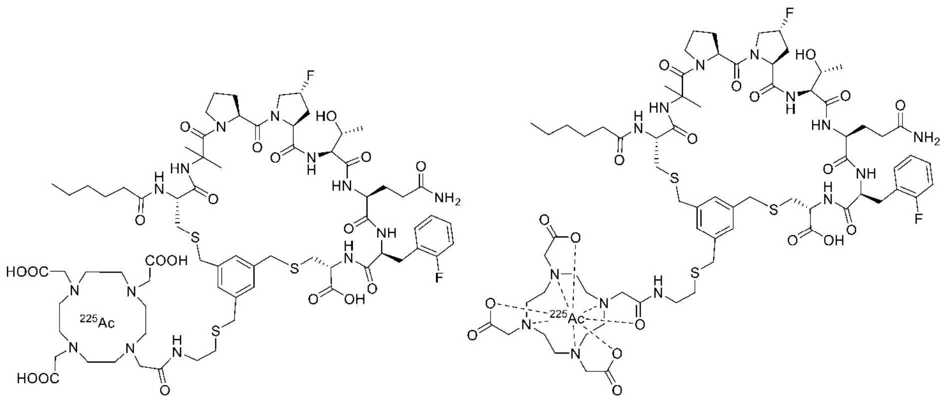

Hex-[Cys-(tMeBn($^{225}$Ac-DOTA-AET))-Aib-Pro-((4R)-F-Pro)-Thr-Gln-(2-F-Phe)-Cys]-OH,

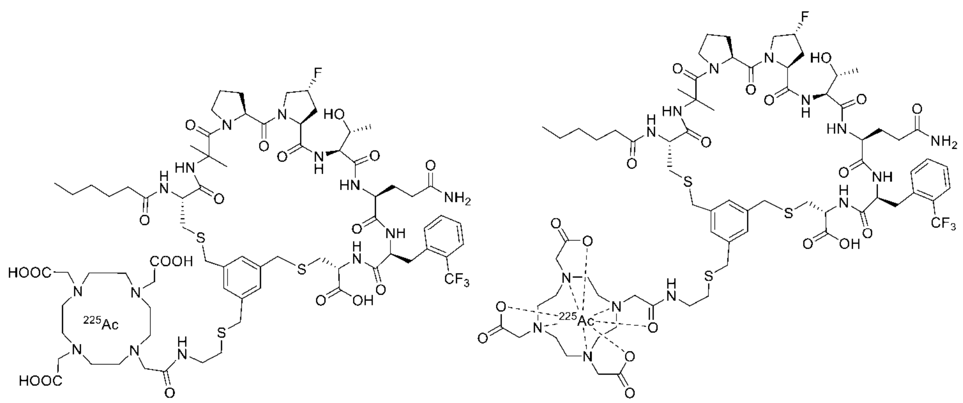

Hex-[Cys-(tMeBn($^{225}$Ac-DOTA-AET))-Aib-Pro-((4R)-F-Pro)-Thr-Gln-(2-$CF_3$-Phe)-Cys]-OH,

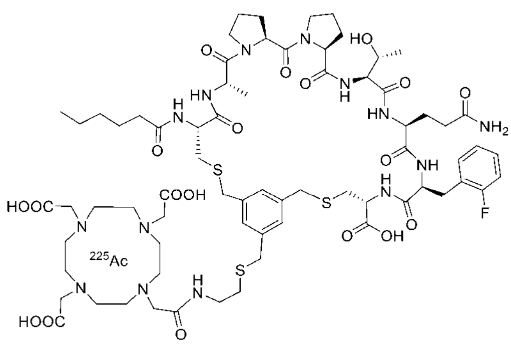

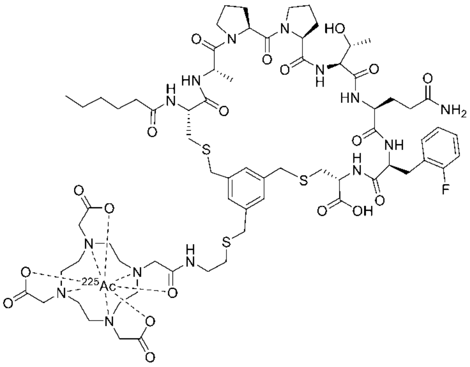

Hex-[Cys-(tMeBn($^{225}$Ac-DOTA-AET))-Ala-Pro-Pro-Thr-Gln-(2-F-Phe)-Cys]-OH,

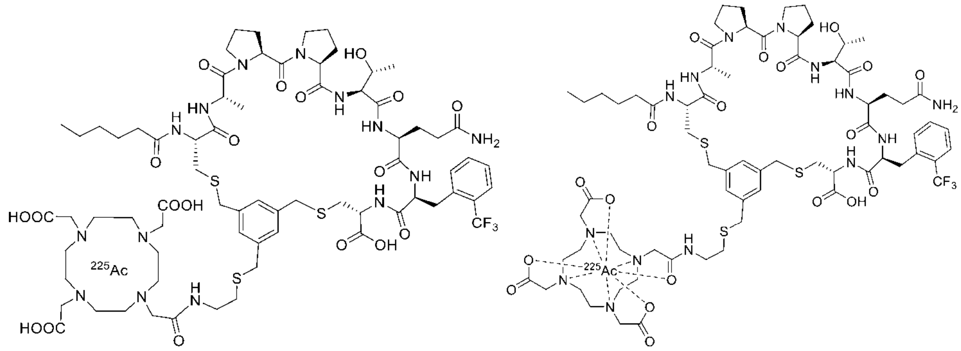

Hex-[Cys-(tMeBn($^{64}$Cu-DOTA-AET))-Ala-Pro-Pro-Thr-Gln-(2-$CF_3$-Phe)-Cys]-OH,

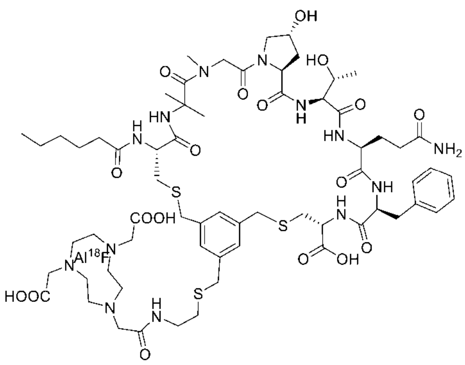

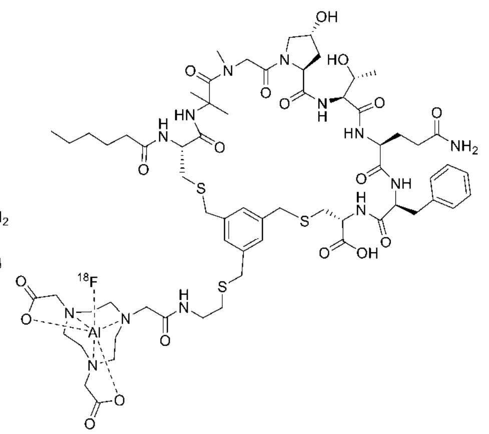

Hex-[Cys-(tMeBn(Al$^{18}$F-NOTA-AET))-Aib-Sar-((4R)-OH-Pro)-Thr-Gln-Phe-Cys]-OH,

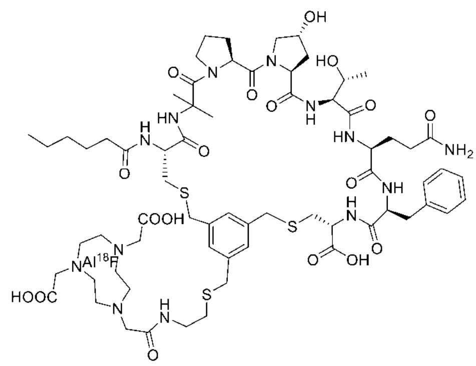
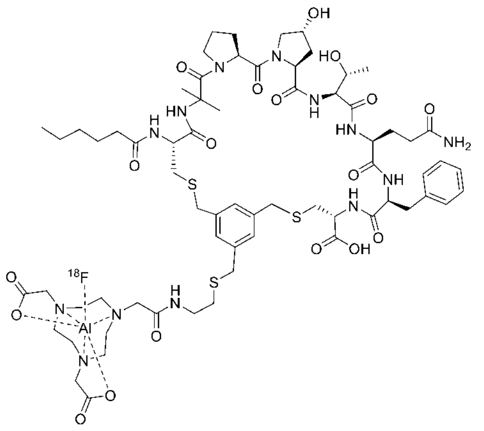

Hex-[Cys-(tMeBn(Al$^{18}$F-NOTA-AET))-Aib-Pro-((4R)-OH-Pro)-Thr-Gln-Phe-Cys]-OH,

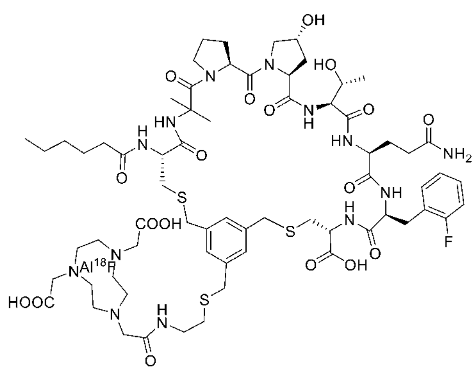
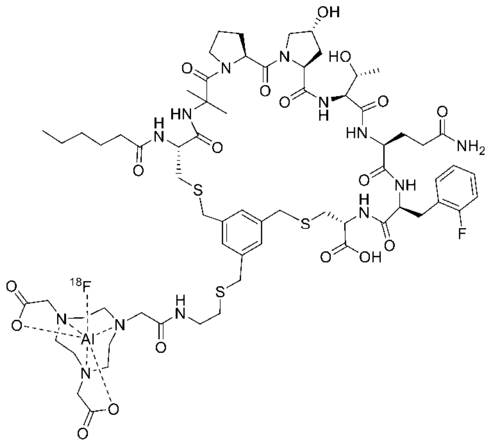

Hex-[Cys-(tMeBn(Al$^{18}$F-NOTA-AET))-Aib-Pro-((4R)-OH-Pro)-Thr-Gln-(2-F-Phe)-Cys]-OH,

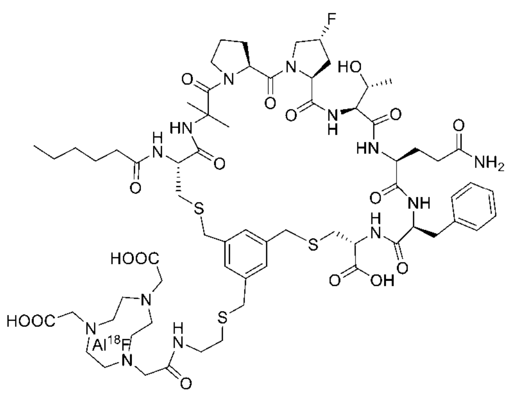
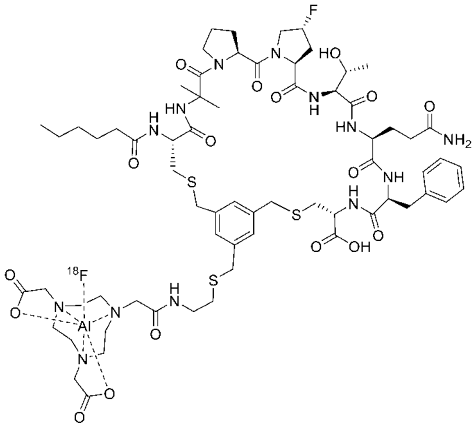

Hex-[Cys-(tMeBn(Al$^{18}$F-NOTA-AET))-Aib-Pro-((4R)-F-Pro)-Thr-Gln-Phe-Cys]-OH,

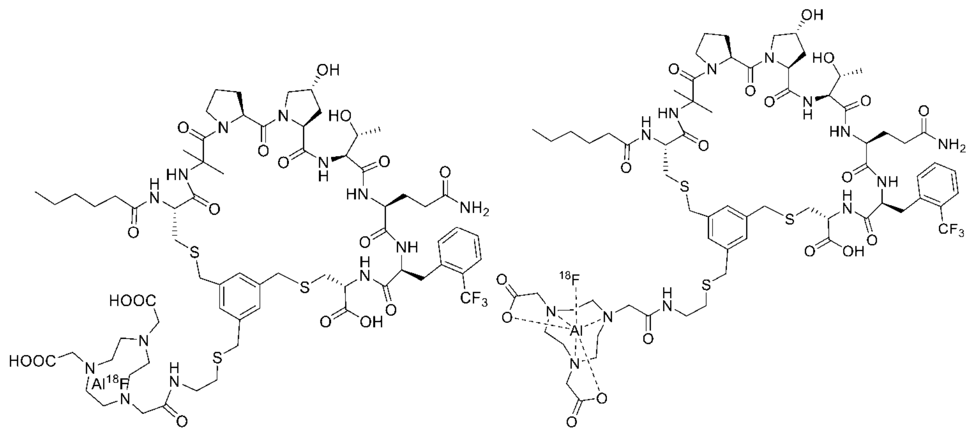

Hex-[Cys-(tMeBn(Al$^{18}$F-NOTA-AET))-Aib-Pro-((4R)-OH-Pro)-Thr-Gln-(2-$CF_3$-Phe) -Cys]-OH,

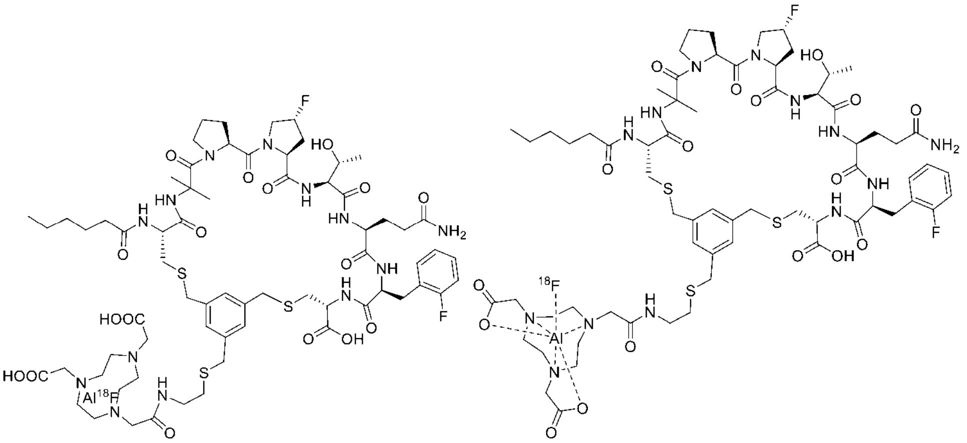

Hex-[Cys-(tMeBn(Al$^{18}$F-NOTA-AET))-Aib-Pro-((4R)-F-Pro)-Thr-Gln-(2-F-Phe)-Cys]-OH,

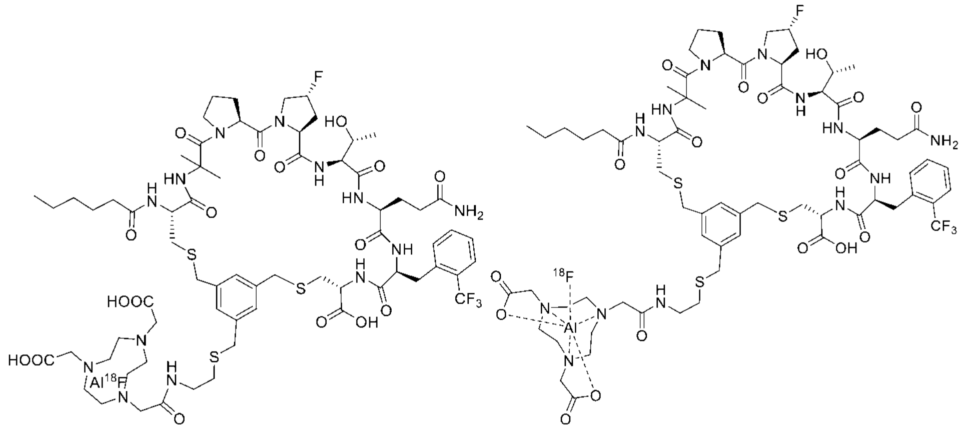

Hex-[Cys-(tMeBn(Al$^{18}$F-NOTA-AET))-Aib-Pro-((4R)-F-Pro)-Thr-Gln-(2-$CF_3$-Phe)-Cys]-OH,

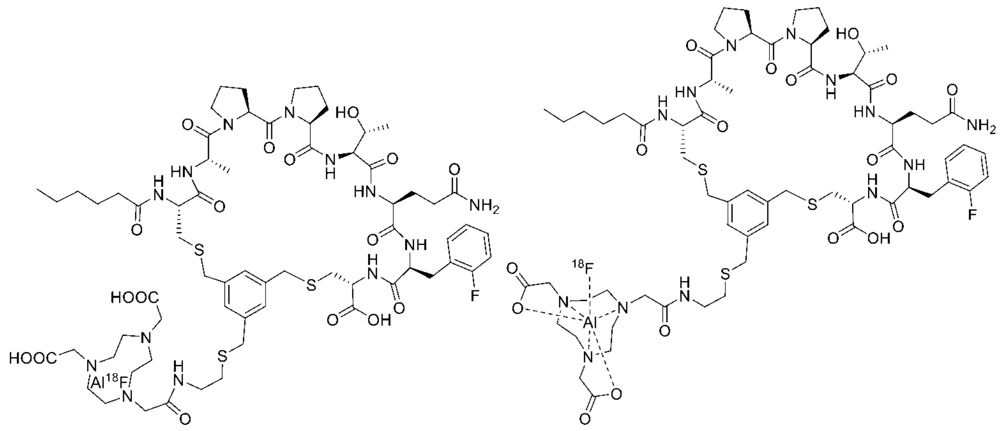

Hex-[Cys-(tMeBn(Al$^{18}$F-NOTA-AET))-Ala-Pro-Pro-Thr-Gln-(2-F-Phe)-Cys]-OH,

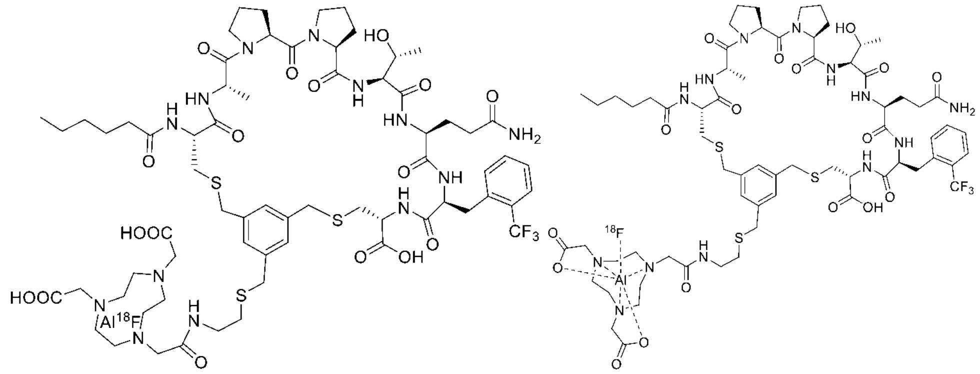

Hex-[Cys-(tMeBn(Al$^{18}$F-NOTA-AET))-Ala-Pro-Pro-Thr-Gln-(2-$CF_3$-Phe)-Cys]-OH,

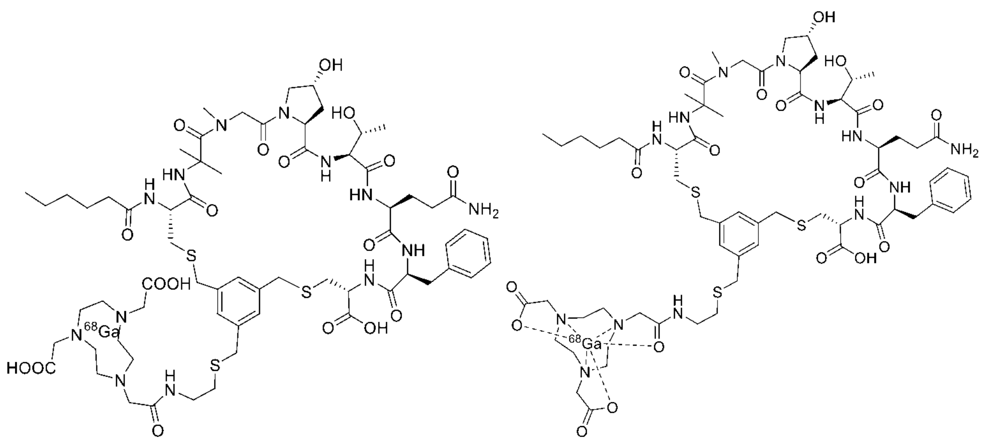

Hex-[Cys-(tMeBn($^{68}$Ga-NOTA-AET))-Aib-Sar-((4R)-OH-Pro)-Thr-Gln-Phe-Cys]-OH,

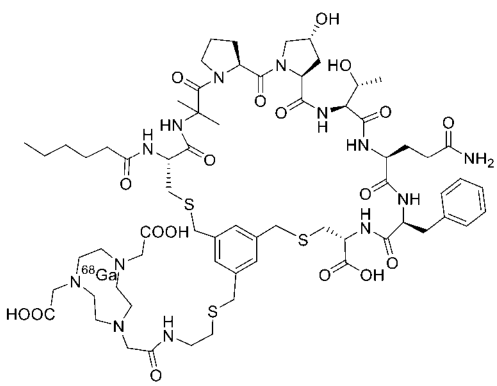 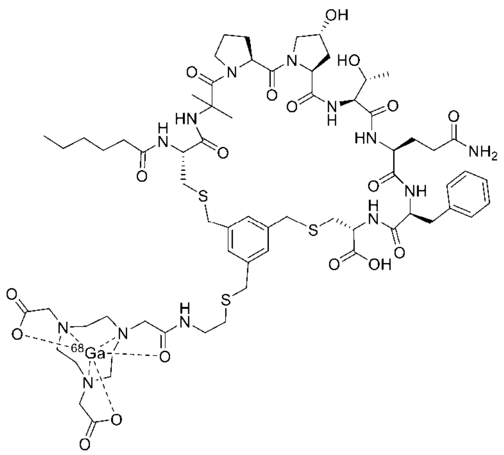

Hex-[Cys-(tMeBn($^{68}$Ga-NOTA-AET))-Aib-Pro-((4R)-OH-Pro)-Thr-Gln-Phe-Cys]-OH,

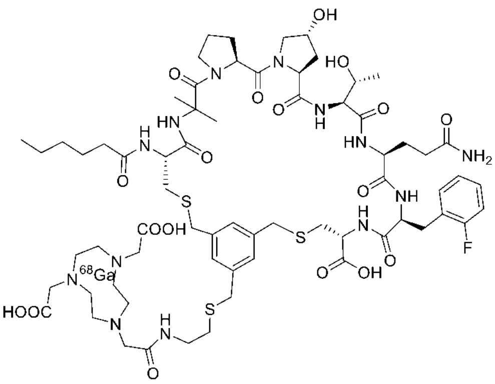 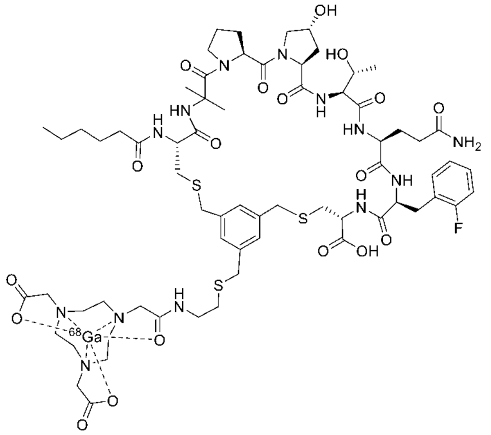

Hex-[Cys-(tMeBn($^{68}$Ga-NOTA-AET))-Aib-Pro-((4R)-OH-Pro)-Thr-Gln-(2-F-Phe)-Cys]-OH,

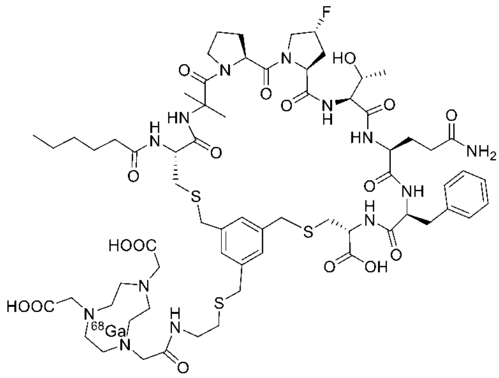 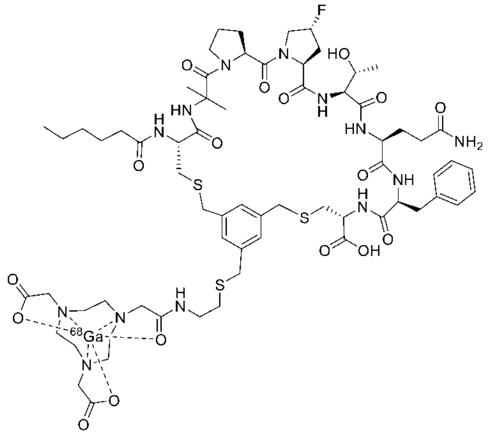

Hex-[Cys-(tMeBn($^{68}$Ga-NOTA-AET))-Aib-Pro-((4R)-F-Pro)-Thr-Gln-Phe-Cys]-OH,

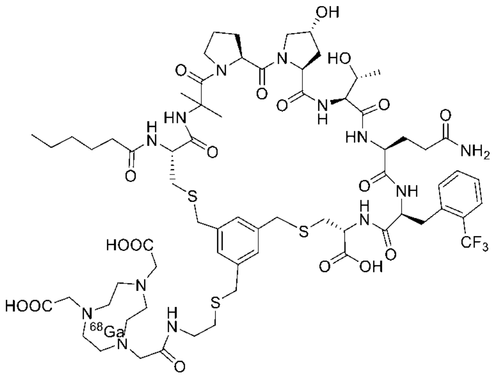
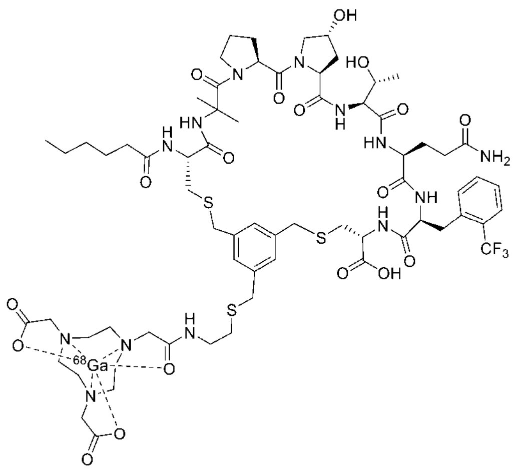

Hex-[Cys-(tMeBn($^{68}$Ga-NOTA-AET))-Aib-Pro-((4R)-OH-Pro)-Thr-Gln-(2-$CF_3$-Phe)-Cys]-OH,

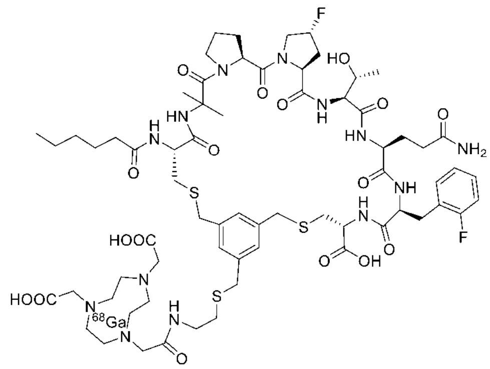
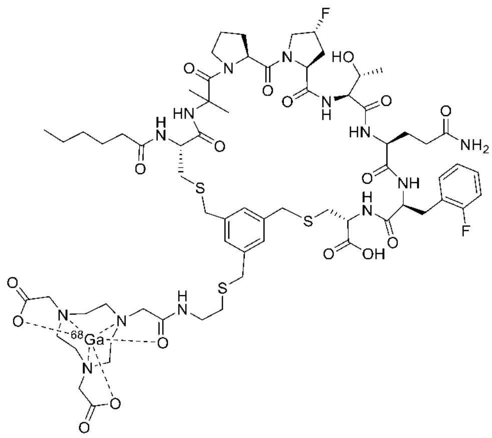

Hex-[Cys-(tMeBn($^{68}$Ga-NOTA-AET))-Aib-Pro-((4R)-F-Pro)-Thr-Gln-(2-F-Phe)-Cys]-OH,

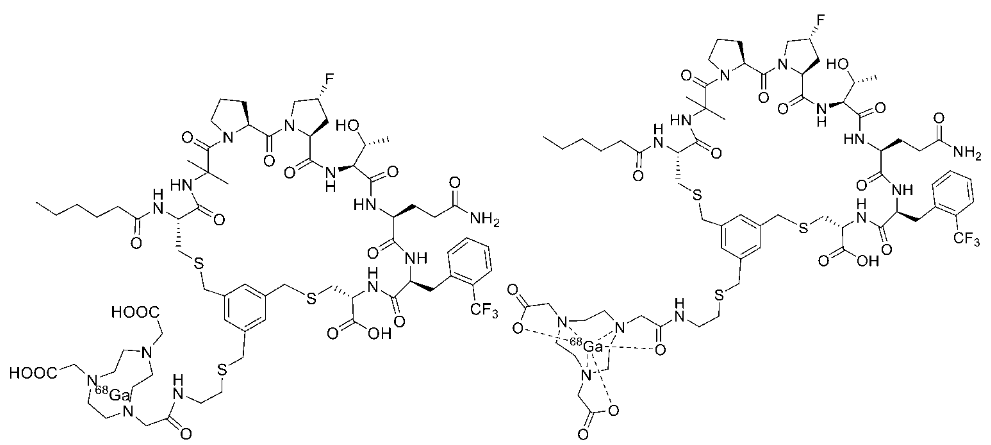

Hex-[Cys-(tMeBn($^{68}$Ga-NOTA-AET))-Aib-Pro-((4R)-F-Pro)-Thr-Gln-(2-$CF_3$-Phe)-Cys]-OH,

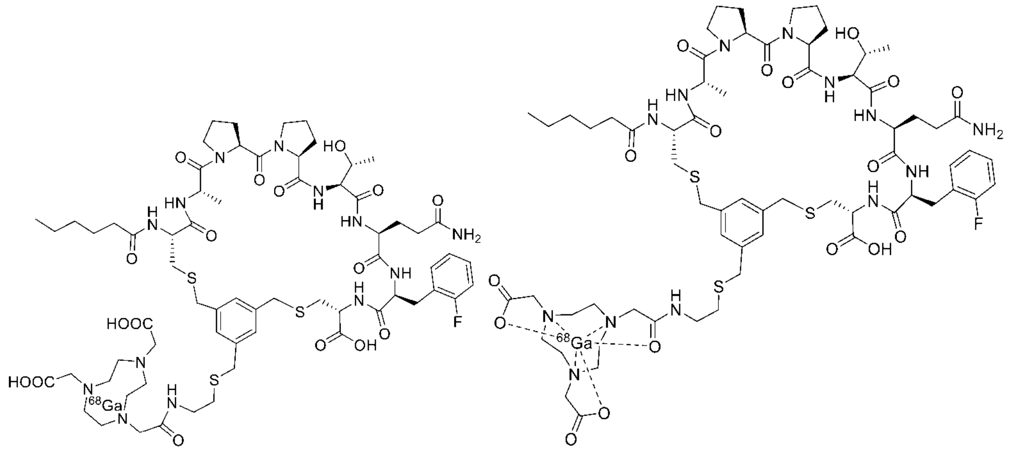

Hex-[Cys-(tMeBn($^{68}$Ga-NOTA-AET))-Ala-Pro-Pro-Thr-Gln-(2-F-Phe)-Cys]-OH,

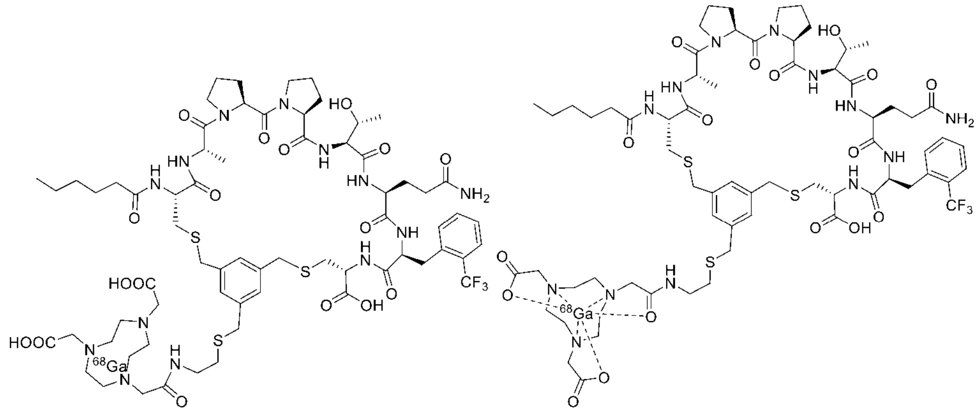

Hex-[Cys-(tMeBn($^{68}$Ga-NOTA-AET))-Ala-Pro-Pro-Thr-Gln-(2-$CF_3$-Phe)-Cys]-OH,

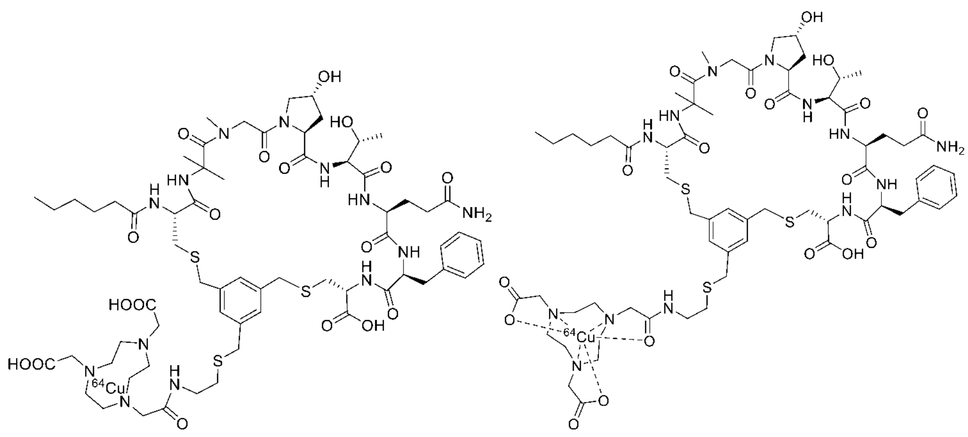

Hex-[Cys-(tMeBn($^{64}$Cu-NOTA-AET))-Aib-Sar-((4R)-OH-Pro)-Thr-Gln-Phe-Cys]-OH,

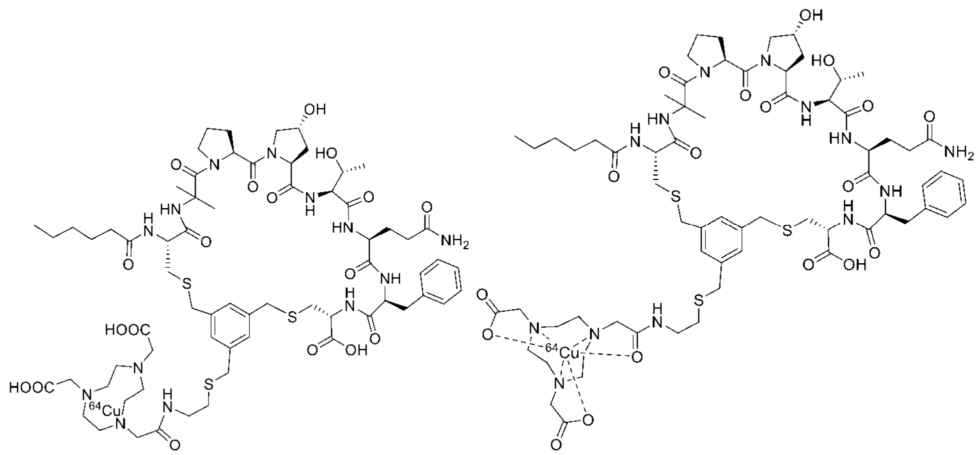

Hex-[Cys-(tMeBn($^{64}$Cu-NOTA-AET))-Aib-Pro-((4R)-OH-Pro)-Thr-Gln-Phe-Cys]-OH,

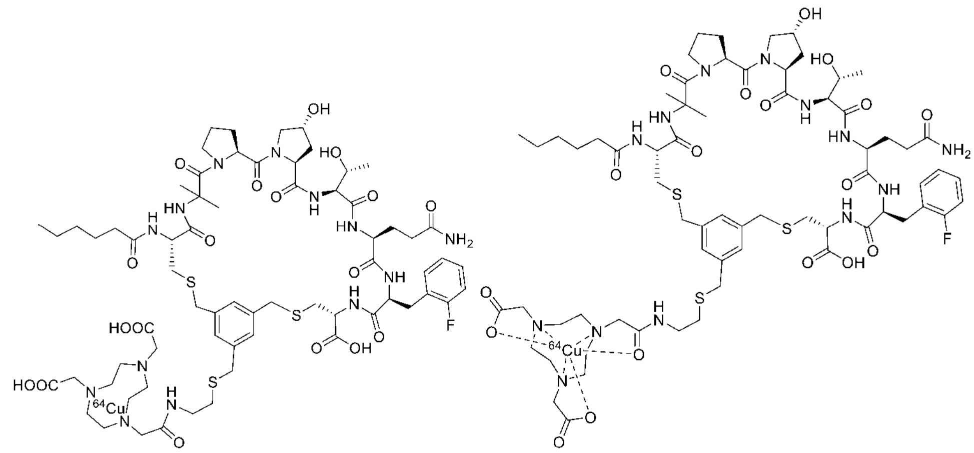

Hex-[Cys-(tMeBn($^{64}$Cu-NOTA-AET))-Aib-Pro-((4R)-OH-Pro)-Thr-Gln-(2-F-Phe)-Cys]-OH,

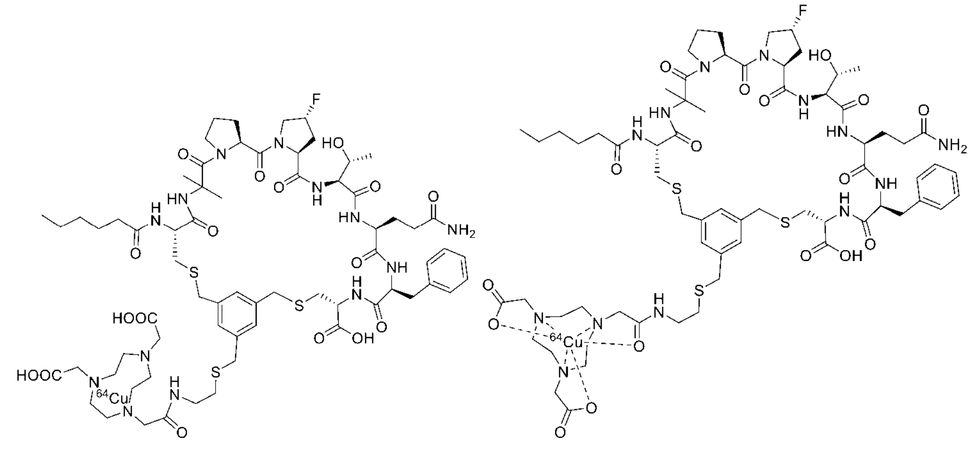

Hex-[Cys-(tMeBn($^{64}$Cu-NOTA-AET))-Aib-Pro-((4R)-F-Pro)-Thr-Gln-Phe-Cys]-OH,

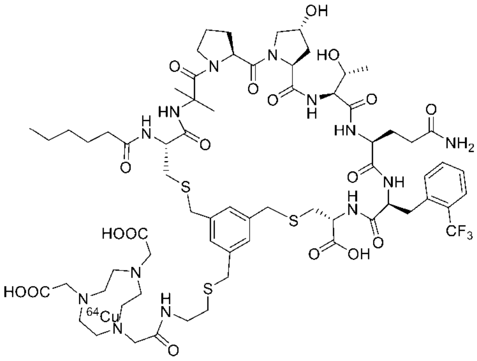
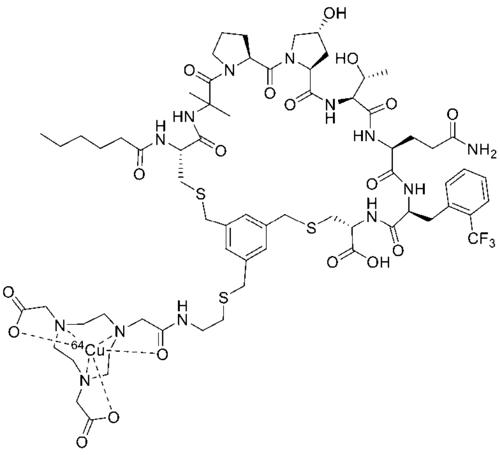

Hex-[Cys-(tMeBn($^{64}$Cu-NOTA-AET))-Aib-Pro-((4R)-OH-Pro)-Thr-Gln-(2-$CF_3$-Phe)-Cys]-OH,

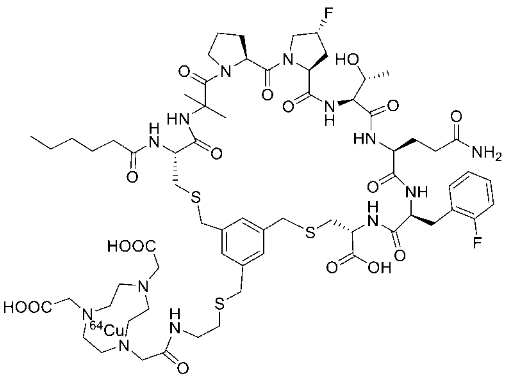
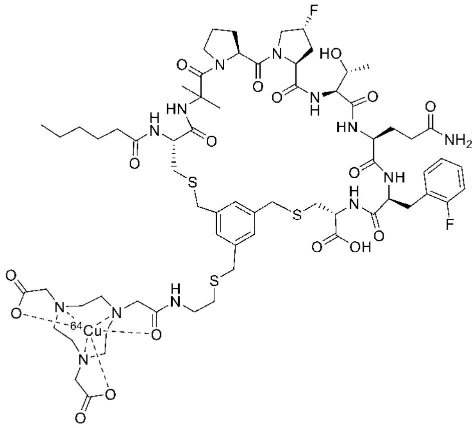

Hex-[Cys-(tMeBn($^{64}$Cu-NOTA-AET))-Aib-Pro-((4R)-F-Pro)-Thr-Gln-(2-F-Phe)-Cys]-OH,

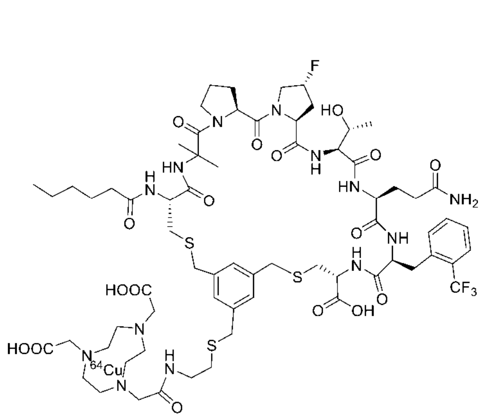
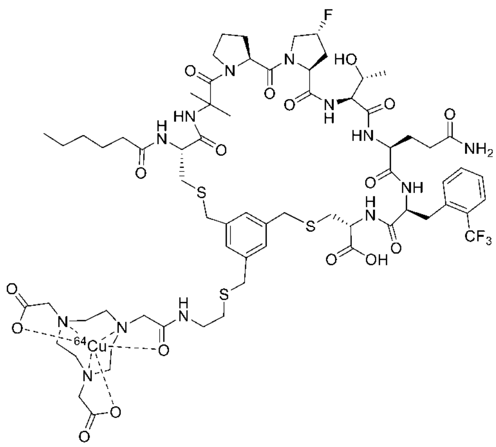

Hex-[Cys-(tMeBn($^{64}$Cu-NOTA-AET))-Aib-Pro-((4R)-F-Pro)-Thr-Gln-(2-$CF_3$-Phe)-Cys]-OH,

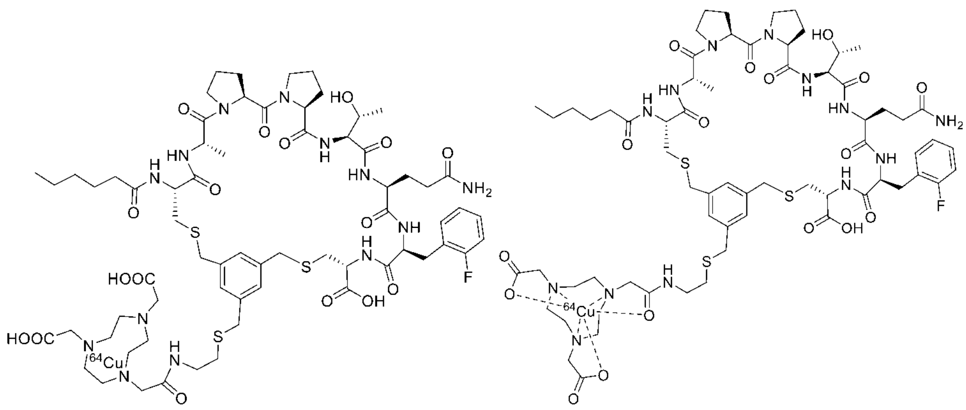

Hex-[Cys-(tMeBn($^{64}$Cu-NOTA-AET))-Ala-Pro-Pro-Thr-Gln-(2-F-Phe)-Cys]-OH, and

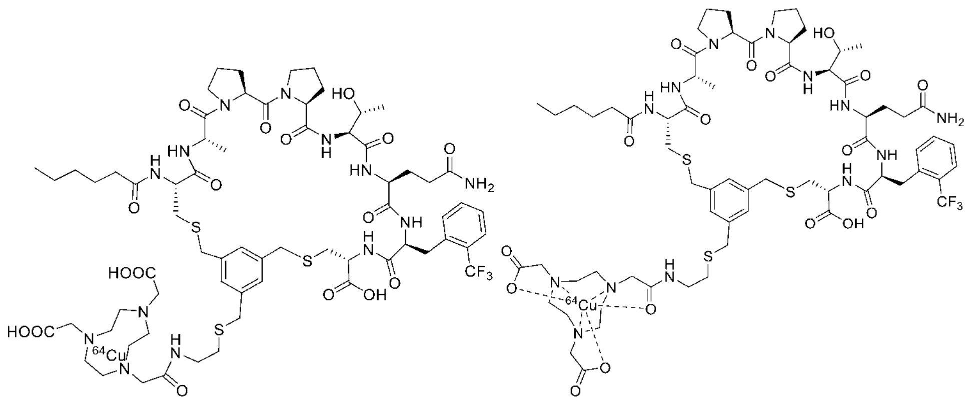

Hex-[Cys-(tMeBn($^{64}$Cu-NOTA-AET))-Ala-Pro-Pro-Thr-Gln-(2-$CF_3$-Phe)-Cys]-OH.

**26.** A pharmaceutical composition, **characterised by** comprising the compound as shown in formula (I) or the pharmaceutically acceptable salt thereof according to any one of claims 1 to 25, and one or more pharmaceutically acceptable carriers, diluents or excipients.

**27.** A method for preparing the compound as shown in formula (I) or the pharmaceutically acceptable salt thereof according to any one of claims 1 to 25 or the pharmaceutical composition according to claim 26, **characterised in that** the method comprises a step of complexing the compound or the pharmaceutically acceptable salt thereof with a nuclide.

**28.** Use of the compound as shown in formula (I) or the pharmaceutically acceptable salt thereof according to any one of claims 1 to 25 or the pharmaceutical composition according to claim 26 in the manufacture of a medicament for diagnosing or treating a disease, **characterised in that** the disease is a disease related to fibroblast activation protein (FAP); preferably, the disease related to fibroblast activation protein (FAP) is a disease associated with upregulation of fibroblast activation protein (FAP) expression; and more preferably, the disease is related to cells that exhibit upregulation of fibroblast activation protein (FAP) expression or a diseased tissue containing cells that exhibit upregulation of fibroblast activation protein (FAP) expression, e.g., a tumour or cancer.

**29.** Use of the compound as shown in formula (I) or the pharmaceutically acceptable salt thereof according to any one of claims 1 to 25 or the pharmaceutical composition according to claim 26 in the manufacture of a medicament for diagnosing or treating a disease, **characterised in that** the disease is a tumour or cancer selected from solid tumour, epithelioma, bladder cancer, breast cancer, cervical cancer, colorectal cancer, cholangiocarcinoma, endometrial cancer, oesophageal cancer, gastric cancer, gastrointestinal stromal tumour, head and neck cancer, liver cancer, lung cancer, melanoma, mesothelioma, neuroendocrine tumour and carcinoma, ovarian cancer, pancreatic cancer, prostate cancer, renal cell carcinoma, salivary gland cancer, sarcoma, squamous cell carcinoma, glioma (e.g., human brain astroglioblastoma cells), and thyroid cancer (e.g., medullary thyroid carcinoma).

**30.** Use of the compound as shown in formula (I) or the pharmaceutically acceptable salt thereof according to any one of claims 1 to 25 or the pharmaceutical composition according to claim 26 in the manufacture of a medicament for diagnosing or treating a disease, **characterised in that** the disease is selected from an inflammatory disease, a cardiovascular disease, an autoimmune disease and a fibrotic disease.

**31.** A method for preparing the compound as shown in formula (I) or the pharmaceutically acceptable salt thereof according to either claim 24 or claim 25, or the pharmaceutical composition according to claim 26, **characterised in that** the method comprises a step of complexing the compound as shown in formula (I) or the pharmaceutically acceptable salt thereof with a nuclide,

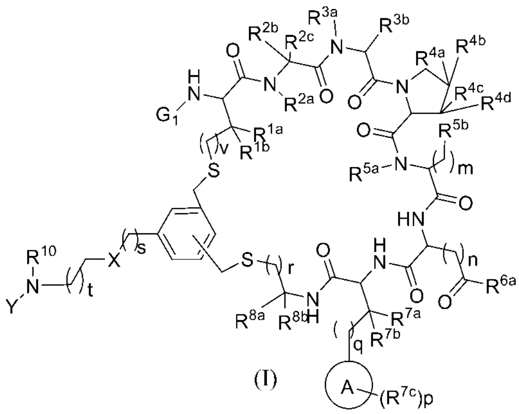

(I)

wherein the Y is a chelating agent, the chelating agent complexes a nuclide, and the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t and $R^{10}$ are as defined in claims 1 to 25.

**32.** A method for preparing the compound as shown in formula (I) or the pharmaceutically acceptable salt thereof according to any one of claims 1 to 25 or the pharmaceutical composition according to claim 26, **characterised in that** the method comprises a step of reacting a compound as shown in formula (I-1) with a chelating agent,

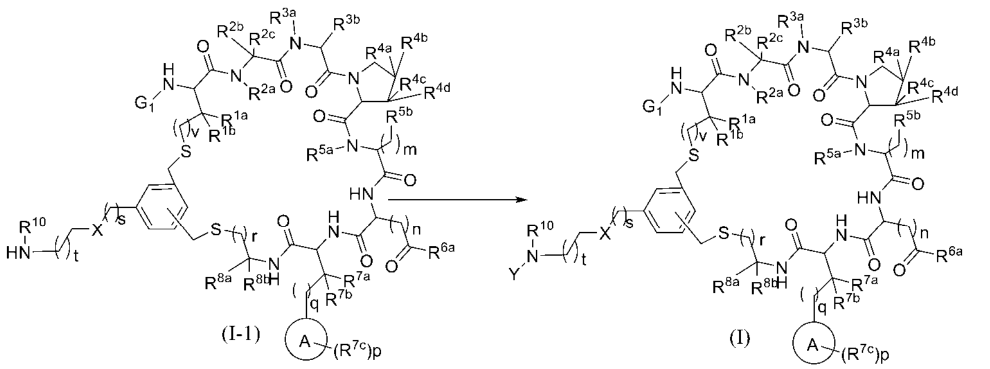

(I-1) (I)

wherein the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$, r, s, X, t and $R^{10}$ are as defined in claims 1 to 25.

**33.** A compound as shown in formula (I-2) or a pharmaceutically acceptable salt thereof,

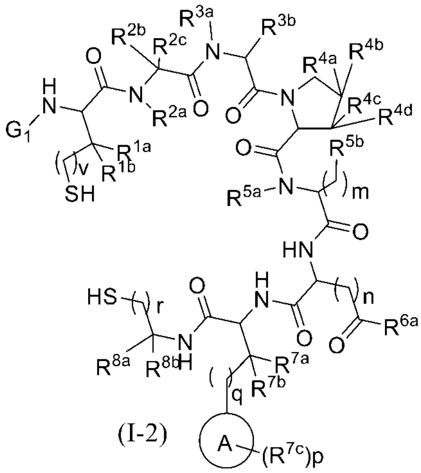

(I-2)

**characterised in that** the $R^{1a}$, $R^{1b}$, v, $G_1$, $R^{2a}$, $R^{2b}$, $R^{2c}$, $R^{3a}$, $R^{3b}$, $R^{4a}$, $R^{4b}$, $R^{4c}$, $R^{4d}$, $R^{5a}$, $R^{5b}$, m, $R^{6a}$, n, $R^{7a}$, $R^{7b}$, q, ring A, $R^{7c}$, p, $R^{8a}$, $R^{8b}$ and r are as defined in claims 1 to 25.

**34.** A method for preparing the compound as shown in formula (I) or the pharmaceutically acceptable salt thereof according to any one of claims 13 to 25, **characterised in that** the method comprises a step of reacting a compound as shown in formula (III-1) or a pharmaceutically acceptable salt thereof with 2-aminoethanethiol and 1,3,5-tris(bromomethyl)benzene,

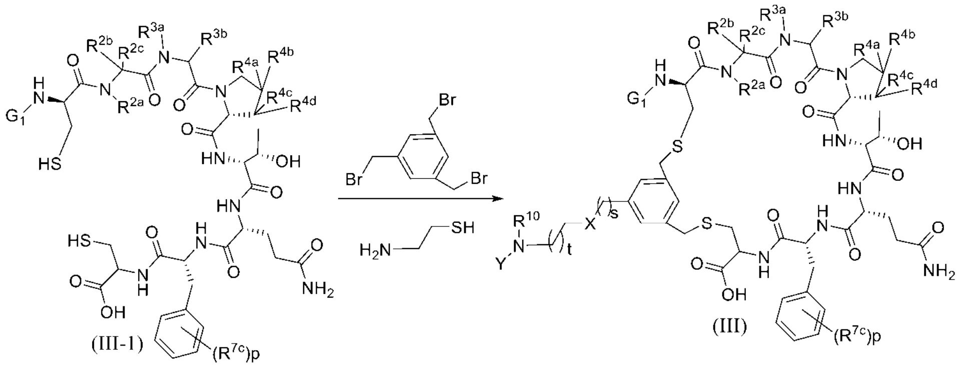

wherein the Y is hydrogen; $R^{10}$ is hydrogen; the t is 1; the X is a sulphur atom; the s is 1;
the $R^{2a}$, $R^{2b}$, $R^{2c}$, $R^{3a}$, $R^{3b}$, $R^{4a}$, $R^{4b}$, $R^{4c}$, $R^{4d}$, $R^{7c}$, p and $G_1$ are as defined in claims 13 to 25.

**35.** The preparation method according to claim 31 or 32, **characterised in that** the method further comprises the step in the preparation method according to claim 34.

**36.** A compound as shown in formula (III-1) or a pharmaceutically acceptable salt thereof,

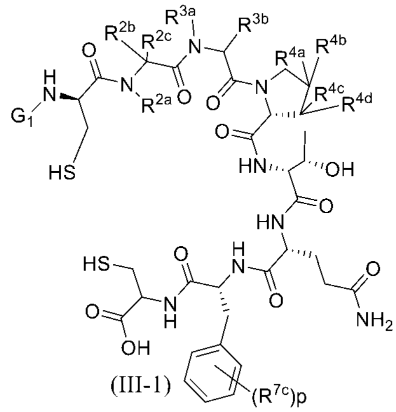

(III-1)

**characterised in that** the $R^{2a}$, $R^{2b}$, $R^{2c}$, $R^{3a}$, $R^{3b}$, $R^{4a}$, $R^{4b}$, $R^{4c}$, $R^{4d}$, $R^{7c}$, p and $G_1$ are as defined in claims 13 to 25.

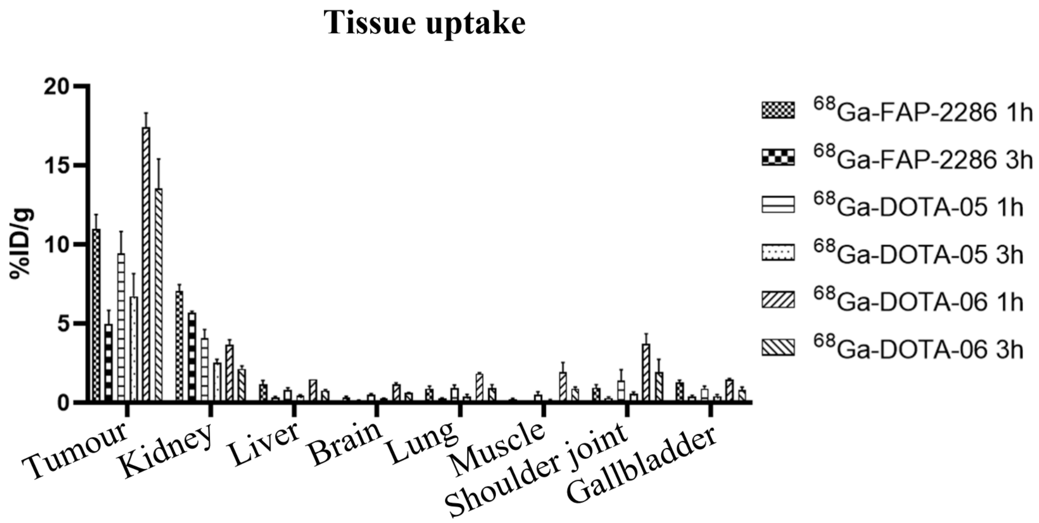


*FIG. 1*

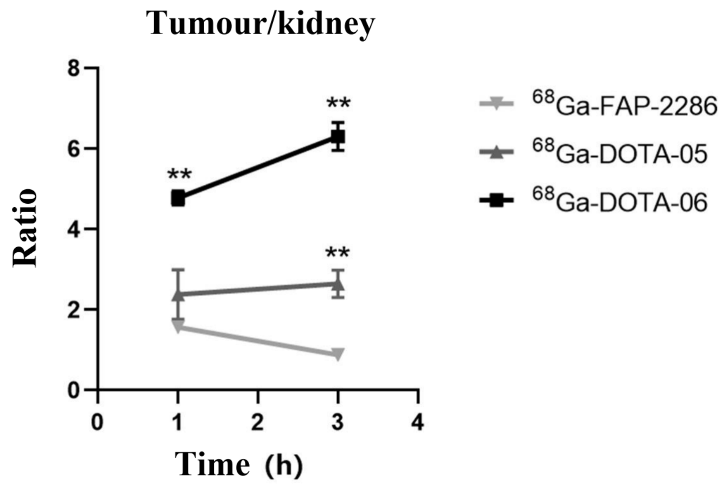


*FIG. 2*

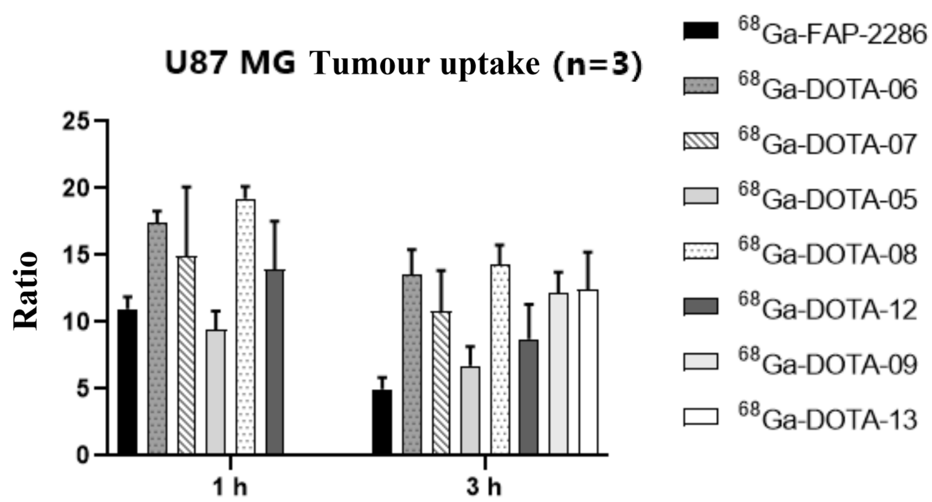


*FIG. 3*

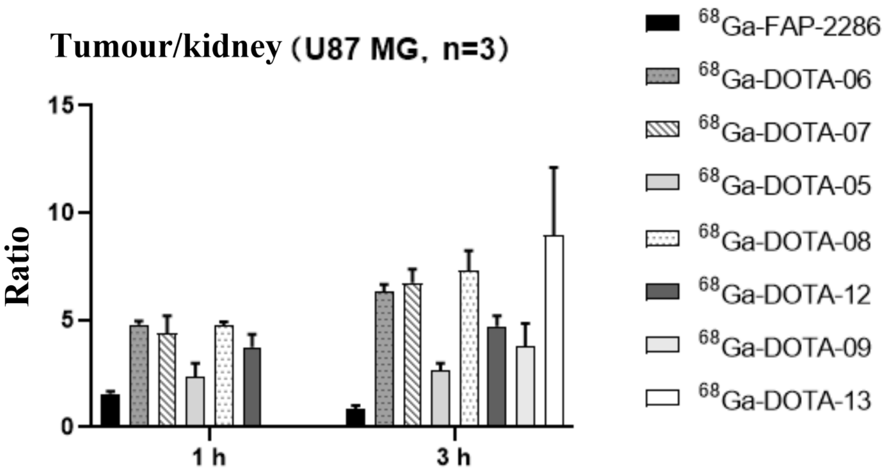


*FIG. 4*

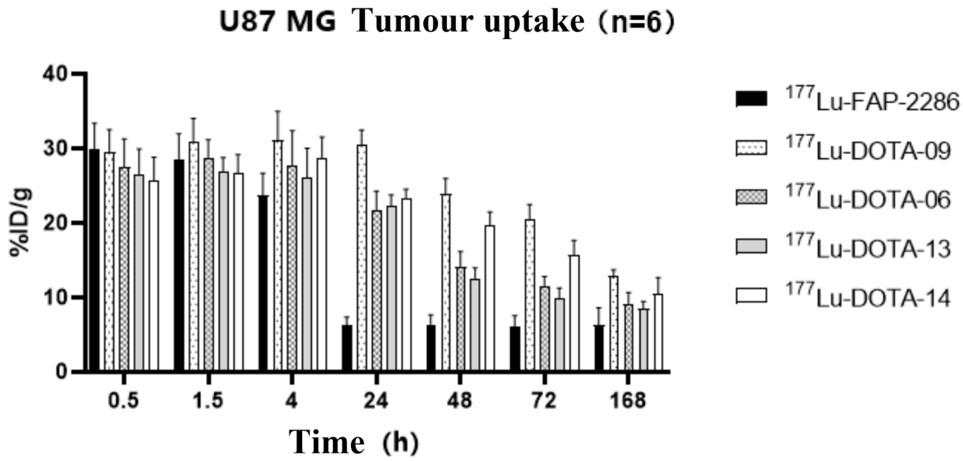


*FIG. 5*

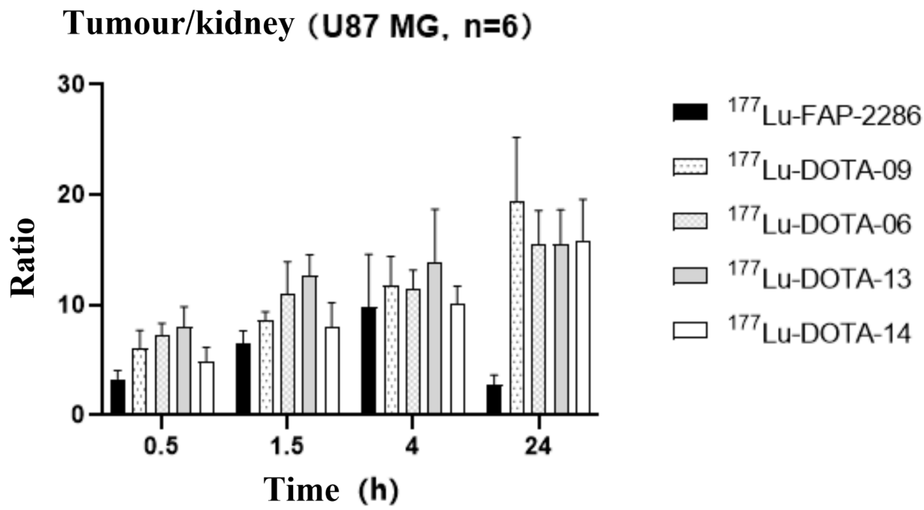


*FIG. 6*

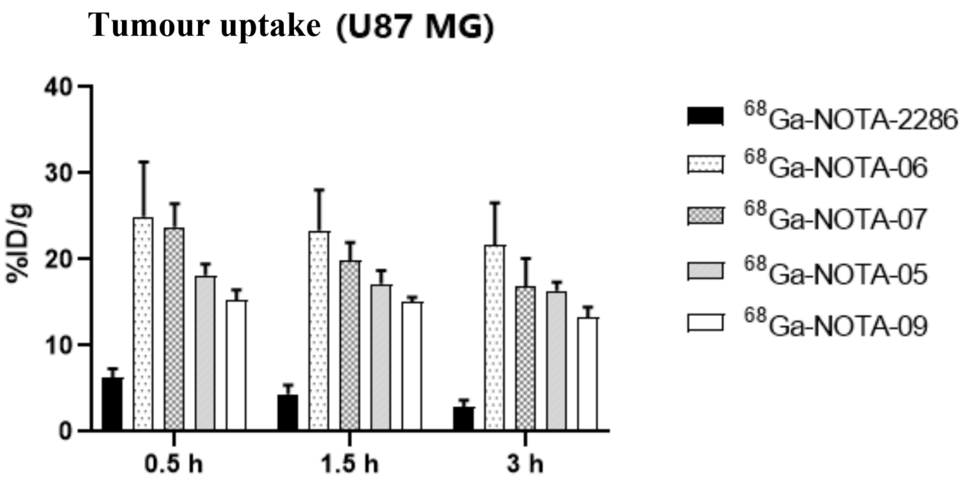


*FIG. 7*

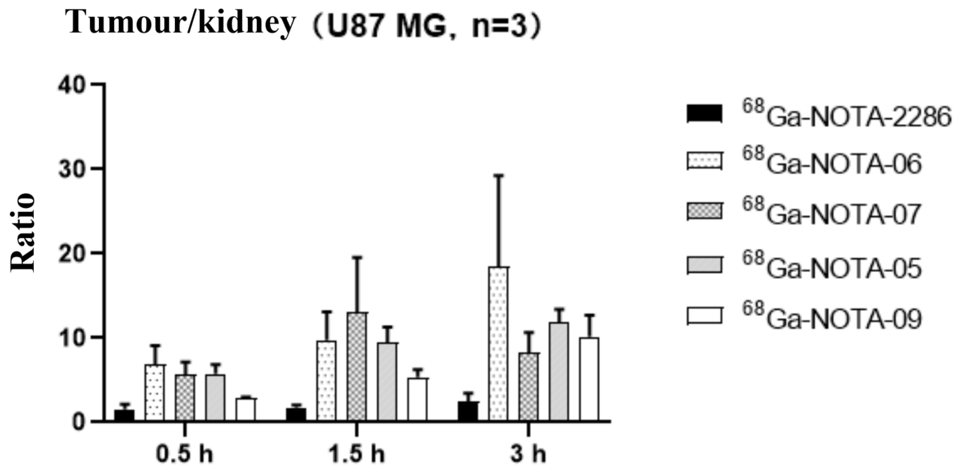


*FIG. 8*

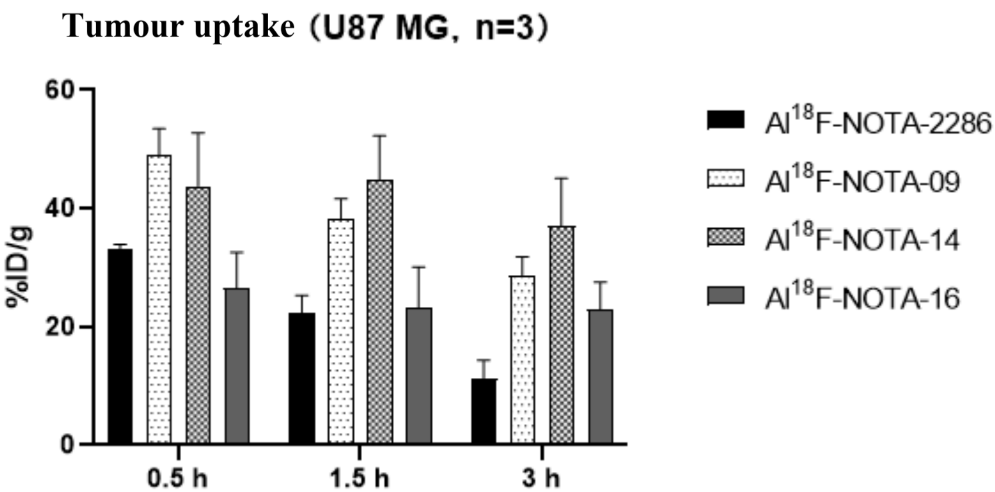


*FIG. 9*

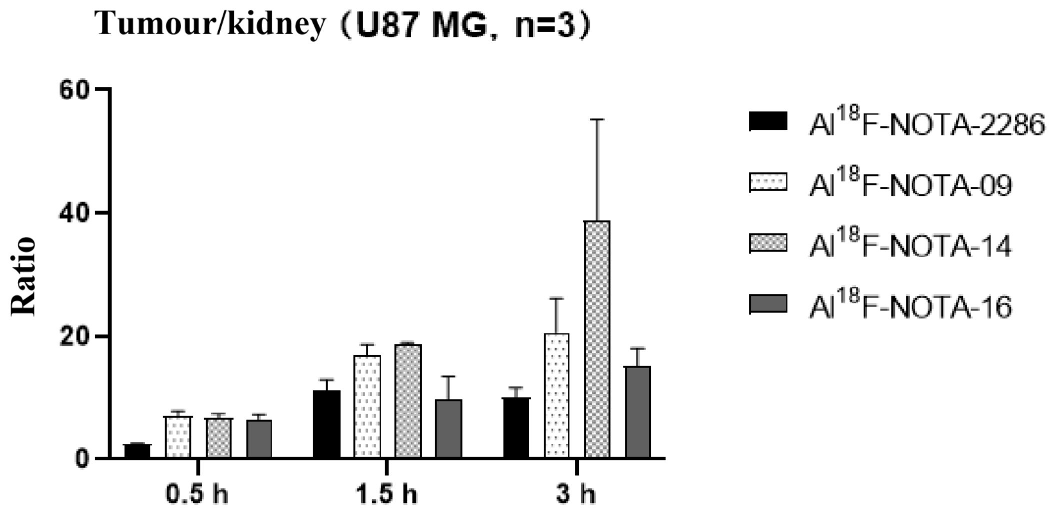


*FIG. 10*

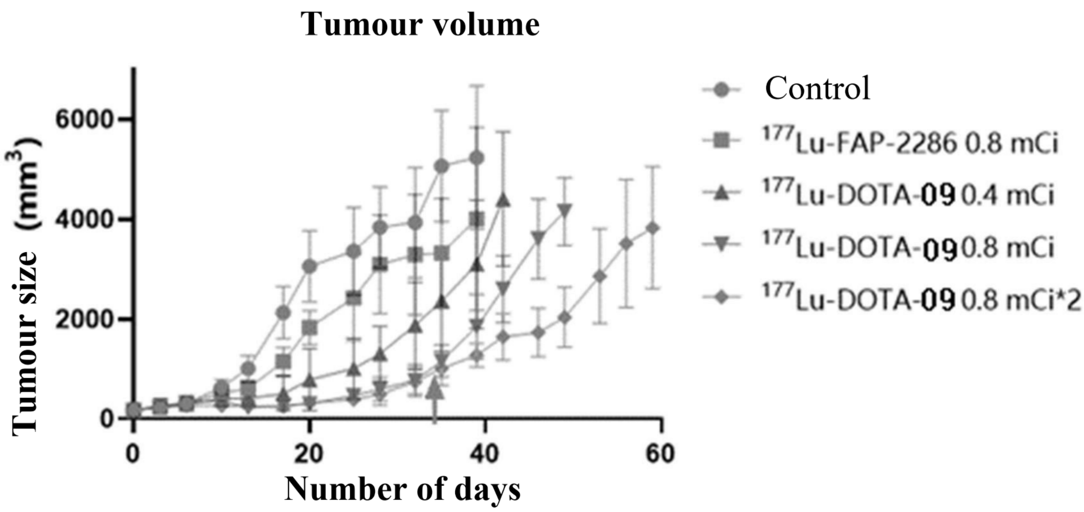


*FIG. 11*

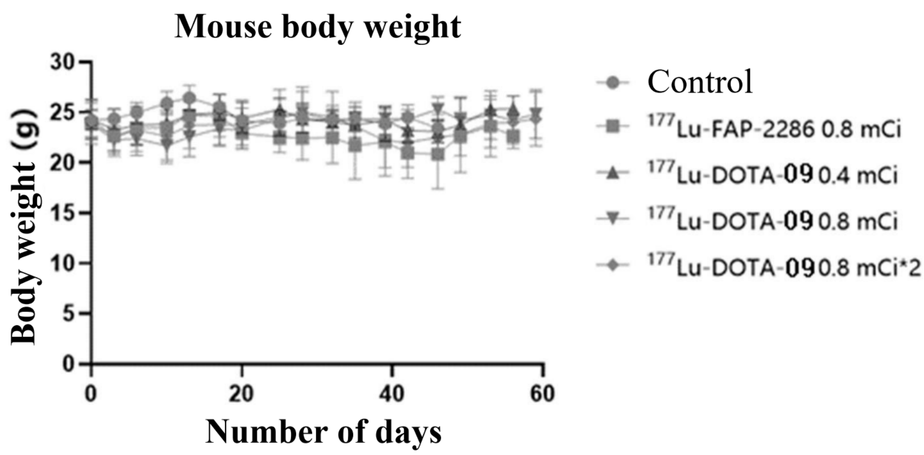


*FIG. 12*

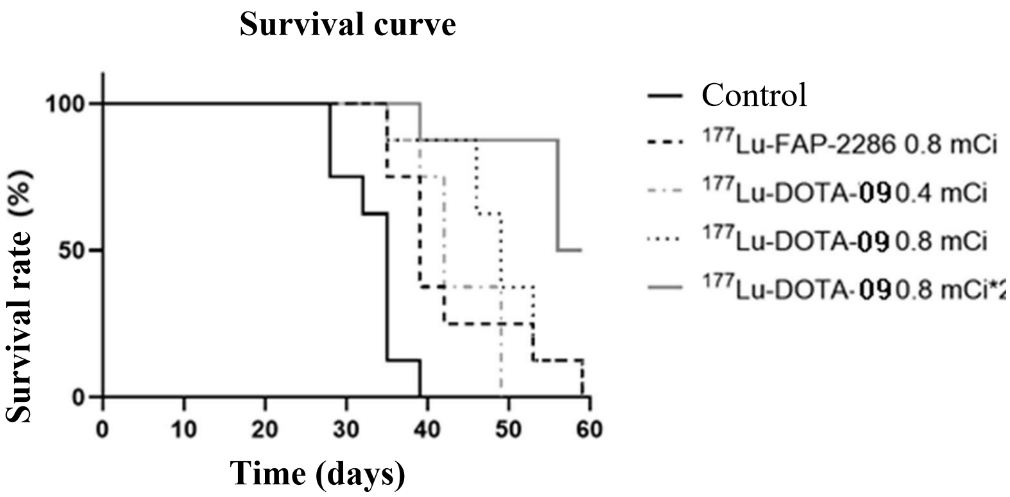
Survival curve
Survival rate (%)
100
50
0
0
10
20
30
40
50
60
Time (days)
Control
177Lu-FAP-2286 0.8 mCi
177Lu-DOTA-09 0.4 mCi
177Lu-DOTA-09 0.8 mCi
177Lu-DOTA-09 0.8 mCi*2

*FIG. 13*

**INTERNATIONAL SEARCH REPORT**

International application No.
**PCT/CN2024/104918**

**A. CLASSIFICATION OF SUBJECT MATTER**

C07K7/08(2006.01)i; C07K14/81(2006.01)i; C07K7/06(2006.01)i; A61K38/08(2019.01)i

According to International Patent Classification (IPC) or to both national classification and IPC

**B. FIELDS SEARCHED**

Minimum documentation searched (classification system followed by classification symbols)

IPC: C07K,A61K

Documentation searched other than minimum documentation to the extent that such documents are included in the fields searched

Electronic data base consulted during the international search (name of data base and, where practicable, search terms used)

DWPI, VEN, CNABS, CNTXT, CNKI, PubMed, GenBank, EBI, STN, WPABSC, WPABS, ENTXT, ENTXTC, Wanfang Database, ISI web of Knowledge: DOTA, NOTA, FAP, tMeBn, AET, HEX, Aib, fibroblast activation protein, 成纤维细胞, 活化蛋白配体, FAP-2286, CAPPTQFC, 3BP, 抑制剂, inhibit, 标记, 放射, 核素, Cys-Ala-Pro-Pro-Thr-Gln-Phe-Cys, 恒瑞

**C. DOCUMENTS CONSIDERED TO BE RELEVANT**

| Category* | Citation of document, with indication, where appropriate, of the relevant passages | Relevant to claim No. |
|---|---|---|
| X | EP 3763726 A1 (3B PHARMACEUTICALS GMBH) 13 January 2021 (2021-01-13)<br>description, the embodiments | 1-20, 22, 24, 26-36 |
| X | CN 114341159 A (3B PHARMACEUTICALS GMBH) 12 April 2022 (2022-04-12)<br>claims 1-8, and description, the mbodiments | 1-20, 22, 24, 26-36 |
| X | US 2022273831 A1 (3B PHARMACEUTICALS GMBH) 01 September 2022 (2022-09-01)<br>description, the embodiments | 1-20, 22, 24, 26-36 |
| X | US 2022315554 A1 (3B PHARMACEUTICALS GMBH) 06 October 2022 (2022-10-06)<br>description, the embodiments | 1-20, 22, 24, 26-36 |
| X | US 2023212549 A1 (3B PHARMACEUTICALS GMBH) 06 July 2023 (2023-07-06)<br>description, the embodiments | 1-20, 22, 24, 26-36 |
| X | CN 114341158 A (3B PHARMACEUTICALS GMBH) 12 April 2022 (2022-04-12)<br>description, the embodiments | 1-20, 22, 24, 26-36 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 August 2024** | **12 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/104918**

**C. DOCUMENTS CONSIDERED TO BE RELEVANT**

| Category* | Citation of document, with indication, where appropriate, of the relevant passages | Relevant to claim No. |
|---|---|---|
| A | CN 114790194 A (SUZHOU YAOMING BORUI BIOTECHNOLOGY CO., LTD.) 26 July 2022 (2022-07-26)<br>claims 1-14 | 1-36 |
| A | CN 116265484 A (SHANGHAI JUNNA MEDICAL TECHNOLOGY CO., LTD.) 20 June 2023 (2023-06-20)<br>entire document | 1-36 |

Form PCT/ISA/210 (second sheet) (July 2022)

**INTERNATIONAL SEARCH REPORT**
**Information on patent family members**

International application No.
**PCT/CN2024/104918**

| Patent document cited in search report | | | Publication date (day/month/year) | Patent family member(s) | | | Publication date (day/month/year) |
|---|---|---|---|---|---|---|---|
| EP | 3763726 | A1 | 13 January 2021 | None | | | |
| CN | 114341159 | A | 12 April 2022 | JP | 2022541753 | A | 27 September 2022 |
| | | | | BR | 112022000122 | A2 | 26 April 2022 |
| | | | | US | 2022273831 | A1 | 01 September 2022 |
| | | | | KR | 20220032079 | A | 15 March 2022 |
| | | | | EP | 3997103 | A1 | 18 May 2022 |
| | | | | AU | 2020310538 | A1 | 27 January 2022 |
| | | | | CL | 2021003580 | A1 | 05 August 2022 |
| | | | | IL | 289675 | A | 01 March 2022 |
| | | | | CA | 3145340 | A1 | 14 January 2021 |
| | | | | MX | 2022000252 | A | 21 February 2022 |
| | | | | WO | 2021005125 | A1 | 14 January 2021 |
| US | 2022273831 | A1 | 01 September 2022 | None | | | |
| US | 2022315554 | A1 | 06 October 2022 | MX | 2022000251 | A | 21 February 2022 |
| | | | | CA | 3145872 | A1 | 14 January 2021 |
| | | | | WO | 2021005131 | A1 | 14 January 2021 |
| | | | | EP | 3997104 | A1 | 18 May 2022 |
| | | | | BR | 112022000144 | A2 | 22 February 2022 |
| | | | | JP | 2022541752 | A | 27 September 2022 |
| | | | | CL | 2022000016 | A1 | 19 August 2022 |
| | | | | AU | 2020309161 | A1 | 27 January 2022 |
| | | | | IL | 289673 | A | 01 March 2022 |
| | | | | KR | 20220032078 | A | 15 March 2022 |
| US | 2023212549 | A1 | 06 July 2023 | CA | 3206863 | A1 | 14 July 2022 |
| | | | | WO | 2022148843 | A1 | 14 July 2022 |
| | | | | AU | 2022205523 | A1 | 13 July 2023 |
| | | | | JP | 2024503637 | A | 26 January 2024 |
| | | | | IL | 303925 | A | 01 August 2023 |
| | | | | CL | 2023001991 | A1 | 15 December 2023 |
| | | | | MX | 2023007869 | A | 22 September 2023 |
| | | | | EP | 4274835 | A1 | 15 November 2023 |
| | | | | KR | 20230129261 | A | 07 September 2023 |
| CN | 114341158 | A | 12 April 2022 | US | 2022315554 | A1 | 06 October 2022 |
| | | | | MX | 2022000251 | A | 21 February 2022 |
| | | | | CA | 3145872 | A1 | 14 January 2021 |
| | | | | WO | 2021005131 | A1 | 14 January 2021 |
| | | | | EP | 3997104 | A1 | 18 May 2022 |
| | | | | BR | 112022000144 | A2 | 22 February 2022 |
| | | | | JP | 2022541752 | A | 27 September 2022 |
| | | | | CL | 2022000016 | A1 | 19 August 2022 |
| | | | | AU | 2020309161 | A1 | 27 January 2022 |
| | | | | IL | 289673 | A | 01 March 2022 |
| | | | | KR | 20220032078 | A | 15 March 2022 |
| CN | 114790194 | A | 26 July 2022 | None | | | |
| CN | 116265484 | A | 20 June 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J Nucl Med*, 2022, vol. 63, 415-423
- *European Journal of Nuclear Medicine and Molecular Imaging*, 2022, vol. 49, 3651-3667